(19) Europäisches Patentamt / European Patent Office / Office européen des brevets

(11) **EP 4 786 446 A1**

(12) **EUROPEAN PATENT APPLICATION**
published in accordance with Art. 153(4) EPC


(43) Date of publication:
**05.08.2026 Bulletin 2026/32**

(21) Application number: **24872187.0**

(22) Date of filing: **24.09.2024**

(51) International Patent Classification (IPC):
***C07C 31/20*** (2006.01) ***B32B 15/09*** (2006.01)
***B32B 27/00*** (2006.01) ***B32B 27/32*** (2006.01)
***B32B 27/36*** (2006.01) ***B65D 65/40*** (2006.01)
***C07C 29/10*** (2006.01) ***C07C 43/10*** (2006.01)
***C08G 63/12*** (2006.01) ***C08G 63/16*** (2006.01)
***C08G 63/183*** (2006.01) ***C08L 67/02*** (2006.01)
***C12P 7/06*** (2006.01) ***C12P 7/625*** (2022.01)
***C23C 14/20*** (2006.01) ***C25B 3/03*** (2021.01)
***C25B 3/07*** (2021.01) ***C25B 3/26*** (2021.01)
***C25B 9/00*** (2021.01) ***D01F 6/62*** (2006.01)

(52) Cooperative Patent Classification (CPC):
**B32B 15/09; B32B 27/00; B32B 27/32; B32B 27/36; B65D 65/40; C07C 29/10; C07C 31/20; C07C 43/10; C08G 63/12; C08G 63/16; C08G 63/183; C08L 67/02; C12P 7/06; C12P 7/625; C23C 14/20;** (Cont.)

(86) International application number:
**PCT/JP2024/033946**

(87) International publication number:
**WO 2025/070397 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:
**25.09.2023 JP 2023161496**
**07.02.2024 JP 2024017367**
**07.03.2024 JP 2024035324**
**20.06.2024 JP 2024099759**
**24.06.2024 JP 2024101528**
**03.07.2024 JP 2024107724**
**09.07.2024 JP 2024110366**
**10.07.2024 JP 2024111165**



(71) Applicant: **Dai Nippon Printing Co., Ltd.**
**Tokyo 162-8001 (JP)**

(72) Inventors:
- **EGUCHI Makoto**
  **Tokyo 162-8001 (JP)**
- **SEKINE Akitomo**
  **Tokyo 162-8001 (JP)**
- **HASEGAWA Takayuki**
  **Tokyo 162-8001 (JP)**
- **BORAH Angana**
  **Tokyo 162-8001 (JP)**
- **TAKAHASHI Hideaki**
  **Tokyo 162-8001 (JP)**

(74) Representative: **Beck Greener LLP**
**Fulwood House**
**12 Fulwood Place**
**London WC1V 6HR (GB)**


(54) **ETHYLENE GLYCOL, POLYESTER, POLYESTER PELLETS, MOLDED ARTICLE, FILM, FIBERS, FIBER ARTICLE, MOLDED ARTICLE, PREFORM, POLYESTER FILM, LAMINATED FILM, LAMINATE, AND PACKAGE**


(57) Ethylene glycol is made using at least one gas selected from the group consisting of carbon monoxide and carbon dioxide as a raw material.


EP 4 786 446 A1

(52) Cooperative Patent Classification (CPC): (Cont.)
**C25B 3/03; C25B 3/07; C25B 3/26; C25B 9/00;
D01F 6/62**

## Description

### Technical Field

The present disclosure relates to ethylene glycol, a polyester, polyester pellets, a molded article, a film, fibers, a fiber article, a molded article, a preform, a polyester film, a laminated film, a laminate, and a package.

### Background Art

Ethylene glycol is widely used in various applications, for example as a raw material for polymers or as a humectant. Ethylene glycol is industrially manufactured using ethylene obtained from petroleum, which is a fossil fuel, as a starting material. For example, by producing ethylene oxide from ethylene by the chlorohydrin method or by oxidation using a silver catalyst, and hydrolyzing the ethylene oxide, ethylene glycol is manufactured.

In recent years, with a growing call for building a recycling-oriented society, the materials field, similarly to energy, has also been facing the need for transition away from fossil fuels, and the use of raw materials other than fossil fuels, such as biomass, has been considered. Biomass is an industrial resource originating in substances constituting living organisms, rather than a depleting resource. The carbon contained in biomass is derived from carbon dioxide that the organisms absorb from the atmosphere through photosynthesis during their process of growth. Even if carbon dioxide is emitted as a result of combustion of biomass, therefore, it probably does not increase the amount of carbon dioxide in the atmosphere as a whole. For this reason, biomass is attracting attention as a type of carbon-neutral renewable energy.

Nowadays, ethylene glycol made using such biomass as a raw material has been rapidly put to practical use, and attempts to manufacture polyesters and other resins from ethylene glycol made using biomass as a raw material (biomass ethylene glycol) have also been ongoing (e.g., PTL 1 and 2). Using biomass ethylene glycol instead of ethylene glycol manufactured using a fossil fuel-derived raw material makes it possible to reduce environmental impact by reducing the amount of fossil fuel used.

Bioethanol for use in the manufacture of biomass ethylene glycol can be manufactured from sugarcane molasses, which is an inedible raw material, by sugar fermentation. It is, however, empirically known that the yields of crops, including sugarcane, are significantly influenced by meteorological factors. Accordingly, there is a concern that climate changes can make stable procurement of sugarcane as a raw material for bioethanol difficult.

To make it possible to address such concerns, researchers have also been attempting the manufacture of ethanol and other organic compounds from a resource that is neither a fossil fuel nor biomass. For example, the manufacture of ethanol by microbial fermentation using carbon monoxide existing in, for example, exhaust gas emitted from ironworks or plants as a raw material has been attempted (e.g., PTL 3). The production of ethane, ethylene, and other hydrocarbons by reducing carbon dioxide using an electrolytic cell has also been tried (e.g., PTL 4). Using such organic compounds instead of organic compounds manufactured using fossil fuel-derived raw materials also makes it possible to reduce environmental impact by reducing the amount of fossil fuel used.

### Citation List

### Patent Literature

PTL 1: Japanese Unexamined Patent Application Publication (Translation of PCT Application) No. 2011-527348

PTL 2: Japanese Unexamined Patent Application Publication (Translation of PCT Application) No. 2012-519748

PTL 3: Japanese Unexamined Patent Application Publication (Translation of PCT Application) No. 2011-512869

PTL 4: Japanese Unexamined Patent Application Publication No. 2012-112001

### Summary of Invention

### Technical Problem

An object of the present disclosure, therefore, is to provide ethylene glycol manufactured from carbon monoxide gas and/or carbon dioxide gas through reduction by microbial fermentation or using an electrolytic cell, with which environmental impact can be reduced.

Another object of the present disclosure, furthermore, is to provide a polyester made using the ethylene glycol.
Moreover, another object of the present disclosure is to provide polyester pellets containing the polyester.
Another object of the present disclosure, furthermore, is to provide a molded article, a film, fibers, and a fiber product made using the polyester pellets.
Moreover, another object of the present disclosure is to provide an article molded from the polyester.
Another object of the present disclosure, furthermore, is to provide a preform for use in the manufacture of the molded article.
Moreover, another object of the present disclosure is to provide a polyester film containing the polyester.
Another object of the present disclosure, furthermore, is to provide a laminate including the polyester film and a package including the laminate.
Moreover, another object of the present disclosure is to provide a laminated film including a base material layer containing the polyester.
Another object of the present disclosure, furthermore, is to provide a laminate including the laminated film and a package including the laminate.

Solution to Problem

The inventors found that ethylene glycol can be manufactured from ethanol manufactured by microbial fermentation using carbon monoxide gas as a raw material.
The inventors, furthermore, also found that ethylene glycol can be manufactured by performing reduction of carbon dioxide gas through electrolysis.
The present disclosure was completed based on these findings after subsequent further investigations.
The present disclosure is solved by the following embodiments.

<1>
Ethylene glycol made using at least one gas selected from the group consisting of carbon monoxide and carbon dioxide as a raw material.
<2>
The ethylene glycol according to <1>, wherein a quantity of carbon dioxide molecules is $2.2 \times 10^{18}$ molecules/g or more and $1.5 \times 10^{19}$ molecules/g or less.
<3>
The ethylene glycol according to <1> or <2>, wherein an amount of Kjeldahl nitrogen is 700 μg/g or less.
<4>
A polyester composed of a diol unit and a dicarboxylic acid unit, wherein:
the diol unit is the ethylene glycol according to any one of <1> to <3>.
<5>
The polyester according to <4>, wherein the dicarboxylic acid unit is at least one selected from the group consisting of fossil fuel-derived terephthalic acid, biomass-derived terephthalic acid, and terephthalic acid made using carbon dioxide gas as a raw material.
<6>
Polyester pellets containing the polyester according to <4> or <5>, wherein:
an intrinsic viscosity of the polyester pellets is 0.50 dL/g or more and 0.90 dL/g or less.
<7>
The polyester pellets according to <6>, wherein an amount of Kjeldahl nitrogen is 260 μg/g or less.
<8>
The polyester pellets according to <6> or <7>, wherein a quantity of carbon dioxide molecules is $4.8 \times 10^{17}$ molecules/g or more and $3.0 \times 10^{18}$ molecules/g or less.
<9>
The polyester pellets according to any one of <6> to <8>, wherein a quantity of water molecules is $1.8 \times 10^{19}$ molecules/g or more and $1.0 \times 10^{20}$ molecules/g or less.
<10>
A molded article made using the polyester pellets according to any one of <6> to <9>.
<11>
A film made using the polyester pellets according to any one of <6> to <9>.
<12>
Fibers made using the polyester pellets according to <6>.
<13>
The fibers according to <12>, wherein an amount of heat of fusion of the polyester pellets during a second heating run

obtained by differential scanning calorimetry (DSC) according to JIS K7122: 2012 is 8 J/g or more.

<14>

The fibers according to <12> or <13>, wherein an amount of heat of solidification of the polyester pellets during a second cooling run obtained by differential scanning calorimetry (DSC) according to JIS K7122: 2012 is 7 J/g or more.

<15>

The fibers according to any one of <12> to <14>, wherein an amount of Kjeldahl nitrogen in the polyester pellets is 90 μg/g or less.

<16>

The fibers according to any one of <12> to <15>, wherein a quantity of hydrogen molecules in the polyester pellets is $7.5 \times 10^{17}$ molecules/g or less.

<17>

A fiber article made using the fibers according to any one of <12> to <16>.

<18>

An article molded from the polyester according to <4> or <5>.

<19>

The molded article according to <18>, wherein a quantity of carbon dioxide molecules is $9.2 \times 10^{17}$ molecules/g or more and $3.0 \times 10^{18}$ molecules/g or less.

<20>

The molded article according to <18> or <19>, wherein an amount of Kjeldahl nitrogen is 18 μg/g or less.

<21>

The molded article according to any one of <18> to <20>, wherein the molded article is a container.

<22>

A preform for use in manufacture of the molded article according to <21>.

<23>

A polyester film containing the polyester according to <4> or <5>.

<24>

The polyester film according to <23>, wherein a quantity of carbon dioxide molecules is $1.1 \times 10^{18}$ molecules/g or more and $3.0 \times 10^{18}$ molecules/g or less.

<25>

The polyester film according to <23> or <24>, wherein a quantity of water molecules is $8.6 \times 10^{19}$ molecules/g or more and $2.5 \times 10^{20}$ molecules/g or less.

<26>

A laminate including the polyester film according to any one of <23> to <25>.

<27>

A laminated film including at least a base material layer and a vapor deposition film, wherein:

the base material layer contains the polyester according to <4> or <5>; and
the vapor deposition film is a vapor deposition film of a metal or a metal oxide.

<28>

The laminated film according to <27>, wherein a quantity of carbon dioxide molecules in the base material layer is $1.1 \times 10^{18}$ molecules/g or more and $3.0 \times 10^{18}$ molecules/g or less.

<29>

The laminated film according to <27> or <28>, wherein a quantity of water molecules in the base material layer is $8.6 \times 10^{19}$ molecules/g or more and $2.5 \times 10^{20}$ molecules/g or less.

<30>

The laminated film according to any one of <27> to <29>, further including a surface coating layer between the base material layer and the vapor deposition film, wherein:
the surface coating layer contains a resin material having a polar group.

<31>

The laminated film according to any one of <27> to <30>, wherein:

the laminated film includes the base material layer, the vapor deposition film, and a gas barrier coating film in this order; and

the gas barrier coating film contains an alkoxide and at least one water-soluble polymer selected from the group consisting of a polyvinyl alcohol resin and an ethylene-vinyl alcohol copolymer.

<32>
A laminate including the laminated film according to any one of <27> to <31> and a sealant layer.
<33>
The laminate according to <32>, further including a support, wherein:
the support is a stretched polyester resin layer, a stretched polyamide resin layer, or a stretched polypropylene resin layer.
<34>
The laminate according to <33>, wherein the laminate includes the support, the base material layer, the vapor deposition film, and the sealant layer in this order.
<35>
A laminate including at least the polyester film according to any one of <23> to <25> and a sealant layer.
<36>
The laminate according to <35>, wherein a resin constituting the sealant layer is low-density polyethylene, linear low-density polyethylene, or polypropylene.
<37>
The laminate according to <35> or <36>, further including a barrier layer between the polyester film and the sealant layer.
<38>
The laminate according to <37>, wherein the barrier layer is a metal foil.
<39>
A package including the laminate according to any one of <32> to <38>.

Advantageous Effects of Invention

The present disclosure can provide ethylene glycol with which environmental impact can be reduced.
The present disclosure, furthermore, can provide a polyester made using the ethylene glycol.
Moreover, the present disclosure can provide polyester pellets containing the polyester.
The present disclosure, furthermore, can provide a molded article, a film, fibers, and a fiber product made using the polyester pellets.
Moreover, the present disclosure can provide an article molded from the polyester.
The present disclosure, furthermore, can provide a preform for use in the manufacture of the molded article.
Moreover, the present disclosure can provide a polyester film containing the polyester.
The present disclosure, furthermore, can provide a laminate including the polyester film and a package including the laminate.
Moreover, the present disclosure can provide a laminated film including a base material layer containing the polyester.
The present disclosure, furthermore, can provide a laminate including the laminated film and a package including the laminate.

Brief Description of Drawings

[Fig. 1] Fig. 1 is a schematic view illustrating the composition of a reducing catalyst used in the manufacture of ethylene glycol according to the present disclosure.

[Fig. 2] Fig. 2 is a schematic view illustrating an example of a composition of the reducing catalyst in Fig. 1 in detail.

[Fig. 3] Fig. 3 is a cross-sectional view illustrating the structure of a photochemical reaction cell used in the manufacture of ethylene glycol according to the present disclosure.

[Fig. 4] Fig. 4 is a cross-sectional view illustrating an example of a principle of operation of the photochemical reaction cell in Fig. 3.

[Fig. 5] Fig. 5 is a cross-sectional view illustrating another example of a principle of operation of the photochemical reaction cell in Fig. 3.

[Fig. 6] Fig. 6 is a perspective view illustrating the structure of a chemical reaction apparatus used in the manufacture of

ethylene glycol according to the present disclosure.

[Fig. 7] Fig. 7 is a cross-sectional view illustrating the structure of a chemical reaction apparatus used in the manufacture of ethylene glycol according to the present disclosure.

[Fig. 8] Fig. 8 is a cross-sectional view illustrating another example of a structure of a chemical reaction apparatus used in the manufacture of ethylene glycol according to the present disclosure.

[Fig. 9] Fig. 9 is a cross-sectional view illustrating another example of a structure of a chemical reaction apparatus used in the manufacture of ethylene glycol according to the present disclosure.

[Fig. 10] Fig. 10 is a schematic half-sectional view illustrating an embodiment of a preform used in the manufacture of a molded article according to the present disclosure.

[Fig. 11] Fig. 11 is a schematic half-sectional view illustrating an embodiment of a molded article according to the present disclosure.

[Fig. 12] Fig. 12 is a schematic cross-sectional view illustrating an embodiment of a film according to the present disclosure.

[Fig. 13] Fig. 13 is a perspective view illustrating a spoon that is an embodiment of a molded article according to the present disclosure.

[Fig. 14] Fig. 14 is a cross-sectional view briefly illustrating an example of a structure of a polyester film according to the present disclosure.

[Fig. 15] Fig. 15 is a cross-sectional view briefly illustrating an example of a structure of a laminate including a polyester film according to the present disclosure.

[Fig. 16] Fig. 16 is a perspective view briefly illustrating an example of a stand-up pouch.

[Fig. 17] Fig. 17 is a cross-sectional view briefly illustrating an example of a structure of a laminate that forms side sheets of a stand-up pouch.

[Fig. 18] Fig. 18 is a cross-sectional view briefly illustrating an example of another structure of a laminate that forms side sheets of a stand-up pouch.

[Fig. 19] Fig. 19 is a front view briefly illustrating an example of a pillow bag.

[Fig. 20] Fig. 20 is a front view briefly illustrating an example of a three-side-seal bag.

[Fig. 21] Fig. 21 is a front view briefly illustrating an example of a four-side-seal bag.

[Fig. 22] Fig. 22 is a partial cross-sectional view briefly illustrating an example of a tube container.

[Fig. 23] Fig. 23 is a cross-sectional view briefly illustrating an example of a structure of a laminate that forms a cylindrical body of a tube container.

[Fig. 24] Fig. 24 is a cross-sectional view briefly illustrating an example of another structure of a laminate that forms a cylindrical body of a tube container.

[Fig. 25] Fig. 25 is a perspective view briefly illustrating an example of a paper container for liquids.

[Fig. 26] Fig. 26 is a cross-sectional view briefly illustrating an example of a structure of a laminate that forms a paper container for liquids.

[Fig. 27] Fig. 27 is a perspective view briefly illustrating an example of a partially cut-away paper cup.

[Fig. 28] Fig. 28 is a cross-sectional view briefly illustrating an example of a structure of a laminate that forms the body of a paper cup.

[Fig. 29] Fig. 29 is an explanatory view illustrating an example of an outline of a method for manufacturing a paper cup.

[Fig. 30] Fig. 30 is a cross-sectional view briefly illustrating an example of a structure of a laminated film according to the present disclosure.

[Fig. 31] Fig. 31 is a cross-sectional view briefly illustrating an example of a structure of a laminated film according to the present disclosure.

[Fig. 32] Fig. 32 is a cross-sectional view briefly illustrating an example of a structure of a laminated film according to the present disclosure.

[Fig. 33] Fig. 33 is a cross-sectional view briefly illustrating an example of a structure of a laminate according to the present disclosure.

[Fig. 34] Fig. 34 is a cross-sectional view briefly illustrating an example of a structure of a laminate according to the present disclosure.

Description of Embodiments

[Ethylene Glycol]

In the present disclosure, "ethylene glycol" does not refer to ethylene glycol that is pure as a compound (ethylene glycol represented by the chemical formula: $HOCH_2CH_2OH$); it refers to a composition containing impurities (contaminant components) that are inevitably contained in ethylene glycol manufactured via synthesis or purification.
Ethylene glycol according to the present disclosure is made using at least one gas selected from the group consisting of carbon monoxide and carbon dioxide as a raw material. By using ethylene glycol according to the present disclosure instead of known, fossil fuel-derived ethylene glycol, the amount of fossil fuel used can be reduced, whereby environmental impact can be decreased.
The amount of Kjeldahl nitrogen in the ethylene glycol according to the present disclosure tends to be smaller compared with that in known, fossil fuel-derived ethylene glycol. For example, the amount of Kjeldahl nitrogen in the ethylene glycol according to the present disclosure is preferably 700 $\mu$g/g or less, more preferably 560 $\mu$g/g or less, even more preferably 330 $\mu$g/g or less. The amount of Kjeldahl nitrogen in the ethylene glycol according to the present disclosure, furthermore, is 0 $\mu$g/g or more. In practice, the amount of Kjeldahl nitrogen is 10 $\mu$g/g or more, more practically 30 $\mu$g/g or more.
In the present disclosure, Kjeldahl nitrogen is nitrogen quantified according to 44.1 (Kjeldahl method) and 44.2 (indophenol blue absorptiometry) of JIS K0102: 2019. For the specific method for measuring the amount of Kjeldahl nitrogen, measurement is performed by the method described in the Examples section below.
The quantity of carbon dioxide molecules in the ethylene glycol according to the present disclosure, furthermore, tends to be greater than that in known, fossil fuel-derived ethylene glycol. For example, the quantity of carbon dioxide molecules in the ethylene glycol according to the present disclosure is preferably $2.2 \times 10^{18}$ molecules/g or more, more preferably $2.8 \times 10^{18}$ molecules/g or more, even more preferably $3.8 \times 10^{18}$ molecules/g or more. The quantity of carbon dioxide molecules in the ethylene glycol according to the present disclosure, furthermore, is preferably $1.5 \times 10^{19}$ molecules/g or less, more preferably $1.2 \times 10^{19}$ molecules/g or less, even more preferably $1.0 \times 10^{19}$ molecules/g or less.
The quantity of carbon dioxide molecules in the ethylene glycol is measured by thermal desorption spectroscopy-mass spectrometry (TDS-MS). Specifically, measurement is performed by the method described in the Examples section below.
Among types of ethylene glycol according to the present disclosure, ethylene glycol made using carbon monoxide gas as a raw material can be manufactured from ethanol made using carbon monoxide gas as a raw material. In the following, ethanol made using carbon monoxide gas as a raw material, ethylene glycol made using carbon monoxide gas as a raw material, and ethylene glycol made using carbon dioxide gas as a raw material will be described individually.

[Ethanol Made Using Carbon Monoxide Gas as a Raw Material]

Ethanol made using carbon monoxide gas as a raw material can be obtained by microbial fermentation. In the following, a method for manufacturing ethanol from carbon monoxide gas by microbial fermentation will be described.

<Microbial Fermentation>

The microbial fermentation is performed in, for example, a fermenter filled with a culture broth containing water and microorganisms. The inside of the fermenter is supplied with a feedstock gas containing carbon monoxide gas, and the carbon monoxide gas is converted into ethanol inside the fermenter. It should be noted that the feedstock gas may contain carbon dioxide, nitrogen, oxygen, etc., in addition to carbon monoxide.

The fermenter is preferably a continuous fermentation system and may be any of the stirredtank type, the airlift type, the bubble-column type, the loop-reactor type, the open-bond type, or the photobioreactor type.

The fermenter may be supplied with the feedstock gas and the culture broth continuously. It is, however, not necessary to supply the feedstock gas and the culture broth simultaneously; a fermenter preliminarily supplied with the culture broth may be supplied with the feedstock gas. The feedstock gas is typically introduced into the fermenter through, for example, a sparger.

The culture broth is not particularly limited as long as it has an appropriate composition for the cultivation of the microorganisms. It is, however, a liquid containing water as the main component and nutrients (e.g., vitamins and phosphoric acid) dissolved or dispersed in the water.

The temperature of the fermenter is preferably controlled to 40°C or below. As a result of the temperature being controlled to 40°C or below, ethanol is efficiently produced through the contact of the feedstock gas with the microorganisms, without the microorganisms in the fermenter dying out.

The temperature of the fermenter is more preferably 38°C or below. The temperature, furthermore, is preferably 10°C or above, more preferably 20°C or above, even more preferably 30°C or above, for increased activity of the microorganisms.

The microorganisms (species) that induce microbial fermentation of the feedstock gas are not particularly limited as long as they allow ethanol to be manufactured through microbial fermentation of the feedstock gas with carbon monoxide as the primary raw material. For example, the microorganisms (species) are preferably ones that produce ethanol from the feedstock gas through fermentation effects of gas-utilizing bacteria. Among gas-utilizing bacteria, the genus *Clostridium* is particularly preferred from the viewpoints of gas utilization and cultivation stability, with *Clostridium autoethanogenum* being more preferred. In the following, more specific examples will be presented.

The gas-utilizing bacteria include both true bacteria and archaebacteria.

Examples of true bacteria include bacteria in the genus *Clostridium*, bacteria in the genus *Moorella*, bacteria in the genus *Acetobacterium*, bacteria in the genus *Carboxydocella*, bacteria in the genus *Rhodopseudomonas*, bacteria in the genus *Eubacterium*, bacteria in the genus *Butyribacterium*, bacteria in the genus *Oligotropha*, bacteria in the genus *Bradyrhizobium*, and bacteria in the genus *Ralsotonia*, which are aerobic hydrogen-oxidizing bacteria.

Examples of archaebacteria, on the other hand, include bacteria in the genus *Methanobacterium*, bacteria in the genus *Methanobrevibacter*, the genus *Methanocalculus*, bacteria in the genus *Methanococcus*, bacteria in the genus *Methanosarcina*, bacteria in the genus *Methanosphaera*, bacteria in the genus *Methanothermobacter*, bacteria in the genus *Methanothrix*, bacteria in the genus *Methanoculleus*, bacteria in the genus *Methanofollis*, bacteria in the genus *Methanogenium*, bacteria in the genus *Methanospirillium*, bacteria in the genus *Methanosaeta*, bacteria in the genus *Thermococcus*, bacteria in the genus *Thermofilum*, and bacteria in the genus *Arcaheoglobus*. Of these, for archaebacteria, bacteria in the genus *Methanosarcina*, bacteria in the genus *Methanococcus*, bacteria in the genus *Methanothermobacter*, bacteria in the genus *Methanothrix*, bacteria in the genus *Thermococcus*, bacteria in the genus *Thermofilum*, and bacteria in the genus *Archaeoglobus* are particularly preferred.

For archaebacteria, furthermore, bacteria in the genus *Methanosarcina*, bacteria in the genus *Methanothermobactor*, or bacteria in the genus *Methanococcus* are preferred because they are superior in the utilization of carbon monoxide and carbon dioxide, with bacteria in the genus *Methanosarcina* or bacteria in the genus *Methanococcus* being particularly preferred. It should be noted that specific examples of bacteria in the genus *Methanosarcina* include *Methanosarcina barkeri*, *Methanosarcina mazei*, and *Methanosarcina acetivorans*.

It is particularly preferred to select and use bacteria with high ethanol production capability from among such gas-utilizing bacteria. For example, bacteria such as *Clostridium autoethanogenum*, *Clostridium ljungdahlii*, *Clostridium aceticum*, *Clostridium carboxidivorans*, *Moorella thermoacetica*, and *Acetobacterium woodii* are preferred.

The ethanol produced through the microbial fermentation is obtained as, for example, an ethanol-containing liquid in a state in which the ethanol is mixed with the culture broth. From this ethanol-containing liquid, the ethanol can be separated using a separator. Examples of separators include a solid-liquid separator, a distillation apparatus, and a separation membrane, but it is preferred to use a solid-liquid separator and a distillation apparatus in combination. In the following, a separation step performed using a solid-liquid separator and a distillation apparatus in combination will be specifically described.

The ethanol-containing liquid obtained through the microbial fermentation separates within the solid-liquid separator into a solid component that is primarily the microorganisms and a liquid component containing ethanol. Since the ethanol-containing liquid obtained through the microbial fermentation contains materials such as microorganisms and their carcasses originally present in the fermenter in addition to the ethanol as the desired product, solid-liquid separation is

performed to remove them. Examples of solid-liquid separators include a filter, a centrifuge, and an apparatus that utilizes solution precipitation. The solid-liquid separator, furthermore, may be an apparatus that evaporates the liquid component containing ethanol from the ethanol-containing liquid to separate it from the solid component (e.g., a heater-dryer). During this, the entire liquid component containing the ethanol as the desired product may be evaporated, or the liquid component may be partially evaporated such that the target ethanol evaporates preferentially.

The liquid component separated through the solid-liquid separation is subjected in the distillation apparatus to distillation for further separation of the ethanol as the desired product. Through separation by distillation, a large amount of ethanol can be purified to high purity with a simple operation.

When distillation is performed, a known distillation apparatus, such as a distillation column, can be used. During the distillation, furthermore, the operation is performed such that the distillate contains the ethanol as the desired product with high purity, whereas the bottoms (i.e., distillation residue) contain water as its main component (e.g., 70% by mass or more, preferably 90% by mass or more). By performing the operation in such a manner, the ethanol as the desired product and water can be substantially completely separated.

The temperature inside the distillation apparatus during the distillation of the ethanol is not particularly limited, but preferably is 100°C or below, more preferably 95°C or below, and preferably is 70°C or above. By setting the temperature inside the distillation apparatus within these ranges, it can be ensured that the separation between the ethanol and other components, such as water, takes place.

The pressure inside the distillation apparatus during the distillation of the ethanol may be normal pressure, but preferably is lower than atmospheric pressure, more preferably 60 kPa or more and 150 kPa or less (gauge pressure). By setting the pressure inside the distillation apparatus within these ranges, efficiency in the separation of the ethanol can be improved, whereby the yield of the ethanol can be improved.

[Ethylene Glycol Made Using Carbon Monoxide Gas as a Raw Material]

Ethylene glycol made using carbon monoxide gas as a raw material can be manufactured from ethanol made using carbon monoxide gas as a raw material. For example, ethanol obtained as described above is heated in the presence of a catalyst, whereby ethylene is produced through an intramolecular dehydration reaction.

Then the ethylene is oxidized through a gas-phase catalytic reaction of the ethylene using a silver catalyst, through which a gas containing ethylene oxide is generated. It is known that in a gas-phase catalytic reaction of ethylene, not only ethylene oxide but also small amounts of organic acids, such as formic acid and acetic acid, and aldehyde compounds, such as formaldehyde and acetaldehyde, are produced as by-products. A gas generated through a gas-phase catalytic reaction of ethylene, therefore, contains organic acids and aldehyde compounds in addition to ethylene oxide.

The generated gas is washed with water to allow the ethylene oxide to be absorbed into water, whereby the ethylene oxide is separated from the generated gas. The resulting aqueous solution of ethylene oxide is supplied to a distillation column, at which the ethylene oxide is allowed to evaporate, and a high-concentration aqueous solution of ethylene oxide is collected as overhead distillate.

Then the high-concentration aqueous solution of ethylene oxide is transferred to a reactor. Typically, at a reaction temperature of 150°C or above and 180°C or below and a reaction pressure of 2.5 MPa or less, a reaction for producing ethylene glycol from the ethylene oxide is conducted. The amount of water in the aqueous solution of ethylene oxide is adjusted as appropriate. In general, it is preferred to use the solution at an ethylene oxide concentration of 9% by mass or more and 13% by mass or less. The generated aqueous solution of ethylene glycol is subjected to water removal, for example at a dehydration column.

Then distillation under reduced pressure is performed to fractionate the ethylene glycol from diethylene glycol and triethylene glycol. In this manner, ethylene glycol made using carbon monoxide gas as a raw material is obtained.

The distillation under reduced pressure is performed using a vacuum distillation column. The temperature during the fractionation of the ethylene glycol is preferably 125°C or above, more preferably 135°C or above, and preferably is 225°C or below, more preferably 195°C or below. The pressure during the fractionation of the ethylene glycol is preferably 10 hPa or more, more preferably 15 hPa or more, and preferably is 550 hPa or less, more preferably 79 hPa or less. Within these ranges of temperatures and pressures, the ethylene glycol can be fractionated and purified efficiently.

[Ethylene Glycol Made Using Carbon Dioxide Gas as a Raw Material]

Ethylene glycol made using carbon dioxide gas as a raw material can be obtained through electrolysis using a chemical reaction apparatus employing a predetermined reducing catalyst. First, the reducing catalyst is described.

<Reducing Catalyst>

The reducing catalyst includes a current collector having a metal layer on its surface and organic molecules bound to the surface of the metal layer. The organic molecules are represented by any of general formulae I to V below and contains at least one quaternary nitrogen cation.

[Chem. 1]

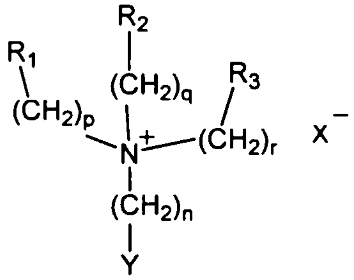

... General formula I

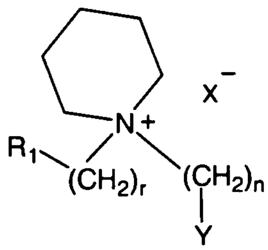

... General formula II

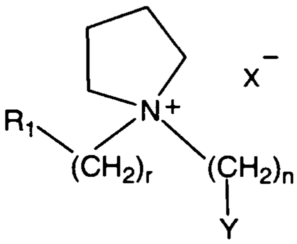

... General formula III

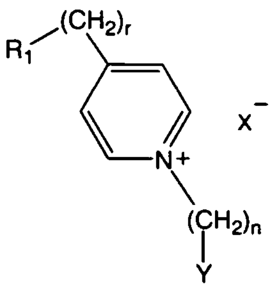

... General formula IV

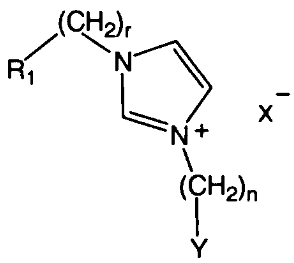

... General Formula V

In general formulae I to V, $R_1$ is a primary, secondary, or tertiary amino group. $R_2$ and $R_3$ may be the same or may be different, and each is independently H or a primary, secondary, or tertiary amino group. Each of p, q, r, and n is independently an integer of 1 or greater and 12 or less. Y is a reactive functional group, and $X^-$ denotes a counteranion.

In the following, a reducing catalyst according to a first embodiment will be described with reference to Fig. 1 and Fig. 2. Fig. 1 is a diagram illustrating the composition of a reducing catalyst 1, and Fig. 2 is a diagram illustrating an example of a composition of the reducing catalyst 1 in detail. As illustrated in Fig. 1, the reducing catalyst 1 includes a current collector 101 and organic molecules 112 containing at least one quaternary nitrogen cation. The current collector 101 has a metal layer 102 on its surface. The organic molecules 112 containing at least one quaternary nitrogen cation are bound to the metal layer 102, forming a self-assembled monolayer (SAM). It should be noted that hereinafter, the organic molecules containing at least one quaternary nitrogen cation are referred to as modifying organic molecules.

The current collector 101 is composed of a material having electrical conductivity. The current collector 101 can be, for example, a stainless-steel substrate.

The metal layer 102 on the surface of the current collector 101 contains at least one metal selected from the group consisting of Au, Ag, Cu, Zn, Pt, Fe, Ti, Ni, Sn, In, and Bi. The metal layer 102 may contain constituents other than metals, but preferably is formed solely of metal(s). The metal layer 102 and the current collector 101 may be composed of the same material(s). In that case, the metal constituting the metal layer 102 may also serve as the current collector 101.

The metal contained in the metal layer 102 functions as a catalyst that activates the reduction reaction. The metal contained in the metal layer 102 is preferably in a state of fine particles because in that case the activity of the catalyst improves.

The average particle diameter of metal fine particles contained in the metal layer 102 is preferably 1 nm or more and 300 nm or less. When the average particle diameter is 300 nm or less, the activation efficiency of the catalyst can be increased. Metal fine particles having an average particle diameter of less than 1 nm, furthermore, are difficult to manufacture. The average particle diameter of the metal fine particles is more preferably 150 nm or less because in that case the activation efficiency of the catalyst further improves. It should be noted that the metal fine particles may be primary particles having an average particle diameter of 50 nm or less, but may alternatively be secondary particles formed through aggregation of such primary particles.

Each modifying organic molecule 112 has a backbone 110 containing at least one quaternary nitrogen cation, a reactive functional group 109 located at one end, and an amino group 111 located at the other end. The quaternary nitrogen cation contained in the modifying organic molecule 112 is at least one cation selected from an alkylammonium cation, a pyridinium cation, a piperidinium cation, a pyrrolidinium cation, and an imidazolium cation. As described later, the quaternary nitrogen cation is more preferably an imidazolium cation because it is highly effective in improving reducing activity.

The backbone 110 is composed of at least one quaternary nitrogen cation and alkyl groups that are substituents on the cation. The number of carbons in the alkyl groups corresponds to p, q, r, and n in general formulae I to V, each of which is independently an integer of 1 or greater and 12 or less. When p, q, r, and n are 1 or greater and 12 or less in general formulae I to V, the quaternary nitrogen cation and the amino group, and the quaternary nitrogen cation and the metal layer 102, are not excessively distant. In that case, as described later, the advantage of improved reducing efficiency owing to the quaternary nitrogen cation can be achieved. p, q, r, and n are more preferably integers of 2 or greater and 6 or less.

The reactive functional group 109 corresponds to Y in general formulae I to V. The reactive functional group 109 has affinity for the metal layer 102 and chemically binds to the metal layer 102. As a result of this, the modifying organic molecules 112 are immobilized on the metal layer 102. The reactive functional group 109 is preferably a functional group capable of covalent bonding with the metal layer 102, and preferably is selected from, for example, a thiol group, a disulfide group, and a thiocyanate group. It is more preferred that the reactive functional group be a thiol group because it is superior in binding strength.

The amino group 111 corresponds to $R_1$, and optionally $R_2$ and $R_3$, in general formulae I to V. The amino group 111 may be a primary, secondary, or tertiary amino group. When the amino group is a secondary or tertiary amino group, the substituent(s) is preferably one or two $C_1$ to $C_{12}$ alkyl groups. When the number of carbon atoms in the alkyl group(s) is 12 or less, the quaternary nitrogen cation and the amino group are not excessively distant. In that case, as described later, the advantage of improved reducing efficiency owing to the amino group can be achieved. The number of carbon atoms in the alkyl group(s) is more preferably 2 or greater and 6 or less. The amino group 111 may be in the form of a salt, for example with hydrogen fluoride, hydrogen chloride, hydrogen bromide, hydrogen iodide, sulfuric acid, nitric acid, or phosphoric acid.

The modifying organic molecules 112 are in the form of a salt with a counteranion represented by $X^-$ in general formulae I to V. Specifically, they are in the form of an ammonium salt, a piperidinium salt, a pyrrolidinium salt, a pyridinium salt, or an imidazolium salt. It should be noted that in Fig. 1, the counteranion is omitted. In Fig. 2, a bromide ion is presented as the counteranion.

The counteranion may be a fluoride ion, a chloride ion, a bromide ion, an iodide ion, $HCO_3^-$, $BF_4^-$, $PF_6^-$, $CF_3COO^-$, $CF_3SO_3^-$, $NO_3^-$, $SCN^-$, $N(CN)_2^-$, $C(CN)_3^-(CF_3SO_2)_3C^-$, a bis(trifluoromethoxysulfonyl)imide anion, a bis(trifluoromethoxysulfonyl)imide anion, or a bis(perfluoroethylsulfonyl)imide anion, although not limited to these.

Examples of modifying organic molecules 112 include the following molecules: 1-(2-mercaptoethyl)-3-aminomethylimidazolium bromide, 1-(3-mercaptopropyl)-3-aminomethylimidazolium bromide, 1-(4-mercaptobutyl)-3-aminomethylimidazolium bromide, 1-(5-mercaptopentyl)-3-aminomethylimidazolium bromide, 1-(6-mercaptohexyl)-3-aminomethylimidazolium bromide, 1-(8-mercaptooctyl)-3-aminomethylimidazolium bromide, 1-(9-mercaptononyl)-3-aminomethylimidazolium bromide, 1-(10-mercaptodecyl)-3-aminomethylimidazolium bromide, 1-(11-mercaptoundecyl)-3-aminomethylimidazolium bromide, 1-(12-mercaptododecyl)-3-aminomethylimidazolium bromide,

1-(2-mercaptoethyl)-3-(2-aminoethyl)imidazolium bromide, 1-(2-mercaptoethyl)-3-(3-aminopropyl)imidazolium bromide, 1-(2-mercaptoethyl)-3-(4-aminobutyl)imidazolium bromide, 1-(2-mercaptoethyl)-3-(5-aminopentyl)imidazolium bromide, 1-(2-mercaptoethyl)-3-(6-aminohexyl)imidazolium bromide, 1-(2-mercaptoethyl)-3-(8-aminooctyl)imidazolium bromide, 1-(2-mercaptoethyl)-3-(9-aminononyl)imidazolium bromide, 1-(2-mercaptoethyl)-3-(10-aminodecyl)imidazolium bromide, 1-(2-mercaptoethyl)-3-(11-aminoundecyl)imidazolium bromide, 1-(2-mercaptoethyl)-3-(12-aminododecyl)imidazolium bromide, 1-(4-mercaptobutyl)-3-(2-methylaminoethyl)imidazolium bromide, 1-(6-mercaptohexyl)-3-(3-dimethylaminopropyl)imidazolium bromide, 1-(8-mercaptohexyl)-3-(4-ethylmethylaminobutyl)imidazolium bromide,

1-(2-mercaptoethyl)-4-aminomethylpyridinium bromide, 1-(3-mercaptopropyl)-4-aminomethylpyridinium bromide, 1-(4-mercaptobutyl)-4-aminomethylpyridinium bromide, 1-(5-mercaptopentyl)-4-aminomethylpyridinium bromide, 1-(6-mercaptohexyl)-4-aminomethylpyridinium bromide, 1-(8-mercaptooctyl)-4-aminomethylpyridinium bromide, 1-(9-mercaptononyl)-4-aminomethylpyridinium bromide, 1-(10-mercaptodecyl)-4-aminomethylpyridinium bromide, 1-(11-mercaptoundecyl)-4-aminomethylpyridinium bromide, 1-(12-mercaptododecyl)-4-aminomethylpyridinium bromide, 1-(2-mercaptoethyl)-4-(2-aminoethyl)pyridinium bromide, 1-(2-mercaptoethyl)-4-(3-aminopropyl)pyridinium bromide, 1-(2-mercaptoethyl)-4-(4-aminobutyl)pyridinium bromide, 1-(2-mercaptoethyl)-4-(5-aminopentyl)pyridinium bromide, 1-(2-mercaptoethyl)-4-(6-aminohexyl)pyridinium bromide, 1-(2-mercaptoethyl)-4-(8-aminooctyl) pyridinium bromide, 1-(2-mercaptoethyl)-4-(9-aminononyl)pyridinium bromide, 1-(2-mercaptoethyl)-4-(10-aminodecyl)pyridinium bromide, 1-(2-mercaptoethyl)-4-(11-aminoundecyl)pyridinium bromide, 1-(2-mercaptoethyl)-4-(12-aminododecyl)pyridinium bromide,

1-(5-mercaptopentyl)-4-(3-methylaminopropyl)pyridinium bromide, 1-(9-mercaptononyl)-4-(4-dimethylaminobutyl) pyridinium bromide, 1-(11-mercaptoundecyl)-4-(6-ethylmethylaminohexyl)pyridinium bromide,

1-(2-mercaptoethyl)-1-aminomethylpyrrolidinium bromide, 1-(3-mercaptopropyl)-1-aminomethylpyrrolidinium bromide, 1-(4-mercaptobutyl)-1-aminomethylpyrrolidinium bromide, 1-(5-mercaptopentyl)-1-aminomethylpyrrolidinium bromide, 1-(6-mercaptohexyl)-1-aminomethylpyrrolidinium bromide, 1-(8-mercaptooctyl)-1-aminomethylpyrrolidinium bromide, 1-(9-mercaptononyl)-1-aminomethylpyrrolidinium bromide, 1-(10-mercaptodecyl)-1-aminomethylpyrrolidinium bromide, 1-(11-mercaptoundecyl)-1-aminomethylpyrrolidinium bromide, 1-(12-mercaptododecyl)-1-aminomethylpyrrolidinium bromide, 1-(2-mercaptoethyl)-1-(2-aminoethyl)pyrrolidinium bromide, 1-(2-mercaptoethyl)-1-(3-aminopropyl)pyrrolidinium bromide, 1-(2-mercaptoethyl)-1-(4-aminobutyl)pyrrolidinium bromide, 1-(2-mercaptoethyl)-1-(6-aminohexyl)pyrrolidinium bromide, 1-(2-mercaptoethyl)-1-(8-aminooctyl)pyrrolidinium bromide, 1-(2-mercaptoethyl)-1-(9-aminononyl)pyrrolidinium bromide, 1-(2-mercaptoethyl)-1-(10-aminodecyl)pyrrolidinium bromide, 1-(2-mercaptoethyl)-1-(11-aminoundecyl)pyrrolidinium bromide, 1-(2-mercaptoethyl)-1-(12-aminododecyl)pyrrolidinium bromide, 1-(2-mercaptoethyl)-1-(4-methylaminobutyl)pyrrolidinium bromide, 1-(3-mercaptopropyl)-1-(8-dimethylaminooctyl)pyrrolidinium bromide, 1-(4-mercaptobutyl)-1-(9-ethylmethylaminononyl)pyrrolidinium bromide,

1-(2-mercaptoethyl)-1-aminomethylpiperidinium bromide, 1-(3-mercaptopropyl)-1-aminomethylpiperidinium bromide, 1-(4-mercaptobutyl)-1-aminomethylpiperidinium bromide, 1-(5-mercaptopentyl)-1-aminomethylpiperidinium bromide, 1-(6-mercaptohexyl)-1-aminomethylpiperidinium bromide, 1-(8-mercaptooctyl)-1-aminomethylpiperidinium bromide, 1-(9-mercaptononyl)-1-aminomethylpiperidinium bromide, 1-(10-mercaptodecyl)-1-aminomethylpiperidinium bromide, 1-(11-mercaptoundecyl)-1-aminomethylpiperidinium bromide, 1-(12-mercaptododecyl)-1-aminomethylpiperidinium bromide, 1-(2-mercaptoethyl)-1-(2-aminoethyl)piperidinium bromide, 1-(2-mercaptoethyl)-1-(3-aminopropyl)piperidinium bromide, 1-(2-mercaptoethyl)-1-(4-aminobutyl)piperidinium bromide, 1-(2-mercaptoethyl)-1-(6-aminohexyl)piperidinium bromide, 1-(2-mercaptoethyl)-1-(8-aminooctyl)piperidinium bromide, 1-(2-mercaptoethyl)-1-(9-aminononyl)piperidinium bromide, 1-(2-mercaptoethyl)-1-(10-aminodecyl)piperidinium bromide, 1-(2-mercaptoethyl)-1-(11-aminoundecyl)piperidinium bromide, 1-(2-mercaptoethyl)-1-(12-aminododecyl)piperidinium bromide, 1-(10-mercaptodecyl)-1-(9-methylaminononyl)piperidinium bromide, 1-(11-mercaptoundecyl)-1-(10-dimethylaminodecyl)piperidinium bromide, 1-(12-mercaptododecyl)-1-(12-ethylmethylaminododecyl) piperidinium bromide,

2-mercaptoethyl-(aminomethyl)dimethylammonium bromide, 3-mercaptopropyl-(aminomethyl)dimethylammonium bromide, 4-mercaptobutyl-(aminomethyl)dimethylammonium bromide, 5-mercaptopentyl-(aminomethyl)dimethylammonium bromide, 6-mercaptohexyl-(aminomethyl)dimethylammonium bromide, 8-mercaptooctyl-(aminomethyl)dimethylammonium bromide, 9-mercaptononyl-(aminomethyl)dimethylammonium bromide, 10-mercaptodecyl-(aminomethyl)dimethylammonium bromide, 11-mercaptoundecyl-(aminomethyl)dimethylammonium bromide, 12-mercaptododecyl-(aminomethyl)dimethylammonium bromide,

2-mercaptoethyl-(3-aminopropyl)dimethylammonium bromide, 2-mercaptoethyl-(4-aminobutyl)dimethylammonium bromide, 2-mercaptoethyl-(5-aminopentyl)dimethylammonium bromide, 2-mercaptoethyl-(6-aminohexyl)dimethylammonium bromide, 2-mercaptoethyl-(8-aminooctyl)dimethylammonium bromide, 2-mercaptoethyl-(9-aminononyl)dimethylammonium bromide, 2-mercaptoethyl-(10-aminodecyl)dimethylammonium bromide, 2-mercaptoethyl-(11-aminoundecyl)dimethylammonium bromide, 2-mercaptoethyl-(12-aminododecyl)dimethylammonium bromide,

5-mercaptopentyl-(8-methylaminononyl)ethylmethylammonium bromide, 6-mercaptohexyl-(6-dimethylaminohexyl)methylpropylammonium bromide, and 8-mercaptooctyl-(4-ethylmethylaminobutyl)butylhexylammonium bromide.

A method for manufacturing the reducing catalyst 1 will now be described.

First, a metal layer 102 is formed on the surface of a current collector 101. The method for this can be a known vacuum film formation method, such as sputtering, vapor deposition, or ALD (atomic layer deposition).

Then, by binding reactive functional groups 109 to the metal layer 102, modifying organic molecules 112 are immobilized on the metal layer 102. The method for this can be a known method. For example, methods such as the method of bringing the current collector 101 including the metal layer 102 into contact with a solution in which the modifying organic molecules 112 are dissolved, the method of evaporating the modifying organic molecules 112 in a high vacuum and forming a film of them on the surface of the current collector 101, and the method of atomizing the modifying organic molecules 112 onto the surface of the current collector 101, for example using a spray, can be used.

In the method in which a solution in which the modifying organic molecules 112 are dissolved is used, modifying organic molecules 112 chemically adsorbed onto the metal layer 102 spontaneously form an assembly through van der Waals forces or hydrophobic interactions between the adsorbed molecules. Then, as a result of dense assembly of the adsorbed molecules, a monolayer with aligned orientations is formed.

The solvent in which the modifying organic molecules 112 are dissolved may be any solvent in which the organic molecules can be dissolved. For example, it can be selected from alcohols, such as ethanol, and aromatic or aliphatic organic solvents, such as toluene and hexane. It is preferred to use ethanol because it exhibits high solubility for the modifying organic molecules 112 and is easy to handle.

An example of a method for immobilizing the modifying organic molecules 112 on the metal layer 102 will be described in further detail.

First, a formulated solution in which the modifying organic molecules 112 are dissolved is prepared. Then, into this formulated solution, the current collector 101 with the metal layer 102 formed thereon is immersed. The duration of immersion is from several minutes to several hours. As a result of this, the modifying organic molecules 112 are immobilized on the surface of the metal layer 102. The conditions, such as the concentration of the modifying organic molecules 112, the immersion duration, and the immersion temperature, can be changed as appropriate, for example according to the structure of the modifying organic molecules 112. Such conditions influence the state of formation of the monolayer of the modifying organic molecules 112.

When the concentration of the formulated solution is too low, the formation of the monolayer requires a long time. When the concentration is too high, however, a multilayer membrane may be formed as a result of additional adsorption of molecules onto the monolayer. The concentration of the modifying organic molecules 112, therefore, is preferably 0.1 mM or more and 100 mM or less, more preferably 1 mM or more and 10 mM or less.

The immersion duration is preferably a length of time sufficient for the formation of a dense monolayer with aligned orientations. The immersion duration is preferably 1 minute or longer and 100 hours or shorter, preferably 12 hours or longer and 72 hours or shorter.

The temperature of the formulated solution during the immersion influences the formation of a dense monolayer with aligned orientations. It is, therefore, desirable that the temperature be room temperature (25°C) or above and 60°C or below, considering factors such as the vapor pressure and boiling point of the solvent.

The immobilization of the modifying organic molecules 112 on the surface of the metal layer 102 can be confirmed by a known electrochemical method or surface analysis method.

An electrochemical method that can be used is cyclic voltammetry. A specific example is described in the following. First, a 0.2 M aqueous solution of potassium chloride (KCl) in which 1 mM of potassium hexacyanoferrate(III) ($K_3[Fe(CN)_6]$) or 1 mM of hexaammineruthenium(III) chloride ($[Ru(NH_3)_6]Cl_3$) is dissolved is prepared. An electrochemical response of the current collector 101 is measured in this aqueous solution before and after the step of adsorption of the modifying organic molecules 112, and the results are compared.

The electrochemical response measured is the kinetic current caused by an electrochemical redox reaction of hexacyanoferrate(III) anions or hexaammineruthenium(III) cations. The kinetic current with the current collector 101 with immobilized modifying organic molecules 112 decreases compared with the kinetic current with the current collector 101 without immobilized modifying organic molecules 112. This owes to inhibition of the redox reaction of hexacyanoferrate(III) anions or hexaammineruthenium(III) cations caused by the immobilization of the modifying organic molecules 112 on the metal layer 102. As can be seen from this, by measuring the kinetic current as described above, the immobilization of the modifying organic molecules 112 can be confirmed indirectly.

A surface analysis method that can be used is a Fourier-transform infrared spectrophotometer (FT-IR) using a reflection method. With this method, the infrared spectrum of thin films and adsorbed molecular species on the surface of the current collector 101 can be measured with high sensitivity. That is, information on the structure, in particular functional groups, of the organic molecules, can be obtained. X-ray photoelectron spectroscopy (XPS), furthermore, is also a surface analysis method that can be used. With this method, the composition of the modifying organic molecules 112, and of the anion included in the modifying organic molecules 112 when an anion is present, can be measured. Moreover, it is also

possible to determine whether or not the modifying organic molecules 112 are present based on a difference in wettability with water using a contact angle meter.

A reduction reaction at the reducing catalyst 1 will now be described, taking the example of the reduction of $CO_2$. In an elementary reaction in the reduction reaction of $CO_2$, $CO_2$ becomes a $CO_2$ radical anion through a single-electron reduction reaction. This reaction requires a high overpotential. This overpotential is a loss of energy and causes a decrease in energy conversion efficiency. Concurrently with the reduction reaction of $CO_2$, furthermore, reduction reactions of water and hydrogen ions occur as side reactions, generating hydrogen. Due to these side reactions, the Faraday efficiency of the reduction reaction of $CO_2$ decreases. With the reducing catalyst 1, however, it is possible to achieve a high reduction efficiency.

Within the reducing catalyst 1, the quaternary nitrogen cation forms a reaction intermediate with $CO_2$, thereby contributing to the formation and stabilization of $CO_2$ radical anions. By virtue of this, the reducing catalyst 1 can induce the reduction reaction of $CO_2$ a small amount of energy. The energy conversion efficiency of the reducing catalyst, therefore, can be improved. In addition, the quaternary nitrogen cation has the effect of inhibiting access of water and hydrogen ions to the metal layer 102. By virtue of this, the quaternary nitrogen cation can impart reaction selectivity to reduction reactions at the metal layer 102. The generation of hydrogen from side reactions, therefore, can be reduced, whereby the Faraday efficiency can be improved.

The amino groups in the modifying organic molecules 112, furthermore, form carbonates by reacting with $CO_2$ molecules. With these amino groups, therefore, $CO_2$ molecules required for the reduction reaction can be attracted and supplied to the quaternary nitrogen cation and the metal layer 102. The amino groups, furthermore, form salts with carboxylic acids (e.g., formic acid, acetic acid, and oxalic acid) resulting from the reduction of $CO_2$, and thus have the effect of promoting a multi-electron reduction reaction, in which reduction continuously occurs. As a result, it is possible to improve reduction efficiency.

The reducing catalyst 1 allows carbon dioxide to be used as the source material for reduction. The reduction product varies according to interactions among the quaternary nitrogen cation, the metal layer 102, and the source material for reduction. For example, when $CO_2$ is used as the source material, carbon monoxide (CO), formic acid (HCOOH), formaldehyde (HCHO), methanol ($CH_3OH$), acetic acid ($CH_3COOH$), acetaldehyde ($CH_3CHO$), ethanol ($CH_3CH_2OH$), oxalic acid ($(COOH)_2$), glycolic acid ($C_2H_2O_3$), glycolaldehyde ($C_2H_2O_2$), and ethylene glycol ($HOCH_2CH_2OH$) can form. With the reducing catalyst 1, however, ethylene glycol can be produced with high selectivity.

<Photochemical Reaction Cell>

A photochemical reaction cell included in the chemical reaction apparatus will now be described with reference to Figs. 3 to 5.

The photochemical reaction cell includes an oxidizing catalyst layer, a reducing catalyst layer containing a reducing catalyst, and a power supply element coupled to the oxidizing catalyst layer and the reducing catalyst layer. The power supply element preferably includes a semiconductor layer that undergoes charge separation in response to optical energy. For example, it can be a solar cell. The semiconductor layer is preferably positioned between the oxidizing catalyst layer and the reducing catalyst layer.

Fig. 3 is a cross-sectional view illustrating the structure of a photochemical reaction cell 30. As illustrated in Fig. 3, the photochemical reaction cell 30 is a multilayer body in which a reducing catalyst layer 20, a substrate 11, a reflective layer 12, a reducing electrode layer 13, a multi-junction solar cell 17, an oxidizing electrode layer 18, and an oxidizing catalyst layer 19 are stacked in this order. The reducing catalyst layer 20 side of the photochemical reaction cell 30 is the back surface, and the oxidizing catalyst layer 19 side is the front surface, on which light is incident.

The substrate 11 is provided to support the photochemical reaction cell and increase its mechanical strength. The substrate 11 is composed of a material having electroconductivity. For example, it can be configured as a metal plate formed of a metal selected from the group consisting of Au, Ag, Cu, Pt, Zn, Fe, Ti, Sn, In, Bi, and Ni or an alloy plate containing at least one of these metals. An alloy plate can be, for example, a plate of an alloy such as SUS. Alternatively, the substrate 11 can be composed of, for example, a resin having electroconductivity. The substrate 11, furthermore, can be configured as a semiconductor plate, for example of Si or Ge. Moreover, the substrate 11 can be configured as an ion-exchange membrane.

The reflective layer 12 is formed on the surface of the substrate 11. The reflective layer 12 is composed of a material capable of optical reflection. For example, it can be configured as a metal layer or as a distributed Bragg reflection layer formed by a semiconductor multilayer membrane. This reflective layer 12 is positioned between the substrate 11 and the multi-junction solar cell 17, allowing light that is not absorbed at the multi-junction solar cell 17 to be reflected and brought again into the multi-junction solar cell 17. Thereby, optical absorption at the multi-junction solar cell 17 can be improved.

The reducing electrode layer 13 is positioned on the reflective layer 12 and is sandwiched between the reflective layer 12 and an n-type semiconductor layer of the multi-junction solar cell 17 (an n-type amorphous silicon layer 14a, which

will be described later). The reducing electrode layer 13, therefore, is preferably composed of a material capable of ohmic contact with an n-type semiconductor layer. The reducing electrode layer 13 is composed of, for example, a metal, such as Ag, Au, Al, or Cu, or an alloy containing at least one of such metals. Alternatively, the reducing electrode layer 13 is composed of a transparent conductive oxide, such as ITO (indium tin oxide) or zinc oxide (ZnO), FTO (fluorine-doped tin oxide), AZO (aluminum-doped zinc oxide), or ATO (antimony-doped tin oxide). The reducing electrode layer 13, furthermore, may have a structure in which, for example, layers of a metal and a transparent conductive oxide are stacked, a structure in which a metal and another electroconductive material are combined, or a structure in which a transparent conductive oxide and another electroconductive material are combined.

The multi-junction solar cell 17 is positioned on the reducing electrode layer 13. The multi-junction solar cell 17 has a structure in which a first solar cell 14, a second solar cell 15, and a third solar cell 16 are stacked, in order from the reducing electrode layer 13 side. They are all solar cells made using a pin-junction semiconductor. The first solar cell 14, the second solar cell 15, and the third solar cell 16 each differ in the wavelength of light absorbed. By virtue of these solar cells being stacked flat, the multi-junction solar cell 17 can absorb the broad wavelength spectrum of light included in sunlight. As a result, it becomes possible to utilize solar energy more efficiently. The solar cells, furthermore, are coupled in series, and thus a high open-circuit voltage can be achieved.

The first solar cell 14 includes, in order from the reducing electrode layer 13 side, an n-type amorphous silicon (a-Si) layer 14a, an intrinsic amorphous silicon germanium (a-SiGe) layer 14b, and a p-type microcrystalline silicon (μc-Si) layer 14c. The a-SiGe layer 14b is a layer that absorbs light in a short-wavelength region, approximately 400 nm. The first solar cell 14, therefore, undergoes charge separation in response to optical energy in the short-wavelength region.

The second solar cell 15 includes, in order from the reducing electrode layer 13 side, an n-type a-Si layer 15a, an intrinsic a-SiGe layer 15b, and a p-type μc-Si layer 15c. The a-SiGe layer 15b is a layer that absorbs light in an intermediate-wavelength region, approximately 600 nm. The second solar cell 15, therefore, undergoes charge separation in response to optical energy in the intermediate-wavelength region.

The third solar cell 16, furthermore, includes, in order from the reducing electrode layer 13 side, an n-type a-Si layer 16a, an intrinsic a-Si layer 16b, and a p-type μc-Si layer 16c. The a-Si layer 16b is a layer that absorbs light in a long-wavelength region, approximately 700 nm. The third solar cell 16, therefore, undergoes charge separation in response to optical energy in the long-wavelength region.

The multi-junction solar cell 17 undergoes charge separation in response to light in each wavelength region. That is, holes and electrons separate, moving toward the positive electrode (front surface) and the negative electrode (back surface), respectively. As a result of this, an electromotive force is produced in the multi-junction solar cell 17.

It should be noted that although a multi-junction solar cell 17 configured in a multilayer structure composed of three solar cells has been described by way of example here, the configuration is not limited to this; it is also possible to use a multi-junction solar cell configured in a multilayer structure composed of two or four or more solar cells. Alternatively, a single solar cell may be used instead of a multi-junction solar cell 17. Although solar cells made using a pin-junction semiconductor is used have been described, furthermore, solar cells made using a pn-junction semiconductor may be used. Moreover, a semiconductor layer formed of a compound such as GaAs, GaInP, AlGaInP, CdTe, or CuInGaSe, for example, may be used. In addition, various forms of layers, such as single-crystal, polycrystalline, or amorphous, can be applied as semiconductor layers.

The oxidizing electrode layer 18 is positioned on the multi-junction solar cell 17 and is sandwiched between a p-type semiconductor layer of the multi-junction solar cell 17 and the oxidizing catalyst layer 19. The oxidizing electrode layer 18 is preferably composed of a material capable of ohmic contact with a p-type semiconductor layer. The oxidizing electrode layer 18 is composed of a transparent conductive oxide, such as ITO (indium tin oxide) or zinc oxide (ZnO), FTO (fluorine-doped tin oxide), AZO (aluminum-doped zinc oxide), or ATO (antimony-doped tin oxide). The oxidizing electrode layer 18, furthermore, may have a structure in which, for example, layers of a metal and a transparent conductive oxide are stacked, a structure in which a metal and another electroconductive material are combined, or a structure in which a transparent conductive oxide and another electroconductive material are combined.

The oxidizing catalyst layer 19 is positioned closer to the positive electrode of the multi-junction solar cell 17 and is formed on the oxidizing electrode layer 18. The oxidizing catalyst layer 19 oxidizes $H_2O$ and produces $O_2$ and $H^+$ when the pH of the electrolytic solution is lower than 7 (pH < 7). When the pH of the electrolytic solution is greater than 7 (pH > 7), however, it oxidizes $OH^-$ and produces $O_2$ and $H_2O$. For this purpose, the oxidizing catalyst layer 19 is composed of a material that reduces the activation energy for carrying out the oxidation reaction. In other words, it is composed of a material that reduces the overpotential during the reaction for oxidizing $H_2O$ or $OH^-$ and extracting electrons.

Examples of such materials include binary metal oxides, such as manganese oxide (Mn-O), iridium oxide (Ir-O), nickel oxide (Ni-O), cobalt oxide (Co-O), iron oxide (Fe-O), tin oxide (Sn-O), indium oxide (In-O), or ruthenium oxide (Ru-O), ternary metal oxides, such as Ni-Co-O, La-Co-O, Ni-La-O, or Sr-Fe-O, quaternary metal oxides, such as Pb-Ru-Ir-O or La-Sr-Co-O, or metal complexes, such as Ru complexes or Fe complexes.

The form of the oxidizing catalyst layer 19 is not limited to a thin-film form; it may be in a grid form, a particulate form, or a wire form.

In the photochemical reaction cell 30, the irradiation light reaches the multi-junction solar cell 17 by passing through the oxidizing catalyst layer 19 and the oxidizing electrode layer 18. Accordingly, the oxidizing electrode layer 18 and the oxidizing catalyst layer 19, which are positioned closer to the surface irradiated with light, have optical permeability to the irradiation light. More specifically, the transmittance of the oxidizing electrode layer 18 and the oxidizing catalyst layer 19, which are positioned closer to the irradiation surface, is at least 10% or more, more desirably 30% or more, of the amount of irradiation of the irradiation light.

The reducing catalyst layer 20 is positioned closer to the negative electrode of the multi-junction solar cell 17 and is formed on the back surface of the substrate 11. The reducing catalyst layer 20 reduces a source material for reduction, such as $CO_2$, and produces carbon compounds (e.g., carbon monoxide, formic acid, formaldehyde, methane, methanol, acetic acid, acetaldehyde , ethanol, and ethylene glycol). For this purpose, the reducing catalyst layer 20 is composed of a material that reduces the activation energy for reducing the source material for reduction.

As such a reducing catalyst layer 20, a reducing catalyst 1 as described above is applied. That is, the reducing catalyst layer 20 includes a current collector 101 and organic molecules 112 containing at least one quaternary nitrogen cation. The current collector 101 may be used as the substrate 11.

In addition, on the front surface of the multi-junction solar cell 17, or between the electrode layer and the catalyst layer closer to the surface irradiated with light (for the photochemical reaction cell 30, between the oxidizing electrode layer 18 and the oxidizing catalyst layer 19), a protective layer having electroconductivity may be placed. The protective layer prevents corrosion of the multi-junction solar cell 17 in the redox reactions. As a result, the life of the multi-junction solar cell 17 can be extended. The protective layer, furthermore, optionally has optical transparency. An example of a protective film is a dielectric thin film, for example of $TiO_2$, $ZrO_2$, $Al_2O_3$, $SiO_2$, or $HfO_2$. Moreover, its thickness is preferably 10 nm or less, more preferably 5 nm or less, so that electroconductivity is obtained through the tunnel effect.

Figs. 4 and 5 are cross-sectional views for describing the principle of operation of the photochemical reaction cell 30. In these drawings, the reflective layer 12, the reducing electrode layer 13, and the oxidizing electrode layer 18 are omitted.

As illustrated in Figs. 4 and 5, when light L is incident on the front surface side (the oxidizing electrode layer 18 side), the incident light passes through the oxidizing catalyst layer 19 (and the oxidizing electrode layer 18) and reaches the multi-junction solar cell 17. Upon absorption of the light by the multi-junction solar cell 17, photoexcited electrons and their counterpart holes are generated, and they are separated. Specifically, in each solar cell (the first solar cell 14, the second solar cell 15, and the third solar cell 16), photoexcited electrons move toward the n-type semiconductor layer (toward the reducing catalyst layer 20), and holes generated as counterparts of the photoexcited electrons move toward the p-type semiconductor layer (toward the oxidizing catalyst layer 19). That is, charge separation occurs. As a result of this, an electromotive force arises in the multi-junction solar cell 17.

The photoexcited electrons generated inside the multi-junction solar cell 17 are used in the reduction reaction at the reducing catalyst layer 20, which is the negative electrode. The holes are used in the oxidation reaction at the oxidizing catalyst layer 19, which is the positive electrode.

An example in the case in which the electrolytic solution is an acidic solution, having a pH smaller than 7, is illustrated in Fig. 4. Near the oxidizing catalyst layer 19, the reaction in formula (1) below occurs. That is, $H_2O$ is oxidized, whereby $O_2$, $H^+$, and electrons are produced. The $H^+$ produced in this reaction moves toward the reducing catalyst layer 20 via the pathway for ion movement described later. Near the reducing catalyst layer 20, the reaction in formula (2) below occurs. That is, $CO_2$ is reduced by incoming $H^+$ and electrons, whereby carbon monoxide (CO) and $H_2O$ are produced.

$$2H_2O \rightarrow 4H^+ + O_2 + 4e^- \ldots \quad (1)$$

$$2CO_2 + 4H^+ + 4e^- \rightarrow 2CO + 2H_2O \ldots \quad (2)$$

An example in the case in which, by contrast, the electrolytic solution is a basic solution, having a pH greater than 7, is illustrated in Fig. 5. Near the oxidizing catalyst layer 19, the reaction in formula (3) below occurs. That is, $OH^-$ is oxidized, whereby $O_2$, $H_2O$, and electrons are produced. Near the reducing catalyst layer 20, the reaction in formula (4) below occurs. That is, $CO_2$ undergoes a reduction reaction, in which it receives electrons, together with $H_2O$, whereby carbon monoxide (CO) and $OH^-$ are produced. The $OH^-$ produced near the reducing catalyst layer 20 moves toward the oxidizing catalyst layer 19 via the pathway for ion movement described later.

$$4OH^- \rightarrow O_2 + 2H_2O + 4e^- \ldots \quad (3)$$

$$2CO_2 + 2H_2O + 4e^- \rightarrow 2CO + 4OH^- \ldots \quad (4)$$

The multi-junction solar cell 17 needs to have an open-circuit voltage equal to or greater than the potential difference between the standard redox potential of the oxidation reaction that occurs at the oxidizing catalyst layer 19 and

the standard redox potential of the reduction reaction that occurs at the reducing catalyst layer 20. For example, when the pH of the reaction solution is = 0, the standard redox potential of the oxidation reaction in formula (1) is +1.23 [V], and the standard redox potential of the reduction reaction in formula (2) is -0.1 [V]. The open-circuit voltage of the multi-junction solar cell 17, therefore, needs to be 1.33 [V] or more.

More specifically, the open-circuit voltage needs to be equal to or greater than the potential difference including overpotentials. For example, when the overpotentials for the oxidation reaction in formula (1) and the reduction reaction in formula (2) are each 0.2 [V], the open-circuit voltage is preferably 1.73 [V] or more.

It should be noted that although the reduction reactions in formula (2) and formula (4) above are presented as reduction reactions from $CO_2$ to CO, the reaction scheme is not limited to these; reduction reactions from $CO_2$ to HCOOH, HCHO, $CH_4$, $CH_3OH$, $C_2H_5OH$, $HOCH_2CH_2OH$, etc., can occur. All reduction reactions consume $H^+$ or produce $OH^-$. Accordingly, when $H^+$ produced at the oxidizing catalyst layer 19 cannot move to the reducing catalyst layer 20 at the counter electrode, or, alternatively, when $OH^-$ produced at the reducing catalyst layer 20 cannot move to the oxidizing catalyst layer 19 at the counter electrode, the overall reaction efficiency decreases. With a chemical reaction apparatus according to this embodiment, by contrast, the transportation of $H^+$ or $OH^-$ can be improved because the reaction apparatus has pathways for ion movement through which $H^+$ or $OH^-$ is moved. A high photoreaction efficiency, therefore, can be achieved.

<Chemical Reaction Apparatus>

A chemical reaction apparatus in which a photochemical reaction cell as described above is used will now be described using Figs. 6 to 9. In this section, redox reactions in the case in which the electrolytic solution is an acidic solution, having a pH smaller than 7 (formula (1) and formula (2) above), are described by way of example. When the electrolytic solution is a basic solution, having a pH greater than 7, redox reactions according to formula (3) and formula (4) above occur.

Fig. 6 is a perspective view illustrating the structure of a chemical reaction apparatus 200 according to this embodiment. Fig. 7 is a cross-sectional view illustrating the structure of the chemical reaction apparatus 200. The chemical reaction apparatus 200 includes a photochemical reaction cell 30, electrolytic cells 31 in which the photochemical reaction cell is housed, and at least one electrolytic cell channel 41 connected to the electrolytic cells 31 as a pathway for ion movement. It should be noted that in Fig. 6, the pathway for ion movement is omitted. The photochemical reaction cell 30 includes, as described above, a multilayer body composed of an oxidizing catalyst layer 19, a reducing catalyst layer 20, a multi-junction solar cell 17 formed therebetween, and a substrate 11.

The electrolytic cells 31 include an oxidation reaction electrolytic cell 31a, in which the oxidizing catalyst layer 19 is positioned, and a reduction reaction electrolytic cell 31b, in which the reducing catalyst layer 20 is positioned. In the oxidation reaction electrolytic cell 31a, $H_2O$ is oxidized by the oxidizing catalyst layer 19, whereby $O_2$ and $H^+$ are produced. In the reduction reaction electrolytic cell 31b, $CO_2$ is reduced by the reducing catalyst layer 20, whereby CO and $H_2O$ are produced. These two electrolytic cells are separated into the two by the substrate 11 of the photochemical reaction cell 30. It should be noted that although the ends of the substrate 11 extend farther away than the ends of the multi-junction solar cell 17, the oxidizing catalyst layer 19, and the reducing catalyst layer 20 in this example, the geometry is not limited to this; the substrate 11, the multi-junction solar cell 17, the oxidizing catalyst layer 19, and the reducing catalyst layer 20 may be in a flat-plate shape occupying the same area.

The oxidation reaction electrolytic cell 31a and the reduction reaction electrolytic cell 31b can be supplied with different electrolytic solutions. The electrolytic cell channel 41, which allows ions to move through, links the oxidation reaction electrolytic cell 31a and the reduction reaction electrolytic cell 31b together.

As is seen from this, with a configuration including at least one electrolytic cell channel 41, $H^+$ produced on the oxidizing catalyst layer 19 side can be moved to the reducing catalyst layer 20, and carbon dioxide can be decomposed on the reducing catalyst layer 20 side with the $H^+$. A high photoreaction efficiency, therefore, can be achieved.

The electrolytic cell channel 41 is described in further detail. The electrolytic cell channel 41 is provided at, for example, the side of the electrolytic cells 31. One end of the electrolytic cell channel 41 is connected to the oxidation reaction electrolytic cell 31a, and the other end is connected to the reduction reaction electrolytic cell 31b. That is, the electrolytic cell channel 41 connects the oxidation reaction electrolytic cell 31a and the reduction reaction electrolytic cell 31b together. As a result of this, the movement of ions between the oxidizing catalyst layer 19 and the reducing catalyst layer 20 becomes possible.

Part of the inside of the electrolytic cell channel 41 is packed with an ion-exchange membrane 43, and the ion-exchange membrane 43 allows only specific ions to pass through. By virtue of this, it is possible to move only specific ions while separating the electrolytic solution between the oxidation reaction electrolytic cell 31a and the reduction reaction electrolytic cell 31b.

The ion-exchange membrane 43 is a proton-exchange membrane, through which $H^+$ produced in the oxidation reaction electrolytic cell 31a can be moved to the reduction reaction electrolytic cell 31b side. Examples of proton-exchange membranes include a cation-exchange membrane, such as Nafion or FLEMION, and an anion-exchange membrane, such as NEOSEPTA or SELEMION.

It should be noted that instead of an ion-exchange membrane 43, a material through which ions can move and with which the electrolytic solution can be separated, such as agar, may be used. For example, a salt bridge is used. In general, when a proton-exchange solid polymer film, such as ones typified by Nafion, is used, good ion movement performance can be achieved.

In addition, a circulating mechanism 42, such as a pump, may be provided in the electrolytic cell channel 41. By promoting the circulation of the electrolytic solution using a circulating mechanism 42, the circulation of ions ($H^+$) can be improved between the oxidation reaction electrolytic cell 31a and the reduction reaction electrolytic cell 31b. Two electrolytic cell channels 41, furthermore, may be provided, and at least one circulating mechanism 42 provided in at least one of them may be used to move ions from the oxidation reaction electrolytic cell 31a to the reduction reaction electrolytic cell 31b via one electrolytic cell channel 41 and move ions from the reduction reaction electrolytic cell 31b to the oxidation reaction electrolytic cell 31a via the other electrolytic cell channel 41. Moreover, multiple circulating mechanisms 42 may be provided. In addition, multiple (three or more) electrolytic cell channels 41 may be provided to reduce the diffusion of ions and allow ions to circulate more efficiently.

By creating a flow of liquid using a circulating mechanism 42, persistence of bubbles of generated gas on the electrode surfaces and the surfaces of the electrolytic cells can also be limited, whereby an efficiency loss and light intensity variations caused by sunlight scattering due to the bubbles can be reduced.

When the reaction apparatus is irradiated with light, furthermore, heat can arise on the surface of the multi-junction solar cell 17. The temperature difference in the electrolytic solution caused by this heat may be utilized to create convection and circulate ions more efficiently. In this case, the movement of ions can be promoted compared with ionic diffusion.

A temperature adjustment mechanism 44 that adjusts the temperature of the electrolytic solution may be provided inside the electrolytic cell channel 41 or inside the electrolytic cells 31. Through temperature control using a temperature adjustment mechanism 44, solar cell performance and catalyst performance can be controlled. For example, by equalizing the temperature of the reaction system, the performance of the solar cells and the catalyst can be stabilized and can even be improved. By virtue of the stability of the system, furthermore, temperature increases can also be prevented. Through temperature control, the selectivity of the solar cells and the catalyst can be changed, and their products can also be controlled.

The oxidation reaction electrolytic cell 31a is filled with an electrolytic solution containing any electrolyte. In this electrolytic solution, the oxidizing catalyst layer 19 is immersed. The electrolytic solution is preferably one that promotes the oxidation reaction of $H_2O$. For example, a liquid containing $H_2O$ is used.

The reduction reaction electrolytic cell 31b is filled with any electrolytic solution. In this electrolytic solution, the reducing catalyst layer 20 is immersed. The electrolytic solution preferably reduces the reduction potential of $CO_2$, exhibits high ionic conductivity, and contains a $CO_2$ absorber, which absorbs $CO_2$. For example, a liquid containing $CO_2$ is used.

An example of an electrolytic solution with which the reduction reaction electrolytic cell 31b can be filled is an ionic liquid, which is a salt of a cation such as an imidazolium ion or pyridinium ion and an anion such as $BF_4^-$ or $PF_6^-$ and is in a liquid state within a broad temperature range, or its aqueous solution. An amine solution, such as ethanolamine, imidazole, or pyridine, or its aqueous solution, furthermore, is also an example. The amine may be any of a primary amine, a secondary amine, or a tertiary amine. Examples of primary amines include methylamine, ethylamine, propylamine, butylamine, pentylamine, and hexylamine. For all of a primary amine, a secondary amine, and a tertiary amine, the substituent hydrocarbon group(s) on the amine may be alcohol group(s) or may alternatively be substituted with halogens. Examples of such amines include methanolamine and ethanolamine, as well as chloromethylamine. The substituent hydrocarbon group(s) on the amine may have unsaturated bonds.

Examples of secondary amines include dimethylamine, diethylamine, dipropylamine, dibutylamine, dipentylamine, dihexylamine, dimethanolamine, diethanolamine, dipropanolamine, methylethylamine, and methylpropylamine.

Examples of tertiary amines include trimethylamine, triethylamine, tripropylamine, tributylamine, trihexylamine, trimethanolamine, triethanolamine, tripropanolamine, tributanolamine, tripropanolamine, trihexanolamine, methyldiethylamine, and methyldipropylamine.

The cation in an ionic liquid can be an ion such as an imidazolium ion or a pyridinium ion. Examples of imidazolium ions include a 1-ethyl-3-methylimidazolium ion, a 1-methyl-3-propylimidazolium ion, a 1-butyl-3-methylimidazole ion, a 1-methyl-3-pentylimidazolium ion, and a 1-hexyl-3-methylimidazolium ion. Position 2 of such imidazolium ions may be substituted, and examples of such types include a 1-ethyl-2,3-dimethylimidazolium ion, a 1,2-dimethyl-3-propylimidazolium ion, a 1-butyl 2,3-dimethylimidazolium ion, a 1,2-dimethyl-3-pentylimidazolium ion, and a 1-hexyl-2,3-dimethylimidazolium ion. Examples of pyridinium ions include methylpyridinium, ethylpyridinium, propylpyridinium, butylpyridinium, pentylpyridinium, and hexylpyridinium. For both imidazolium ions and pyridinium ions, alkyl groups therein may be substituted, and unsaturated bonds may exist.

Examples of anions that can be in an ionic liquid include a fluoride ion, a chloride ion, a bromide ion, an iodide ion,

$BF_4^-$, $PF_6^-$, $CF_3COO^-$, $CF_3SO_3^-$, $NO_3^-$, $SCN^-$, $(CF_3SO_2)_3C^-$, bis(trifluoromethoxysulfonyl)imide, bis(trifluoromethoxysulfonyl)imide, and bis(perfluoroethylsulfonyl)imide. A zwitterion formed by linking the cation and the anion in an ionic liquid with a hydrocarbon, furthermore, can also be used.

The temperatures of the electrolytic solution filling the oxidation reaction electrolytic cell 31a and the electrolytic solution filling the reduction reaction electrolytic cell 31b may be equal or may be different, depending on the environment in which the solutions are used. For example, the electrolytic solution used for the reduction reaction electrolytic cell 31b can be a $CO_2$-containing amine-based absorbent discharged from a plant. In that case, the temperature of this electrolytic solution is higher than the atmospheric temperature. For example, it is 30°C or above and 150°C or below, more specifically 40°C or above and 120°C or below.

As the reducing catalyst layer 20, a reducing catalyst 1 in the first embodiment is used. To the metal layer 102 of the reducing catalyst, a reduction potential has been applied. Due to this, $CO_2$-containing ions (e.g., hydrogencarbonate ions) or physically dissolved $CO_2$, among other constituents of the electrolytic solution, are subjected to electrostatic attractive forces in the vicinity of the metal layer 102 and the quaternary nitrogen cations contained in the modifying organic molecules 112 immobilized on its surface. As a result, $CO_2$ forms an electric double layer with the metal layer 102 and the quaternary nitrogen cations at the catalyst/electrolytic solution interface.

At this interface, a $CO_2$-reducing reaction driven by a charge transfer reaction proceeds. In the reduction reaction electrolytic cell 31b, $CO_2$ is reduced by the reducing catalyst layer 20, whereby carbon compounds are produced. Specifically, the $CO_2$ is converted into carbon monoxide (CO), formic acid (HCOOH), formaldehyde (HCHO), methanol ($CH_3OH$), acetic acid ($CH_3COOH$), acetaldehyde ($CH_3CHO$) ethanol ($CH_3CH_2OH$), and ethylene glycol ($HOCH_2CH_2OH$). As a side reaction, furthermore, hydrogen ($H_2$) is also produced through the reduction of water ($H_2O$).

A two-electron reduction reaction of carbon dioxide produces formic acid in addition to carbon monoxide. A two-electron reduction reaction of formic acid produces formaldehyde. A two-electron reduction reaction of formaldehyde, furthermore, produces methanol. When methanol is produced using the reducing catalyst 1, formic acid or formaldehyde may be selected as a source material for reduction in addition to carbon dioxide. The electrolytic solution in the reduction reaction electrolytic cell 31b, therefore, desirably contains at least one source material for reduction absorbed therein selected from carbon dioxide, formic acid, and formaldehyde. For instance, an example of an electrolytic solution that can be in the reduction reaction electrolytic cell 31b is a sodium hydrogencarbonate solution.

A two-electron reduction reaction of carbon dioxide, furthermore, may produce oxalic acid. A two-electron reduction reaction of oxalic acid produces glycolic acid. A two-electron reduction reaction of glycolic acid, furthermore, produces glyoxal or glycolic acid. Moreover, a two-electron reduction reaction of glyoxal or glycolic acid produces glycolaldehyde. In addition, a two-electron reduction reaction of glycolaldehyde produces ethylene glycol. When ethylene glycol is produced using the reducing catalyst 1, oxalic acid, glycolic acid, or glycolaldehyde may be selected as a source material for reduction in addition to carbon dioxide. The electrolytic solution in the reduction reaction electrolytic cell 31b, therefore, may contain at least one source material for reduction absorbed therein selected from oxalic acid, glycolic acid, or glycolaldehyde.

An eight-electron reduction reaction of carbon dioxide may produce acetic acid. An eight-electron reduction reaction of acetic acid produces acetaldehyde. An eight-electron reduction reaction of acetaldehyde, furthermore, produces ethanol. When ethanol is produced using the reducing catalyst 1, acetic acid or acetaldehyde may be selected as a source material for reduction in addition to carbon dioxide. The electrolytic solution in the reduction reaction electrolytic cell 31b, therefore, may contain at least one source material for reduction absorbed therein selected from carbon dioxide, acetic acid, and acetaldehyde.

As stated above, the reactions in which carbon dioxide is reduced and formic acid, formaldehyde, and methanol are produced, the reactions in which carbon dioxide is reduced and oxalic acid, glycolic acid, glyoxal or glycolic acid, glycolaldehyde, and ethylene glycol are produced, and the reactions in which carbon dioxide is reduced and acetic acid, acetaldehyde, and ethanol are produced depend on the density of the modifying organic molecules 112 in the reducing catalyst 1. For example, when the density of the modifying organic molecules 112 with respect to the metal layer 102 is $1 \times 10^{11}$ atoms/$cm^2$ or less, the reactions in which formic acid, formaldehyde, and methanol are produced primarily occur. By contrast, when the density of the modifying organic molecules 112 is, for example, $1 \times 10^{12}$ atoms/$cm^2$ or more and $10^{15}$ atoms/$cm^2$ or less, reactions in which acetic acid, acetaldehyde, and ethanol are produced in addition to formic acid, formaldehyde, and methanol occur. In particular, when the density of the modifying organic molecules 112 is $1 \times 10^{13}$ atoms/$cm^2$ or more and $10^{15}$ atoms/$cm^2$ or less, the reactions in which acetic acid, acetaldehyde, and ethanol are produced primarily occur.

The bonding state and molecular density of the modifying organic molecules 112 can be calculated based on analytical results obtained by X-ray photoelectron spectroscopy (XPS). The analytical conditions can be the conditions specified below. It should be noted that the detection angle indicates the angle formed by the normal to the sample and the axis of the input lens of the detector.

Model used Quantera-SXM, manufactured by PHI

Irradiation X-ray source Single-crystal spectral AlK$\alpha$ radiation

Power 50 W

Region analyzed $\phi$200 $\mu$m

Pass Energy

Wide Scan - 280.0 eV (1.0 eV/Step)

Narrow Scan - 69.0 eV (0.125 eV/Step)

Detection angle 45°

Electron gun for charge neutralization Used for both $Ar^+$ and $e^-$

As charge correction (correction of energy on the horizontal axis), the C-C/H bond component of the C1s spectrum is aligned with 284.80 eV.

The bonding density (molecular density) of the modifying organic molecules 112 is calculated according to equation (6) below from an approximate number of Au atoms per unit area estimated using equation (5) below and the number of S atoms normalized by the number of Au atoms (S/Au) obtained as a result of a semiquantitative analysis.

$$\text{Au (atoms/cm}^2\text{)} = \text{Density (g/cm}^3\text{)} \times \text{detection depth (nm)} \times \text{N/Mw} \ldots (5)$$

$$\text{Molecular density (atoms/cm}^2\text{)} = \text{Au (atoms/cm}^2\text{)} \times \text{S/Au (ratio by the number of atoms)} \ldots (6)$$

In these equations, the density is 19.3 g/cm$^3$, the detection depth is 5 nm, N is Avogadro's number (atoms/mol), and Mw is 197 g/mol.

At the reducing catalyst 1, the reactions in which carbon dioxide is reduced and ethylene glycol is produced via oxalic acid, glycolic acid, or glycolaldehyde occur selectively as a result of the electrode being held at the reduction potential. That is, as a result of the electrode being held at the reduction potential, the orientations of the modifying organic molecules 112 become uniformly aligned, and thus the reactions in which ethylene glycol is produced occur. For the electrolytic conditions under which the electrode is held at the reduction potential, it is preferred that a potential of -0.8 V to -1.3 V be applied to the working electrode for 5 hours or longer in a three-electrode cell in which the electrode substrate is the working electrode, silver/silver chloride is the reference electrode, and Pt is used as the counter electrode. It is more preferred that the potential be held for 3 hours or longer, even more preferably 1 hour or longer. The orientation of the modifying organic molecules 112 can be observed using a scanning tunneling microscope (STM).

Variations of chemical reaction apparatuses according to this embodiment will now be described. Fig. 8 is a cross-sectional view illustrating the structure of a chemical reaction apparatus 210. Fig. 9 is a cross-sectional view illustrating the structure of a chemical reaction apparatus 220. In the following, structures different from that of the above-described chemical reaction apparatus 200 will be described.

As illustrated in Fig. 8, the chemical reaction apparatus 210 includes a photochemical reaction cell 30, electrolytic cells 31 in which the photochemical reaction cell 30 is housed, and an opening 51 created in the substrate 11 as a pathway for ion movement.

The opening 51 is provided such that it extends through an end of the substrate 11 from the oxidation reaction electrolytic cell 31a side to the reduction reaction electrolytic cell 31b side. Part of the opening 51 is packed with an ion-exchange membrane 43, and the oxidation reaction electrolytic cell 31a and the reduction reaction electrolytic cell 31b are separated by the substrate 11 and the ion-exchange membrane 43. The ion-exchange membrane 43 allows only specific ions to pass through.

With such a configuration, it is possible to move only specific ions via the ion-exchange membrane 43 while separating the electrolytic solution between the oxidation reaction electrolytic cell 31a and the reduction reaction electrolytic cell 31b.

As illustrated in Fig. 9, the chemical reaction apparatus 220 includes a photochemical reaction cell 30, electrolytic cells 31 in which the photochemical reaction cell 30 is housed, and an opening 52 created as a pathway for ion movement. The opening 52 is provided such that it extends through the reducing catalyst layer 20, the substrate 11, the multi-junction solar cell 17, and the oxidizing catalyst layer 19 from the oxidation reaction electrolytic cell 31a side to the reduction reaction electrolytic cell 31b side.
Part of the opening 52 is packed with an ion-exchange membrane 43, and the oxidation reaction electrolytic cell 31a and the reduction reaction electrolytic cell 31b are separated by the substrate 11 and the ion-exchange membrane 43. The ion-exchange membrane 43 allows only specific ions to pass through.
With such a configuration, it is possible to move only specific ions via the ion-exchange membrane 43 while separating the electrolytic solution between the oxidation reaction electrolytic cell 31a and the reduction reaction electrolytic cell 31b.
It should be noted that although the chemical reaction apparatus 220 in Fig. 9 includes the ion-exchange membrane 43 only in part of the opening 52, the ion-exchange membrane 43 may be disposed in the entire opening 52.

[Applications of Ethylene Glycol]

Ethylene glycol according to the present disclosure can be used as a solvent or antifreeze. Ethylene glycol according to the present disclosure, furthermore, can be used as a raw material for manufacturing an ethylene glycol compound derivative or as a raw material for manufacturing a polyester.
In the following, a polyester will be described as an example of an application of ethylene glycol according to the present disclosure.

[Polyester]

A polyester according to the present disclosure is one that is composed of a diol unit and a dicarboxylic acid unit and is obtained through polycondensation using ethylene glycol according to the present disclosure as a diol unit and a dicarboxylic acid as the dicarboxylic acid unit. That is, in the polyester according to the present disclosure, a diol unit is ethylene glycol made using at least one gas selected from the group consisting of carbon monoxide and carbon dioxide as a raw material.

<Diol Unit>

The ethylene glycol according to the present disclosure does not need to be the only diol unit used in the polyester. That is, ethylene glycol made using at least one gas selected from the group consisting of carbon monoxide and carbon dioxide as a raw material and other diols may be used in combination. This is because as long as ethylene glycol made using at least one gas selected from the group consisting of carbon monoxide and carbon dioxide as a raw material is used, the reduction of environmental impact can be sought, even if additional diols are used in combination with the ethylene glycol.
Examples of additional diols include fossil fuel-derived diols and biomass-derived diols.
For fossil fuel-derived diols, compounds having two or more, preferably two or more and eight or fewer, hydroxyl groups in one molecule can be used. Specifically, fossil fuel-derived diols that can be used are not particularly limited; types known in the related art can be used. For example, diols such as polypropylene glycol (PPG), neopentyl glycol (NPG), ethylene glycol (EG), diethylene glycol (DEG), butylene glycol (BG), hexamethylene glycol, triethylene glycol, dipropylene glycol, 1,4-cyclohexanedimethanol, 1,9-nonanediol, and 3-methyl-1,5-pentanediol can be used. Such diols may be used individually or as a combination of two or more.
For biomass-derived diols, aliphatic diols obtained from vegetable raw materials, such as corn, sugarcane, cassava, and sago palm, can be used. Examples of biomass-derived aliphatic diols include polypropylene glycol (PPG), neopentyl glycol (NPG), ethylene glycol (EG), diethylene glycol (DEG), butylene glycol (BG), and hexamethylene glycol obtained from vegetable raw materials by methods such as those described below, and all such diols can be used. Such diols may be used individually or as a combination.
Biomass-derived polypropylene glycol is manufactured by fermentation, in which glucose is obtained by decomposing a vegetable raw material, via glycerol and then 3-hydroxypropylaldehyde (HPA). Compared with polypropylene glycol obtained by an EO-based manufacturing method, polypropylene glycol manufactured by a bio-based method such as this fermentation method provides by-products that are useful from safety perspectives, such as lactic acid. This type of polypropylene glycol, furthermore, is also preferred because the manufacturing cost can be lowered as well.
Biomass-derived butylene glycol can be manufactured from biomass-derived succinic acid. Specifically, by producing glycol from a vegetable raw material and fermenting this glycol, biomass-derived succinic acid can be obtained.

By adding water to the biomass-derived succinic acid, biomass-derived butylene glycol can be manufactured.
Biomass-derived ethylene glycol can be manufactured via, for example, ethylene from bioethanol obtained by an ordinary method.

<Dicarboxylic Acid Unit>

The dicarboxylic acid unit used in the polyester according to the present disclosure is, for example, a fossil fuel-derived dicarboxylic acid. For fossil fuel-derived dicarboxylic acids, aromatic dicarboxylic acids, aliphatic dicarboxylic acids, and their derivatives can be used without restrictions.
Examples of aromatic dicarboxylic acids include terephthalic acid and isophthalic acid. Examples of derivatives of aromatic dicarboxylic acids include lower alkyl esters of aromatic dicarboxylic acids, specifically methyl esters, ethyl esters, propyl esters, and butyl esters. Of these, terephthalic acid is particularly preferred. For derivatives of aromatic dicarboxylic acids, dimethyl terephthalate is particularly preferred.
Specific examples of aliphatic dicarboxylic acids, furthermore, include chain or alicyclic dicarboxylic acids, typically containing 2 or more and 40 or fewer carbons, such as oxalic acid, succinic acid, glutaric acid, adipic acid, sebacic acid, dodecanedioic acid, dimer acid, and cyclohexanedicarboxylic acid. Moreover, examples of derivatives of aliphatic dicarboxylic acids include lower alkyl esters of the listed aliphatic dicarboxylic acids, such as methyl esters, ethyl esters, propyl esters, and butyl esters, and cyclic acid anhydrides of the listed aliphatic dicarboxylic acids, such as succinic anhydride. Of these, adipic acid, succinic acid, dimer acid, or a mixture thereof is particularly preferred, with a mixture containing succinic acid as its main component being especially preferred. For derivatives of aliphatic dicarboxylic acids, the methyl esters of adipic acid and succinic acid or a mixture thereof is more preferred.
The dicarboxylic acid used as the dicarboxylic acid unit, furthermore, may be a biomass-derived dicarboxylic acid.
Biomass-derived dicarboxylic acids that can be used include aliphatic dicarboxylic acids obtained from vegetable raw materials such as reproducible oils of vegetable origin, such as soybean oil, linseed oil, tung oil, coconut oil, palm oil, and castor oil, and recycled oils obtained by recycling, for example, waste cooking oils that are primarily such reproducible oils. Examples of biomass-derived aliphatic dicarboxylic acids include sebacic acid, succinic acid, phthalic acid, adipic acid, glutaric acid, and dimer acid. For example, sebacic acid is produced through alkali pyrolysis of ricinoleic acid obtained from castor oil, with heptyl alcohol as a by-product.
Biomass-derived aromatic dicarboxylic acids that can be used, furthermore, include biomass-derived terephthalic acid. Biomass-derived terephthalic acid can be manufactured by, for example, producing isobutanol from corn, saccharides, or wood, then converting the isobutanol into isobutylene, then dimerizing it to produce isooctene, then synthesizing p-xylene by the method described in Chemische Technik, vol. 38, No. 3, pp. 116-119; 1986, i.e., via radical ring opening, recombination, and cyclization, and then oxidizing it (International Publication No. 2009/079213).
When a biomass-derived dicarboxylic acid is used as the dicarboxylic acid unit, it is particularly preferred, in the present disclosure, to use biomass-derived terephthalic acid.
Moreover, the dicarboxylic acid used as the dicarboxylic acid unit may be a dicarboxylic acid made using carbon dioxide gas as a raw material.
Dicarboxylic acids made using carbon dioxide gas as a raw material that can be used include terephthalic acid made using carbon dioxide gas as a raw material. Terephthalic acid made using carbon dioxide gas as a raw material can be manufactured by, for example, producing p-xylene from carbon dioxide gas and hydrogen gas as raw materials using a composite catalyst containing chromium oxide and a predetermined type of H-ZSM-5 zeolite and then oxidizing the p-xylene (Japanese Unexamined Patent Application Publication No. 2019-205969).
Such dicarboxylic acids can be used individually or as a mixture of two or more.
The polyester may be a copolymeric polyester containing a copolymeric component added as a third component in addition to the diol unit and the dicarboxylic acid unit described above. Specific examples of copolymeric components include bifunctional oxycarboxylic acids and, for the formation of a crosslinked structure, at least one polyfunctional compound selected from the group consisting of trifunctional or higher-functionality polyhydric alcohols, trifunctional or higher-functionality polycarboxylic acids and/or their anhydrides, and trifunctional or higher-functionality oxycarboxylic acids. Among these copolymeric components, bifunctional and/or trifunctional or higher-functionality oxycarboxylic acid(s) is particularly suitable for use because with such acid(s), it tends to be easier to manufacture a copolymeric polyester with a high degree of polymerization. Of these, the use of a trifunctional or higher-functionality oxycarboxylic acid is the most preferred because with this acid, a polyester with a high degree of polymerization can be easily manufactured with an extremely small amount of it, without using a chain extender, which is described below.
The polyester described above, furthermore, may be a high-molecular-weight polyester obtained by subjecting such a copolymeric polyester to chain extension (coupling). The chain extender can be a chain extender such as a carbonate compound or a diisocyanate compound, but its amount is typically 10 mol% or less on a carbonate linkage or urethane linkage basis, preferably 5 mol% or less, more preferably 3 mol% or less, typically when the amount of all

monomer units constituting the polyester is defined as 100 mol%.

Specific examples of carbonate compounds include diphenyl carbonate, ditolyl carbonate, bis(chlorophenyl) carbonate, m-cresyl carbonate, dinaphthyl carbonate, dimethyl carbonate, diethyl carbonate, dibutyl carbonate, ethylene carbonate, diamyl carbonate, and dicyclohexyl carbonate. In addition to these, carbonate compounds derived from hydroxy compounds, such as phenols and alcohols, and composed of hydroxy compounds of the same type or different types can be used.

Specific examples of diisocyanate compounds include known diisocyanates, such as 2,4-tolylene diisocyanate, a mixture of 2,4-tolylene diisocyanate and 2,6-tolylene diisocyanate, diphenylmethane diisocyanate, 1,5-naphthylene diisocyanate, xylylene diisocyanate, hydrogenated xylylene diisocyanate, hexamethylene diisocyanate, and isophorone diisocyanate.

The polyester according to the present disclosure can be obtained by a method known in the related art in which the diol unit and the dicarboxylic acid unit described above are polycondensed. Specifically, it can be manufactured by a common method for melt polymerization, such as performing an esterification reaction and/or a transesterification reaction between the diol unit and the dicarboxylic acid unit described above and then performing polycondensation under reduced pressure, or a known method for dehydrative condensation in a heated solution in which an organic solvent is used.

The amount of the diol(s) used during the manufacture of the polyester is substantially equimolar to 100 moles of the dicarboxylic acid(s) or derivative(s) thereof. In general, however, the diol(s) is used in excess by 0.1 mol% or more and 20 mol% or less because distillation during the esterification and/or transesterification reaction(s) and/or the polycondensation reaction occurs.

The polycondensation, furthermore, is preferably performed in the presence of a polymerization catalyst. The time of addition of the polymerization catalyst is not particularly limited as long as it is before the polycondensation; it may be added in advance during the introduction of the raw materials or may be added at the start of pressure reduction.

Examples of polymerization catalysts generally include compounds containing a group-1 to group-14 metal element excluding hydrogen and carbon in the periodic table. Specific examples include compounds containing an organic group, such as carboxylates, alkoxides, organic sulfonates, or β-diketonates, and also containing at least one or more metals selected from the group consisting of titanium, zirconium, tin, antimony, cerium, germanium, zinc, cobalt, manganese, iron, aluminum, magnesium, calcium, strontium, sodium, and potassium, and even inorganic compounds of the listed metals, such as oxides and halides, and mixtures thereof. Of these, metal compounds containing titanium, zirconium, germanium, zinc, aluminum, magnesium, and calcium and mixtures thereof are particularly preferred, with titanium compounds, zirconium compounds, and germanium compounds being especially preferred. The catalyst, furthermore, is preferably a compound that is liquid or dissolves in the lower ester polymer or polyester during the polymerization for the reason that when it is in a molten or dissolved state during the polymerization, the polymerization speed is high.

For titanium compounds, tetraalkyl titanates are preferred. Specific examples include tetra-n-propyl titanate, tetraisopropyl titanate, tetra-n-butyl titanate, tetra-t-butyl titanate, tetraphenyl titanate, tetracyclohexyl titanate, tetrabenzyl titanate, and titanate mixtures thereof. Compounds such as titanium(oxy) acetylacetonate, titanium tetraacetylacetonate, titanium (diisopropoxide) acetylacetonate, titanium bis(ammonium lactate) dihydroxide, titanium bis(ethyl acetoacetate) diisopropoxide, titanium (triethanolaminate) isopropoxide, polyhydroxy titanium stearate, titanium lactate, titanium triethanolaminate, and the butyl titanate dimer, furthermore, are also suitable for use. Moreover, titanium oxide and composite oxides containing titanium and silicon are also suitable for use. Of these, tetra-n-propyl titanate, tetraisopropyl titanate, and tetra-n-butyl titanate, titanium(oxy) acetylacetonate, titanium tetraacetylacetonate, titanium bis(ammonium lactate) dihydroxide, polyhydroxy titanium stearate, titanium lactate, the butyl titanate dimer, titanium oxide, and titania/silica composite oxides (e.g., a product manufactured by Acordis Industrial Fibers GmbH under the name: C-94) are particularly preferred. In particular, tetra-n-propyl titanate, polyhydroxy titanium stearate, titanium(oxy) acetylacetonate, titanium tetraacetylacetonate, and titania/silica composite oxides (e.g., a product manufactured by Acordis Industrial Fibers GmbH under the name: C-94) are preferred.

Specific examples of zirconium compounds include zirconium tetraacetate, zirconium acetate hydroxide, zirconium tris(butoxy)stearate, zirconyl diacetate, zirconium oxalate, zirconyl oxalate, zirconium potassium oxalate, polyhydroxy zirconium stearate, zirconium ethoxide, zirconium tetra-n-propoxide, zirconium tetraisopropoxide, zirconium tetra-n-butoxide, zirconium tetra-t-butoxide, zirconium tributoxyacetylacetonate, and mixtures thereof. Furthermore, zirconium oxide or a composite oxide containing, for example, zirconium and silicon may also be used. Of these, zirconyl diacetate, zirconium tris(butoxy)stearate, zirconium tetraacetate, zirconium acetate hydroxide, zirconium ammonium oxalate, zirconium potassium oxalate, polyhydroxy zirconium stearate, zirconium tetra-n-propoxide, zirconium tetraisopropoxide, zirconium tetra-n-butoxide, and zirconium tetra-t-butoxide are particularly preferred.

For germanium compounds, specific examples include inorganic germanium compounds, such as germanium oxide and germanium chloride, and organic germanium compounds, such as tetraalkoxygermanium. For reasons such as price and availability, compounds such as germanium oxide, tetraethoxygermanium, and tetrabutoxygermanium are preferred. In particular, germanium oxide is preferred.

For the amount of catalyst used when a metal compound is used as such a polymerization catalyst, the lower limit is typically 5 ppm or more, preferably 10 ppm or more, as the amount of metal relative to the resulting polyester. The upper limit is typically 30000 ppm or less, preferably 1000 ppm or less, more preferably 250 ppm or less, particularly preferably 130 ppm or less. When the amount of catalyst used is too large, not only is it economically disadvantageous, but also the thermal stability of the polymer is low. Conversely, when the amount is too small, the polymerization activity is low, and the decomposition of the polymer is accordingly likely to be induced during the manufacture of the polymer. For the amount of catalyst used here, the quantity of terminal carboxyl groups in the resulting polyester is reduced with decreasing amount used. A method in which the amount of catalyst used is reduced, therefore, is a preferred configuration.

The reaction temperature for the esterification reaction and/or the transesterification reaction between the diol unit and the dicarboxylic acid unit typically falls within the range of 150°C to 260°C, inclusive, and the reaction atmosphere is typically in an atmosphere of an inert gas, such as nitrogen or argon. The reaction pressure, furthermore, is typically normal pressure or higher and 10 kPa or lower. Moreover, the reaction duration is typically 1 hour or longer and 10 hours or shorter.

In the manufacturing process described above, a chain extender (coupling agent) may be added to the reaction system. The chain extender is added to the reaction system after completion of the polycondensation in a uniform molten state without using a solvent, and is allowed to react with the polyester obtained through the polycondensation.

A high-molecular-weight polyester made using such a chain extender (coupling agent) can be manufactured using known technologies. The chain extender is added to the reaction system after completion of the polycondensation in a uniform molten state without using a solvent, and is allowed to react with the polyester obtained through the polycondensation. Specifically, by allowing a chain extender as described above to react with a polyester prepolymer that is obtained by subjecting diol(s) and dicarboxylic acid(s) to a catalytic reaction, whose terminal groups substantially have a hydroxy group, and that has a mass-average molecular weight (Mw) of 20,000 or more, preferably 40,000 or more, a polyester with a higher molecular weight can be obtained. A prepolymer having a mass-average molecular weight of 20,000 or more can be extended with a small amount of coupling agent and is not influenced by residual catalyst, even under harsh conditions such as a molten state. With such a prepolymer, therefore, a high-molecular-weight polyester can be manufactured without producing gel during the reaction.

[Polyester Pellets]

Polyester pellets according to the present disclosure are obtained by molding, by a known method, a polyester obtained as described above. For example, they are obtained by extracting the polyester from the polymerization reaction chamber in a strand form and pelletizing it using a stationary or rotary cutter or a pelletizer while or after cooling with water or air. Examples of shapes of the pellets after molding include a cylindrical shape having a circular or elliptical cross-section or a spherical shape.

After the polyester is pelletized, solid-phase polymerization may optionally be performed to further increase the degree of polymerization or to remove oligomers, such as cyclic trimers. Specifically, it is performed by pelletizing and drying the polyester, then heating the pellets at a temperature of 100°C or above and 180°C or below for 1 hour or longer and 8 hours or shorter to precrystallize the polyester, and subsequently heating the pellets for 1 hour or longer and several tens of hours or shorter under a stream of an inert gas or under reduced pressure at a temperature of 190°C or above and 230°C or below.

The size of the polyester pellets is adjusted through the adjustment of parameters such as the size of the opening for extraction from the polymerization reaction chamber, the speed of extraction of the strand, the speed of taking up, and the cutting speed. Specifically, it is adjusted by adjusting the pressure in the polymerization reaction chamber during the polymer extraction or by adjusting the cutting speed of a rotary strand cutter.

The size of the resulting polyester pellets is typically 0.1 mm or more, preferably 0.2 mm or more, more preferably 0.5 mm or more, the most preferably 1 mm or more for the lower limit (minimum size), and is 20 mm or less, preferably 10 mm or less, more preferably 7 mm or less, the most preferably 4 mm or less for the upper limit (maximum size). When the size is too small, degradation due to hydrolysis during the storage of the pellets tends to be significant. When the size is too large, furthermore, unevenness tends to occur in the product due to insufficient resin penetration during the molding.

It should be noted that the size of the polyester pellets in this context indicates the diameter of the cross-section or the length of the polyester pellets. The cross-section of the polyester pellets, furthermore, indicates the cross-section at which the cross-sectional area of the polyester pellets reaches a maximum.

In the polyester pellets according to the present disclosure, it is preferred that the percentage of powdery matter having a maximum size of less than 1 mm be 2.0% by weight or less, and it is more preferred that the percentage of powdery matter having a maximum size of less than 1 mm be 1.0% by weight or less. When the percentage of powdery matter having a maximum size of less than 1 mm is large, this powdery matter causes insufficient resin penetration into the screws of the molding machine during melt molding, resulting in an extended retention time in the molding machine. Due to a large surface area, furthermore, the powdery matter is likely to undergo thermal degradation. The degraded powder is mixed into

the molded article as contaminants, such as burnt resin and particulate defects, leading to problems such as reduced mechanical strength and inferior appearance of the molded article.

The intrinsic viscosity of the polyester pellets according to the present disclosure is 0.50 dL/g or more and 0.90 dL/g or less, preferably 0.60 dL/g or more and 0.85 dL/g or less, more preferably 0.65 dL/g or more and 0.80 dL/g or less. When the intrinsic viscosity is less than 0.50 dL/g, mechanical properties required for the molded article, film, fibers, or fiber product manufactured using the polyester pellets may be insufficient. When the intrinsic viscosity exceeds 0.90 dL/g, however, productivity in the process for manufacturing the raw materials, in the process for manufacturing the molded article, in the process for forming the film, in the process for manufacturing the fibers, and in the process for manufacturing the fiber article is impaired.

The preferred values of the intrinsic viscosity of the polyester pellets according to the present disclosure vary according to the intended use of the polyester pellets. For example, when the polyester pellets are used for a molded article, the intrinsic viscosity of the polyester pellets is preferably 0.70 or more, more preferably 0.75 or more, and preferably is 0.85 or less, more preferably 0.80 or less. When the polyester pellets are used for a film, the intrinsic viscosity of the polyester pellets is preferably 0.55 or more, more preferably 0.60 or more, and preferably is 0.70 or less, more preferably 0.65 or less.

The intrinsic viscosity of the polyester pellets according to the present disclosure is a value calculated from a melt viscosity measured at 35°C using a phenol/tetrachloroethane solvent (component ratio: 3/2).

By adjusting parameters such as the temperature for the polycondensation, the pressure during the polycondensation, the duration of the polycondensation, the temperature for the solid-state polymerization, the pressure during the solid-state polymerization, and the duration of the solid-state polymerization, polyester pellets having a desired intrinsic viscosity can be obtained.

The higher the temperature for the polycondensation, the more the intrinsic viscosity of the resulting polyester pellets tends to increase. The lower the temperature for the polycondensation, the more the intrinsic viscosity of the resulting polyester pellets tends to decrease.

The greater the pressure during the polycondensation, the more the intrinsic viscosity of the resulting polyester pellets tends to increase. The smaller the pressure during the polycondensation, the more the intrinsic viscosity of the resulting polyester pellets tends to decrease.

The longer the duration of the polycondensation, the more the intrinsic viscosity of the resulting polyester pellets tends to increase. The shorter the duration of the polycondensation, the more the intrinsic viscosity of the resulting polyester pellets tends to decrease.

The higher the temperature for the solid-state polymerization, the more the intrinsic viscosity of the resulting polyester pellets tends to increase. The lower the temperature for the solid-state polymerization, the more the intrinsic viscosity of the resulting polyester pellets tends to decrease.

The greater the pressure during the solid-state polymerization, the more the intrinsic viscosity of the resulting polyester pellets tends to increase. The smaller the pressure during the solid-state polymerization, the more the intrinsic viscosity of the resulting polyester pellets tends to decrease.

The longer the duration of the solid-state polymerization, the more the intrinsic viscosity of the resulting polyester pellets tends to increase. The shorter the duration of the solid-state polymerization, the more the intrinsic viscosity of the resulting polyester pellets tends to decrease.

For the polyester pellets according to the present disclosure, the amount of heat of fusion during the second heating run and the amount of heat of solidification during the second cooling run obtained by differential scanning calorimetry (DSC) according to JIS K7122: 2012 (Testing Methods for Heat of Transitions of Plastics) tend to be greater compared with those of known, fossil fuel-derived polyester pellets.

For example, the amount of heat of fusion of the polyester pellets according to the present disclosure during the second heating run obtained by DSC according to JIS K7122: 2012 is preferably 8 J/g or more, more preferably 11 J/g or more, even more preferably 13 J/g or more. The amount of heat of fusion of the polyester pellets according to the present disclosure during the second heating run obtained by DSC in JIS K7122: 2012, furthermore, is preferably 25 J/g or less, more preferably 20 J/g or less, even more preferably 15 J/g or less.

For example, the amount of heat of solidification of the polyester pellets according to the present disclosure during the second cooling run obtained by DSC according to JIS K7122: 2012 is 7 J/g or more, more preferably 9 J/g or more, even more preferably 12 J/g or more. The amount of heat of solidification of the polyester pellets according to the present disclosure during the second cooling run obtained by DSC according to JIS K7122: 2012, furthermore, is 25 J/g or less, more preferably 20 J/g or less, even more preferably 15 J/g or less.

For the specific measurement method in DSC, measurement is performed using a differential scanning calorimeter by the method described in the Examples section below.

In the polyester pellets according to the present disclosure, the amount of Kjeldahl nitrogen tends to be smaller compared with that in known, fossil fuel-derived polyester pellets. For example, the amount of Kjeldahl nitrogen in the polyester pellets according to the present disclosure is preferably 260 μg/g or less, more preferably 230 μg/g or less, even

more preferably 200 μg/g or less, still more preferably 90 μg/g or less, particularly preferably 80 μg/g or less, especially preferably 75 μg/g or less. The amount of Kjeldahl nitrogen in the polyester pellets according to the present disclosure, furthermore, is 0 μg/g or more. In practice, the amount of Kjeldahl nitrogen is 10 μg/g or more, more practically 30 μg/g or more, even more practically 100 μg/g or more, still more practically 150 μg/g or more.

In the present disclosure, Kjeldahl nitrogen is nitrogen quantified according to 44.1 (Kjeldahl method) and 44.2 (indophenol blue absorptiometry) of JIS K0102: 2019. For the specific method for measuring the amount of Kjeldahl nitrogen, measurement is performed by the method described in the Examples section below.

In the polyester pellets according to the present disclosure, the quantity of carbon dioxide molecules tends to be greater compared with that in known, fossil fuel-derived polyester pellets. For example, the quantity of carbon dioxide molecules in the polyester pellets according to the present disclosure is preferably $4.8 \times 10^{17}$ molecules/g or more, more preferably $7.0 \times 10^{17}$ molecules/g or more, even more preferably $1.0 \times 10^{18}$ molecules/g or more. The quantity of carbon dioxide molecules in the polyester pellets according to the present disclosure, furthermore, is preferably $3.0 \times 10^{18}$ molecules/g or less, more preferably $2.0 \times 10^{18}$ molecules/g or less, even more preferably $1.5 \times 10^{18}$ molecules/g or less.

The quantity of carbon dioxide molecules in the polyester pellets is measured by thermal desorption spectroscopy-mass spectrometry (TDS-MS). Specifically, measurement is performed by the method described in the Examples section below.

In the polyester pellets according to the present disclosure, furthermore, the quantity of water molecules tends to be greater compared with that in known, fossil fuel-derived polyester pellets. For example, the quantity of water molecules in the polyester pellets according to the present disclosure is preferably $1.8 \times 10^{19}$ molecules/g or more, more preferably $2.0 \times 10^{19}$ molecules/g or more, even more preferably $4.0 \times 10^{19}$ molecules/g or more. Moreover, the quantity of water molecules in the polyester pellets according to the present disclosure is preferably $1.0 \times 10^{20}$ molecules/g or less, more preferably $7.5 \times 10^{19}$ molecules/g or less, even more preferably $5.0 \times 10^{19}$ molecules/g or less.

The quantity of water molecules in the polyester pellets is measured by thermal desorption spectroscopy-mass spectrometry (TDS-MS). Specifically, measurement is performed by the method described in the Examples section below.

In the polyester pellets according to the present disclosure, furthermore, the quantity of hydrogen molecules tends to be smaller compared with that in known, fossil fuel-derived polyester pellets. For example, the quantity of hydrogen molecules in the polyester pellets according to the present disclosure is preferably $7.5 \times 10^{17}$ molecules/g or less, more preferably $7.0 \times 10^{17}$ molecules/g or less, even more preferably $6.6 \times 10^{17}$ molecules/g or less. Moreover, the quantity of hydrogen molecules in the polyester pellets according to the present disclosure is 0 molecules/g or more. In practice, the quantity of hydrogen molecules is $1.0 \times 10^{17}$ molecules/g, more practically $3.0 \times 10^{17}$ molecules/g or more.

The quantity of hydrogen molecules in the polyester pellets is measured by thermal desorption spectroscopy-mass spectrometry (TDS-MS). Specifically, measurement is performed by the method described in the Examples section below.

During the process for manufacturing the polyester pellets, or to the manufactured polyester pellets, various additives may be added, provided that the characteristics of the pellets are not impaired. For example, additives such as plasticizers, ultraviolet stabilizers, anti-discoloration agents, matting agents, deodorants, flame retardants, weathering agents, antistatic agents, yarn friction reducers, release agents, antioxidants, ion-exchange agents, and coloring pigments can be added. Such additives are added within the range of 5% to 50% by mass relative to the entire polyester pellets.

The polyester pellets according to the present disclosure can be, as described later, used for the manufacture of a molded article or a film. The polyester pellets according to the present disclosure, furthermore, can be used for the manufacture of fibers or a fiber product, as described later. The applications of the polyester pellets according to the present disclosure, however, are not limited to these; the pellets do not need to be used for the manufacture of fibers.

[Molded Article]

A molded article according to the present disclosure is one made using polyester pellets as described above. Examples of molded articles include molded articles such as compression-molded articles, injection-molded articles, blow-molded articles, and thermoformed articles. Specific examples of containers include a bottle, a vial, a cup, a tray, and a pack.

To process the polyester pellets into the molded article, known methods for processing polyester pellets into a molded article can be employed. In the following, an example of a method for processing the polyester pellets into a bottle will be described.

By melting polyester pellets as described above and injecting the melt using an injection molding machine, a preform 30X as illustrated in Fig. 10 is produced.

The preform 30X illustrated in Fig. 10 includes a mouth 31X, a body 32X connected to the mouth 31X, and a bottom 33X connected to the body 32X. Among these, the mouth 31X is a portion corresponding to the mouth 41X of a bottle 40X, which will be described later, and has substantially the same shape as the mouth 41X. The body 32X,

furthermore, is a portion corresponding to the neck 44X, the shoulder 48X, and the body 42X of the bottle 40X, and has a substantially cylindrical shape. The bottom 33X is a portion corresponding to the bottom 43X of the bottle 40X and has a substantially hemispherical shape.

In an embodiment, the mouth 31X includes a thread 34X corresponding to the thread 46X, over which a cap is screwed, of the bottle 40X, a bead 35X disposed beneath the thread 34X, and a support ring 36X disposed beneath the bead 35X and corresponding to the support ring 47X of the bottle 40X. The shape of the mouth 31X may be a shape known in the related art.

The thickness of the cross-section of the preform 30X according to the present disclosure at the body 32X is preferably 1.3 mm or more, more preferably 1.7 mm or more. The thickness of the cross-section of the preform 30X at the body 32X, however, is preferably 4.7 mm or less, more preferably 4.0 mm or less.

By setting the thickness of the cross-section of the preform 30X at the body 32X within these ranges, the molded article according to the present disclosure can be manufactured.

It should be noted that the thickness of the cross-section of the preform 30X at the body 32X represents the thickness of the cross-section of the preform 30X at the body 32X at a point at which the thickness of the cross-section is the smallest.

Then the preform is heated, and biaxial orientation blow molding is performed in a blow mold. As a result of this, a bottle as illustrated in Fig. 11 can be molded.

The bottle 40X illustrated in Fig. 11 includes a mouth 41X, a substantially cylindrical body 42X, and a bottom 43X. The body 42X is disposed beneath the mouth 41X to be continuous with the mouth 41X. The bottom 43X is disposed beneath the body 42X to be continuous with the body 42X. Between the mouth 41X and the body 42X, a neck 44X is positioned. Between the neck 44X and the body 42X, a shoulder 48X is positioned.

On the outer circumference of the mouth 41X, a thread 46X for screwing a cap, not illustrated, over it is disposed. In a portion beneath the thread 46X on the outer circumference of the mouth 41X, an annular support ring 47X protruding outward is disposed.

The body 42X has, as mentioned above, a substantially cylindrical shape. The body 42X has multiple horizontal grooves 45X created in it. The horizontal cross-section of the shoulder 48X has a substantially circular shape, and the area of the horizontal cross-section of the shoulder 48X gradually increases from the neck 44X toward the body 42X.

An outer portion in the radial direction of the bottom 43X has a heel 49X formed in it. The heel 49X is provided annularly and throughout in the circumferential direction of the bottom 43X. The heel 49X is a portion that is stretched to be the thinnest portion of the bottom 43X during blow molding.

The size of such a bottle 40X is not limited; the bottle 40X may be a bottle of any size. The overflow capacity of the bottle 40X may be, for example, 100 mL or more, may be 200 mL or more, or may be 300 mL or more. The overflow capacity of the bottle 40X may be, for example, 600 mL or less, may be 550 mL or less, or may be 530 mL or less.

The thickness of the bottom 43X at the heel 49X, furthermore, may be, for example, 0.07 mm or more, may be 0.08 mm or more, or may be 0.09 mm or more, although the thickness is not limited thereto. The thickness at the heel 49X may be, for example, 0.30 mm or less, may be 0.25 mm or less, or may be 0.20 mm or less. By setting the thickness at the heel 49X to 0.30 mm or less, weight reduction of the bottle 40X can be sought. By setting the thickness at the heel 49X to 0.07 mm or more, by contrast, permanent deformation of the bottom 43X due to bulging outward that can occur when the bottle 40X is housed in a vending machine or when the bottle 40X falls can be reduced.

When considered from the viewpoint of the strength of the bottle, it is preferred that the thickness of the cross-section of the bottle 40X at the body 42X be 0.05 mm or more, more preferably 0.10 mm or more.

When considered from the viewpoint of reducing the amount of polyester used, however, it is preferred that the thickness of the cross-section of the bottle 40X at the body 42X be 0.54 mm or less, more preferably 0.50 mm or less.

It should be noted that the thickness of the cross-section of the bottle 40X at the body 42X represents the thickness of the cross-section of the bottle 40X at the body 42X at a point at which the thickness of the cross-section is the smallest.

For a bottle that is an embodiment of the present disclosure, the ratio of its capacity to its mass (capacity/mass) is preferably 5 mL/g or more, more preferably 8 mL/g or more, for bottle moldability reasons.

The capacity/mass, however, is preferably 50 mL/g or less, more preferably 45 mL/g or less, for bottle strength reasons.

A bottle that is an embodiment of the present disclosure may have a single-layer structure or may have a multilayer structure, including two or more layers. When the bottle has a multilayer structure, furthermore, the layers may have the same composition or may have different compositions.

In an embodiment, the bottle may include a vapor deposition film on its surface. Thereby, the gas barrier properties of the bottle can be improved. It should be noted that the vapor deposition film may be positioned on the inner surface of the bottle or may be positioned on the outer surface, but preferably, it is positioned on the inner surface.

Examples of vapor deposition films include vapor deposition films that are composed of metals, such as aluminum, inorganic oxides, such as aluminum oxide, silicon oxide, magnesium oxide, calcium oxide, zirconium oxide,

titanium oxide, boron oxide, hafnium oxide, or barium oxide, organic silicon compounds, such as hexamethyldisiloxane, or that are hard carbon films, such as DLC (Diamond Like Carbon) films.

It should be noted that a hard carbon film that is a DLC film represents a hard carbon film that is also referred to as an i-carbon film or a hydrogenated amorphous carbon film (a-C:H), and is an amorphous carbon film in which $SP^3$ bonds are predominant.

The thickness of the vapor deposition film, furthermore, is not particularly limited. For example, it can be 1 nm or more and 150 nm or less.

The formation of the vapor deposition film can be performed using a method known in the related art. Examples include physical vapor deposition (PVD) methods, such as vacuum deposition, sputtering, and ion plating, and chemical vapor deposition (CVD) methods, such as plasma chemical vapor deposition, thermal chemical vapor deposition, and photochemical vapor deposition.

It should be noted that the thickness of the vapor deposition film can be measured at the body of the bottle, and represents the thickness of the cross-section of the vapor deposition film at a point at which the thickness of the cross-section is the smallest. When the vapor deposition film is multilayer, the thickness of the vapor deposition film is the total of the thicknesses of all vapor deposition films.

[Film]

A film according to the present disclosure is one made using polyester pellets as described above. To process the polyester pellets into the film, known methods for forming a film from polyester pellets can be employed. Specifically, polyester pellets as described above are dried and then fed into a hot-melt extruder heated to a temperature from a temperature equal to or higher than the melting point of the polyester pellets (Tm) to Tm + 70°C to melt the polyester pellets. The molten polyester pellets are extruded to form a sheet through a die, such as a T-die. The extruded sheet-shaped material is rapidly solidified, for example using a rotating cooling drum. In such a manner, a film 1X as illustrated in Fig. 12 can be formed.

The hot-melt extruder can be a single-screw extruder, a twin-screw extruder, a vented extruder, a tandem extruder, or another type of extruder according to the purpose.

It should be noted that in the present disclosure, film is a term encompassing all cases of a sheet, a film, a film-shaped material, and a sheet-shaped material; it is not a term that gives a special meaning to the referent.

The film according to the present disclosure has preferably been biaxially oriented. The biaxial orientation can be performed by methods known in the related art, and it may be sequential biaxial orientation or simultaneous biaxial orientation.

In the following, sequential biaxial orientation and simultaneous biaxial orientation will be described.

[Sequential Biaxial Orientation]

In sequential biaxial orientation, a film extruded onto a cooling drum as described above is stretched in the machine direction, and then stretching of the film in the transverse direction is performed.

The stretching in the machine direction is typically applied using a difference in circumferential speed between rollers, and may be performed in a single stage or may be performed in multiple stages using multiple pairs of rollers. The stretch ratio in the machine direction is preferably 2.0 or greater, more preferably 2.5 or greater, and preferably is 15.0 or less, more preferably 7.0 or less, even more preferably 5.0 or less, still more preferably 4.2 or less. Thereby, variations in optical characteristics, such as in-plane retardation, can be reduced.

The stretching temperature is preferably 50°C or above, more preferably 80°C or above, even more preferably 95°C or above, and preferably is 120°C or below, more preferably 115°C or below, even more preferably 110°C or below. Thereby, variations in optical characteristics, such as in-plane retardation, can be reduced.

The stretching in the transverse direction is typically performed using the tenter method, by stretching the film in the transverse direction while transporting it with both ends thereof held by clips. The stretch ratio in the transverse direction is preferably 2 or greater, more preferably 2.5 or greater, and preferably is 15 or less, more preferably 5 or less. Thereby, variations in optical characteristics, such as in-plane retardation, can be reduced.

The stretching temperature is preferably 50°C or above, more preferably 90°C or above, even more preferably 95°C or above, and preferably is 120°C or below, more preferably 110°C or below, even more preferably 105°C or below. Thereby, variations in optical characteristics, such as in-plane retardation, can be reduced.

The film that has undergone sequential biaxial orientation as described above is preferably subjected to heat treatment at or above the stretching temperature and at or below the melting point within the tenter in order to impart planarity and dimensional stability to the film. Specifically, it is preferred to perform heat setting, preferably at 120°C or above, more preferably 190°C or above, and preferably at 235°C or below, more preferably 225°C or below. From the viewpoint of reducing variations in optical characteristics, such as in-plane retardation, furthermore, it is preferred to

perform additional stretching by heat treatment by 1% or more and 10% or less during the first half of the heat treatment. Thereby, variations in optical characteristics, such as in-plane retardation, can be reduced.

After the heat treatment of the film, the film is slowly cooled to room temperature and then is rolled. Optionally, furthermore, relaxation treatment, for example, may be simultaneously performed during the heat treatment or slow cooling. The percentage relaxation during the heat treatment is preferably 0.5% or more, more preferably 0.8% or more, even more preferably 1% or more, and preferably is 5% or less, more preferably 3% or less, even more preferably 2.5% or less, still more preferably 2% or less. Thereby, variations in optical characteristics, such as in-plane retardation, can be reduced. The percentage relaxation during the slow cooling is preferably 0.5% or more, and preferably is 3% or less, more preferably 2% or less, even more preferably 1.5% or less, still more preferably 1.0% or less. Thereby, variations in optical characteristics, such as in-plane retardation, can be reduced. For planarity reasons, the temperature during the slow cooling is preferably 80°C or above, more preferably 90°C or above, even more preferably 100°C or above °C, and preferably is 150°C or below, more preferably 130°C or below, even more preferably 120°C or below.

[Simultaneous Biaxial Orientation]

In simultaneous biaxial orientation, a film extruded onto a cooling drum as described above is guided to a simultaneous biaxial tenter, in which the film is stretched in the machine direction and the transverse direction simultaneously and/or stepwise while being transported with both ends thereof held by clips. Examples of simultaneous biaxial orientation machines include a pantograph type, a screw type, a driving motor type, and a linear motor type, but a driving motor type or a linear motor type, in which the stretch ratios can be changed to desired values and relaxation treatment can be performed at any point, is preferred.

The ratio of simultaneous biaxial orientation is preferably 2 or greater, more preferably 3 or greater, even more preferably 6 or greater, still more preferably 10 or greater, and preferably is 50 or less, more preferably 30 or less, even more preferably 25 or less, still more preferably 20 or less, particularly preferably 15 or less, as a ratio by area. Thereby, variations in optical characteristics, such as in-plane retardation, can be reduced.

In the case of simultaneous biaxial orientation, furthermore, it is preferred to set the stretch ratios in the machine direction and in the transverse direction equal, and to make the stretching speeds in the two directions substantially equal as well, so that in-plane differences in orientation are reduced.

The stretching temperature in simultaneous biaxial orientation is preferably 50°C or above, more preferably 90°C or above, even more preferably 100°C or above, and preferably is 160°C or below, more preferably 150°C or below, even more preferably 140°C or below. Thereby, variations in optical characteristics, such as in-plane retardation, can be reduced.

The film that has undergone simultaneous biaxial orientation is preferably subsequently subjected to heat treatment at or above the stretching temperature and at or below the melting point in a heat-setting chamber within the tenter in order to impart planarity and dimensional stability to the film. The conditions for this heat treatment are the same as the conditions for the heat treatment after sequential biaxial orientation.

The thickness of the film according to the present disclosure can be freely selected according to the intended use. Typically, however, the thickness is 5 μm or more and 500 μm or less. The fracture strength of such a film is 1 kg/mm$^2$ or more and 40 kg/mm$^2$ or less in the MD direction, and 1 kg/mm$^2$ or more and 35 kg/mm$^2$ or less in the TD direction. The elongation at break, furthermore, is 10% or more and 350% or less in the MD direction, and 3% or more and 300% or less in the TD direction. As can be seen from these, the film according to the present disclosure has physical characteristics comparable to those of known polyester films manufactured from fossil fuel-derived materials or biomass-derived materials.

To the film according to the present disclosure, secondary processing may be applied for the purpose of imparting, for example, surface functions, such as chemical functions, electrical functions, magnetic functions, mechanical functions, frictional/wear/lubricating functions, optical functions, thermal functions, and biocompatibility. Examples of secondary processing include embossing, painting, bonding, printing, metallization (e.g., plating), machining, and surface treatments (e.g., antistatic treatment, corona discharge treatment, plasma treatment, photochromic treatment, physical vapor deposition, chemical vapor deposition, and coating).

The film according to the present disclosure is suitable for use in applications such as laminates, packages, various labeling materials, lids, sheet-shaped articles, and laminated tubes.

[Fibers]

Fibers according to the present disclosure are ones made using polyester pellets as described above. The fibers according to the present disclosure may be in any form, such as filaments or staples. In the case of filaments, the fibers may be monofilaments, each of which is a single filament, or may be multifilaments, each of which is composed of multiple filaments. In the case of staples, there are no limitations on the cut length and on the number of crimps as well.

The cross-sectional shape of the fibers according to the present disclosure is not limited to a circular cross-section; a wide variety of cross-sectional shapes, such as flat, Y-shaped, T-shaped, hollow shape, grid-shaped, and lattice-shaped, can be employed.

The fineness of the fibers can be set as appropriate according to the intended use. When the fibers are filaments for clothing, it is practically preferred that the fineness be 8 dtex or more and 150 dtex or less.

The strength, furthermore, is preferably 1.5 cN/dtex or more for use in clothing. Even with a strength of 1.5 cN/dtex or less, however, the fibers can be used without problems by taking measures such as using them in combination with a different type of fiber when producing the fabric.

The elongation can be set as appropriate according to the intended use. Preferably, the elongation is 25% or more and 60% or less for processability when the fibers are processed into fabric. When post-processing is applied, it is suitable to set the elongation to 60% or more and 250% or less.

To process the polyester pellets into the fibers, known methods for processing polyester pellets into fibers can be employed. In the following, melt spinning, which is an example of a method for processing polyester pellets into fibers, will be described.

The raw-material polyester pellets are melted, and the melt is metered and transported using a gear pump and discharged through a spinneret. The resulting yarn is cooled to room temperature by blowing quench air onto them using a yarn-cooling device, such as a chimney, oiled using an oiling device, and collected, and the resulting bundle is entangled using a fluid-entangling nozzle device. The entangled bundle passes through take-up rollers and drawing rollers, during which the yarn is drawn according to the ratio of circumferential speed between the take-up rollers and the drawing rollers. In this manner, the polyester pellets can be processed into fibers.

In addition, the yarn may be heat-set using the drawing rollers and wound using a winder (winding apparatus).

Alternatively, undrawn yarn may be produced once by setting the circumferential speeds of the take-up rollers and the drawing rollers to equal speeds and then winding the bundle using a winder at the same speed, and drawing may be performed in a separate step.

The fibers can be subjected to post-processing, such as false twisting or twisting. For weaving and knitting as well, the fibers can be handled in the same manner as general fibers.

[Fiber Article]

Fibers according to the present disclosure can be made into a fiber article in accordance with known methods. Examples of fiber articles that can be made using fibers according to the present disclosure include gauze, filters, carpets, automotive interior materials, clothing, woven fabrics, knitted fabrics, pile fabrics, nonwoven fabrics, spun yarns, and stuffing.

The fiber article according to the present disclosure, furthermore, may have any weave structure or knit structure. Structures such as plain weave, twill weave, satin weave, and variations thereof, and warp knitting, weft knitting, circular knitting, lace knitting, and variations thereof are suitable for employment.

[Molded Article]

A molded article according to the present disclosure is an article molded from a polyester as described above. Examples of molded articles include molded articles such as compression-molded articles, injection-molded articles, blow-molded articles, and thermoformed articles. Molded articles also include containers for housing things therein. Specific examples of containers include a bottle, a vial, a cup, a tray, and a pack.

Examples of molded articles other than containers include pellets, a film, a sheet, chopsticks, and cutlery. Specific examples of cutlery include a knife, a fork, and a spoon.

To process the polyester into the molded article, known methods for processing a polyester into a molded article can be employed.

In the molded article according to the present disclosure, the amount of Kjeldahl nitrogen tends to be smaller compared with that in articles molded from known, fossil fuel-derived polyesters. For example, the amount of Kjeldahl nitrogen in the molded article according to the present disclosure is preferably 18 $\mu$g/g or less, more preferably 16 $\mu$g/g or less, even more preferably 15 $\mu$g/g or less. The amount of Kjeldahl nitrogen in the molded article according to the present disclosure, furthermore, is 0 $\mu$g/g or more. In practice, the amount of Kjeldahl nitrogen is 1 $\mu$g/g or more, more practically 3 $\mu$g/g or more.

In the present disclosure, Kjeldahl nitrogen is nitrogen quantified according to 44.1 (Kjeldahl method) and 44.2 (indophenol blue absorptiometry) of JIS K0102: 2019. For the specific method for measuring the amount of Kjeldahl nitrogen, measurement is performed by the method described in the Examples section below.

In the molded article according to the present disclosure, the quantity of carbon dioxide molecules tends to be greater compared with that in articles molded from known, fossil fuel-derived polyesters. For example, the quantity of

carbon dioxide molecules in the molded article according to the present disclosure is preferably $9.2 \times 10^{17}$ molecules/g or more, more preferably $9.4 \times 10^{17}$ molecules/g or more, even more preferably $9.6 \times 10^{17}$ molecules/g or more. The quantity of carbon dioxide molecules in the molded article according to the present disclosure, furthermore, is preferably $3.0 \times 10^{18}$ molecules/g or less, more preferably $2.7 \times 10^{18}$ molecules/g or less, even more preferably $2.5 \times 10^{18}$ molecules/g or less.

The quantity of carbon dioxide molecules in the polyester resin layer is measured by thermal desorption spectroscopy-mass spectrometry (TDS-MS). Specifically, measurement is performed by the method described in the Examples section below.

[Container]

An example of a container that is an embodiment of a molded article according to the present disclosure is the bottle 40X illustrated in Fig. 11. Fig. 11 is a front view illustrating an embodiment of a molded article according to the present disclosure. The details of the bottle 40X are the same as described above, and thus the description thereof is omitted in this section.

[Preform]

Herein, a preform is a preliminary molded article before blow molding into a container.

A preform according to the present disclosure is one used for the manufacture of a container that is an embodiment of the present disclosure. The preform according to the present disclosure, therefore, is composed of a polyester in which ethylene glycol made using at least one gas selected from the group consisting of carbon monoxide and carbon dioxide as a raw material is used as a diol unit, and in which a dicarboxylic acid is used as a dicarboxylic acid unit. By configuring the preform in such a manner, the molded article according to the present disclosure can be manufactured.

It should be noted that polyesters and other materials that can be used for the preform according to the present disclosure are the same as in the molded article according to the present disclosure.

An example of a preform according to the present disclosure is the preform 30X illustrated in Fig. 10. Fig. 10 is a schematic half-sectional view illustrating an embodiment of a preform 30X according to the present disclosure. The details of the preform 30X are the same as described above, and thus the description thereof is omitted in this section.

[Spoon]

In the following, the structure of a spoon that is an embodiment of a molded article according to the present disclosure will be described by presenting Fig. 13 by way of example.

The spoon 50X includes a measuring section 51X and a handle 52X. The measuring section 51X and the handle 52X have both been integrally formed by injection molding of the polyester. The tip of the measuring section 51X is rounded. The width of the handle 52X has been formed to become greater toward the end. The measuring section 51X has a measuring space 53X formed to open upward.

The thicknesses of the measuring section 51X and the handle 52X may be the same or may be different. As an example, the thickness of the measuring section 51X is set to 1.2 mm, and the thickness of the handle 52X is set to 1.4 mm. The thicknesses of the measuring section 51X and the handle 52X can be designed as appropriate, for example according to the weight of the material for measurement that is to be measured using the spoon 50X. The dimensions of the measuring section 51X and the dimensions of the handle 52X can also be designed as appropriate.

[Polyester Film]

A polyester film according to the present disclosure is a film containing a polyester according to the present disclosure. That is, it is a film containing a polyester made using ethylene glycol made using at least one gas selected from the group consisting of carbon monoxide and carbon dioxide as a raw material (Hereinafter also referred to as "gas-derived polyester.").

For the reduction of environmental impact, the amount of the gas-derived polyester in the polyester film is preferably 50% by mass or more, preferably 80% by mass or more, preferably 95% by mass or more. The amount of the gas-derived polyester in the polyester film, furthermore, is 100% by mass or less. In practice, the amount is 99% by mass or less, more practically 97% by mass or less.

Since the polyester film according to the present disclosure contains a gas-derived polyester, the quantity of carbon dioxide molecules in the polyester film tends to be greater than that in films made using known, fossil fuel-derived polyesters. For example, the quantity of carbon dioxide molecules in the polyester film according to the present disclosure is preferably $1.1 \times 10^{18}$ molecules/g or more, more preferably $1.5 \times 10^{18}$ molecules/g or more, even more preferably $2.0 \times$

$10^{18}$ molecules/g or more. The quantity of carbon dioxide molecules in the polyester film according to the present disclosure, furthermore, is preferably $3.0 \times 10^{18}$ molecules/g or less, more preferably $2.7 \times 10^{18}$ molecules/g or less, even more preferably $2.4 \times 10^{18}$ molecules/g or less.

The quantity of carbon dioxide molecules in the polyester film is measured by thermal desorption spectroscopy-mass spectrometry (TDS-MS). Specifically, measurement is performed by the method described in the Examples section below.

The quantity of water molecules in the polyester film according to the present disclosure, furthermore, tends to be greater than that in films made using known, fossil fuel-derived polyesters. For example, the quantity of water molecules in the polyester film according to the present disclosure is preferably $8.6 \times 10^{19}$ molecules/g or more, more preferably $9.0 \times 10^{19}$ molecules/g or more, even more preferably $1.0 \times 10^{20}$ molecules/g or more. The quantity of water molecules in the polyester film according to the present disclosure, furthermore, is preferably $2.5 \times 10^{20}$ molecules/g or less, more preferably $2.0 \times 10^{20}$ molecules/g or less, even more preferably $1.5 \times 10^{20}$ molecules/g or less.

The quantity of water molecules in the polyester film is measured by thermal desorption spectroscopy-mass spectrometry (TDS-MS). Specifically, measurement is performed by the method described in the Examples section below.

The polyester film may contain various additives, provided that the features of the present disclosure are not impaired. Examples of additives include plasticizers, ultraviolet stabilizers, anti-discoloration agents, matting agents, deodorants, flame retardants, weathering agents, antistatic agents, yarn friction reducers, release agents, antioxidants, ion-exchange agents, and coloring pigments. The amount of such additives is, for example, 5% by mass or more and 50% by mass or less.

To process the polyester into the form of a film and obtain the polyester film according to the present disclosure, known methods for forming a film from a polyester can be adopted. Specifically, pellets of the gas-derived polyester are dried and then fed into a hot-melt extruder heated to a temperature equal to or higher than the melting point (Tm) of the pellets and equal to or lower than Tm + 70°C to melt the pellets. The molten pellets are extruded to form a sheet through a die, such as a T-die. By rapidly solidifying the extruded sheet-shaped material, for example using a rotating cooling drum, a polyester film 10Y as illustrated in Fig. 14 can be formed. A polyester film 10Y obtained in such a manner is composed of a single layer.

The hot-melt extruder can be a single-screw extruder, a twin-screw extruder, a vented extruder, a tandem extruder, or another type of extruder according to the purpose.

The polyester film according to the present disclosure has preferably been biaxially oriented. The biaxial orientation can be performed by methods known in the related art, and it may be sequential biaxial orientation or simultaneous biaxial orientation.

The details of the sequential biaxial orientation and the simultaneous biaxial orientation are the same as described above, and thus the description thereof is omitted in this section.

The thickness of the polyester film can be freely selected according to the intended use. Typically, however, the thickness is 5 μm or more and 500 μm or less. The fracture strength of such a film is 1 kg/mm$^2$ or more and 40 kg/mm$^2$ or less in the MD direction, and 1 kg/mm$^2$ or more and 35 kg/mm$^2$ or less in the TD direction. The elongation at break, furthermore, is 10% or more and 350% or less in the MD direction, and 3% or more and 300% or less in the TD direction. As can be seen from these, the polyester film according to the present disclosure has physical characteristics comparable to those of known polyester films manufactured from fossil fuel-derived materials or biomass-derived materials.

Although the polyester film 10Y illustrated in Fig. 14 is composed of a single layer, the polyester film according to the present disclosure is not limited to a single-layer configuration; it may be in a multilayer configuration. When the polyester film according to the present disclosure is in a multilayer configuration, it suffices that at least one layer contains the gas-derived polyester; the other layers may be free of the gas-derived polyester. An example of polyester(s) that can constitute the layers free of the gas-derived polyester is at least one selected from the group consisting of a fossil fuel-derived polyester, a biomass-derived polyester, and a recycled polyester. It should be noted that recycled polyester refers to a polyester recycled from collected post-consumer containers or other products that have been placed on the market.

To the polyester film according to the present disclosure, secondary processing may be applied for the purpose of imparting, for example, surface functions, such as chemical functions, electrical functions, magnetic functions, mechanical functions, frictional/wear/lubricating functions, optical functions, thermal functions, and biocompatibility. Examples of secondary processing include embossing, painting, bonding, printing, metallization (e.g., plating), machining, and surface treatments (e.g., antistatic treatment, corona discharge treatment, plasma treatment, photochromic treatment, physical vapor deposition, chemical vapor deposition, and coating).

The polyester film according to the present disclosure is suitable for use in applications such as laminates, packages, various labeling materials, lids, sheet-shaped articles, and laminated tubes. In the following, a laminate, a package, a stand-up pouch, a lid, a tube container, a paper container for liquids, and a paper cup will be described as examples of applications of the polyester film according to the present disclosure.

[Laminate]

A laminate according to the present disclosure includes a polyester film according to the present disclosure. For example, the laminate according to the present disclosure is one that includes a polyester film according to the present disclosure and at least one sealant layer provided on any one or both surfaces of the polyester film according to the present disclosure. For example, the laminate according to the present disclosure includes at least a polyester film and at least one sealant layer. A cross-sectional view briefly illustrating an example of a structure of a laminate according to the present disclosure is presented in Fig. 15. As illustrated in Fig. 15, the laminate 20A includes a polyester film 10Y and a sealant layer 21Y and is constructed by laminating the sealant layer 21Y above the polyester film 10Y. It should be noted that although the laminate 20A in the embodiment illustrated in Fig. 15 is in a configuration in which the sealant layer 21Y is laminated above a surface of the polyester film 10Y, the laminate 20A may be in a configuration in which the sealant layer 21Y is laminated beneath a surface of the polyester film 10Y, or may be in a configuration in which sealant layers 21Y are laminated on both above and beneath the surfaces of the polyester film 10Y.

The laminate 20A, furthermore, may have at least one additional layer, such as a barrier layer, a support, a resin increase, a printed layer, an adhesive agent layer, or an adhesive resin layer, above and/or beneath surface(s) of the polyester film 10Y in addition to the sealant layer 21Y. The additional layers will be described later.

[Sealant Layer]

The sealant layer 21Y is provided, for example, above the polyester film 10Y in the laminate 20A as illustrated in Fig. 15. The sealant layer 21Y is a layer formed of a film of a heat-sealable resin, which can undergo mutual fusion in response to heat.

The material that forms the sealant layer 21Y is not particularly limited, as long as it is a resin that undergoes mutual fusion in response to heat. Examples include resins such as polyolefin resins, such as low-density polyethylene (LDPE), medium-density polyethylene (MDPE), high-density polyethylene (HDPE), linear-chain (linear) low-density polyethylene (LLDPE), ethylene-$\alpha$-olefin copolymers polymerized using a metallocene catalyst, ethylene-polypropylene random copolymers (random polypropylene), ethylene-polypropylene block copolymers (block polypropylene), polypropylene, ethylene-vinyl acetate copolymers (EVA), ethylene-acrylic acid copolymers (EAA), ethylene-ethyl acrylate copolymers (EEA), ethylene-methacrylic acid copolymers (EMAA), ethylene-methyl methacrylate copolymers (EMMA), ionomer resins, heat-sealable ethylene-vinyl alcohol resins, ethylene-propylene copolymers, methylpentene polymers, polybutene polymers, or cyclic olefin copolymers, acid-modified polyolefin resins resulting from modifying polyolefin resins with acrylic acid, methacrylic acid, maleic acid, maleic anhydride, fumaric acid, itaconic acid, or other unsaturated carboxylic acids, polyvinyl acetate resins, poly(meth)acrylic resins, and polyvinyl chloride resins. Of these, low-density polyethylene, linear low-density polyethylene, or polypropylene is particularly preferred.

Such resins may be used individually or as a mixture of two or more. The sealant layer 21Y can be used as a film or sheet of such resin(s) or as, for example, a coating film of such resin(s).

When polyethylene is used as the material that forms the sealant layer 21Y, polyethylene resulting from polymerizing not only ethylene obtained from a fossil fuel but also biomass-derived ethylene as a raw material may be used. The biomass-derived ethylene can be, for example, that described in Japanese Unexamined Patent Application Publication No. 2012-251006. By using polyethylene obtained by polymerizing biomass-derived ethylene as the material constituting the sealant layer 21Y, the sealant layer can be formed as a layer composed of a carbon-neutral material. By using it in combination with a gas-derived polyester as described above, therefore, the amount of fossil fuel used can be more significantly reduced, whereby environmental impact can be further decreased.

The polyethylene resulting from polymerizing biomass-derived ethylene can be a commercially available one. For example, "C4LL-LL118 (d = 0.916, MFR = 1.0 g/10 minutes)" sugarcane-derived linear low-density polyethylene resin, manufactured by Braskem S.A., can be used.

It should be noted that although the laminate 20A illustrated in Fig. 15 has one sealant layer 21Y, two or more sealant layers 21Y may be provided. When the laminate has two or more sealant layers 21Y, the individual layers may have the same composition or may have different compositions.

The thickness of the sealant layer 21Y is preferably 20 $\mu$m or more, more preferably 30 $\mu$m or more, and preferably is 200 $\mu$m or less, more preferably 130 $\mu$m or less.

Examples of methods for laminating a sealant layer on one surface of the laminate (e.g., the inner surface when the laminate is made into a packaging bag) include dry lamination and melt extrusion lamination. When this lamination is performed, furthermore, pretreatments, such as corona treatment, ozonation, and flaming, can optionally be applied to the film. Of the listed methods, dry lamination is superior in bonding strength and is a more preferred method.

Although the laminate 20A illustrated in Fig. 15 is in a configuration including a polyester film 10Y and a sealant layer 21Y, furthermore, the configuration is not limited to this; as long as these layers are included, the laminate 20A may have at least one additional layer in addition to the polyester film 10Y and the sealant layer 21Y. For example, the laminate

20A may include another layer according to the intended use of the laminate, for example between the polyester film 10Y and the sealant layer 21Y, on the surface of the sealant layer 21Y opposite the surface where the polyester film 10Y is laminated, or on the surface of the polyester film 10Y opposite the surface where the sealant layer 21Y is laminated.

Examples of additional layers include a barrier layer, a support, a resin layer, a printed layer, an adhesive agent layer, and an adhesive resin layer. When the laminate has two or more additional layers, the individual layers may have the same composition or may have different compositions. Such additional layers can be laminated together, with a bonding layer interposed therebetween by dry lamination or with an adhesive resin layer interposed therebetween by melt extrusion lamination.

[Barrier Layer]

The laminate preferably further includes at least one barrier layer. The position at which the barrier layer is included is not particularly limited, as long as the advantages of the present disclosure are provided. Preferably, however, the barrier layer is included between the polyester film and the sealant layer.

The barrier layer functions as a layer having gas barrier properties, which prevent penetration of gases, such as oxygen gas, or water vapor barrier properties, which prevent penetration of, for example, water vapor. The barrier layer can be, for example, a layer formed of a metal foil obtained by rolling a metal, such as an aluminum foil, a layer formed of a vapor deposition film of a metal, such as aluminum, a layer formed of a vapor deposition film of an inorganic oxide, such as alumina, a layer formed of a gas barrier coating film, or a saponified form of an ethylene-vinyl acetate copolymer (EVOH). When the barrier layer is formed as a layer formed of a vapor deposition film of an inorganic oxide, such as alumina, furthermore, at least one layer formed of a gas barrier coating film as mentioned above may be provided on at least any one surface of the layer formed of a vapor deposition film of an inorganic oxide to impart or improve gas barrier properties. The barrier layer can be formed by methods known in the related art, and its composition and formation method are not particularly limited. A metal foil or vapor deposition film constituting the barrier layer can be, for example, one as described in Japanese Unexamined Patent Application Publication No. 2012-96469. A gas barrier coating film is a film that contains one or more alkoxides and a polyvinyl alcohol resin and/or an ethylene-vinyl alcohol copolymer and that is obtained through polycondensation by the sol-gel method. For example, a gas barrier coating film as described in Japanese Unexamined Patent Application Publication No. 2012-96469 can be used. An EVOH constituting the barrier layer can be, for example, one as described in Japanese Unexamined Patent Application Publication No. 2008-307847. It should be noted that two or more barrier layers may be provided. When the laminate has two or more barrier layers, the individual layers may have the same composition or may have different compositions. One or more barrier layers, furthermore, may be provided on each of the two surfaces of the polyester film 10Y.

[Support]

A support is not particularly limited and can be formed using a known material, as long as it is a material that can support the laminate and improve the strength characteristics and impact resistance, for example, of the laminate, such as a stretched polyester resin layer, a stretched polyamide resin layer, a stretched polyolefin resin layer, or a paper layer (paper base material). A biomass-derived material, furthermore, may be used as a support. Moreover, a support can be one such layer used alone or a combination of two or more such layers.

A stretched polyester resin layer that can be used as a support can be made using a biomass-derived polyester or a gas-derived polyester, as well as a fossil fuel-derived polyester known in the related art. The stretched polyester resin layer, furthermore, may be a layer resulting from mixing a resin material containing a known, fossil fuel-derived raw material and a resin material containing a biomass-derived raw material.

It should be noted that to improve bonding, a component including a dicarboxylic acid containing a sulfonic acid group may be used as a dicarboxylic acid component. Examples of dicarboxylic acids containing a sulfonic acid group include dicarboxylic acids containing a sulfonic acid metal salt. Examples of dicarboxylic acids containing a sulfonic acid metal salt include metal salts (e.g., alkali metal salts or alkaline earth metal salts) of acids such as sulfoterephthalic acid, 5-sulfoisophthalic acid, 4-sulfophthalic acid, 4-sulfonaphthalene-2,7-dicarboxylic acid, and 5-[4-sulfophenoxy]isophthalic acid. In particular, when considered from the viewpoint of achieving good bonding and resistance to deformation, it is preferred to use sodium sulfoterephthalate or 5-sodium sulfoisophthalate. In a sulfone-containing polyester, the proportion of the dicarboxylic acid containing a sulfonic acid group in copolymerization is preferably 0.5 mol% or more and 10 mol% or less relative to all acid components. The limiting viscosity thereof, furthermore, is preferably 0.3 dL/g or more and 0.8 dL/g or less.

According to the intended use of a package including the laminate according to the present disclosure, the stretched polyester resin layer can be made using various polyester resins, and a mixture of two or more resins may also be used. For example, when a polyester is used, a support having excellent thermal dimensional stability, aroma retention, and heat resistance is obtained. When a sulfone-containing polyester is used, furthermore, a coextruded film having high

interlayer bonding strength is obtained.

In the stretched polyester resin layer, various additives, such as a slipping agent, may optionally be contained.

A stretched polyamide resin layer that can be used as a support, furthermore, may be made primarily using at least one aliphatic polyamide, but may contain other polyamide components, such as an aromatic polyamide. The aliphatic polyamide can be, for example, an aliphatic polyamide obtained through polycondensation between an aliphatic, alicyclic, or other diamine, such as hexamethylenediamine, decamethylenediamine, dodecamethylenediamine, 2,2,4- or 2,4,4-trimethylhexamethylenediamine, 1,3- or 1,4-bis(aminomethyl)cyclohexane, or bis(p-aminocyclohexylmethane), and a dicarboxylic acid or a derivative thereof, such as adipic acid, suberic acid, sebacic acid, cyclohexanedicarboxylic acid, terephthalic acid, or isophthalic acid, a polyamide resin obtained through condensation of, for example, ε-aminocaproic acid or 11-aminoundecanoic acid, a polyamide resin obtained from a lactam compound, such as ε-caprolactam or ω-laurolactam, or a mixture thereof. Specifically, aliphatic polyamide resins such as nylon 6, nylon 6,6, nylon 9, nylon 11, nylon 12, nylon 6/66, nylon 66/610, and nylon MXD6 can be used. Of these, examples of particularly suitable aliphatic polyamides include nylon 6, nylon 6,6, and nylon-6/6,6. Examples of two or more aliphatic polyamides include combinations of nylon 6 and nylon 6/6,6 at any proportions.

Moreover, a stretched polyolefin resin layer that can be used as a support can be made using, for example, low-density polyethylene (LDPE), medium-density polyethylene (MDPE), high-density polyethylene (HDPE), linear-chain (linear) low-density polyethylene (LLDPE), polypropylene (PP), an ethylene-α-olefin copolymer polymerized using a metallocene catalyst, or an ethylene-polypropylene random or block copolymer. Of these, it is particularly preferred that the layer be a stretched polypropylene resin layer, made using polypropylene.

When a stretched polyester resin layer or a stretched polyamide resin layer is formed as a support, a modified polyolefin resin layer, which is formed of a modified polyolefin, may be provided between the support and the sealant layer 21Y to improve bonding between the support and the sealant layer 21Y. A modified polyolefin is a polyolefin resin modified by a method such as replacing part of the polyolefin serving as the main component with another substance (monomer), for example through copolymerization or co-condensation, or allowing an appropriate substance (monomer) to locally react. Examples include acid-modified polyolefin resins resulting from modifying a polyolefin resin, such as low-density polyethylene (LDPE), medium-density polyethylene (MDPE), high-density polyethylene (HDPE), linear-chain (linear) low-density polyethylene (LLDPE), an ethylene-α-olefin copolymer polymerized using a metallocene catalyst, polypropylene (PP), an ethylene-vinyl acetate copolymer (EVA), an ionomer resin, an ethylene-acrylic acid copolymer (EAA), an ethylene-ethyl acrylate copolymer (EEA), an ethylene-methacrylic acid copolymer (EMAA), an ethylene-propylene copolymer, a methylpentene polymer, polyethylene, a polyethylene resin, or a polypropylene resin, with acrylic acid, methacrylic acid, maleic anhydride, fumaric acid, or other unsaturated carboxylic acids. It should be noted that, needless to say, the aforementioned polyethylene resin can be one in which biomass-derived ethylene as mentioned above is used as a monomer unit.

Examples of preferred modified polyolefins include copolymers having a structure in which a polyolefin segment and a non-olefin segment having polarity are bonded in a block, graft, and/or random manner, such as copolymers of a propylene-based polyolefin segment and a segment containing lactic acid as a constituent, copolymers of an ethylene-based polyolefin segment and a segment containing an acrylic acid unit as a constituent, and copolymers of a propylene-based polyolefin segment and a segment containing an acrylic acid unit as a constituent. Specifically, as maleic anhydride-modified polyolefin resins, ADMER SE800, SF740, SF731, and SF730, manufactured by Mitsui Chemicals, Inc., can be used.

Biomass-derived materials that may be used as constituents of a support, furthermore, also include commercially available polylactic acid films, in addition to the materials mentioned above. For example, polylactic acid films available from Mitsui Chemicals Tohcello, Inc. are suitable for use.

Moreover, a paper layer (paper base material) that can be used as a support can be any type of paper selected according to, for example, the desired rigidity. For example, known types of paper, such as woodfree paper, imitation Japanese vellum, art paper, coated paper, pure-white machine-glazed paper, kraft paper, labeling paper with enhanced waterproofness, base paper for drinking cups, paperboards, such as card stock, ivory board, and manila-lined board, base paper for milk cartons, base paper for cups in general, synthetic paper, and clay-coated paper, can be used.

The thickness of a polyester-based resin layer as described above, for example, when used as a support may be adjusted as appropriate according to the application in which the laminate is used. For example, when the laminate is used as a packaging material for an application in which rigidity is required, the support can be a relatively thick one.

By forming the support using a resin material containing a biomass-derived raw material, furthermore, the support is formed as a layer of a carbon-neutral resin. As a result of the laminate being formed by the polyester film 10Y and a support that is a layer formed of a carbon-neutral resin, a laminate having two or more layers containing a carbon-neutral resin can be manufactured. Thereby, the amount of fossil fuel used for forming the support can be significantly reduced, whereby environmental impact can be decreased.

The method for forming the support can be a method known in the related art and is not particularly limited. The support may be formed using extrusion lamination from one or more of the materials described above, or may be laminated

onto a printed layer, for example using dry lamination, as a film formed in advance using the T-die method, inflation, or other techniques.

[Printed Layer]

A printed layer can optionally be provided, and can be provided between, for example, the polyester film 10Y and a support. A printed layer is a layer that forms any print design, such as characters, pictures, figures, symbols, and/or patterns, is formed for decoration, indicating the contents, indicating the best-before date, or indicating information such as the manufacturer and the distributor. The printed layer may be provided over the entire surface or may be provided in part.

The printed layer can be formed using pigments and dyes known in the related art. It is possible to use an ink composition obtained by using one or two or more common ink vehicles as its main component(s), optionally adding one or two or more additives, such as a plasticizer, a stabilizer, an antioxidant, a photostabilizer, an ultraviolet absorber, a curing agent, a crosslinking agent, a slipping agent, an antistatic agent, and/or a filler, as needed, then adding a coloring agent, such as a dye or a pigment, and sufficiently kneading the mixture, for example with a solvent or a diluent.

The ink vehicles can be one or two or more of known vehicles, such as linseed oil, tung oil, soybean oil, hydrocarbon oils, rosin, rosin esters, rosin-modified resins, shellac, alkyd resins, phenolic resins, maleic acid resins, natural resins, hydrocarbon resins, polyvinyl chloride resins, polyacetate resins, polystyrene resins, polyvinyl butyral resins, acrylic or methacrylic resins, polyamide resins, polyester resins, polyurethane resins, epoxy resins, urea resins, melamine resins, amino-alkyd resins, nitrocellulose, ethyl cellulose, chlorinated rubber, and cyclized rubber.

The method for forming the printed layer is not particularly limited. For example, common printing methods, such as gravure printing, offset printing, letterpress printing, screen printing, transfer printing, and flexography, can be used. By printing the desired print design on the outer surface of the support with the ink composition using such a method for forming the printed layer, the printed layer can be formed.

The thickness of the printed layer is preferably 0.1 μm or more, more preferably 0.3 μm or more, and preferably is 8 μm or less, more preferably 5 μm or less.

The printed layer is preferably formed after performing surface treatment in advance on the side of the support where the printed layer is to be formed. Examples of such surface treatments include pretreatments such as corona discharge treatment, ozonation, low-temperature plasma treatment using, for example, oxygen gas or nitrogen gas, glow discharge treatment, and oxidation treatment in which treatment is performed using, for example, chemicals. It is, furthermore, also possible to applying, for example, a primer coating agent, an undercoating agent, or an anchor coating agent as appropriate in advance and then perform surface treatment.

[Metal Foil]

A metal foil is a material obtained by rolling a metal, such as an aluminum foil or a copper foil. By including a metal foil in the laminate, gas barrier properties can be further improved.

The metal that constitutes the metal foil is not particularly limited. Examples include aluminum, copper, and magnesium.

The thickness of the metal foil is preferably 3 μm or more, more preferably 5 μm or more, and preferably is 50 μm or less, more preferably 10 μm or less. By setting the thickness of the metal foil to 3 μm or more, gas barrier properties can be further improved.

The metal foil can be laminated onto any layer with an adhesive resin layer, which will be described later, interposed therebetween.

[Adhesive Agent Layer]

An adhesive agent layer, which is provided when two layers are bonded by dry lamination, can be formed by applying an adhesive for use in lamination (a lamination adhesive) to the surface of the layer to be laminated and drying the applied adhesive. The lamination adhesive can be, for example, a vinyl, (meth)acrylic, polyamide, polyester, polyether, polyurethane, epoxy, or rubber adhesive of a onecomponent or two-component curing type or of a non-curing type, for example in a solvent-based, water-based, or emulsion form. For the method for coating with the lamination adhesive, the adhesive can be applied to the application surface of a layer constituting the laminate by, for example, direct gravure roll coating, gravure roll coating, kiss coating, reverse roll coating, fountain coating, transfer roll coating, or other methods. The thickness of the adhesive agent layer is preferably 0.5 μm or more, more preferably 1 μm or more. This makes it possible to ensure sufficient bonding strength. The thickness of the adhesive agent layer, furthermore, is preferably 10 μm or less, more preferably 5 μm or less. This makes misaligned winding during lamination processing less likely to occur.

[Adhesive Resin Layer]

An adhesive resin layer, which is provided when two layers are bonded by melt extrusion lamination, is formed by melt extrusion lamination using at least one thermoplastic resin. Thermoplastic resins that can be used for an adhesive resin layer include polyethylene resins, polypropylene resins, or cyclic olefin polymer resins, and copolymeric resins, modified resins, or mixtures (including alloys) containing such resins as their main component. For polyolefin resins, examples of resins that can be used include low-density polyethylene (LDPE), medium-density polyethylene (MDPE), high-density polyethylene (HDPE), linear-chain (linear) low-density polyethylene (LLDPE), polypropylene (PP), ethylene-$\alpha$-olefin copolymers polymerized using a metallocene catalyst, ethylene-polypropylene random or block copolymers, ethylene-vinyl acetate copolymers (EVA), ethylene-acrylic acid copolymers (EAA), ethylene-ethyl acrylate copolymers (EEA), ethylene-methacrylic acid copolymers (EMAA), ethylene-methyl methacrylate copolymers (EMMA), ethylene-maleic acid copolymers, and ionomer resins. To improve interlayer adhesion, furthermore, resins such as acid-modified polyolefin resins resulting from modifying such polyolefin resins with acrylic acid, methacrylic acid, maleic acid, maleic anhydride, fumaric acid, itaconic acid, or other unsaturated carboxylic acids can be used. Moreover, resins such as ones resulting from graft-polymerizing or copolymerizing an unsaturated carboxylic acid, an unsaturated carboxylic acid anhydride, or an ester monomer to/with polyolefin resins can be used. One such material alone or two or more in combination can be used. For cyclic olefin polymer resins, examples of resins that can be used include cyclic olefin polymers such as ethylene-propylene copolymers, polymethylpentene, polybutene, and polynorbornene. Such resins can be used individually or as a combination of multiple ones. It should be noted that, needless to say, the aforementioned polyethylene resins can be ones in which biomass-derived ethylene as mentioned above is used as a monomer unit.

To the adhesive resin layer, materials such as various compounding ingredients for plastics and additives can be added for the purpose of improving or modifying, for example, the workability, heat resistance, weatherability, mechanical properties, dimensional stability, oxidation resistance, lubricity, releasability, flame retardancy, antifungal properties, electrical properties, and strength of the film. For the amounts of materials added, any amount can be added, from an extremely small amount to several tens of percent, according to the purpose. In the foregoing, examples of common additives that can be used include slipping agents, plasticizers, fillers, antistatic agents, anti-blocking agents, crosslinking agents, antioxidants, ultraviolet absorbers, photostabilizers, and coloring agents (e.g., dyes and pigments), and materials such as modifying resins can also be used.

The thickness of the adhesive resin layer can be set as appropriate. Preferably, the thickness is 5 $\mu$m or more, more preferably 10 $\mu$m or more, and preferably is 800 $\mu$m or less, more preferably 500 $\mu$m or less. When the thickness is smaller than the lower limit, water barrier properties are insufficient. When the thickness is equal to or greater than the upper limit, excessive quality arises, and formability also decreases.

The adhesive resin layer, furthermore, may be in a single-layer configuration or may be in a multilayer configuration.

It should be noted that when, as an adhesive resin layer, an adhesive resin layer is laminated on the polyester film 10Y by melt extrusion lamination using a polyolefin resin having a polar group, such as an acid-modified polyolefin resin as mentioned above, the adhesive resin layer can be laminated without performing surface treatment, such as an anchor coating agent.

To one or both surfaces of the laminate, furthermore, secondary processing may be applied for the purpose of imparting, for example, surface functions, such as chemical functions, electrical functions, magnetic functions, mechanical functions, frictional/wear/lubricating functions, optical functions, thermal functions, and biocompatibility. Examples of secondary processing include embossing, painting, bonding, printing, metallization (e.g., plating), machining, and surface treatments (e.g., antistatic treatment, corona discharge treatment, plasma treatment, photochromic treatment, physical vapor deposition, chemical vapor deposition, and coating). Moreover, it is also possible to manufacture, for example, a package, by applying lamination processing (dry lamination or extrusion lamination), processing for shaping into a bag, and other posttreatment processing to the laminate.

As has been described in the foregoing, the laminate 20A is a film including a polyester film 10Y and a sealant layer 21Y. Since the polyester film 10Y is formed as a layer of a carbon-neutral material and is superior in hygienic properties, by using the polyester film 10Y in the laminate 20A, the reduction of the amount of fossil fuel used in the entire laminate 20A can be sought, and a laminate that provides a high $CO_2$ emission-reducing effect and is superior in hygienic properties can be obtained.

To one or both surfaces of the laminate, furthermore, secondary processing may be applied for the purpose of imparting, for example, surface functions, such as chemical functions, electrical functions, magnetic functions, mechanical functions, frictional/wear/lubricating functions, optical functions, thermal functions, and biocompatibility. Examples of secondary processing include embossing, painting, bonding, printing, metallization (e.g., plating), machining, and surface treatments (e.g., antistatic treatment, corona discharge treatment, plasma treatment, photochromic treatment, physical vapor deposition, chemical vapor deposition, and coating). Moreover, it is also possible to manufacture, for example, a package, by applying lamination processing (dry lamination or extrusion lamination), processing for shaping into a bag,

and other posttreatment processing to the laminate.

In the following, packages will be described.

<Package>

A laminate including a polyester film according to the present disclosure is useful as a packaging material, for example for foods, because, as mentioned above, it seeks the reduction of the amount of fossil fuel used, provides a high $CO_2$ emission-reducing effect, and is superior in hygienic properties. For example, the laminate is suitable for use as a packaging film in a package. Examples of packages include packaging bags, lids, laminated tubes, containers for liquids, paper cups, and various labeling materials. Examples of packaging bags include packaging bags in various forms, such as the stand-up pouch type, the side-seal type, the two-side-seal type, the three-side-seal type, the four-side-seal type, the centerseal type, the pillow-seal type, the pleated-seal type, the flat-bottom-seal type, the square-bottom-seal type, and the gusset type. The thickness of the laminate can be determined as appropriate according to the intended use. Preferably, the thickness is 30 μm or more, more preferably 35 μm or more, and preferably is 300 μm or less, more preferably 180 μm or less. Examples of packages made using a laminate including a polyester film according to the present disclosure will be described below. It should be noted that in the present disclosure, although the packages described by way of example below have one layer for each layer type other than the sealant layer 21Y, the configuration is not limited to this; the packages may have two or more layers of the same type. Although the packages described by way of examples below include a printed layer, furthermore, the configuration is not limited to this; the packages may lack a printed layer.

[Stand-Up Pouch]

A case in which a laminate including a polyester film according to the present disclosure is applied to a stand-up pouch is described. Fig. 16 is a perspective view briefly illustrating an example of a stand-up pouch. As illustrated in Fig. 16, the stand-up pouch 30Y is composed of two side sheets 31Y and one bottom sheet 32Y. The stand-up pouch 30Y includes the side sheets 31Y and the bottom sheet 32Y constructed as different components. The stand-up pouch 30Y is a package formed by shaping the laminate constituting the side sheets 31Y into a bag such that the sealant layer becomes the innermost layer. It should be noted that in the present disclosure, although the stand-up pouch 30Y includes the side sheets 31Y and the bottom sheet 32Y constructed as different components, the configuration is not limited to this; the side sheets 31Y and the bottom sheet 32Y may be constructed as the same component.

The side sheets 31Y can be formed using a laminate including a polyester film according to the present disclosure. Fig. 17 is a cross-sectional view briefly illustrating an example of a structure of the laminate that forms the side sheets 31Y. As illustrated in Fig. 17, the laminate 20B that forms the side sheets 31Y includes, for example, a sealant layer 21Y, a support 33Y, a printed layer 34Y, and a polyester film 10Y, and can be constructed by laminating the sealant layer 21Y, the support 33Y, the printed layer 34Y, and the polyester film 10Y in this order from the inner surface side toward the outer surface side. The stand-up pouch 30Y is shaped as a bag by bonding the sealant layers 21Y of the side sheets 31Y together by heat sealing. The laminate 20B, furthermore, may include a barrier layer as described above between any layers constituting the laminate 20B, such as between the polyester film 10Y and the printed layer 34Y.

It should be noted that although the printed layer 34Y in this embodiment is provided between the polyester film 10Y and the support 33Y, the printed layer 34Y may be provided between the sealant layer 21Y and the support 33Y when the support 33Y is transparent.

In addition, although the side sheets 31Y in this embodiment are formed using a laminate including a polyester film according to the present disclosure, the configuration is not limited to this. The bottom sheet 32Y may be formed using a laminate including a polyester film according to the present disclosure, or both the side sheets 31Y and the bottom sheet 32Y may be formed using a laminate including a polyester film according to the present disclosure.

The laminate 20B that forms the side sheets 31Y, furthermore, is not limited to a layer configuration like that illustrated in Fig. 17; it may be in the layer configuration illustrated in Fig. 15, or a laminate including additional layers such as those described above may be used. Moreover, each layer may be changed to any position as appropriate according to the material constituting the support 33Y. For example, as illustrated in Fig. 18, the laminate 20C that forms the side sheets 31Y may include a sealant layer 21Y, a polyester film 10Y, a support 33Y, and a printed layer 34Y, and the laminate 20C may be in a layer configuration in which the sealant layer 21Y, the polyester film 10Y, the support 33Y, and the printed layer 34Y are laminated in this order from the inner surface side toward the outer surface side. In addition, the laminate 20C may also include a barrier layer as described above between any layers constituting the laminate 20C, such as between the polyester film 10Y and the sealant layer 21Y.

The stand-up pouch 30Y can be manufactured using a manufacturing method known in the related art. For example, it is manufactured by placing two side sheets 31Y one over the other, with the side sheets 31Y positioned face-to-face such that their sealant layers 21Y become the innermost layers, inserting a bottom sheet 32Y between the two side sheets 31Y at the same time, and heat-sealing the side sheets 31Y and the bottom sheet 32Y.

For the method for heat sealing, it can be performed by a known method, such as bar sealing, revolving roller sealing, belt sealing, impulse sealing, radiofrequency sealing, or ultrasonic sealing.

The stand-up pouch 30Y is suitable for use as a packaging bag to be filled with a liquid, for packaging of articles such as chemical products, pharmaceutical products, "quasi-drugs" (a product category defined under Japanese regulation for items having milder effects than pharmaceuticals), cosmetic products, and foods, for example as an outer bag for a patch or as a stand-up pouch for use with refill contents, such as liquid detergent, liquid softener, or liquid soap.

According to the form in which the laminate is heat-sealed, furthermore, various packaging bags can be formed in addition to a stand-up pouch. Fig. 19 is a front view briefly illustrating an example of a pillow bag, Fig. 20 is a front view briefly illustrating an example of a three-side-seal bag, and Fig. 21 is a front view briefly illustrating an example of a four-side-seal bag. It should be noted that in Figs. 19 to 21, heat-sealed portions are indicated by hatching. The pillow bag 41Y illustrated in Fig. 19 is obtained by shaping a laminate 20A to 20C like those illustrated in Fig. 15, Fig. 17, and Fig. 18 into a bag such that its sealant layer 21Y becomes the innermost layer, and bonding the two opposite surfaces of the laminate by heat sealing. The three-side-seal bag 42Y illustrated in Fig. 20, furthermore, is obtained by shaping a laminate 20A to 20C like those illustrated in Fig. 15, Fig. 17, and Fig. 18 into a bag such that its sealant layer 21Y becomes the innermost layer, and bonding three sides of the laminate 20A to 20C by heat sealing. Moreover, the four-side-seal bag 43Y illustrated in Fig. 21 is obtained by shaping a laminate 20A to 20C like those illustrated in Fig. 15, Fig. 17, and Fig. 18 into a bag such that its sealant layer 21Y becomes the innermost layer, and bonding the four sides of the laminate 20A to 20C by heat sealing.

[Lid]

A case in which a lid is formed using a laminate including a polyester film according to the present disclosure will now be described. The lid represents a case in which a support 33Y constituting the laminate is constructed as a paper layer, and can be formed using a laminate 20A to 20C in a layer configuration like those illustrated in Fig. 15, Fig. 17, and Fig. 18.

The lid is suitable for use as the lid of a cup-shaped packaging container, in particular a packaging container for hermetically sealing contents, such as an instant food intended to be cooked by pouring hot water, e.g., instant ramen or instant deep-fried noodles, a food intended to be microwaved or another precooked food, a warmed beverage, a beverage intended to be microwaved, a snack food, confectionery, or jelly.

[Tube Container]

A case in which a tube container is formed using a laminate including a polyester film according to the present disclosure will now be described. Fig. 22 is a partial cross-sectional view briefly illustrating an example of a tube container. As illustrated in Fig. 22, the tube container 50Y includes a head 51Y and a cylindrical body 52Y.

The head 51Y is composed of a hollow conical shoulder 53Y and an outlet 54Y and is formed as a single integral structure.

The cylindrical body 52Y is connected to the shoulder 53Y of the head 51Y. The cylindrical body 52Y can be formed using a laminate. A cross-sectional view briefly illustrating an example of a structure of the laminate that forms the cylindrical body of the tube container is presented in Fig. 23. As illustrated in Fig. 23, the laminate 20D that forms the cylindrical body 52Y includes a first sealant layer 21A, a polyester film 10Y, a printed layer 34Y, and a second sealant layer 21B, and is constructed by laminating the first sealant layer 21A, the polyester film 10Y, the printed layer 34Y, and the second sealant layer 21B in this order from the side that becomes the inner surface of the cylindrical body 52Y toward the outer surface side. The laminate 20D, furthermore, may include a barrier layer as described above between any layers constituting the laminate 20D, such as between the polyester film 10Y and the printed layer 34Y.

The laminate 20D that forms the cylindrical body 52Y, furthermore, is not limited to the layer configuration illustrated in Fig. 23; the layer configuration of the laminate 20D can optionally be adjusted as appropriate. A cross-sectional view briefly illustrating an example of another layer configuration of the laminate that forms the cylindrical body 52Y is presented in Fig. 24. As illustrated in Fig. 24, the laminate 20E that forms the cylindrical body 52Y is a laminate resulting from providing an adhesive resin layer 55Y between the polyester film 10Y and the printed layer 34Y in the laminate 20D illustrated in Fig. 23, and providing a support 33Y between the printed layer 34Y and the second sealant layer 21B. The laminate 20E, furthermore, may also include a barrier layer as described above between any layers constituting the laminate 20E, such as between the polyester film 10Y and the adhesive resin layer 55Y.

The cylindrical body 52Y is produced by placing the first sealant layer 21A and the second sealant layer 21B at the ends of the cylindrical body 52Y one over the other, and melt-bonding the overlapping portions by heat sealing. The head 51Y is connected to an upper portion of one opening of the cylindrical body 52Y. It should be noted that the ends of the cylindrical body 52Y are not limited to the method of placing the first sealant layer 21A and the second sealant layer 21B one over the other; the second sealant layers 21B may be placed one over the other.

For the method for heat sealing, it can be performed by a known method, such as bar sealing, revolving roller

sealing, belt sealing, impulse sealing, radiofrequency sealing, ultrasonic sealing, or flame sealing.

An example of a method for manufacturing the tube container 50Y is described. To one opening of the cylindrical body 52Y, the head 51Y is connected by an ordinary method, such as compression molding. Thereafter, the contents are introduced into the cylindrical body 52Y through the open end opposite the end connected to the head 51Y, and the open end is heat-bonded to form a bottom seal 56Y. As a result of this, a tube container 50Y that has been filled with the contents and in which the contents are packaged can be obtained. To the outlet 54Y, a cap can be attached by various methods, such as screwing or engagement, corresponding to the shape of the outlet 54Y.

The tube container 50Y is suitable for use as a tube container for contents such as toothpaste, cosmetic products, glue, mustard, *wasabi* paste, creams, paints, cartilage, pharmaceutical products, and other products known in the related art.

[Paper Container for Liquids]

A case in which a paper container for liquids is formed using a laminate including a polyester film according to the present disclosure will now be described. Fig. 25 is a perspective view illustrating an example of a paper container for liquids. As illustrated in Fig. 25, the paper container for liquids 60Y has a body 61Y shaped like a quadrilateral tube and including side surfaces, a bottom 62Y shaped like a quadrilateral panel, and a top 63Y.

The top 63Y has a pair of opposite sloping panels 63a and a pair of tucked portions 64Y positioned between the sloping panels 63a and tucked between the sloping panels 63a. The pair of sloping panels 63a have bonding margins 65Y at their respective upper ends, and the pair of sloping panels 63a are bonded together by the bonding margins 65Y provided at their respective upper ends. It should be noted that an outlet may be attached to one sloping panel 63a of the pair of sloping panels 63a, and the outlet may be hermetically sealed with a cap.

The paper container for liquids 60Y can be formed using a laminate. Fig. 26 is a cross-sectional view illustrating an example of a structure of a laminate used for a paper container for liquids. As illustrated in Fig. 26, the laminate 20F that forms the paper container for liquids 60Y includes a first sealant layer 21A, a polyester film 10Y, an adhesive resin layer 55Y, a paper layer 66Y, a second sealant layer 21B, and a printed layer 34Y, and can be constructed by laminating the first sealant layer 21A, the polyester film 10Y, the adhesive resin layer 55Y, the paper layer 66Y, the second sealant layer 21B, and the printed layer 34Y in this order from the side that becomes the inner surface of the paper container for liquids 60Y toward the outer surface side. The laminate 20F, furthermore, may include a barrier layer as described above between any layers constituting the laminate 20F, such as between the polyester film 10Y and the adhesive resin layer 55Y, and a layer formed of a vapor deposition film of a metal, a layer formed of a vapor deposition film of an inorganic oxide, and a layer formed of a gas barrier coating film, for example, may be provided on the polyester film 10Y.

The paper layer 66Y can be any type of paper selected according to, for example, the desired rigidity. For example, known types of paper, such as woodfree paper, imitation Japanese vellum, art paper, coated paper, pure-white machine-glazed paper, kraft paper, labeling paper with enhanced waterproofness, base paper for drinking cups, paperboards, such as card stock, ivory board, and manila-lined board, base paper for milk cartons, base paper for cups in general, synthetic paper, and clay-coated paper, can be used.

Before the second sealant layer 21B is laminated onto the paper layer 66Y, furthermore, corona discharge treatment or flame treatment, for example, may be applied to the surface of the paper layer 66Y. By applying such a treatment, interlayer bonding strength can be improved. Corona discharge treatment can be performed by generating a corona atmosphere using a known corona discharge generator and passing the paper layer through it. Flame treatment can be performed by roasting the surface of the paper layer with fire using a known flame treatment device.

The paper container for liquids 60Y can be manufactured by methods known in the related art using a laminate as described above. For example, paper containers for liquids 60Y in various shapes, such as the gable-top type and the brick type, can be manufactured by shaping the laminate into cartons.

The paper container for liquids 60Y is suitable for use as a paper container for liquids in general, including foods, such as *sake*, Japanese spirits, wines, and other alcoholic drinks, milk and other lactic drinks, and orange juice, teas, and other soft drinks, and chemical products, such as car wax, shampoo, and detergents.

[Paper Cup]

A case in which a paper cup is formed using a laminate including a polyester film according to the present disclosure is described. Fig. 27 is a perspective view of a partially cut-away paper cup. As illustrated in Fig. 27, the paper cup 70Y includes a cylindrical body 72Y having a flange 71Y in its upper portion and a diameter gradually increasing toward its opening, and a bottom 73Y provided at the lower end (one end) of the body 72Y. The body 72Y has a flange 71Y with its upper end curled outward. It should be noted that the paper cup 70Y is, after contents are housed, hermetically sealed by pasting a lid along the flange 71Y of the body 72Y. The lid preferably has gas barrier properties, and a lid having gas barrier properties known in the related art can also be used.

The body 72Y can be formed using a laminate including a polyester film according to the present disclosure. Fig. 28 is a cross-sectional view illustrating an example of a structure of a laminate used for the body of a paper cup. As illustrated in Fig. 28, the laminate 20G that forms the body 72Y includes a sealant layer 21Y, a polyester film 10Y, an adhesive resin layer 55Y, a paper layer 66Y, and a printed layer 34Y, and can be constructed by laminating the sealant layer 21Y, the polyester film 10Y, the adhesive resin layer 55Y, the paper layer 66Y, and the printed layer 34Y in this order from the side that becomes the inner surface of the body 72Y toward the outer surface side. The laminate 20G, furthermore, may include a barrier layer as described above between any layers constituting the laminate 20G, such as between the polyester film 10Y and the adhesive resin layer 55Y, and a layer formed of a vapor deposition film of a metal, a layer formed of a vapor deposition film of an inorganic oxide, and a layer formed of a gas barrier coating film, for example, may be provided on the polyester film 10Y.

The laminate 20G that forms the body 72Y can be, for example, a laminate in which a low-density polyethylene layer as a sealant layer 21Y, a polyester film 10Y, a low-density polyethylene layer as an adhesive resin layer 55Y, and a support 33Y are laminated in this order. The bottom 73Y, furthermore, can also be made using a laminate in which a low-density polyethylene layer as a sealant layer 21Y, a polyester film 10Y, a low-density polyethylene layer as an adhesive resin layer 55Y, and a paper layer 66Y are laminated in this order, like the body 72Y.

Although the laminate 20G in this embodiment has the printed layer 34Y on the outer surface side of the paper layer 66Y, the configuration is not limited to this; an additional sealant layer 21Y may be provided on the outer surface side of the printed layer 34Y or between the paper layer 66Y and the printed layer 34Y.

An example of an outline of a method for manufacturing a paper cup is illustrated in Fig. 29. As illustrated in Fig. 29, a body blank 72' cut out in a sector shape from the laminate 20G illustrated in Fig. 28 and given a predetermined external shape is rolled into a cylindrical shape such that the printed layer 34Y is positioned outside and the printed layer 34Y is positioned inside. With the ends 72a' placed one over the other, the ends are bonded by heat-sealing processing. As a result of this, a body seam 75Y is formed on the body 72Y.

A bottom blank 73', furthermore, is cut out in a circular shape from base paper for drinking cups not illustrated, and the outer periphery of the bottom blank 73' is bent downward to form a bent portion 73a'. It should be noted that the bottom blank 73' may be cut out from the laminate 20G.

Then the bottom blank 73' that has completed the shaping processing is placed beneath the body blank 72' processed into a cylindrical shape. Subsequently, the lower end of the body blank 72' is folded such that the bent portion 73a' of the bottom blank 73' is wrapped by the lower end of the body blank 72'. With this state maintained, the overlapping portions of the bent portion 73a' of the bottom blank 73' and the lower end of the body blank 72' are melted using hot air and unified by applying a predetermined amount of pressure thereto. Thereafter, the flange 71Y is formed at the upper end of the body 72Y, whereby the paper cup 70Y is finished. The laminate 20G that forms the body 72Y is configured such that a sealant layer 21Y, a polyester film 10Y, an adhesive resin layer 55Y, a paper layer 66Y, and a printed layer 34Y are laminated in this order from the side that becomes the inner surface of the paper cup 70Y toward the outer surface side, and that the printed layer 34Y is positioned outermost.

The paper cup 70Y is suitable for use as a paper container for putting contents such as a snack food, an instant food intended to be cooked by pouring hot water, a food intended to be microwaved or another precooked food, a warmed beverage, or a beverage intended to be microwaved.

Although a case in which the body 72Y is formed by the laminate 20G has been described in this embodiment, the configuration is not limited to this; the bottom 73Y alone, or both the body 72Y and the bottom 73Y, may be formed by the laminate 20G.

[Laminated Film]

A laminated film according to the present disclosure includes a base material layer and at least one vapor deposition film on at least one surface of the base material layer. For example, as illustrated in Fig. 30, the laminated film 15Z according to the present disclosure includes a base material layer 10Z and at least one vapor deposition film 11Z on at least one surface of the base material layer 10Z.

It should be noted that although the laminated film 15Z in the embodiment illustrated in Fig. 30 is in a configuration in which it includes a vapor deposition film 11Z above a surface of the base material layer 10Z, the laminated film 15Z may be in a configuration in which it includes a vapor deposition film 11Z beneath a surface of the base material layer 10Z, or may be in a configuration in which it includes vapor deposition films 11Z both above and beneath the surfaces of the base material layer 10Z.

An embodiment of a laminated film according to the present disclosure further includes a surface coating layer between the base material layer and the vapor deposition film. For example, as illustrated in Fig. 31, the laminated film 15Z includes a base material layer 10Z, at least one surface coating layer 13Z on at least one surface of the base material layer 10Z, and at least one vapor deposition film 11Z on the surface coating layer 13Z.

An embodiment of a laminated film according to the present disclosure further includes a gas barrier coating film

on the vapor deposition film. For example, as illustrated in Fig. 32, the laminated film 15Z includes a base material layer 10Z, at least one vapor deposition film 11Z on at least one surface of the base material layer 10Z, and at least one gas barrier coating film 12Z on the vapor deposition film 11Z.

[Base Material Layer]

The base material layer included in the laminated film according to the present disclosure is a film containing a polyester according to the present disclosure. That is, the base material layer is a film containing a gas-derived polyester.
The details of the features of the base material layer included in the laminated film according to the present disclosure are the same as those of a polyester film according to the present disclosure, and thus the description thereof is omitted in this section.

[Vapor Deposition Film]

The vapor deposition film included in the laminated film according to the present disclosure is at least one vapor deposition film of a metal or a metal oxide. The vapor deposition film can be formed by methods known in the related art using a metal or a metal oxide known in the related art, and its composition and formation method are not particularly limited. By virtue of the laminated film having vapor deposition film(s), gas barrier properties, which prevent penetration of, for example, oxygen gas and water vapor, and light-shielding properties, which prevent transmission of, for example, visible light and ultraviolet radiation, can be imparted or improved. It should be noted that the laminated film may have two or more vapor deposition films. When the laminated film has two or more vapor deposition films, the individual films may have the same composition or may have different compositions.
The vapor deposition film can be, for example, a vapor deposition film of a metal, such as silicon (Si), aluminum (Al), magnesium (Mg), calcium (Ca), potassium (K), tin (Sn), sodium (Na), boron (B), titanium (Ti), lead (Pb), zirconium (Zr), or yttrium (Y). Alternatively, a vapor deposition film of an oxide of such a metal can be used. In particular, examples of types suitable for, for example, a packaging material include a vapor deposition film of aluminum, a vapor deposition film of an aluminum oxide, and a vapor deposition film of a silicon oxide.
For the notation of a metal oxide, it is represented by, for example, $MO_X$ (Note that in the formula, M represents a metal element, and the value of X varies within different ranges depending on the metal element.), as in $SiO_X$ and $AlO_X$. For the range of the values of X, X can take a value within the range of 0 to 2, inclusive, for silicon (Si), 0 to 1.5, inclusive, for aluminum (Al), 0 to 1, inclusive, for magnesium (Mg), 0 to 1, inclusive, for calcium (Ca), 0 to 0.5, inclusive, for potassium (K), 0 to 2, inclusive, for tin (Sn), 0 to 0.5, inclusive, for sodium (Na), 0 to 1.5, inclusive, for boron (B), 0 to 2, inclusive, for titanium (Ti), 0 to 1, inclusive, for lead (Pb), 0 to 2, inclusive, for zirconium (Zr), and 0 to 1.5, inclusive, for yttrium (Y). When X = 0 in the foregoing, the material is a completely pure metal (pure substance) and is not transparent. The upper limit of the range of X, furthermore, represents a value for complete oxidation. For a packaging material, silicon (Si) and aluminum (Al) are suitable for use. For silicon (Si), oxides in which X ranges from 1.0 to 2.0, inclusive, can be used, and for aluminum (Al), oxides in which X ranges from 0.5 to 1.5, inclusive, can be used.
In the present disclosure, the thickness of such a vapor deposition film of a metal or a metal oxide is preferably 50 Å or more, more preferably 100 Å or more, and preferably is 2000 Å or less, more preferably 1000 Å or less, although preferred thicknesses vary depending on the type of metal or metal oxide used.
To describe this more specifically, when the vapor deposition film is a vapor deposition film of aluminum, the thickness is preferably 50 Å or more, more preferably 100 Å or more, and preferably is 600 Å or less, more preferably 450 Å or less.
When the vapor deposition film is a vapor deposition film of aluminum oxide or silicon oxide, furthermore, the thickness is preferably 50 Å or more, more preferably 100 Å or more, and preferably is 500 Å or less, more preferably 300 Å or less.
Examples of methods for forming the vapor deposition film include physical vapor deposition (PVD), such as vacuum deposition, sputtering, or ion plating, or chemical vapor deposition (CVD), such as plasma chemical vapor deposition, thermal chemical vapor deposition, or photochemical vapor deposition.
As an aspect of the present disclosure, the vapor deposition film will be described in further detail below.
On one surface of the base material layer, a vapor deposition film of a metal oxide, such as silicon oxide, can be formed using low-temperature plasma chemical vapor deposition utilizing, for example, a low-temperature plasma generator in which a monomer gas for vapor deposition, for example of an organic silicon compound, is used as a raw material, an inert gas, such as argon gas or helium gas, is used as a carrier gas, and oxygen gas, for example, is used as an oxygen supply gas. In the foregoing, the low-temperature plasma generator can be a generator of, for example, radiofrequency plasma, pulsed-wave plasma, or microwave plasma. To obtain stable plasma with high activity, it is desirable to use a generator of the radiofrequency plasma type.
A vapor deposition film of silicon oxide formed using a vapor deposition monomer gas, for example of an organic

silicon compound, is a film resulting from a chemical reaction between the vapor deposition monomer gas, for example of an organic silicon compound, and, for example, oxygen gas, close adhesion of the reaction product to the surface of the base material layer, and formation of a dense thin film superior in, for example, flexibility. Typically, it is a continuous thin film that is primarily silicon oxide represented by the general formula $SiO_X$ (Note that X represents a number of 0 or greater and 2 or less.). This vapor deposition film of silicon oxide is preferably a thin film that is primarily a vapor deposition film of silicon oxide represented by the general formula $SiO_X$ (Note that X represents a number of 1.3 or greater and 1.9 or less.), for reasons such as transparency and barrier properties. In the foregoing, the value of X varies depending on factors such as the molar ratio between the vapor deposition monomer gas and the oxygen gas and the energy of the plasma. In general, however, as the value of X decreases, the gas transmission rate becomes smaller, but the film itself assumes yellowness, and transparency worsens.

The above vapor deposition film of silicon oxide, furthermore, is one that is primarily silicon oxide and that contains, for example via chemical bonding, at least one compound composed of one or two or more elements selected from the group consisting of carbon, hydrogen, silicon, and oxygen. Examples of compounds that can be contained include compounds having a C-H bond, compounds having a Si-H bond, compounds in which carbon units are in, for example, the graphite form, the diamond form, or the fullerene form, or the raw-material organic silicon compound or derivatives thereof. More specific examples include hydrocarbons having a $CH_3$ moiety, hydrosilicas, such as $SiH_3$ silyl and $SiH_2$ silylene, and hydroxyl-group derivatives, such as $SiH_2OH$ silanol. In addition to these, the types, amounts, etc., of compounds contained in the vapor deposition film of silicon oxide can be changed, for example by changing the conditions for the deposition process. The amount of such compound(s) contained in the vapor deposition film of silicon oxide, furthermore, is preferably 0.1% by mass or more, more preferably 5% by mass or more, and preferably is 50% by mass or less, more preferably 20% by mass or less. When the amount is less than 0.1% by mass, the impact resistance, ductility, and flexibility, for example, of the vapor deposition film of silicon oxide are insufficient. In that case, scratches, cracks, and other defects easily occur, for example in response to bending, and it becomes difficult to maintain high barrier properties in a stable manner. When the amount exceeds 50% by mass, furthermore, barrier properties decrease.

Moreover, in the present disclosure, it is preferred that the amount of such compound(s) in the vapor deposition film of silicon oxide decrease from the surface of the vapor deposition film of silicon oxide in the depth direction. This provides the advantage that at the surface of the vapor deposition film of silicon oxide, impact resistance, for example, can be increased by virtue of, for example, the compound(s), whereas at the interface with the base material layer, the close adhesion between the base material layer and the vapor deposition film of silicon oxide becomes firmer because the amount of the compound(s) is small.

For the above vapor deposition film of silicon oxide, physical characteristics such as those mentioned above can be confirmed by performing an elemental analysis of the vapor deposition film of silicon oxide by utilizing the method of ion-etching or otherwise analyzing the film in the depth direction using a surface analyzer, such as an Xray photoelectron spectroscopy (XPS) apparatus or a secondary ion mass spectroscopy (SIMS) apparatus.

In the present disclosure, furthermore, the thickness of the above vapor deposition film of silicon oxide is preferably 50 or more, more preferably 100 or more, and preferably is 4000 or less, more preferably 1000 or less. When the thickness exceeds 4000 , defects such as cracks are likely to occur. When the thickness is less than 50 , furthermore, it becomes difficult to achieve the advantage of barrier properties. In the foregoing, the thickness can be measured by the fundamental parameter method using an X-ray fluorescence analyzer manufactured by Rigaku Corporation (model name, RIX2000).

Moreover, in the foregoing, examples of means for changing the thickness of the above vapor deposition film of silicon oxide include increasing the volumetric flow rate of the vapor deposition film, or the method of increasing the amounts of the monomer gas and the oxygen gas, and slowing down the rate of deposition.

Then, in the foregoing, the vapor deposition monomer gas, for example of an organic silicon compound, that forms the vapor deposition film of a metal oxide, such as silicon oxide, can be, for example, 1.1.3.3-tetramethyldisiloxane, hexamethyldisiloxane, vinyltrimethylsilane, methyltrimethylsilane, hexamethyldisilane, methylsilane, dimethylsilane, trimethylsilane, diethylsilane, propylsilane, phenylsilane, vinyltriethoxysilane, vinyltrimethoxysilane, tetramethoxysilane, tetraethoxysilane, phenyltrimethoxysilane, methyltriethoxysilane, or octamethylcyclotetrasiloxane.

From viewpoints such as ease of handling and the characteristics of the resulting continuous film, it is particularly preferred to use 1.1.3.3-tetramethyldisiloxane or hexamethyldisiloxane as a raw material, among such organic silicon compounds. In the foregoing, furthermore, the inert gas can be, for example, argon gas or helium gas.

Then, in the present disclosure, to describe a vapor deposition film of a metal oxide produced by physical vapor deposition as mentioned above in further detail, such a vapor deposition film of a metal oxide produced by physical vapor deposition can be a vapor deposition film of a metal oxide formed using physical vapor deposition (PVD), such as vacuum deposition, sputtering, ion plating, or ion cluster beam deposition.

In the present disclosure, specifically, the vapor deposition film can be formed using, for example, vacuum deposition, in which a metal or an oxide of a metal is used as a raw material, it is vaporized by heating, and the resulting vapor is deposited on the surface of the base material layer, or, alternatively, oxidation deposition, in which a metal or a

metal oxide is used as a raw material, and it is oxidized by introducing oxygen and deposited on the surface of the base material layer, or even plasma-assisted oxidation deposition, in which the oxidation reaction is assisted using plasma. In the foregoing, the method for heating the deposition material can be, for example, resistance heating, radiofrequency induction heating, or electron beam heating (EB).

A specific example of a preferred type of the above vapor deposition film of a metal oxide produced by physical vapor deposition is an amorphous thin film of aluminum oxide. To describe this more specifically, it is possible to use a vapor deposition film that is an amorphous thin film of aluminum oxide represented by the formula $AlO_X$ (In the formula, X represents a number within the range of 0.5 to 1.5, inclusive.) and in which the amorphous thin film of aluminum oxide is formed of an amorphous thin film of aluminum oxide in which the value of X decreases from the surface of the thin film in the depth direction toward the inner surface. Alternatively, in the present disclosure, the amorphous thin film of aluminum oxide can be an amorphous thin film of aluminum oxide that is an amorphous thin film of aluminum oxide represented by the formula $AlO_X$ (In the formula, X represents a number within the range of 0.5 to 1.5, inclusive.) and in which the amorphous thin film of aluminum oxide has values of X increasing from the surface of the thin film in the depth direction toward the inner surface. It should be noted that in the present disclosure, regarding the value of X in the above formula, oxides in which X is 0.5 or greater can basically be used. In the present disclosure, however, when X is less than 1.0, coloration is significant, and the film is inferior in transparency; therefore, it is desirable to use an oxide in which X is 1.0 or greater. An oxide in which X is 1.5, furthermore, is one in which aluminum and oxygen are in a completely oxidized state. For the upper limit, therefore, oxides in which X is 1.5 or less can be used.

Then, in the present disclosure, the thickness of the amorphous thin film of aluminum oxide is preferably 10 Å or more, more preferably 60 Å or more, and preferably is 3000 Å or less, more preferably 1000 Å or less.

In the present disclosure, furthermore, a roll-to-roll vacuum deposition apparatus can be used to form the amorphous thin film of aluminum oxide. In this type of vapor deposition, the degree of vacuum in the vacuum chamber is preferably $10^{-5}$ mbar or more, more preferably $10^{-4}$ mbar or more, preferably is $10^{0}$ mbar or less, more preferably $10^{-1}$ mbar or less. Before oxygen introduction, furthermore, the degree of vacuum in the deposition chamber is preferably $10^{-8}$ mbar or more, more preferably $10^{-7}$ mbar or more, preferably $10^{-2}$ mbar or less, more preferably $10^{-3}$ mbar or less. After oxygen introduction, the degree of vacuum is preferably $10^{-6}$ mbar or more, more preferably $10^{-5}$ mbar or more, preferably $10^{-1}$ mbar or less, more preferably $10^{-2}$ mbar or less. The speed of transportation of the base material layer is preferably 10 m/minute or more, more preferably 50 m/minute or more, and preferably is 800 m/minute or less, more preferably 600 m/minute or less. It should be noted that the amount of oxygen introduced, for example, varies depending on factors such as the size of the deposition machine.

In the present disclosure, for the above thin film layer formed of a metal oxide produced by physical vapor deposition, the percentage transmission of ultraviolet radiation (wavelength, 366 nm) of a vapor deposition film formed of aluminum oxide during deposition and immediately after deposition is preferably 85% or more, more preferably 87% or more, and preferably is 96% or less, more preferably 94% or less. When the percentage transmission of ultraviolet radiation is too small, transparency decreases. A vapor deposition film of aluminum oxide with too large a percentage transmission of ultraviolet radiation, furthermore, is likely to crack because the degree of oxidation of the aluminum oxide is high and the resulting film is hard. For postprocessing suitability reasons, the thickness of a vapor deposition film of aluminum oxide is preferably 150 Å or more and 600 Å or less.

Meanwhile, for a vapor deposition film formed of silicon oxide, a vapor deposition film of silicon oxide produced by physical vapor deposition is preferred that is derived from a mixture of silicon monoxide and silicon as a raw material and whose thickness falls within the range of 50 to 300 , inclusive, for postprocessing suitability reasons.

It should be noted that in the present disclosure, when a vapor deposition film of a metal oxide is formed on a base material layer, it is preferred to provide, for example, a plasma-treated surface by applying plasma treatment with an inert gas to the surface of the base material layer in advance, from the viewpoint of improving, for example, adhesion between the surface of the base material layer and the surface of the vapor deposition film of a metal oxide and, ultimately, firmly joining them together to prevent the occurrence of defects such as delamination.

In the present disclosure, to describe a plasma-treated surface produced using an inert gas, such a plasma-treated surface can be a plasma-treated surface formed on one surface of the base material layer by utilizing, for example, plasma surface treatment, in which surface modification is performed by utilizing plasma gas generated by ionizing a gas by arc discharge. That is, in the present disclosure, a plasma-treated surface can be formed by performing plasma treatment by plasma surface treatment in which an inert gas, such as nitrogen gas, argon gas, or helium gas, is used as the plasma gas.

It should be noted that in the present disclosure, the plasma gas can be a mixed gas resulting from further adding oxygen gas to an inert gas as described above.

In the present disclosure, furthermore, when a plasma-treated surface produced using an inert gas is formed, performing the plasma treatment in-line immediately before forming the vapor deposition film of a metal oxide produced by physical vapor deposition or chemical vapor deposition, for example, is desirable because it removes water, dust, etc., on the surface of the base material layer and also enables surface treatments, such as smoothing and activation of the

surface.

Moreover, in the present disclosure, the above plasma treatment is preferably plasma discharge treatment because of conditions such as plasma power, the type of plasma gas, the amount of plasma gas supplied, and the treatment duration. In the present disclosure, furthermore, the method for generating the plasma can be the utilization of an apparatus for, for example, direct-current glow discharge, radiofrequency discharge, or microwave discharge. Moreover, in the present disclosure, atmospheric-pressure plasma treatment, for example, can be utilized to form the plasma-treated surface.

In addition, when a laminated film including a surface coating layer as described below is manufactured, a laminated film including a surface coating layer between a base material layer and a vapor deposition film can be manufactured by forming the vapor deposition film on the surface coating layer in the same manner as in the foregoing.

[Surface Coating Layer]

The laminated film according to the present disclosure may further include a surface coating layer between the base material layer and the vapor deposition film. The surface coating layer is a layer containing a resin material having a polar group. By including the surface coating layer, a vapor deposition film having high adhesion can be formed on the surface coating layer, whereby gas barrier properties can be improved. A package produced using a laminate including such a surface coating layer, furthermore, has high lamination strength.

In an embodiment, the surface coating layer may be provided on the base material layer. That is, the surface coating layer may be adjacent to the base material layer.

The surface coating layer contains a resin material having a polar group. In the present disclosure, polar group refers to a group containing one or more heteroatoms. Examples of polar groups include an ester group, an epoxy group, a hydroxyl group, an amino group, an amide group, a carboxyl group, a carbonyl group, a carboxylic anhydride group, a sulfone group, a thiol group, and a halogen group. Of these, when considered from the viewpoint of the lamination properties of the package, a carboxyl group, a carbonyl group, an ester group, a hydroxyl group, and an amino group are particularly preferred, with a carboxyl group and a hydroxyl group being more preferred.

For use as the resin material having a polar group, resins such as polyesters, polyethyleneimines, hydroxyl-containing (meth)acrylic resins, nylon 6, nylon 6,6, MXD nylon, amorphous nylon, and other polyamides, and polyurethanes are preferred.

By using such a resin material, the adhesion of the vapor deposition film formed on the surface coating layer can be significantly improved, whereby its gas barrier properties can be effectively enhanced.

In an embodiment, the resin material having a polar group is preferably a hydroxyl-containing (meth)acrylic resin. Thereby, the heat resistance of the laminated film can be improved. Even after the laminated film is subjected to retort treatment or boiling treatment, furthermore, the decreases in gas barrier properties, furthermore, can be limited.

In an embodiment of the present disclosure, a hydroxyl-containing (meth)acrylic resin used to form the surface coating layer is a polymer of a neutral monomer and a hydroxyl-containing (meth)acrylic monomer.

Examples of neutral monomers include methyl (meth)acrylate, ethyl (meth)acrylate, isopropyl (meth)acrylate, n-butyl (meth)acrylate, 2-ethylhexyl (meth)acrylate, lauryl (meth)acrylate, styrene, vinyltoluene, and vinyl acetate.

Examples of hydroxyl-containing (meth)acrylic monomers include 2-hydroxyethyl (meth)acrylate and hydroxypropyl (meth)acrylate.

The glass transition temperature (Tg) of the hydroxyl-containing (meth)acrylic resin is preferably 50°C or above, more preferably 70°C or above. The glass transition temperature (Tg) of the hydroxyl-containing (meth)acrylic resin is preferably 200°C or below, more preferably 150°C or below.

By setting the glass transition temperature (Tg) of the hydroxyl-containing (meth)acrylic resin to 50°C or above, anti-blocking properties can be improved.

By setting the glass transition temperature (Tg) of the hydroxyl-containing (meth)acrylic resin to 200°C or below, reactivity between the hydroxyl-containing (meth)acrylic resin and an isocyanate compound can be improved when the surface coating layer is formed using the isocyanate compound in combination with the resin.

It should be noted that in the present disclosure, Tg can be measured according to JIS K7121: 2012 (Testing Methods for Transition Temperatures of Plastics). Specifically, a DSC curve is measured at a temperature elevation rate of 10°C/minute using a differential scanning calorimetry (DSC) apparatus, from which Tg can be determined.

The number-average molecular weight of the hydroxyl-containing (meth)acrylic resin is preferably 10000 or more. The number-average molecular weight of the hydroxyl-containing (meth)acrylic resin is preferably 100000 or less.

For the number-average molecular weight of the hydroxyl-containing (meth)acrylic resin, anti-blocking properties can be improved by setting it to 10000 or more.

For the number-average molecular weight of the hydroxyl-containing (meth)acrylic resin, the ease of formation of the surface coating layer can be improved by setting it to 100000 or more.

It should be noted that in the present disclosure, the number-average molecular weight can be measured by gel

permeation chromatography (GPC). In GPC measurement, in general, the number-average molecular weight of a polymer is measured as a standard polystyrene-equivalent value.

The hydroxyl value of the hydroxyl-containing (meth)acrylic resin is preferably 20 mg KOHL/g or more, more preferably 30 mg KOHL/g or more. The hydroxyl value of the hydroxyl-containing (meth)acrylic resin is preferably 200 mg KOHL/g or less, more preferably 150 mg KOHL/g or less.

By setting the hydroxyl value of the hydroxyl-containing (meth)acrylic resin to 20 mg KOHL/g or more, reactivity between the hydroxyl-containing (meth)acrylic resin and an isocyanate compound can be improved when the surface coating layer is formed using the isocyanate compound in combination with the resin.

By setting the hydroxyl value of the hydroxyl-containing (meth)acrylic resin to 200 mg KOHL/g or less, the amount of isocyanate compound used can be reduced, whereby the manufacturing cost can be lowered.

It should be noted that in the present disclosure, hydroxylation can be measured according to JIS K0070: 1992 (Test methods for oxidation, saponification value, ester value, iodine value, hydroxyl value and unsaponifiable matter of chemical products).

In the present disclosure, the surface coating layer can be formed using a water-based emulsion or a solvent-based emulsion. Specific examples of water-based emulsions include a polyamide emulsion, a polyethylene emulsion, and a polyurethane emulsion. Specific examples of solvent-based emulsions include a polyester emulsion.

The amount of the resin material having a polar group in the surface coating layer is preferably 70% by mass or more, more preferably 80% by mass or more, even more preferably 90% by mass or more.

Unless the characteristics of the present invention are impaired, the surface coating layer may contain resin materials other than the resin material having a polar group.

In an embodiment of the present disclosure, the surface coating layer may contain an isocyanate compound.

Unless the characteristics of the present disclosure are impaired, the surface coating layer can contain additives. Examples of additives include crosslinking agents, antioxidants, anti-blocking agents, slipping agents, ultraviolet absorbers, photostabilizers, fillers, reinforcing agents, antistatic agents, pigments, and modifying resins.

The percentage of the thickness of the surface coating layer relative to the total of the thickness of the base material layer and the thickness of the surface coating layer is preferably 0.08% or more, more preferably 0.2% or more, even more preferably 1% or more, still more preferably 3% or more. The percentage of the thickness of the surface coating layer relative to the total of the thickness of the base material layer and the thickness of the surface coating layer is preferably 20% or less, more preferably 10% or less.

By setting the percentage of the thickness of the surface coating layer relative to the total of the thickness of the base material layer and the thickness of the surface coating layer to 0.08% or more, the adhesion of the vapor deposition film can be further improved, whereby gas barrier properties can be further improved. The lamination strength of the package, furthermore, can be further improved.

By setting the percentage of the thickness of the surface coating layer relative to the total of the thickness of the base material layer and the thickness of the surface coating layer to 20% or less, the film formation and working suitability of the surface coating layer can be further improved. The recycling suitability of a package produced using the laminated film according to the present disclosure, furthermore, can be improved.

The thickness of the surface coating layer is preferably 0.02 μm or more, more preferably 0.05 μm or more, even more preferably 0.1 μm or more, still more preferably 0.2 μm or more. The thickness of the surface coating layer is preferably 10 μm or less, more preferably 5 μm or less.

By setting the thickness of the surface coating layer to 0.02 μm or more, the adhesion of the vapor deposition film can be further improved, whereby gas barrier properties can be further improved. The lamination strength of the package, furthermore, can be further improved.

By setting the thickness of the surface coating layer to 10 μm or less, the film formation and working suitability of the surface coating layer can be further improved. The recycling suitability of a package produced using the laminated film according to the present disclosure, furthermore, can be improved.

A laminate composed of a base material layer and a surface coating layer can be manufactured off-line. Specifically, it can be produced by shaping a resin composition containing a predetermined polyester into a resin film, for example by utilizing the T-die method or inflation, then stretching the resin film, applying a coating solution for coating formation onto the resin film, and drying the applied solution.

A laminate composed of a base material layer and a surface coating layer, furthermore, can be manufactured in-line. Specifically, it can be produced by shaping a resin composition containing a predetermined polyester into a resin film, for example by utilizing the T-die method or inflation, then stretching the resin film in the machine direction (MD direction), applying a coating solution for coating formation onto the resin film and drying the applied solution, and then stretching the film in the transverse direction (TD direction). It should be noted that stretching in the transverse direction may be performed first. Moreover, stretching in both directions of the machine direction and the transverse direction may be performed before the application of the coating solution for coating formation or after the application of the coating solution for coating formation.

[Gas Barrier Coating Film]

The laminated film according to the present disclosure may further include a gas barrier coating film on the vapor deposition film. The gas barrier coating film can be manufactured through a manufacturing process including, for example, a step of preparing a gas barrier composition that contains at least one alkoxide represented by the general formula $R^1{}_nM(OR^2)_m$ (Note that in the formula, $R^1$ and $R^2$ each independently represent an organic group containing one or more and eight or fewer carbons, M represents a metal atom, n represents an integer of 0 or greater, m represents an integer of 1 or greater, and n + m represents the valence of M.) and at least one water-soluble polymer selected from the group consisting of a polyvinyl alcohol resin and an ethylene-vinyl alcohol copolymer (Hereinafter also referred to simply as "water-soluble polymer.") and that undergoes polycondensation by the sol-gel process in the presence of a sol-gel catalyst, an acid, water, and an organic solvent, a step of providing a coating film by coating the above gas barrier composition that undergoes polycondensation by the sol-gel process onto a vapor deposition film formed of a metal oxide provided on one surface of the base material layer, optionally with a plasma-treated surface produced using oxygen gas therebetween, and a step of forming a gas barrier coating film of the gas barrier composition on the vapor deposition film formed of a metal oxide provided on one surface of the base material layer, optionally with a plasma-treated surface produced using oxygen gas therebetween, by subjecting the laminated film with the provided coating film to heating treatment at a temperature of 20°C or above and 180°C or below and equal to or below the melting point of the base material layer for 10 seconds or longer and 10 minutes or shorter.

It should be noted that in the present disclosure, the gas barrier coating film can also be manufactured by preparing a gas barrier composition that contains at least one or more alkoxides represented by the general formula $R^1{}_nM(OR^2)_m$ and at least one water-soluble polymer and that undergoes polycondensation by the sol-gel process in the presence of a sol-gel catalyst, an acid, water, and an organic solvent, stacking two or more layers using it on a vapor deposition film formed of a metal oxide provided on one surface of the base material layer, and forming a composite polymer layer in which two or more gas barrier coating films of the gas barrier composition are stacked.

In the foregoing, the alkoxide that forms the gas barrier coating film, represented by the general formula $R^1{}_nM(OR^2)_m$, can be at least one or more alkoxide partial hydrolysates or alkoxide hydrolytic condensates. The alkoxide partial hydrolysates, furthermore, do not need to have all alkoxy groups hydrolyzed; they may be ones in which one or more alkoxy groups are hydrolyzed or mixtures thereof. Moreover, the condensates from hydrolysis can be dimers or larger multimers of partially hydrolyzed alkoxides, specifically dimers to hexamers.

In the above alkoxide represented by the general formula $R^1{}_nM(OR^2)_m$, the metal atom represented by M can be, for example, silicon, zirconium, titanium, aluminum, or another. In the present disclosure, examples of preferred metals include silicon and titanium. In the present disclosure, furthermore, the usage of the alkoxide can be using an alkoxide of one kind of metal atom alone or alkoxides of two or more different kinds of metal atoms as a mixture in the same solution.

In the above alkoxide represented by the general formula $R^1{}_nM(OR^2)_m$, furthermore, specific examples of organic groups represented by $R^1$ include alkyl groups, such as a methyl group, an ethyl group, an n-propyl group, an i-propyl group, an n-butyl group, an i-butyl group, a sec-butyl group, a t-butyl group, an n-hexyl group, an n-octyl group, and others. Moreover, in the above alkoxide represented by the general formula $R^1{}_nM(OR^2)_m$, specific examples of organic groups represented by $R^2$ include a methyl group, an ethyl group, an n-propyl group, an i-propyl group, an n-butyl group, a sec-butyl group, and others. It should be noted that in the present disclosure, such alkyl groups in the same molecule may be the same or may be different.

In the present disclosure, the above alkoxide represented by the general formula $R^1{}_nM(OR^2)_m$ is preferably, for example, an alkoxysilane, in which M is Si. This alkoxysilane is one represented by the general formula $Si(ORa)_4$ (Note that in the formula, Ra represents a lower alkyl group.). In the foregoing, Ra can be a methyl group, an ethyl group, an n-propyl group, an n-butyl group, or another. Specific examples of such alkoxysilanes that can be used include tetramethoxysilane $Si(OCH_3)_4$, tetraethoxysilane $Si(OC_2H_5)_4$, tetrapropoxysilane $Si(OC_3H_7)_4$, tetrabutoxysilane $Si(OC_4H_9)_4$, and others.

The polyvinyl alcohol resin that forms the gas barrier coating film can be a polyvinyl alcohol resin or an ethylene-vinyl alcohol copolymer used alone, or, alternatively, a polyvinyl alcohol resin and an ethylene-vinyl alcohol copolymer can be used in combination. By using a water-soluble polymer, the gas barrier properties, waterproofness, weatherability, and other physical characteristics of the gas barrier coating film can be significantly improved. In particular, in the present disclosure, by using a polyvinyl alcohol resin and an ethylene-vinyl alcohol copolymer in combination, a gas barrier coating film significantly superior in, for example, resistance to hot water and gas barrier properties after hot-water treatment, in addition to the gas barrier properties, waterproofness, weatherability, and other physical characteristics, can be formed.

In the present disclosure, when a polyvinyl alcohol resin and an ethylene-vinyl alcohol copolymer are used in combination, their blend ratio is preferably polyvinyl alcohol resin:ethylene-vinyl alcohol copolymer = 10:0.05 to 10:6, inclusive, as a ratio by weight. More preferably, they are used in a blend ratio of 10:1.

In the present disclosure, furthermore, the amount of the water-soluble polymer is preferably 5 parts by mass or more, more preferably 20 parts by mass or more, and preferably is 500 parts by mass or less, more preferably 200 parts by mass or less, per 100 parts by mass of the total amount of the alkoxide. When the amount exceeds 500 parts by mass, the

brittleness of the gas barrier coating film tends to be large, and the waterproofness and weatherability, for example, of the resulting gas barrier laminated film also tend to decrease. When the amount falls below 5 parts by mass, gas barrier properties decrease.

In the present disclosure, the polyvinyl alcohol resin and/or the ethylene-vinyl alcohol copolymer can be as follows. First, the polyvinyl alcohol resin can generally be one obtained by saponifying polyvinyl acetate. Such a polyvinyl alcohol resin is not particularly limited; it may be a partially saponified polyvinyl alcohol resin in which several tens of percent of acetic acid groups remain, may be a completely saponified polyvinyl alcohol in which no acetic acid group remains, or may be a modified polyvinyl alcohol resin in which OH groups are modified. Specific examples of such polyvinyl alcohol resins that can be used include RS-110 (degree of saponification = 99%; degree of polymerization = 1000), which is an RS polymer manufactured by Kuraray Co., Ltd., KURARAY POVAL LM-20SO (degree of saponification = 40%; degree of polymerization = 2000), manufactured by the same, and GOHSENOL NM-14 (degree of saponification = 99%; degree of polymerization = 1400), manufactured by The Nippon Synthetic Chemical Industry Co., Ltd.

In the present disclosure, furthermore, the ethylene-vinyl alcohol copolymer can be a saponified copolymer of ethylene and vinyl acetate, that is, a product obtained by saponifying an ethylene-vinyl acetate random copolymer. Specifically, copolymers that can be used range from partially saponified copolymers in which several tens of molar percent of acetic acid groups remain to completely saponified copolymers in which only several molar percent of acetic acid groups remain or no acetic acid group remains, and are not particularly limited. For gas barrier properties reasons, however, the degree of saponification is preferably 80 mol% or more, more preferably 90 mol% or more, even more preferably 95 mol% or more. For the amount of ethylene-derived repeating units in the ethylene-vinyl alcohol copolymer (hereinafter also referred to as "ethylene content"), furthermore, it is typically preferred to use a copolymer having an ethylene content of 0 to 50 mol%, preferably 20 to 45 mol%. Specific examples of such ethylene-vinyl alcohol copolymers that can be used include EVAL EP-F101 (ethylene content, 32 mol%), manufactured by Kuraray Co., Ltd., and Soarnol D2908 (ethylene content, 29 mol%), manufactured by The Nippon Synthetic Chemical Industry Co., Ltd.

Then, in the present disclosure, to describe a gas barrier composition that forms a gas barrier coating film constituting the laminated film according to the present disclosure, such a gas barrier composition is prepared as a gas barrier composition that contains at least one or more alkoxides represented by the general formula $R^1{}_nM(OR^2)_m$ as stated above and at least one water-soluble polymer as described above and that undergoes polycondensation by the sol-gel process in the presence of a sol-gel catalyst, an acid, water, and an organic solvent.

In the preparation of this gas barrier composition, at least one silane coupling agent, for example, can also be added. The silane coupling agent can be known organoalkoxysilane(s) containing an organic reactive group. In the present disclosure, organoalkoxysilanes having an epoxy group are particularly preferred. For example, compounds such as γ-glycidoxypropyltrimethoxysilane, γ-glycidoxypropylmethyldiethoxysilane, and β-(3,4-epoxycyclohexyl)ethyltrimethoxysilane can be used. One such silane coupling agent or a mixture of two or more may be used.

In the present disclosure, for the amount of such a silane coupling agent used, it can be used within the range of 1 part by mass to 20 parts by mass, inclusive, per 100 parts by mass of the alkoxysilane. When 20 parts by mass or more is used, the rigidity and brittleness of the resulting gas barrier coating film tend to be large, and, furthermore, the insulating properties and workability of the gas barrier coating film tend to decrease.

Then, the sol-gel catalyst used in the above gas barrier composition, primarily a polycondensation catalyst, is a tertiary amine that is substantially insoluble in water and soluble in organic solvents. For example, amines such as N,N-dimethylbenzylamine, tripropylamine, tributylamine, and tripentylamine can be used. In the present disclosure, N,N-dimethylbenzylamine is particularly suitable. The amount of the catalyst used is preferably 0.01 parts by mass or more and 1.0 part by mass or less per 100 parts by mass of the total amount of the alkoxide and the silane coupling agent. For example, the amount is 0.03 parts by mass. The acid used in the above gas barrier composition, furthermore, is used as a catalyst for the sol-gel process, primarily as a catalyst for the hydrolysis of, for example, the alkoxide and the silane coupling agent. This acid can be, for example, a mineral acid, such as sulfuric acid, hydrochloric acid, or nitric acid, or an organic acid, such as acetic acid or tartaric acid. The amount of the acid used is preferably 0.001 moles or more and 0.05 moles or less relative to the total molar amount of the alkoxide and the alkoxide content (e.g., the silicate moiety) of the silane coupling agent. For example, the amount is 0.01 moles.

In the above gas barrier composition, furthermore, water can be used. The amount of water is preferably 0.1 moles or more, more preferably 0.8 moles or more, and preferably is 100 moles or less, more preferably 2 moles or less, per mole of the total molar amount of the alkoxide. When the amount exceeds 100 moles, the polymer obtained from the alkoxysilane and a metal alkoxide forms spherical particles. These spherical particles, furthermore, three-dimensionally crosslink together to form a porous polymer having a low density, and, with such a porous polymer, the gas barrier properties of the laminated film can no longer be improved. Moreover, when the amount falls below 0.1 moles, the hydrolysis reaction tends to be less likely to proceed.

The organic solvent used in the above gas barrier composition can be, for example, methyl alcohol, ethyl alcohol, n-propyl alcohol, isopropyl alcohol, n-butanol, or another. In the above gas barrier composition, furthermore, the water-soluble polymer is preferably in a state in which it is dissolved in a coating solution containing materials such as the alkoxide

and the silane coupling agent, and the type of the organic solvent is selected as appropriate for this purpose. When a polyvinyl alcohol resin and an ethylene-vinyl alcohol copolymer are used in combination, it is preferred to use n-butanol. In the present disclosure, an ethylene-vinyl alcohol copolymer solubilized in a solvent can be, for example, a copolymer commercially available as Soarnol (trade name). The amount of the organic solvent used is typically 30 parts by mass or more and 500 parts by mass or less per 100 parts by mass of the total amount of the alkoxide, the silane coupling agent, the water-soluble polymer, the acid, and the sol-gel catalyst.

Then, a laminated film according to the present disclosure can also be manufactured, for example, as follows.

First, a gas barrier composition (coating solution) is prepared by mixing materials, such as an alkoxysilane or other alkoxide, a silane coupling agent, a water-soluble polymer, a sol-gel catalyst, an acid, water, and an organic solvent as described above and optionally a metal alkoxide. Then, in this gas barrier composition (coating solution), polycondensation gradually proceeds. Subsequently, this gas barrier composition (coating solution) is applied onto a vapor deposition film formed of a metal oxide provided on one surface of the base material layer and dried by typical methods in the usual manner. Then, as a result of this drying, polycondensation of, for example, the alkoxysilane or other alkoxide, the metal alkoxide, the silane coupling agent, and the water-soluble polymer proceeds, and a coating film is formed. In addition, preferably, this application operation is repeated to stack multiple coating films composed of two or more layers. Finally, the base material layer with the coating solution applied thereto is subjected to heating treatment for a duration of 10 seconds or longer and 10 minutes or shorter at a temperature that is preferably 20°C or above, more preferably 50°C or above, and is equal to or below the melting point of the base material layer, preferably 180°C or below, more preferably 160°C or below, whereby one or two or more gas barrier coating films of the gas barrier composition (coating solution) are formed on the vapor deposition film of a metal oxide formed on one surface of the base material layer. In this manner, a laminated film according to the present disclosure can be manufactured. The laminated film according to the present disclosure obtained in such a manner is one superior in gas barrier properties.

It should be noted that in the present disclosure, a laminated film according to the present disclosure manufactured by performing coating, drying, and heating treatments in the same manner as described above using an ethylene-vinyl alcohol copolymer, or both a polyvinyl alcohol resin and an ethylene-vinyl alcohol copolymer, instead of a polyvinyl alcohol resin provides the advantage that gas barrier properties after hot-water treatment, such as boiling treatment or retort treatment, further improve.

In the present disclosure, furthermore, when neither an ethylene-vinyl alcohol copolymer nor a combination of a polyvinyl alcohol resin and an ethylene-vinyl alcohol copolymer is used as in the foregoing, that is, when a laminated film according to the present disclosure is manufactured using a polyvinyl alcohol resin alone, applying, for example, a gas barrier composition made using a polyvinyl alcohol resin to form a first coating layer in advance, then applying a gas barrier composition containing an ethylene-vinyl alcohol copolymer onto this coating layer to form a second coating layer, and thus forming a composite layer thereof in order to improve gas barrier properties after hot-water treatment enables improving the gas barrier properties of the laminated film according to the present disclosure.

Moreover, stacking multiple coating layers each formed by a gas barrier composition containing an ethylene-vinyl alcohol copolymer as described above, or multiple coating layers each formed by a gas barrier composition containing a polyvinyl alcohol resin and an ethylene-vinyl alcohol copolymer in combination, also provides effective means for improving the gas barrier properties of the laminated film according to the present disclosure.

In the present disclosure, the vapor deposition film formed of a metal oxide and the gas barrier coating film form, for example, chemical bonds resulting from hydrolysis-co-condensation reactions, hydrogen bonds, or coordination bonds. Adhesion between the vapor deposition film formed of a metal oxide and the gas barrier coating film improves, and, as a result of synergy between the two layers, the advantage of better gas barrier properties can be provided. Examples of methods for applying the gas barrier composition include forming a coating film by performing one or multiple rounds of application by application means such as a gravure roll coater or another type of roll coating, spray coating, spin coating, dipping, a brush, bar coating, or an applicator. Thereafter, drying by heating is performed to induce condensation, whereby the gas barrier coating film can be formed. The drying temperature is preferably 50°C or above, more preferably 70°C or above, and preferably is 300°C or below, more preferably 200°C or below. The drying duration is preferably 0.005 minutes or longer, more preferably 0.01 minutes or longer, and preferably is 60 minutes or shorter, more preferably 10 minutes or shorter. The thickness of the coating film after drying is preferably 0.01 $\mu$m or more, more preferably 0.1 $\mu$m or more, and preferably is 30 $\mu$m or less, more preferably 10 $\mu$m or less. Optionally, furthermore, when the gas barrier composition is applied, a primer agent, for example, can be applied onto the vapor deposition film formed of a metal oxide in advance. Alternatively, pretreatment, such as corona discharge treatment or plasma treatment, can be applied as appropriate.

As described in the foregoing, in the laminated film according to the present disclosure, a plasma-treated surface formed using an inert gas, a vapor deposition film formed of a metal oxide, a plasma-treated surface formed using oxygen gas or a primer agent layer, and a gas barrier coating film may be laminated in order on one surface of the base material layer.

[Laminate]

An embodiment of a laminate according to the present disclosure includes a laminated film according to the present disclosure and at least one sealant layer. For example, as illustrated in Fig. 33, a laminate 20Z according to the present disclosure includes a laminated film 15Z and a sealant layer 21Z. It should be noted that the laminate 20Z in the embodiment illustrated in Fig. 33 is in a configuration in which the sealant layer 21Z is laminated on the surface of the vapor deposition film 11Z in the laminated film 15Z, the laminate 20Z may be in a configuration in which the sealant layer 21Z is laminated on the surface of the base material layer 10Z of the laminated film 15Z, or may be in a configuration in which sealant layers 21Z are laminated on both surfaces of the laminated film 15Z.
An embodiment of a laminate according to the present disclosure further includes a support. For example, as illustrated in Fig. 34, a laminate 20Z includes a laminated film 15Z, a sealant layer 21Z, and a support 33Z. More specifically, the laminate 20Z includes a support 33Z, a base material layer 10Z, a vapor deposition film 11Z, and a sealant layer 21Z in this order.
An embodiment of a laminate according to the present disclosure, furthermore, may have at least one additional layer, such as a printed layer, a metal foil, an adhesive agent layer, or an adhesive resin layer, in addition to the sealant layer and a support. When the laminate has two or more additional layers, the individual layers may have the same composition or may have different compositions.
The details of the features of the sealant layer, the support, and the additional layer(s) are the same as described above, and thus the description thereof is omitted in this section.
It should be noted that although the laminates 20Z illustrated in Fig. 33 and Fig. 34 have one sealant layer, two or more sealant layers may be provided. When the laminate has two or more sealant layers, the individual layers may have the same composition or may have different compositions.
An embodiment of a laminate according to the present disclosure, furthermore, is not limited to a configuration including only the laminated film and the sealant layer; as long as these layers are included, the laminate may have at least one additional layer in addition to the laminated film and the sealant layer. For example, as illustrated in Fig. 34, the laminate 20Z may include a support 33Z on the surface of the laminated film 15Z opposite the surface where the sealant layer 21Z is laminated.
An embodiment of a laminate according to the present disclosure includes a base material layer and at least one sealant layer. Since the base material layer is formed as a layer made of a carbon-neutral material and is superior in hygienic properties, by using the base material layer in the laminate, the reduction of the amount of fossil fuel used in the entire laminate can be sought, and a laminate that provides a high $CO_2$ emission-reducing effect and is superior in hygienic properties can be achieved.
To one or both surfaces of the laminate, furthermore, secondary processing may be applied for the purpose of imparting, for example, surface functions, such as chemical functions, electrical functions, magnetic functions, mechanical functions, frictional/wear/lubricating functions, optical functions, thermal functions, and biocompatibility. Examples of secondary processing include embossing, painting, bonding, printing, metallization (e.g., plating), machining, and surface treatments (e.g., antistatic treatment, corona discharge treatment, plasma treatment, photochromic treatment, physical vapor deposition, chemical vapor deposition, and coating). Moreover, it is also possible to manufacture a package, for example, by applying lamination processing (dry lamination or extrusion lamination), processing for shaping into a bag, and other posttreatment processing to the laminate.
In particular, an embodiment of a laminate according to the present disclosure is useful as a packaging material, for example for foods, because, as mentioned above, it seeks the reduction of the amount of fossil fuel used, provides a high $CO_2$ emission-reducing effect, and is superior in hygienic properties. For example, it is suitable for use as a packaging film in a package.

EXAMPLES

In the following, the present disclosure will be described in further detail by examples; however, the present disclosure is not limited to the following examples.

[EXAMPLE 1-1]

As a medium to be used for microbial fermentation, a medium containing the following ingredients in 1 L of distilled water was prepared.

(Composition of the Medium)

- $MgCl_2 \cdot 6H_2O$ 0.5 g
- NaCl 0.2 g
- 100 mM Sodium phosphate buffer, pH 6.0 160 mL
- $NH_4Cl$ 0.6 g
- 85% $H_3PO_4$ 0.05 mL
- KCl 0.15 g
- Mixed solution of trace amounts of metals (LS06) 10 mL
- Mixed solution of vitamins B (LS03) 10 mL
- 1000 mg/L of resazurin 1 mL
- $FeCl_3$ 0.0025 g
- Cysteine HCl monohydrate 0.75 g
- Agarose 15 g

The 100 mM sodium phosphate buffer is a solution of 13.2 g of $NaH_2PO_4$ and 1.1 g of $Na_2HPO_4 \cdot 7H_2O$ in 1 L of water.

As a culture to be used for microbial fermentation, furthermore, a *Clostridium autoethanogenum* culture was prepared. The *Clostridium autoethanogenum* in the culture was obtained from the German Resource Centre for Biological Material (DSMZ), and the accession number assigned to the bacterium is DSMZ 10061.

A continuous stirring tank-type reaction apparatus with a capacity of 5 L was filled with 4.9 L of the above medium. The headspace gas in the continuous stirring tank-type reaction apparatus was replaced with 95% CO and 5% $CO_2$ at atmospheric pressure. A 100-mL inoculum of the *Clostridium autoethanogenum* culture was inoculated. While the continuous stirring tank-type reaction apparatus was maintained at 37°C, the mixture was stirred at 200 rpm at the start of cultivation, and stirred at 400 rpm during the growth phase. During stirring, the pH of the culture broth was maintained at 5.5 by automatic addition of 5 M NaOH.

An aseptic serum bottle with a capacity of 234 mL was purged three times with waste gas from a steel mill (composition: 53% CO, 18% $CO_2$, 26% $N_2$, and 3% $H_2$) and then evacuated to a vacuum of -5 psi. 50 mL of the above culture broth was directly transferred to the serum bottle. While the reaction temperature was maintained at 37°C using a shaking incubator, the headspace of the serum bottle was filled with the waste gas and pressurized to 25 psig. Cultivation was performed at 37°C while the culture broth was shaken. The ethanol concentration of the culture broth at 5 hours was 1.675 g/L, confirming that ethanol was produced through microbial fermentation.

The culture broth after the microbial fermentation was separated into the solid component and the liquid component using a solid-liquid separation filter apparatus under the conditions of a culture broth introduction pressure of 200 kPa or more.

Then the liquid component was introduced into a distilling apparatus equipped with a heater using steam at 170°C. After the temperature at the bottom of the distillation column was elevated to 101°C within 15 minutes, the liquid component was introduced through the middle of the distillation column. During continuous operation, the bottom of the column was maintained at 101°C, the middle of the column was maintained at 99°C, and the top was maintained at 91°C, and the apparatus was continuously operated under conditions of 15 seconds/L to purify the ethanol.

The purified ethanol was supplied at 10 L/Hr to a multi-tube externally heated isothermal reactor packed with 1.5 L of a $\gamma$-$Al_2O_3$ catalyst (3 mm $\Phi \times$ 3 mm H). Reaction was performed for 500 hours at a pressure of 10 kg/cm$^2$ G and a temperature of 370°C, whereby ethylene was produced through an intramolecular dehydration reaction of ethanol.

The generated ethylene was introduced into an ethylene oxidation reaction apparatus. In the presence of a silver catalyst, contact gas-phase oxidation was performed using a molecular oxygen-containing gas, giving a reaction product gas containing ethylene oxide.

The reaction product gas containing ethylene oxide was supplied to the lower portion of an ethylene oxide absorption column. Water was introduced through the upper portion of the ethylene oxide absorption column and subjected to counter-current contact with the reaction product gas, allowing the ethylene oxide in the reaction product

gas to be absorbed into the water. Unabsorbed gas near the top of the ethylene oxide absorption column was circulated to the ethylene oxidation reaction apparatus. The bottom liquid in the ethylene oxide absorption column was supplied to the upper portion of an ethylene oxide stripping column operating at a column-top pressure of 0.045 MPa-gauge and a column-bottom temperature of 115°C. Part of the bottom liquid in the ethylene oxide absorption column was circulated to the ethylene oxide absorption column.

Discharged vapor near the top of the ethylene oxide stripping column was condensed using a condenser. Part of the condensate was refluxed to the ethylene oxide stripping column, and part was supplied to an ethylene oxide dehydration column. Vapor near the top of the ethylene oxide dehydration column was condensed using a condenser. Part of the condensate was refluxed to the dehydration column, and the remainder of the condensate was supplied to a light-fraction separation column.

Vapor near the top of the light-fraction separation column was sent to a condenser, and the condensate was refluxed to the light-fraction separation column. The bottom liquid in the light-fraction separation column was supplied to an ethylene oxide rectification column.

Ethylene oxide vapor near the top of the ethylene oxide rectification column was condensed using a condenser. Part of the condensate was refluxed to the ethylene oxide rectification column, and the remainder was extracted as ethylene oxide.

The extracted ethylene oxide was added to the bottom liquid in the ethylene oxide dehydration column, and the resulting mixture was supplied to a hydrolysis reaction apparatus. The mixture was allowed to react at a pressure of 1.8 MPa-gauge and a reaction temperature of 150°C, giving a 15.2% by mass aqueous solution of ethylene glycol.

The aqueous solution of ethylene glycol was supplied to a first evaporator operating at a vessel-top pressure of 0.41 MPa-gauge and a liquid temperature of 153°C. The bottom liquid in the first evaporator was supplied to a second evaporator operating at a vessel-top pressure of 0.17 MPa-gauge and a vessel-bottom temperature of 136°C. The bottom liquid in the second evaporator was supplied to a third evaporator operating at a vessel-top pressure of 0.07 MPa-gauge and a vessel-bottom temperature of 124°C. The bottom liquid in the third evaporator was supplied to a fourth evaporator operating at a vessel-top pressure of -0.03 MPa-gauge and a vessel-bottom temperature of 105°C. The bottom liquid in the fourth evaporator was supplied to an ethylene glycol dehydration column, subjected to water removal, and then supplied to an ethylene glycol distillation column. Through the top of the ethylene glycol distillation column, ethylene glycol was separated.

In such a manner, ethylene glycol in Example 1-1 was obtained.

[EXAMPLE 1-2]

Using a stainless-steel substrate (150 mm × 250 mm; thickness, 150 μm) as a current collector, a metal layer made of Au was formed on its surface by sputtering. The metal layer had a thickness uniform in the plane direction, and the thickness was 100 nm.

The current collector with the metal layer formed thereon was immersed in an ethanol solution containing 10 mL of 1-(2-mercaptoethyl)-4-(3-aminopropyl)-pyridinium bromide at a concentration of 1 mM for 48 hours, whereby modified organic molecules were immobilized on the surface of the metal layer.

Using the reducing catalyst produced in such a manner, a three-electrode cell was fabricated. Specifically, a three-electrode cell was constructed using an H-shaped cell, with the reducing catalyst used as the working electrode, a Ag/AgCl electrode used as the reference electrode, and a Pt electrode used as the counter electrode. The Pt electrode was placed in a cell partitioned by a glass filter.

Then 100% $CO_2$ gas was bubbled into a 5% aqueous solution of $NaHCO_3$, and $CO_2$ was dissolved until the concentration of $CO_2$ absorbed in the solution reached saturation. The inlet and outlet concentrations of the $CO_2$ gas absorbed in the 5% aqueous solution of $NaHCO_3$ were measured. When the two concentrations became equal, it was determined that the concentration within the aqueous solution reached a saturation concentration.

The 5% aqueous solution of $NaHCO_3$ prepared in such a manner was used as a $CO_2$ absorbent and a $CO_2$-reducing electrolytic solution in the three-electrode cell.

In the three-electrode cell, constant-current electrolysis was performed such that the potential applied to the working electrode was -1.2 V vs. Ag/AgCl. The electrolysis duration was set to 60 minutes. During the electrolysis, 100% $CO_2$ gas was bubbled into the electrolytic chamber of the working electrode, and stirring was performed at a stirring speed of 800 rpm using a magnetic stirrer.

Then ethylene glycol produced through the constant-current electrolysis was purified. Specifically, the electrolytic solution was removed from the three-electrode cell and supplied to a first evaporator operating at a vessel-top pressure of 0.41 MPa-gauge and a liquid temperature of 153°C. The bottom liquid in the first evaporator was supplied to a second evaporator operating at a vessel-top pressure of 0.17 MPa-gauge and a vessel-bottom temperature of 136°C. The bottom liquid in the second evaporator was supplied to a third evaporator operating at a vessel-top pressure of 0.07 MPa-gauge and a vessel-bottom temperature of 124°C. The bottom liquid in the third evaporator was supplied to a fourth

evaporator operating at a vessel-top pressure of -0.03 MPa-gauge and a vessel-bottom temperature of 105°C. The bottom liquid in the fourth evaporator was supplied to an ethylene glycol dehydration column, subjected to water removal, and then supplied to an ethylene glycol distillation column. Through the top of the ethylene glycol distillation column, ethylene glycol was separated.

In such a manner, ethylene glycol in Example 1-2 was obtained.

[COMPARATIVE EXAMPLE 1-1]

An aqueous solution of fossil fuel-derived ethylene oxide was diluted with water, whereby an aqueous solution of ethylene oxide in which the ratio by mass between ethylene oxide and water was 10:1 was prepared. The aqueous solution of ethylene oxide was charged into a reactor, and ethylene oxide and water were allowed to react under the conditions of a temperature of 200°C and a pressure of approximately 35 kg/cm$^2$ G, yielding an approximately 15% aqueous solution of ethylene glycol.

The resulting aqueous solution of ethylene glycol was concentrated and completely dehydrated, and then the product was charged into a distillation column. While nitrogen gas was blown into the top of the distillation column at a flow rate of 15 Nm$^2$/Hr, ethylene glycol was separated and purified under a reduced pressure of 20 mmHg. Ethylene glycol in Comparative Example 1-1 was obtained at a distillation rate of approximately 10000 kg/Hr.

[Measurement of the Amount of Kjeldahl Nitrogen]

For each of the ethylene glycols in Examples 1-1 and 1-2 and Comparative Example 1-1 above, the amount of Kjeldahl nitrogen contained in the ethylene glycol was measured according to 44.1 (Kjeldahl method) and 44.2 (indophenol blue absorptiometry) of JIS K0102: 2019. The results are presented in Table 1.

[Measurement of the Quantity of Carbon Dioxide Molecules]

For each of the ethylene glycols in Examples 1-1 and 1-2 and Comparative Example 1-1 above, 35 mg was weighed on a SiC stage, and the weighed fraction was used as a sample.

Thermal desorption spectroscopy-mass spectrometry (TDS-MS) was performed under the measurement conditions below, whereby the quantity of carbon dioxide molecules contained in the sample was measured. The results are presented in Table 1.

(Measurement Conditions)

- Instrument name: EMD-WA1000S/W, manufactured by ESCO, Ltd.
- The sample was heated on a SiC stage
- Ionization method: Electron ionization (EI)
- Measurement mode: SCAN mode
- Mass range measured (m/z): 1 to 200
- Heating conditions: 50°C to 100°C
- Temperature elevation rate: 10°C/min
- Retention temperature and retention time: Held at 100°C for 30 minutes

[Table 1]

| Table 1 | Example 1-1 | Example 1-2 | Comparative Example 1-1 |
|---|---|---|---|
| Ethylene glycol source | Carbon monoxide gas | Carbon dioxide gas | Fossil fuel |

(continued)

| Table 1 | Example 1-1 | Example 1-2 | Comparative Example 1-1 |
|---|---|---|---|
| Amount of Kjeldahl nitrogen [$\mu$g/g] | 327 | 560 | 867 |
| Quantity of carbon dioxide molecules [molecules/g] | $2.8 \times 10^{18}$ | $6.9 \times 10^{18}$ | $2.0 \times 10^{18}$ |

As is clear from Table 1 above, it can be understood that ethylene glycol made using carbon monoxide gas or carbon dioxide gas as a raw material contains a smaller amount of Kjeldahl nitrogen compared with fossil fuel-derived ethylene glycol.

It can be, furthermore, understood that ethylene glycol made using carbon monoxide gas or carbon dioxide gas as a raw material contains a greater quantity of carbon dioxide molecules compared with fossil fuel-derived ethylene glycol.

[EXAMPLE 2-1]

Sixty-two parts by mass of the ethylene glycol in Example 1-1 and 83 parts by mass of fossil fuel-derived terephthalic acid were supplied to a reaction chamber as slurry, and an esterification reaction was performed at 240°C for 5 hours by the ordinary, direct polymerization method. Thereafter, 0.013 parts by mass of trimethyl phosphate (manufactured by Aldrich) (15 mmol% relative to the acid component) was added, after which the system was allowed to proceed to a polymerization reaction under high-temperature vacuum conditions. First, the degree of vacuum was increased until 4000 Pa, and the polymerization temperature was elevated to 280°C, both over 40 minutes. Then, with this polymerization temperature of 280°C maintained, a melt polymerization reaction was performed for 3 hours under a reduced pressure of 200 Pa, whereby a polyester was synthesized. The synthesized polyester was discharged in the form of a strand into flowing water, and the strand was pelletized using a pelletizer. The pellets were dried for 5 hours at 160°C, solid-phase polymerization was performed at 205°C under a vacuum of 50 Pa in a nitrogen atmosphere, and the pellets were discharged when a melt viscosity corresponding to an intrinsic viscosity of 0.61 was reached. In this manner, polyester pellets in Example 2-1 were obtained.

The polyester pellets in Example 2-1 were dried, then supplied to an extruder, and melted at 260°C. The melt was extruded to form a sheet through a T-die and solidified by cooling using cooling rollers. In this manner, an unstretched sheet was obtained.

Subsequently, this unstretched sheet was held in an 88°C environment for 1 minute, and then stretched 3.6 times, as a ratio by area, simultaneously in the machine direction and the transverse direction at a rate of 100 mm/min. In this manner, a polyester film in Example 2-1, having a thickness of 12 $\mu$m, was obtained.

[EXAMPLE 2-2]

Polyester pellets in Example 2-2 and a polyester film in Example 2-2 were obtained in the same manner as in Example 2-1, except that the point in time of pellet discharge in the solid-phase polymerization was changed from when a melt viscosity corresponding to an intrinsic viscosity of 0.61 was reached to when a melt viscosity corresponding to an intrinsic viscosity of 0.65 was reached.

[COMPARATIVE EXAMPLE 2-1]

Polyester pellets in Comparative Example 2-1 and a polyester film in Comparative Example 2-1 were obtained in the same manner as in Example 2-1, except that fossil fuel-derived ethylene glycol was used instead of ethylene glycol derived from carbon monoxide gas.

[COMPARATIVE EXAMPLE 2-2]

Polyester pellets in Comparative Example 2-2 and a polyester film in Comparative Example 2-2 were obtained in the same manner as in Example 2-2, except that fossil fuel-derived ethylene glycol was used instead of ethylene glycol derived from carbon monoxide gas.

[Measurement of Intrinsic Viscosity]

Using a phenol/tetrachloroethane (component ratio: 3/2) solvent, the melt viscosity of the polyester pellets in

each of Examples 2-1 and 2-2 and Comparative Examples 2-1 and 2-2 was measured at 35°C, and the intrinsic viscosity was calculated from the melt viscosity. The results are presented in Table 2.

[Measurement of the Quantities of Carbon Dioxide Molecules and Water Molecules in the Polyester Pellets]

The polyester pellets in each of Examples 2-1 and 2-2 and Comparative Examples 2-1 and 2-2 were cut to give a test specimen weighing 35 mg. For the test specimen, thermal desorption spectroscopy-mass spectrometry (TDS-MS) was performed under the measurement conditions below, whereby the quantities of carbon dioxide molecules and water molecules contained in the polyester pellets were measured. The results are presented in Table 2.

(Measurement Conditions)

- Instrument name: EMD-WA1000S/W, manufactured by ESCO, Ltd.
- The test specimen was heated on a SiC stage
- Ionization method: Electron ionization (EI)
- Measurement mode: SCAN mode
- Mass range measured (m/z): 1 to 200
- Heating conditions: 50°C to 100°C
- Temperature elevation rate: 10°C/min
- Retention temperature and retention time: Held at 100°C for 30 minutes

[Measurement of Haze]

For each of the polyester films in Examples 2-1 and 2-2 and Comparative Examples 2-1 and 2-2, a test specimen having a size of 50 mm long × 50 mm wide was cut out. Using a haze meter (NDH4000, manufactured by Nippon Denshoku Industries Co., Ltd.), the haze of each test specimen was measured in an environment at a temperature of 23°C and a relative humidity of 50 RH%. The measurement was performed according to JIS K7136: 2000.
A smaller haze indicates better transparency of the film. The results are presented in Table 2.

[Measurement of Tensile Strength and Tensile Elongation]

For each of the polyester films in Examples 2-1 and 2-2 and Comparative Examples 2-1 and 2-2, a test specimen having a size of 200 mm in the machine direction (machine flow direction, MD direction) × 15 mm in the transverse direction (width direction, TD direction) was cut out. Using a tensile tester (TENSILON RTC-125A, manufactured by Orientec Corporation), the tensile strength in the machine direction and the tensile elongation in the machine direction were measured for each test specimen in an environment at a temperature of 23°C and a relative humidity of 50 RH%. The tensile speed during the measurement was set to 300 mm/minute.
Test specimens having a size of 15 mm in the machine direction × 200 mm in the transverse direction, furthermore, were cut out, and the tensile strength in the transverse direction and the tensile elongation in the transverse direction were measured for each test specimen in the same manner.
The results are presented in Table 2.

[Measurement of the Quantities of Carbon Dioxide Molecules and Water Molecules in the Polyester Film]

For each of the polyester films in Examples 1 and 2 and Comparative Examples 1 and 2, four test specimens each having a size of 10 mm long × 3 mm wide were cut out. The total mass of the four test specimens was approximately 35 mg.
Thermal desorption spectroscopy-mass spectrometry (TDS-MS) was performed under the measurement conditions below, whereby the quantities of carbon dioxide molecules and water molecules contained in the polyester film were measured. The results are presented in Table 2.

(Measurement Conditions)

- Instrument name: EMD-WA1000S/W, manufactured by ESCO, Ltd.
- The four test specimens were heated on a SiC stage
- Ionization method: Electron ionization (EI)
- Measurement mode: SCAN mode
- Mass range measured (m/z): 1 to 200
- Heating conditions: 50°C to 100°C
- Temperature elevation rate: 10°C/min
- Retention temperature and retention time: Held at 100°C for 30 minutes

[Table 2]

| Table 2 | | Example 2-1 | Example 2-2 | Comparative Example 2-1 | Comparative Example 2-2 |
|---|---|---|---|---|---|
| Ethylene glycol source | | Carbon monoxide gas | Carbon monoxide gas | Fossil fuel | Fossil fuel |
| Intrinsic viscosity [dL/g] | | 0.61 | 0.65 | 0.61 | 0.65 |
| Quantity of carbon dioxide molecules in the polyester pellets [molecules/g] | | $1.3 \times 10^{18}$ | $8.3 \times 10^{17}$ | $6.1 \times 10^{17}$ | $3.4 \times 10^{17}$ |
| Quantity of water molecules in the polyester pellets [molecules/g] | | $4.6 \times 10^{19}$ | $2.0 \times 10^{19}$ | $1.5 \times 10^{19}$ | $8.6 \times 10^{18}$ |
| Thickness [$\mu$m] | | 12 | 12 | 12 | 12 |
| Haze [%] | | 0.30 | 0.32 | 0.26 | 0.30 |
| Tensile strength [N/15 mm] | Machine direction | 19.35 | 18.86 | 19.80 | 18.80 |
| | Transverse direction | 18.76 | 19.48 | 17.63 | 18.50 |
| Tensile elongation [%] | Machine direction | 23.60 | 21.70 | 21.93 | 22.40 |
| | Transverse direction | 4.50 | 4.70 | 4.53 | 5.30 |
| Quantity of carbon dioxide molecules in the polyester film [molecules/g] | | $2.2 \times 10^{18}$ | $1.2 \times 10^{18}$ | $1.0 \times 10^{18}$ | $5.5 \times 10^{17}$ |
| Quantity of water molecules in the polyester film [molecules/g] | | $1.8 \times 10^{20}$ | $9.2 \times 10^{19}$ | $8.4 \times 10^{19}$ | $4.9 \times 10^{19}$ |

As is clear from Table 2 above, it can be understood that polyester pellets according to the present disclosure contain greater quantities of carbon dioxide molecules and water molecules compared with known polyester pellets made using fossil fuel-derived ethylene glycol.

It can be, furthermore, understood that polyester films according to the present disclosure, despite being films produced using polyesters in which the diol unit is ethylene glycol made using carbon monoxide gas as a raw material, exhibit physical characteristics that are not inferior in terms of transparency and mechanical properties compared with films produced using known polyesters in which the diol unit is fossil fuel-derived ethylene glycol.

Moreover, it can be understood that polyester films according to the present disclosure contain greater quantities of carbon dioxide molecules and water molecules compared with films produced using polyesters in which the diol unit is fossil fuel-derived ethylene glycol.

<Production of Polyester Pellets for Molded Articles and Molded Articles>

[EXAMPLE 2-3]

Polyester pellets in Example 2-3 were obtained in the same manner as in Example 2-1, except that the point in time of pellet discharge in the solid-phase polymerization was changed from when a melt viscosity corresponding to an intrinsic viscosity of 0.61 was reached to when a melt viscosity corresponding to an intrinsic viscosity of 0.77 was reached.
The polyester pellets in Example 2-3 were supplied to an injection molding machine and melted at 290°C. Then the melt was injected, whereby a preform as illustrated in Fig. 10 was produced. The thickness of the body of the preform was 3.5 mm, and the weight was 22 g.
Subsequently, this preform was heated to 110°C, and biaxial orientation blow molding was performed in a blow mold, whereby a bottle as illustrated in Fig. 11 was produced with an overflow capacity of 600 mL. The thickness of the cross-section of the bottle at the body was 0.28 mm.
In such a manner, a molded article in Example 2-3, which was in the form of a bottle, was obtained.

[EXAMPLE 2-4]

Polyester pellets in Example 2-4 and a molded article in Example 2-4 were obtained in the same manner as in Example 2-1, except that the point in time of pellet discharge in the solid-phase polymerization was changed from when a melt viscosity corresponding to an intrinsic viscosity of 0.61 was reached to when a melt viscosity corresponding to an intrinsic viscosity of 0.79 was reached.

[EXAMPLE 2-5]

The polyester pellets in Example 2-3 were supplied to an injection molding machine and melted at 290°C. Then the melt was injected, whereby a spoon as illustrated in Fig. 13 was produced. The thickness of the measuring section and the thickness of the handle were both 1.5 mm.
In such a manner, a molded article in Example 2-5, which was in the form of a spoon, was obtained.

[COMPARATIVE EXAMPLE 2-3]

Polyester pellets in Comparative Example 2-3 and a molded article in Comparative Example 2-3 were obtained in the same manner as in Example 2-3, except that fossil fuel-derived ethylene glycol was used instead of ethylene glycol derived from carbon monoxide gas.

[COMPARATIVE EXAMPLE 2-4]

Polyester pellets in Comparative Example 2-4 and a molded article in Comparative Example 2-4 were obtained in the same manner as in Example 2-4, except that fossil fuel-derived ethylene glycol was used instead of ethylene glycol derived from carbon monoxide gas.

[COMPARATIVE EXAMPLE 2-5]

A molded article in Comparative Example 2-5 was obtained in the same manner as in Example 2-5, except that the polyester pellets in Comparative Example 2-3 were used instead of the polyester pellets in Example 2-3.

[Measurement of Intrinsic Viscosity]

Using a phenol/tetrachloroethane (component ratio: 3/2) solvent, the melt viscosity of the polyester pellets in each of Examples 2-3 and 2-4 and Comparative Examples 2-3 and 2-4 was measured at 35°C, and the intrinsic viscosity was calculated from the melt viscosity. The results are presented in Table 3.

[Measurement of the Amount of Kjeldahl Nitrogen in the Polyester Pellets]

For the polyester pellets in each of Examples 2-3 and 2-4 and Comparative Examples 2-3 and 2-4, the amount of Kjeldahl nitrogen contained in the polyester pellets was measured according to 44.1 (Kjeldahl method) and 44.2 (indophenol blue absorptiometry) of JIS K0102: 2019. The results are presented in Table 3.

[Measurement of the Quantity of Carbon Dioxide Molecules in the Polyester Pellets]

The polyester pellets in each of Examples 2-3 and 2-4 and Comparative Examples 2-3 and 2-4 were cut to give a test specimen weighing 35 mg. For the test specimen, thermal desorption spectroscopy-mass spectrometry (TDS-MS) was performed under the measurement conditions below, whereby the quantity of carbon dioxide molecules contained in the polyester pellets was measured. The results are presented in Table 3.

(Measurement Conditions)

- Instrument name: EMD-WA1000S/W, manufactured by ESCO, Ltd.
- The test specimen was heated on a SiC stage
- Ionization method: Electron ionization (EI)
- Measurement mode: SCAN mode
- Mass range measured (m/z): 1 to 200
- Heating conditions: 50°C to 100°C
- Temperature elevation rate: 10°C/min
- Retention temperature and retention time: Held at 100°C for 30 minutes

[Measurement of the Amount of Kjeldahl Nitrogen in the Molded Article]

A test specimen was cut out from each of the molded article in Example 2-3, the molded article in Example 2-5, the molded article in Comparative Example 2-3, and the molded article in Comparative Example 2-5, and the amount of Kjeldahl nitrogen contained in the test specimen was measured according to 44.1 (Kjeldahl method) and 44.2 (indophenol blue absorptiometry) of JIS K0102: 2019.

It should be noted that the lower limit of detection for the amount of Kjeldahl nitrogen was 10 μg/g, and no Kjeldahl nitrogen was detected in Example 2-5. The amount of Kjeldahl nitrogen in the molded article in Example 2-5, therefore, was reported as less than 10 μg/g.

The results are presented in Table 3.

[Measurement of the Quantity of Carbon Dioxide Molecules in the Molded Article]

For each of the molded article in Example 2-3, the molded article in Example 2-5, the molded article in Comparative Example 2-3, and the molded article in Comparative Example 2-5, a test specimen was cut out such that the mass was 35 mg.

Thermal desorption spectroscopy-mass spectrometry (TDS-MS) was performed under the measurement conditions below, whereby the quantity of carbon dioxide molecules contained in the test specimen was measured. The results are presented in Table 3.

(Measurement Conditions)

- Instrument name: EMD-WA1000S/W, manufactured by ESCO, Ltd.

- The four test specimens were heated on a SiC stage
- Ionization method: Electron ionization (EI)
- Measurement mode: SCAN mode
- Mass range measured (m/z): 1 to 200
- Heating conditions: 50°C to 100°C
- Temperature elevation rate: 10°C/min
- Retention temperature and retention time: Held at 100°C for 30 minutes

[Measurement of Buckling Strength]

Each of the molded articles in Examples 2-3 and 2-4 and Comparative Examples 2-3 and 2-4 was filled with 600 mL of water as a content liquid and then hermetically sealed with a cap. With each molded article allowed to stand upright, a buckling strength test was performed. The measurement of buckling strength was performed using Topload Tester (Body bending tester) EH-1000, manufactured by EWIG Co., Ltd. The molded article was placed on the supporting stage in an upright state, and the detection head was moved downward. A load was applied at a constant speed from above the mouth of the molded article, and the maximum load before the so-called yield state was reached was reported as buckling strength. A greater buckling strength indicates better mechanical properties of the molded article. The results are presented in Table 3.

[Impact Resistance Test]

Ten molded articles as in Example 2-5 and ten molded articles as in Comparative Example 2-5 were prepared. Each molded article was dropped from a height of 1.5 m in the horizontal position ten times, and an evaluation was made based on the evaluation criteria below. The results are presented in Table 1.

(Evaluation Criteria)

A: All ten molded articles did not break; the molded articles were superior in mechanical properties.

B: One or more of the ten molded articles broke; the molded articles were not superior in mechanical properties.

[Table 3]

| Table 3 | Example 2-3 | Example 2-4 | Example 2-5 | Comparative Example 2-3 | Comparative Example 2-4 | Comparative Example 2-5 |
|---|---|---|---|---|---|---|
| Ethylene glycol source | Carbon monoxide gas | Carbon monoxide gas | Carbon monoxide gas | Fossil fuel | Fossil fuel | Fossil fuel |
| Intrinsic viscosity [dL/g] | 0.77 | 0.79 | - | 0.77 | 0.79 | - |
| Amount of Kjeldahl nitrogen in the polyester pellets [$\mu$g/g] | 230 | 180 | - | 270 | 310 | - |
| Quantity of carbon dioxide molecules in the polyester pellets [molecules/g] | $4.9 \times 10^{17}$ | $7.4 \times 10^{17}$ | - | $4.6 \times 10^{17}$ | $2.6 \times 10^{17}$ | - |

(continued)

| Table 3 | Example 2-3 | Example 2-4 | Example 2-5 | Comparative Example 2-3 | Comparative Example 2-4 | Comparative Example 2-5 |
|---|---|---|---|---|---|---|
| Amount of Kjeldahl nitrogen in the molded article [$\mu$g/g] | Less than 10 | - | 12 | 21 | - | 30 |
| Quantity of carbon dioxide molecules in the molded article [molecules/g] | $2.1 \times 10^{18}$ | - | $9.7 \times 10^{17}$ | $8.9 \times 10^{17}$ | - | $5.3 \times 10^{17}$ |
| Evaluation of buckling strength [N] | 325 | 338 | - | 322 | 337 | - |
| Impact resistance | - | - | A | - | - | A |

As is clear from Table 3 above, it can be understood that polyester pellets according to the present disclosure contain a smaller amount of Kjeldahl nitrogen and a greater quantity of carbon dioxide molecules compared with known polyester pellets made using fossil fuel-derived ethylene glycol.

It can be, furthermore, understood that molded articles made using polyester pellets according to the present disclosure exhibit physical characteristics that are not inferior in terms of mechanical properties compared with known molded articles produced using polyesters in which the diol unit is fossil fuel-derived ethylene glycol.

As is clear from Table 3 above, molded articles according to the present disclosure, despite being articles molded from polyesters in which the diol unit is ethylene glycol made using carbon monoxide gas as a raw material, exhibit physical characteristics that are not inferior in terms of mechanical properties compared with articles molded from known polyesters in which the diol unit is fossil fuel-derived ethylene glycol.

It can be, furthermore, understood that articles molded from polyesters in which the diol unit is ethylene glycol made using carbon monoxide gas as a raw material contain a smaller amount of Kjeldahl nitrogen and a greater quantity of carbon dioxide molecules compared with articles molded from polyesters in which the diol unit is fossil fuel-derived ethylene glycol.

[EXAMPLE 3-1]

The polyester pellets in Example 2-2 were dried, then supplied to an extruder, and melted at 290°C, and the melt was extruded through a spinneret having 192 orifices. The extruded yarn was passed through a 300-mm long heating cylinder at an atmosphere temperature of 350°C, then cooled by blowing quench air at a temperature of 25°C in the transverse direction, and spin-finish oil was applied using an oiling roller. The amount of spin-finish oil applied was such that the oil pick-up after stretching was 0.5%. The yarn was taken up at a speed of 660 m/minute using take-up rollers, whereby undrawn fibers were obtained.

The resulting undrawn fibers were preheated to 100°C using preheating rollers continuously, without being wound once, and drawn 3.5 times between the preheating rollers and drawing rollers. Subsequently, the surface temperature of the drawing rollers was adjusted to 140°C, the fibers were further preheated to facilitate drawing, and the fibers were drawn 1.43 times between the drawing rollers and heating rollers. The surface temperature of the heating rollers was set to 257°C. The drawn fibers were subjected to relaxation heat treatment between the heating rollers and relaxation rollers at a percentage relaxation of 11%, and wound using a winding machine at a speed of 3300 m/minute. In such a manner, fibers in Example 3-1 were obtained.

Using the fibers in Example 3-1 as the warp and the weft, the warp, which is along the longitudinal direction, and the weft, which is along the width direction, were alternately crossed in plain weave using a loom, whereby 30 cm $\times$ 30 cm cloth was formed. In such a manner, gauze in Example 3-1 was obtained.

[COMPARATIVE EXAMPLE 3-1]

Polyester pellets in Comparative Example 3-1, fibers in Comparative Example 3-1, and gauze in Comparative Example 3-1 were obtained in the same manner as in Example 3-1, except that the polyester pellets in Comparative Example 2-2 were used instead of the polyester pellets in Example 2-2.

[Measurement of Intrinsic Viscosity]

Using a phenol/tetrachloroethane (component ratio: 3/2) solvent, the melt viscosity of the polyester pellets in each of Example 3-1 and Comparative Example 3-1 was measured at 35°C, and the intrinsic viscosity was calculated from the melt viscosity. The results are presented in Table 4.

[Differential Scanning Calorimetry (DSC)]

For the polyester pellets used in each of Example 3-1 and Comparative Example 3-1, measurement was conducted in a nitrogen gas atmosphere using a differential scanning calorimeter (manufactured by Hitachi High-Tech Science Corporation; product name, DSC7000X) according to JIS K7122: 2012 (Testing Methods for Heat of Transitions of Plastics). The measurement sample is 15 mg of razor-sliced polyester pellets. During the measurement, the measurement sample was held at 0°C for 5 minutes, then heated from 0°C to 300°C at a heating rate of 10°C/minute (first heating run), held at 300°C for 1 minute, then cooled from 300°C to 0°C at a cooling rate of 10°C/minute (first cooling run), and held at 0°C for 5 minutes. Thereafter, the sample was heated again from 0°C to 300°C at a heating rate of 10°C/minute (second heating run), held at 300°C for 1 minute, then cooled from 300°C to 0°C at a cooling rate of 10°C/minute (second cooling run), and held at 0°C for 5 minutes. Thereafter, the sample was heated from 0°C to 25°C at a heating rate of 10°C/minute. As a result of this, DSC curves during the second heating run and the second cooling run were obtained. In the DSC curves, the amount of heat of fusion and the amount of heat of solidification were determined from the area enclosed by the baseline (the line formed by connecting the starting point and the ending point of the base-to-base ridge) and the peak. The flow rate of the nitrogen gas was set to 20 mL/minute. The results are presented in Table 4.

[Measurement of the Amount of Kjeldahl Nitrogen]

For the polyester pellets used in each of Example 3-1 and Comparative Example 3-1, the amount of Kjeldahl nitrogen contained in the polyester pellets was measured according to 44.1 (Kjeldahl method) and 44.2 (indophenol blue absorptiometry) of JIS K0102: 2019. The results are presented in Table 4.

[Measurement of the Quantity of Hydrogen Molecules]

The polyester pellets used in each of Example 3-1 and Comparative Example 3-1 were cut to give a test specimen weighing 35 mg. For the test specimen, thermal desorption spectroscopy-mass spectrometry (TDS-MS) was performed under the measurement conditions below, whereby the quantity of hydrogen molecules contained in the polyester pellets was measured. The results are presented in Table 4.

(Measurement Conditions)

- Instrument name: EMD-WA1000S/W, manufactured by ESCO, Ltd.
- The test specimen was heated on a SiC stage
- Ionization method: Electron ionization (EI)
- Measurement mode: SCAN mode
- Mass range measured (m/z): 1 to 200
- Heating conditions: 50°C to 100°C
- Temperature elevation rate: 10°C/min
- Retention temperature and retention time: Held at 100°C for 30 minutes

[Table 4]

| Table 4 | | Example 3-1 | Comparative Example 3-2 |
|---|---|---|---|
| Ethylene glycol source | | Carbon monoxide gas | Fossil fuel |
| Intrinsic viscosity [dL/g] | | 0.65 | 0.65 |
| DSC | Amount of heat of fusion during the second heating run [J/g] | 8.9 | 2.3 |
| | Amount of heat of solidification during the second cooling run [J/g] | 7.0 | 2.7 |
| Amount of Kjeldahl nitrogen [$\mu$g/g] | | 74 | 100 |
| Quantity of hydrogen molecules [molecules/g] | | $6.6 \times 10^{17}$ | $7.8 \times 10^{17}$ |

As is clear from Table 4 above, it can be understood that with pellets of polyesters in which the diol unit is ethylene glycol derived from carbon monoxide, the amount of heat of fusion and the amount of heat of solidification in DSC are greater compared with those with pellets of polyesters in which the diol unit is fossil fuel-derived ethylene glycol.
As is clear from Table 4 above, furthermore, it can also be understood that pellets of polyesters in which the diol unit is ethylene glycol derived from carbon monoxide contain a smaller amount of Kjeldahl nitrogen and a smaller quantity of hydrogen molecules compared with pellets of polyesters in which the diol unit is fossil fuel-derived ethylene glycol.

[EXAMPLE 4-1]

On one surface of the polyester film in Example 2-1, which was a base material layer, an aluminum deposition film having a thickness of 450 was formed. In this manner, a laminated film in Example 4-1 was obtained. The conditions for the formation of the vapor deposition film were as follows.

(Formation Conditions)

- Deposition source: Aluminum
- Degree of vacuum in the deposition chamber: $2 \times 10^{-3}$ mbar
- Degree of vacuum in the winding chamber: $3 \times 10^{-2}$ mbar
- Speed of transportation of film: 350 m/min

[EXAMPLE 4-2]

A hydroxyl-containing (meth)acrylic resin (number-average molecular weight, 25000; glass transition temperature, 99°C; hydroxyl value, 80 mg KOHL/g) was diluted using a solvent mixture of methyl ketone and ethyl acetate (mixing ratio, 1:1) until the solids concentration became 10% by mass, whereby a base ingredient was prepared.
An ethyl acetate solution containing tolylene diisocyanate (solids content, 75% by mass) was added as a curing agent to the base ingredient, yielding a solution for forming surface coating layers. It should be noted that the amount of the curing agent used was 10 parts by mass per 100 parts by mass of the base ingredient.
The solution for forming surface coating layers prepared as described above was applied to one surface of the polyester film in Example 2-2, which was a base material layer. The applied solution was dried, whereby a surface coating layer having a thickness of 0.5 $\mu$m was formed.
On the surface coating layer, an aluminum deposition film having a thickness of 450 was formed. In this manner, a laminated film in Example 4-2 was obtained. The conditions for the formation of the vapor deposition film were the same as in Example 4-1.

[COMPARATIVE EXAMPLE 4-1]

A laminated film in Comparative Example 4-1 was obtained in the same manner as in Example 4-1, except that the polyester film in Comparative Example 2-1 was used instead of the polyester film in Example 2-1.

[COMPARATIVE EXAMPLE 4-2]

A laminated film in Comparative Example 4-2 was obtained in the same manner as in Example 4-2, except that the polyester film in Comparative Example 2-2 was used instead of the polyester film in Example 2-2.

[Measurement of the Oxygen Transmission Rate]

The laminated films in Examples 4-1 and 4-2 and Comparative Examples 4-1 and 4-2 were cut to give test specimens. Each test specimen was set to an oxygen permeation analyzer (trade name, OX-TRAN2/20; manufactured by MOCON, Inc.) such that the base material layer side of the test specimen was the oxygen supply side. According to JIS K 7126-2: 2006 (equal-pressure method), the oxygen transmission rate (unit: cc/$m^2$·day·atm) in an environment at a temperature of 23°C and a relative humidity of 90% RH was measured.

[Measurement of the Water Vapor Transmission Rate]

The laminated films in Examples 4-1 and 4-2 and Comparative Examples 4-1 and 4-2 were cut to give test specimens. Each test specimen was set to a water vapor permeation analyzer (trade name, PERMATRAN-w 3/33; manufactured by MOCON, Inc.) such that the base material layer side of the test specimen was the water vapor supply side. According to JIS K 7129: 2008 (method B), the water vapor transmission rate (unit: g/$m^2$·day) in an environment at a temperature of 40°C and a relative humidity of 90% RH was measured.

Along with the layer configurations of the laminated films in Examples 4-1 and 4-2 and Comparative Examples 4-1 and 4-2, the results are presented in Table 5.

[Table 5]

| Table 5 | | Example 4-1 | Example 4-2 | Comparative Example 4-1 | Comparative Example 4-2 |
|---|---|---|---|---|---|
| Base material layer | Type | Polyester film in Example 2-1 | Polyester film in Example 2-2 | Polyester film in Comparative Example 2-1 | Polyester film in Comparative Example 2-2 |
| | Thickness [μm] | 12 | 12 | 12 | 12 |
| Surface coating layer | Type | - | Hydroxyl-containing (meth)acrylic resin | - | Hydroxyl-containing (meth)acrylic resin |
| | Thickness [μm] | | 0.5 | | 0.5 |
| Vapor deposition film | Type | Aluminum | Aluminum | Aluminum | Aluminum |
| | Thickness [Å] | 450 | 450 | 450 | 450 |
| Oxygen transmission rate [cc/$m^2$·day·atm] | | 0.6 | 0.6 | 0.6 | 0.6 |
| Water vapor transmission rate [g/$m^2$·day] | | 0.6 | 0.6 | 0.6 | 0.6 |

As is clear from Table 5 above, it can be understood that Examples 4-1 and 4-2, which were laminated films made using polyesters in which the diol unit was ethylene glycol made using carbon monoxide gas as a raw material, exhibit comparable oxygen transmission and water vapor transmission rates, and thus physical characteristics that are not inferior in terms of gas barrier properties, compared with Comparative Examples 4-1 and 4-2, which were laminated films made using known polyesters in which the diol unit was fossil fuel-derived ethylene glycol.

[EXAMPLE 5-1]

On one surface of a biaxially oriented polypropylene film (trade name, P2161; thickness, 20 μm; manufactured by Toyobo Co., Ltd.), a printed layer was formed by applying a urethane printing ink (trade name, FINART BM; manufactured by DIC Graphics Corporation) by gravure roll coating. The thickness of the printed layer after drying was 1 μm.
Subsequently, the surface of the biaxially oriented polypropylene film where the printed layer was formed and the surface of the laminated film in Example 4-1 where the vapor deposition film was formed were sandwich-laminated with an adhesive resin layer having a thickness of 15 μm formed by melt extrusion of low-density polyethylene (trade name, LC600A; manufactured by Japan Polyethylene Corporation) interposed therebetween.
The surface of the base material layer of the laminate obtained in such a manner and a cast polypropylene film (trade name, TAF513; thickness, 18 μm; manufactured by Okamoto Industries, Inc.) were sandwich-laminated with an adhesive resin layer having a thickness of 15 μm formed by melt extrusion of low-density polyethylene (trade name, LC600A; manufactured by Japan Polyethylene Corporation) interposed therebetween.
In such a manner, a laminate in Example 5-1, in which a support, a printed layer, an adhesive resin layer, a vapor deposition film, a base material layer, an adhesive resin layer, and a sealant layer were laminated in this order, was obtained.
The main use of the laminate in Example 5-1 is a snack food packaging bag.

[EXAMPLE 5-2]

On one surface of a biaxially oriented polypropylene film (trade name, P2161; thickness, 20 μm; manufactured by Toyobo Co., Ltd.), a printed layer was formed by applying a urethane printing ink (trade name, FINART BM; manufactured by DIC Graphics Corporation) by gravure roll coating. The thickness of the printed layer after drying was 1 μm.
Subsequently, an ester adhesive (trade name, RU-77T/H-7; manufactured by Rock Paint Co., Ltd.) was applied onto the printed layer, and the printed layer was joined to the surface of the laminated film in Example 4-1 where the vapor deposition film was formed.
After that, an ester adhesive (trade name, RU-77T/H-7; manufactured by Rock Paint Co., Ltd.) was applied onto the base material layer of the laminated film, and the base material layer was joined to a linear low-density polyethylene film (trade name, MTNST; thickness, 60 μm; manufactured by Futamura Chemical Co., Ltd.).
In such a manner, a laminate in Example 5-2, in which a support, a printed layer, an adhesive agent layer, a vapor deposition film, a base material layer, an adhesive agent layer, and a sealant layer were laminated in this order, was obtained. In the laminate in Example 5-2, the thicknesses of the adhesive agent layers were both 3 μm.
The main use of the laminate in Example 5-2 is a *furikake* (Japanese seasoning for sprinkling over rice) packaging bag.

[EXAMPLE 5-3]

On one surface of a biaxially oriented polyethylene terephthalate film (trade name, E5102; thickness, 12 μm; manufactured by Toyobo Co., Ltd.), a printed layer was formed by applying a urethane printing ink (trade name, FINART BM; manufactured by DIC Graphics Corporation) by gravure roll coating. The thickness of the printed layer after drying was 1 μm.
Subsequently, an ether adhesive (trade name, RU-3600/H-689; manufactured by Rock Paint Co., Ltd.) was applied onto the printed layer, and the printed layer was joined to the surface of the laminated film in Example 4-1 where the vapor deposition film was formed.
After that, an ether adhesive (trade name, RU-3600/H-689; manufactured by Rock Paint Co., Ltd.) was applied onto the base material layer of the laminated film, and the base material layer was joined to a linear low-density polyethylene film (trade name, MTNST; thickness, 60 μm; manufactured by Futamura Chemical Co., Ltd.).
In such a manner, a laminate in Example 5-3, in which a support, a printed layer, an adhesive agent layer, a vapor deposition film, a base material layer, an adhesive agent layer, and a sealant layer were laminated in this order, was obtained. In the laminate in Example 5-3, the thicknesses of both the adhesive agent layers were both 3 μm.
The main use of the laminate in Example 5-3 is a coffee packaging bag.

[EXAMPLE 5-4]

On one surface of a biaxially oriented polyethylene terephthalate film (trade name, E5102; thickness, 12 μm; manufactured by Toyobo Co., Ltd.), a printed layer was formed by applying a urethane printing ink (trade name, FINART BM; manufactured by DIC Graphics Corporation) by gravure roll coating. The thickness of the printed layer after drying was 1 μm.

Subsequently, an ether adhesive (trade name, RU-3600/H-689; manufactured by Rock Paint Co., Ltd.) was applied onto the printed layer, and the printed layer was joined to the surface of the laminated film in Example 4-1 where the vapor deposition film was formed.

The surface of the base material layer of the laminate obtained in such a manner and a high-density polyethylene film (trade name, HD (White); milk-white; thickness, 60 μm; manufactured by Dai Nippon Printing Co., Ltd.) were sandwich-laminated with an adhesive resin layer having a thickness of 20 μm formed by melt extrusion of low-density polyethylene (trade name, LC600A; manufactured by Japan Polyethylene Corporation) interposed therebetween.

The surface of the high-density polyethylene film in the laminate obtained in such a manner and an aluminum foil (thickness, 10 μm; manufactured by Toyo Aluminium K.K.) were sandwich-laminated with an adhesive resin layer having a thickness of 20 μm formed by melt extrusion of an ethylene-methacrylic acid copolymer (trade name, N0908C; manufactured by Dow-Mitsui Polychemicals Co., Ltd.) interposed therebetween.

The surface of the aluminum foil in the laminate obtained in such a manner and a linear low-density polyethylene film (trade name, L-100N; thickness, 50 μm; manufactured by Aicello Corporation) were sandwich-laminated with an adhesive resin layer having a thickness of 35 μm formed by melt extrusion of an ethylene-methacrylic acid copolymer (trade name, N0908C; manufactured by Dow-Mitsui Polychemicals Co., Ltd.) interposed therebetween.

On the surface of the biaxially oriented polyethylene terephthalate film in the laminate obtained in such a manner, an adhesive resin layer was formed by melt-extruding low-density polyethylene (trade name, CE4009; manufactured by Sumitomo Chemical Co., Ltd.) to a thickness of 20 μm. On the surface of this adhesive resin layer, a sealant layer was formed by melt-extruding low-density polyethylene (trade name, LC602A; manufactured by Japan Polyethylene Corporation) to a thickness of 30 μm.

In such a manner, a laminate in Example 5-4, in which a sealant layer, an adhesive resin layer, a support, a printed layer, an adhesive agent layer, a vapor deposition film, a base material layer, an adhesive resin layer, a support, an adhesive resin layer, a metal foil, an adhesive resin layer, and a sealant layer were laminated in this order, was obtained.

The main use of the laminate in Example 5-4 is a laminated tube.

[EXAMPLE 5-5]

On one surface of a biaxially oriented polyethylene terephthalate film (trade name, E5102; thickness, 12 μm; manufactured by Toyobo Co., Ltd.), a printed layer was formed by applying a urethane printing ink (trade name, FINART BM; manufactured by DIC Graphics Corporation) by gravure roll coating. The thickness of the printed layer after drying was 1 μm.

Subsequently, the surface of the biaxially oriented polyethylene terephthalate film where the printed layer was formed and the surface of the laminated film in Example 4-1 where the vapor deposition film was formed were sandwich-laminated with an adhesive resin layer having a thickness of 15 μm formed by melt extrusion of low-density polyethylene (trade name, LC600A; manufactured by Japan Polyethylene Corporation) interposed therebetween.

The surface of the base material layer of the laminate obtained in such a manner and a linear low-density polyethylene film (trade name, MTNST; thickness, 40 μm; manufactured by Futamura Chemical Co., Ltd.) were sandwich-laminated with an adhesive resin layer having a thickness of 15 μm formed by melt extrusion of low-density polyethylene (trade name, LC600A; manufactured by Japan Polyethylene Corporation) interposed therebetween.

In such a manner, a laminate in Example 5-5, in which a support, a printed layer, an adhesive resin layer, a vapor deposition film, a base material layer, an adhesive resin layer, and a sealant layer were laminated in this order, was obtained.

The main use of the laminate in Example 5-5 is a *furikake* packaging bag or a coffee packaging bag.

[EXAMPLE 5-6]

On one surface of a biaxially oriented nylon film (trade name, N1102; thickness, 15 μm; manufactured by Toyobo Co., Ltd.), a printed layer was formed by applying a urethane printing ink (trade name, FINART BM; manufactured by DIC Graphics Corporation) by gravure roll coating. The thickness of the printed layer after drying was 1 μm.

Subsequently, an ether adhesive (trade name, RU-3600/H-689; manufactured by Rock Paint Co., Ltd.) was applied onto the printed layer, and the printed layer was joined to the surface of the laminated film in Example 4-1 where the vapor deposition film was formed.

After that, an ether adhesive (trade name, RU-3600/H-689; manufactured by Rock Paint Co., Ltd.) was applied onto the base material layer of the laminated film, and the base material layer was joined to a linear low-density polyethylene film (trade name, MTNST; thickness, 40 μm; manufactured by Futamura Chemical Co., Ltd.).

In such a manner, a laminate in Example 5-6, in which a support, a printed layer, an adhesive agent layer, a vapor deposition film, a base material layer, an adhesive agent layer, and a sealant layer were laminated in this order, was obtained. In the laminate in Example 5-6, the thicknesses of the adhesive agent layers were both 3 μm.

The main use of the laminate in Example 5-6 is a refill container.

[EXAMPLE 5-7]

A laminate in Example 5-7 was obtained in the same manner as in Example 5-5, except that a cast polypropylene film (trade name, RS512; thickness, 30 μm; manufactured by Idemitsu Unitech Co., Ltd.) was used instead of a linear low-density polyethylene film.
The main use of the laminate in Example 5-7 is individual coffee packaging.

[EXAMPLE 5-8]

On one surface of a biaxially oriented polyethylene terephthalate film (trade name, E5102; thickness, 12 μm; manufactured by Toyobo Co., Ltd.), a printed layer was formed by applying a urethane printing ink (trade name, FINART BM; manufactured by DIC Graphics Corporation) by gravure roll coating. The thickness of the printed layer after drying was 1 μm.
Subsequently, an ether adhesive (trade name, RU-3600/H-689; manufactured by Rock Paint Co., Ltd.) was applied onto the printed layer, and the printed layer was joined to the surface of the laminated film in Example 4-1 where the vapor deposition film was formed. The thickness of the adhesive agent layer formed by joining was 3 μm.
After that, a solventless ether adhesive (trade name, A246A/A248B; manufactured by Mitsui Chemicals, Inc.) was applied onto the base material layer of the laminated film, and the base material layer was joined to a linear low-density polyethylene film (trade name, MTNST; thickness, 40 μm; manufactured by Futamura Chemical Co., Ltd.). The thickness of the adhesive agent layer formed by joining was 1 μm.
In such a manner, a laminate in Example 5-8, in which a support, a printed layer, an adhesive agent layer, a vapor deposition film, a base material layer, an adhesive agent layer, and a sealant layer were laminated in this order, was obtained.
The main use of the laminate in Example 5-8 is individual coffee packaging.

[EXAMPLE 5-9]

A laminate in Example 5-9 was obtained in the same manner as in Example 5-3, except that a cast polypropylene film (trade name, GLC; thickness, 60 μm; manufactured by Mitsui Chemicals Tohcello, Inc.) was used instead of a linear low-density polyethylene film.
The main use of the laminate in Example 5-9 is individual coffee packaging.

[Measurement of Lamination Strength]

For each of the laminates in Examples 5-1 to 5-3 and 5-5 to 5-9, lamination strength between the laminated film and the support was measured according to JIS Z 0238: 1998.
Specifically, the laminate was cut into a strip shape such that the length in the transverse direction of the base material layer constituting the laminated film was 15 mm, and that the length in the machine direction of the base material layer constituting the laminated film was greater than the length in the transverse direction, yielding a test specimen. For the resulting test specimen, the laminated film and the support were partially separated. Using a tensile tester (trade name, TENSILON STA-1150; manufactured by A&D Co., Ltd.), the laminated film and the support in the test specimen were held by pinching each of them with a chuck of the tensile tester. Then the film and the support were pulled apart by the T-peel method in the vertical direction for 10 mm at 50 mm/min, and the average tensile strength was measured.
The same operation was attempted ten times, and the average of the measurements from the ten attempts was reported as the lamination strength between the laminated film and the support (unit: N/15 mm width).
For the laminate in Example 5-4, lamination strength between the laminated film and the biaxially oriented polyethylene terephthalate film was measured in the same manner.
Along with the layer configurations and main uses of the laminates in Examples 5-1 to 5-9, the results are presented in Table 6.

[Table 6]

| Table 6 | | | Example 5-1 | Example 5-2 | Example 5-3 | Example 5-4 | Example 5-5 | Example 5-6 | Example 5-7 | Example 5-8 | Example 5-9 |
|---|---|---|---|---|---|---|---|---|---|---|---|
| Layer configuration | Sealant layer | Type | - | - | - | LDPE | - | - | - | - | - |
| | | Thickness [μm] | | | | 30 | | | | | |
| | Adhesive resin layer | Type | - | - | - | LDPE | - | - | - | - | - |
| | | Thickness [μm] | | | | 20 | | | | | |
| | Support | Type | OPP | OPP | PET | PET | PET | ONY | PET | ONY | PET |
| | | Thickness [μm] | 20 | 20 | 12 | 12 | 12 | 15 | 12 | 15 | 12 |
| | Printed layer | Type | Urethane printing ink | Urethane printing ink | Urethane printing ink | Urethane printing ink | Urethane printing ink | Urethane printing ink | Urethane printing ink | Urethane printing ink | Urethane printing ink |
| | | Thickness [μm] | 1 | 1 | 1 | 1 | 1 | 1 | 1 | 1 | 1 |
| | Adhesive resin layer | Type | LDPE | - | - | - | LDPE | - | LDPE | - | - |
| | | Thickness [μm] | 15 | | | | 15 | | 15 | | |
| | Adhesive *agent layer* | Type | - | Ester adhesive | Ether adhesive | Ether adhesive | - | Ether adhesive | - | Ether adhesive | Ether adhesive |
| | | Thickness [μm] | | 3 | 3 | 3 | | 3 | | 3 | 3 |
| | Vapor deposition film | Type | Aluminum | Aluminum | Aluminum | Aluminum | Aluminum | Aluminum | Aluminum | Aluminum | Aluminum |
| | | Thickness [Å] | 450 | 450 | 450 | 450 | 450 | 450 | 450 | 450 | 450 |
| | Base material layer | Type | Polyester film in Example 2-1 | Polyester film in Example 2-1 | Polyester film in Example 2-1 | Polyester film in Example 2-1 | Polyester film in Example 2-1 | Polyester film in Example 2-1 | Polyester film in Example 2-1 | Polyester film in Example 2-1 | Polyester film in Example 2-1 |
| | | Thickness [μm] | 12 | 12 | 12 | 12 | 12 | 12 | 12 | 12 | 12 |

(continued)

| Table 6 | | | Example 5-1 | Example 5-2 | Example 5-3 | Example 5-4 | Example 5-5 | Example 5-6 | Example 5-7 | Example 5-8 | Example 5-9 |
|---|---|---|---|---|---|---|---|---|---|---|---|
| | Adhesive agent layer | Type | - | Ester adhesive | Ether adhesive | - | - | Ether adhesive | - | Solventless ether adhesive | Ether adhesive |
| | | Thickness [μm] | | 3 | 3 | | | 3 | | 1 | 3 |
| | Adhesive resin | Type | LDPE | - | - | LDPE | LDPE | - | LDPE | - | - |
| | | Thickness [μm] | 15 | | | 20 | 15 | | 15 | | |
| | Support | Type | - | - | - | HDPE | - | - | - | - | - |
| | | Thickness [μm] | | | | 60 | | | | | |
| | Adhesive resin layer | Type | - | - | -- | EMAA | - | - | - | - | - |
| | | Thickness [μm] | | | | 20 | | | | | |
| | Metal foil | Type | - | - | - | Aluminum foil | - | - | - | - | - |
| | | Thickness [μm] | | | | 10 | | | | | |
| | Adhesive resin layer | Type | - | - | - | EMAA | - | - | - | - | - |
| | | Thickness [μm] | | | | 35 | | | | | |
| | Sealant layer | Type | CPP | LLDPE | LLDPE | LLDPE | LLDPE | LLDPE | CPP | LLDPE | CPP |
| | | Thickness [μm] | 18 | 60 | 60 | 50 | 40 | 40 | 30 | 40 | 60 |
| Lamination strength [N/15 mm width] | | | 1.5 | 2.7 | 2.1 | 2.1 | 1.6 | 2.1 | 1.6 | 2.1 | 2.1 |

(continued)

| Table 6 | Example 5-1 | Example 5-2 | Example 5-3 | Example 5-4 | Example 5-5 | Example 5-6 | Example 5-7 | Example 5-8 | Example 5-9 |
|---|---|---|---|---|---|---|---|---|---|
| Main use | Snack food packaging | *Furikake* packaging | Coffee packaging | Laminated tube | *Furikake* packaging Coffee packaging | Refill container | Individual coffee packaging | Individual coffee packaging | Individual coffee packaging |

ONY: Biaxially oriented nylon film
PET: Polyethylene terephthalate film
OPP: Biaxially oriented polypropylene film
CPP: Cast polypropylene film
EMAA: Ethylene-methacrylic acid copolymer
HDPE: High-density polyethylene
LDPE: Low-density polyethylene
LLDPE: Linear low-density polyethylene

As is clear from Examples 5-1 to 5-9 above, it can be understood that laminates can be manufactured from laminated films made using polyesters in which the diol unit is ethylene glycol made using carbon monoxide gas as a raw material.

The lamination strengths measured with the laminates in Examples 5-1 to 5-9, furthermore, were comparable to lamination strength measured with a laminate made using a known polyester in which the diol unit is fossil fuel-derived ethylene glycol. It can be, therefore, understood that the laminates in Examples 5-1 to 5-9 exhibit physical characteristics that are not inferior in terms of mechanical properties.

[EXAMPLE 6-1]

On one surface of the polyester film in Example 2-1, which was a base material layer, an aluminum oxide deposition film having a thickness of 12 nm was formed using a continuous vapor deposition film formation apparatus that included a pretreatment section, in which an oxygen plasma pretreatment apparatus that had been in practical use was disposed, and a film formation section separated from the pretreatment section. In the pretreatment section, oxygen plasma pretreatment was applied by introducing plasma through a plasma supply nozzle under the conditions specified below while applying tension to the base material layer by the roll-to-roll method. In the film formation section, to which the film was transported continuously, the aluminum oxide deposition film was formed (PVD) on the surface treated with oxygen plasma under the conditions specified below using reactive resistance heating as heating means in vacuum deposition. In this manner, a laminated film in Example 6-1 was obtained.

(Formation Conditions)

(Oxygen Plasma Pretreatment Conditions)

- Plasma intensity: 200 W·sec/$m^2$
- Plasma formation gas ratio: Oxygen:argon = 2:1
- Pretreatment drum-plasma supply nozzle applied voltage: 340 V

(Film Formation Conditions)

- Transportation speed: 400 m/min
- Oxygen gas supply: 20000 sccm

[EXAMPLE 6-2]

A solution for forming surface coating layers prepared in the same manner as in Example 4-2 was applied to one surface of the polyester film in Example 2-1, which was a base material layer. The applied solution was dried, whereby a surface coating layer having a thickness of 0.5 μm was formed.

On the surface coating layer, an aluminum oxide deposition film having a thickness of 12 nm was formed. In this manner, a laminated film in Example 6-2 was obtained. The conditions for the formation of the vapor deposition film were the same as in Example 6-1.

[EXAMPLE 6-3]

To a liquid mixture of composition a prepared according to the composition table presented below, composed of polyvinyl alcohol, isopropyl alcohol, and deionized water, a hydrolysis solution of composition b, composed of ethyl silicate, a silane coupling agent, isopropyl alcohol, hydrochloric acid, and deionized water, was added. The resulting mixture was stirred, yielding a colorless and transparent barrier coating solution.

Composition Table

a

(continued)

| | | |
|---|---|---|
| | Polyvinyl alcohol | 2.30 |
| | Isopropyl alcohol | 2.70 |
| | Water | 51.20 |
| b | | |
| | Ethyl silicate | 16.60 |
| | Silane coupling agent | 0.20 |
| | Isopropyl alcohol | 3.90 |
| | 0.5 N aqueous solution of hydrochloric acid | 0.50 |
| | Water | 22.60 |
| | Total | 100.00 (wt%) |

The barrier coating solution (gas barrier composition) prepared as described above was coated onto the aluminum oxide deposition film in the laminated film in Example 6-1, and the workpiece was subjected to heating treatment at a line speed of 100 m/min at a drying temperature of 180°C, whereby a gas barrier coating film having a thickness of 0.3 μm (dry state) was formed. In this manner, a laminated film in Example 6-3 was obtained.

[EXAMPLE 6-4]

A barrier coating solution prepared in the same manner as in Example 6-3 was coated onto the aluminum oxide deposition film in the laminated film in Example 6-2, and the workpiece was subjected to heating treatment at a line speed of 100 m/min at a drying temperature of 180°C, whereby a gas barrier coating film having a thickness of 0.3 μm (dry state) was formed. In this manner, a laminated film in Example 6-4 was obtained.

[EXAMPLE 6-5]

On one surface of the polyester film in Example 2-1, which was a base material layer, a silicon oxide deposition film having a thickness of 20 nm was formed (PVD) using an induction-heating vacuum film formation apparatus that had been in practical use and that included a plasma gun, while tension was applied to the base material by the roll-to-roll method. In this manner, a laminated film in Example 6-5 was obtained. The conditions for the formation of the vapor deposition film were as follows.

(Formation Conditions)

(Plasma Irradiation Conditions)

- Line speed: 30 m/minute
- Degree of vacuum: $1.7 \times 10^{-2}$ Pa
- Power: 5.7 kw
- Acceleration voltage: 151 V
- Ar gas flow rate: 7.5 sccm

(Film Formation Conditions)

- Material deposited: SiO
- Reactive gas: $O_2$

- Reactive gas flow rate: 100 sccm

[EXAMPLE 6-6]

A solution for forming surface coating layers prepared in the same manner as in Example 4-2 was applied to one surface of the polyester film in Example 2-1, which was a base material layer. The applied solution was dried, whereby a surface coating layer having a thickness of 0.5 μm was formed.

On the surface coating layer, a silicon oxide deposition film having a thickness of 20 nm was formed. In this manner, a laminated film in Example 6-6 was obtained. The conditions for the formation of the vapor deposition film were the same as in Example 6-5.

[EXAMPLE 6-7]

A barrier coating solution prepared in the same manner as in Example 6-3 was coated onto the silicon oxide deposition film in the laminated film in Example 6-5, and the workpiece was subjected to heating treatment at a line speed of 100 m/min at a drying temperature of 180°C, whereby a gas barrier coating film having a thickness of 0.3 μm (dry state) was formed. In this manner, a laminated film in Example 6-7 was obtained.

[EXAMPLE 6-8]

A barrier coating solution prepared in the same manner as in Example 6-3 was coated onto the silicon oxide deposition film in the laminated film in Example 6-6, and the workpiece was subjected to heating treatment at a line speed of 100 m/min at a drying temperature of 180°C, whereby a gas barrier coating film having a thickness of 0.3 μm (dry state) was formed. In this manner, a laminated film in Example 6-8 was obtained.

[COMPARATIVE EXAMPLE 6-1]

A laminated film in Comparative Example 6-1 was obtained in the same manner as in Example 6-1, except that the polyester film in Comparative Example 2-1 was used instead of the polyester film in Example 2-1.

[COMPARATIVE EXAMPLE 6-2]

A laminated film in Comparative Example 6-2 was obtained in the same manner as in Example 6-2, except that the polyester film in Comparative Example 2-1 was used instead of the polyester film in Example 2-1.

[COMPARATIVE EXAMPLE 6-3]

A laminated film in Comparative Example 6-3 was obtained in the same manner as in Example 6-3, except that the polyester film in Comparative Example 2-1 was used instead of the polyester film in Example 2-1.

[COMPARATIVE EXAMPLE 6-4]

A laminated film in Comparative Example 6-4 was obtained in the same manner as in Example 6-4, except that the polyester film in Comparative Example 2-1 was used instead of the polyester film in Example 2-1.

[COMPARATIVE EXAMPLE 6-5]

A laminated film in Comparative Example 6-5 was obtained in the same manner as in Example 6-5, except that the polyester film in Comparative Example 2-1 was used instead of the polyester film in Example 2-1.

[COMPARATIVE EXAMPLE 6-6]

A laminated film in Comparative Example 6-6 was obtained in the same manner as in Example 6-6, except that the polyester film in Comparative Example 2-1 was used instead of the polyester film in Example 2-1.

[COMPARATIVE EXAMPLE 6-7]

A laminated film in Comparative Example 6-7 was obtained in the same manner as in Example 6-7, except that

the polyester film in Comparative Example 2-1 was used instead of the polyester film in Example 2-1.

[COMPARATIVE EXAMPLE 6-8]

A laminated film in Comparative Example 6-8 was obtained in the same manner as in Example 6-8, except that the polyester film in Comparative Example 2-1 was used instead of the polyester film in Example 2-1.

[Measurement of the Oxygen Transmission Rate]

The laminated films in Examples 6-1 to 6-8 and Comparative Examples 6-1 to 6-8 were cut to give test specimens. Each test specimen was set to an oxygen permeation analyzer (trade name, OX-TRAN2/20; manufactured by MOCON, Inc.) such that the base material layer side of the test specimen was the oxygen supply side. According to JIS K 7126-2: 2006 (equal-pressure method), the oxygen transmission rate (unit: cc/$m^2$·day·atm) in an environment at a temperature of 23°C and a relative humidity of 90% RH was measured.

[Measurement of the Water Vapor Transmission Rate]

The laminated films in Examples 6-1 to 6-8 and Comparative Examples 6-1 to 6-8 were cut to give test specimens. Each test specimen was set to a water vapor permeation analyzer (trade name, PERMATRAN-w 3/33; manufactured by MOCON, Inc.) such that the base material layer side of the test specimen was the water vapor supply side. According to JIS K 7129: 2008 (method B), the water vapor transmission rate (unit: g/$m^2$·day) in an environment at a temperature of 40°C and a relative humidity of 90% RH was measured.

Along with the layer configurations of the laminated films in Examples 6-1 to 6-8 and Comparative Examples 6-1 to 6-8, the results are presented in Tables 7 and 8.

[Table 7]

| Table 7 | | Example 6-1 | Example 6-2 | Example 6-3 | Example 6-4 | Example 6-5 | Example 6-6 | Example 6-7 | Example 6-8 |
|---|---|---|---|---|---|---|---|---|---|
| Base material layer | Type | Polyester film in Example 2-1 | Polyester film in Example 2-1 | Polyester film in Example 2-1 | Polyester film in Example 2-1 | Polyester film in Example 2-1 | Polyester film in Example 2-1 | Polyester film in Example 2-1 | Polyester film in Example 2-1 |
| | Thickness [$\mu$m] | 12 | 12 | 12 | 12 | 12 | 12 | 12 | 12 |
| Surface coating layer | Type | - | Hydroxyl-containing (meth)acrylic resin | - | Hydroxyl-containing (meth) acrylic resin | - | Hydroxyl-containing (meth)acrylic resin | - | Hydroxyl-containing (meth)acrylic resin |
| | Thickness [$\mu$m] | | 0.5 | | 0.5 | | 0.5 | | 0.5 |
| Vapor deposition film | Type | Aluminum oxide | Aluminum oxide | Aluminum oxide | Aluminum oxide | Silicon oxide | Silicon oxide | Silicon oxide | Silicon oxide |
| | Thickness [nm] | 12 | 12 | 12 | 12 | 20 | 20 | 20 | 20 |
| Gas barrier coating film | Composition | - | - | Ethyl silicate Polyvinyl alcohol Silane coupling agent | Ethyl silicate Polyvinyl alcohol Silane coupling agent | - | - | Ethyl silicate Polyvinyl alcohol Silane coupling agent | Ethyl silicate Polyvinyl alcohol Silane coupling agent |
| | Thickness [$\mu$m] | | | 0.3 | 0.3 | | | 0.3 | 0.3 |
| Oxygen transmission rate [cc/$m^2$·day·atm] | | 0.4 | 0.4 | 0.2 | 0.2 | 0.4 | 0.4 | 0.2 | 0.2 |
| Water vapor transmission rate [g/$m^2$·day] | | 0.6 | 0.6 | 0.4 | 0.4 | 0.9 | 0.9 | 0.7 | 0.7 |

[Table 8]

| Table 8 | | Comparative Example 6-1 | Comparative Example 6-2 | Comparative Example 6-3 | Comparative Example 6-4 | Comparative Example 6-5 | Comparative Example 6-6 | Comparative Example 6-7 | Comparative Example 6-8 |
|---|---|---|---|---|---|---|---|---|---|
| Base material layer | Type | Polyester film in Comparative Example 2-1 | Polyester film in Comparative Example 2-1 | Polyester film in Comparative Example 2-1 | Polyester film in Comparative Example 2-1 | Polyester film in Comparative Example 2-1 | Polyester film in Comparative Example 2-1 | Polyester film in Comparative Example 2-1 | Polyester film in Comparative Example 2-1 |
| | Thickness [μm] | 12 | 12 | 12 | 12 | 12 | 12 | 12 | 12 |
| Surface coating layer | Type | - | Hydroxyl-containing (meth) acrylic resin | - | Hydroxyl-containing (meth) acrylic resin | - | Hydroxyl-containing (meth) acrylic resin | - | Hydroxyl-containing (meth) acrylic resin |
| | Thickness [μm] | | 0.5 | | 0.5 | | 0.5 | | 0.5 |
| Vapor deposition film | Type | Aluminum oxide | Aluminum oxide | Aluminum oxide | Aluminum oxide | Silicon oxide | Silicon oxide | Silicon oxide | Silicon oxide |
| | Thickness [nm] | 12 | 12 | 12 | 12 | 20 | 20 | 20 | 20 |
| Gas barrier coating film | Composition | - | - | Ethyl silicate Polyvinyl alcohol Silane coupling agent | Ethyl silicate Polyvinyl alcohol Silane coupling agent | - | - | Ethyl silicate Polyvinyl alcohol Silane coupling agent | Ethyl silicate Polyvinyl alcohol Silane coupling agent |
| | Thickness [μm] | | | 0.3 | 0.3 | | | 0.3 | 0.3 |
| Oxygen transmission rate [cc/m$^2$·day·atm] | | 0.4 | 0.4 | 0.2 | 0.2 | 0.4 | 0.4 | 0.2 | 0.2 |
| Water vapor transmission rate [g/m$^2$·day] | | 0.6 | 0.6 | 0.4 | 0.4 | 0.9 | 0.9 | 0.7 | 0.7 |

As is clear from Tables 7 and 8 above, it can be understood that Examples 6-1 to 6-8, which were laminated films made using polyesters in which the diol unit was ethylene glycol made using carbon monoxide gas as a raw material, exhibit comparable oxygen transmission and water vapor transmission rates, and thus physical characteristics that are not inferior in terms of gas barrier properties, compared with Comparative Examples 6-1 to 6-8, which were laminated films made using known polyesters in which the diol unit was fossil fuel-derived ethylene glycol.

[EXAMPLE 7-1]

On one surface of a biaxially oriented polyethylene terephthalate film (trade name, E5202; thickness, 12 μm; manufactured by Toyobo Co., Ltd.), a printed layer was formed by applying a urethane printing ink (trade name, FINART BM; manufactured by DIC Graphics Corporation) by gravure roll coating. The thickness of the printed layer after drying was 1 μm.

Subsequently, the surface of the biaxially oriented polyethylene terephthalate film where the printed layer was formed and a low-density polyethylene film (trade name, DNW-20 Blue; milk-white; thickness, 100 μm; manufactured by Dai Nippon Printing Co., Ltd.) were sandwich-laminated with an adhesive resin layer having a thickness of 25 μm formed by melt extrusion of low-density polyethylene (trade name, LC600A; manufactured by Japan Polyethylene Corporation) interposed therebetween.

After that, an ether adhesive (trade name, RU-3600/H-689; manufactured by Rock Paint Co., Ltd.) was applied onto the low-density polyethylene film, and the film was joined to the surface of the laminated film in Example 6-1 where the vapor deposition film was formed. The thickness of the adhesive agent layer formed by joining was 3 μm.

The surface of the base material layer of the laminate obtained in such a manner and a linear low-density polyethylene film (trade name, SP100AS; thickness, 80 μm; manufactured by Dai Nippon Printing Co., Ltd.) were sandwich-laminated with an adhesive resin layer having a thickness of 25 μm formed by melt extrusion of an ethylene-methacrylic acid copolymer (trade name, N0908C; manufactured by Dow-Mitsui Polychemicals Co., Ltd.) interposed therebetween.

The surface of the biaxially oriented polyethylene terephthalate film in the laminate obtained in such a manner and a linear low-density polyethylene film (trade name, L-100N; thickness, 80 μm; manufactured by Aicello Corporation) were sandwich-laminated with an adhesive resin layer having a thickness of 25 μm formed by melt extrusion of low-density polyethylene (trade name, LC600A; manufactured by Japan Polyethylene Corporation) interposed therebetween.

In such a manner, a laminate in Example 7-1, in which a sealant layer, an adhesive resin layer, a support, a printed layer, an adhesive resin layer, a support, an adhesive agent layer, a vapor deposition film, a base material layer, an adhesive resin layer, and a sealant layer were laminated in this order, was obtained.

The main use of the laminate in Example 7-1 is a laminated tube.

[EXAMPLE 7-2]

On the surface of the laminated film in Example 6-1 where the vapor deposition film was formed, a printed layer was formed by applying a urethane printing ink (trade name, FINART BM; manufactured by DIC Graphics Corporation) by gravure roll coating. The thickness of the printed layer after drying was 1 μm.

Subsequently, an ester adhesive (trade name, RU-004/H-1; manufactured by Rock Paint Co., Ltd.) was applied onto the printed layer, and the printed layer was joined to a biaxially oriented nylon film (trade name, N1202; thickness, 15 μm; manufactured by Toyobo Co., Ltd.).

After that, an ester adhesive (trade name, RU-004/H-1; manufactured by Rock Paint Co., Ltd.) was applied onto the biaxially oriented nylon film, and the film was joined to a linear low-density polyethylene film (trade name, EP-7R; thickness, 60 μm; manufactured by Dai Nippon Printing Co., Ltd.).

In such a manner, a laminate in Example 7-2, in which a base material layer, a vapor deposition film, a printed layer, an adhesive agent layer, a support, an adhesive agent layer, and a sealant layer were laminated in this order, was obtained. In the laminate in Example 7-2, the thicknesses of the adhesive agent layers were both 3 μm.

The main use of the laminate in Example 7-2 is a refill container.

[EXAMPLE 7-3]

A laminate in Example 7-3 was obtained in the same manner as in Example 7-2, except that a cast polypropylene film (trade name, ZK207; thickness, 60 μm; manufactured by Toray Advanced Film Co., Ltd.) was used instead of a linear low-density polyethylene film.

The main use of the laminate in Example 7-3 is microwaveable packaging.

[EXAMPLE 7-4]

On the surface of the laminated film in Example 6-1 where the vapor deposition film was formed, a printed layer was formed by applying a urethane printing ink (trade name, FINART BM; manufactured by DIC Graphics Corporation) by gravure roll coating. The thickness of the printed layer after drying was 1 μm.
Subsequently, an ester adhesive (trade name, RU-004/H-1; manufactured by Rock Paint Co., Ltd.) was applied onto the printed layer, and the printed layer was joined to a biaxially oriented polypropylene film (trade name, P2161; thickness, 20 μm; manufactured by Toyobo Co., Ltd.).
After that, an ester adhesive (trade name, RU-004/H-1; manufactured by Rock Paint Co., Ltd.) was applied onto the base material layer of the laminated film, and the base material layer was joined to a linear low-density polyethylene film (trade name, MTNST; thickness, 40 μm; manufactured by Futamura Chemical Co., Ltd.).
In such a manner, a laminate in Example 7-4, in which a support, an adhesive agent layer, a printed layer, a vapor deposition film, a base material layer, an adhesive agent layer, and a sealant layer were laminated in this order, was obtained. In the laminate in Example 7-4, the thicknesses of the adhesive agent layers were both 3 μm.
The main use of the laminate in Example 7-4 is a deep-draw lid.

[EXAMPLE 7-5]

On the surface of the laminated film in Example 6-1 where the vapor deposition film was formed, a printed layer was formed by applying a urethane printing ink (trade name, FINART BM; manufactured by DIC Graphics Corporation) by gravure roll coating. The thickness of the printed layer after drying was 1 μm.
Subsequently, an ether adhesive (trade name, RU-3600/H-689; manufactured by Rock Paint Co., Ltd.) was applied onto the printed layer, and the printed layer was joined to a linear low-density polyethylene film (trade name, MTNST; thickness, 40 μm; manufactured by Futamura Chemical Co., Ltd.). The thickness of the adhesive agent layer formed by joining was 3 μm.
In such a manner, a laminate in Example 7-5, in which a base material layer, a vapor deposition film, a printed layer, an adhesive agent layer, and a sealant layer were laminated in this order, was obtained.
The main use of the laminate in Example 7-5 is wet-wipe packaging or *tsukemono* (Japanese pickled vegetables) packaging.

[EXAMPLE 7-6]

On one surface of a biaxially oriented polypropylene film (trade name, P2161; thickness, 20 μm; manufactured by Toyobo Co., Ltd.), a printed layer was formed by applying a urethane printing ink (trade name, FINART BM; manufactured by DIC Graphics Corporation) by gravure roll coating. The thickness of the printed layer after drying was 1 μm.
Subsequently, an ester adhesive (trade name, RU-004/H-1; manufactured by Rock Paint Co., Ltd.) was applied onto the printed layer, and the printed layer was joined to the surface of the laminated film in Example 6-1 where the vapor deposition film was formed. The thickness of the adhesive agent layer formed by joining was 3 μm.
On the surface of the base material layer of the laminate obtained in such a manner, an adhesive resin layer having a thickness of 15 μm and a sealant layer having a thickness of 30 μm were formed by coextruding low-density polyethylene (trade name, LC600A; manufactured by Japan Polyethylene Corporation) and linear low-density polyethylene (trade name, 15100C; manufactured by Prime Polymer Co., Ltd.).
In such a manner, a laminate in Example 7-6, in which a support, a printed layer, an adhesive agent layer, a vapor deposition film, a base material layer, an adhesive resin layer, and a sealant layer were laminated in this order, was obtained.
The main use of the laminate in Example 7-6 is *katsuobushi* (dried bonito) packaging.

[EXAMPLE 7-7]

On the surface of the laminated film in Example 6-1 where the vapor deposition film was formed, a printed layer was formed by applying a urethane printing ink (trade name, FINART BM; manufactured by DIC Graphics Corporation) by gravure roll coating. The thickness of the printed layer after drying was 1 μm.
Subsequently, an ester adhesive (trade name, RU-004/H-1; manufactured by Rock Paint Co., Ltd.) was applied onto the printed layer, and the printed layer was joined to a low-density polyethylene film (trade name, SR-WN2 White; milk-white; thickness, 130 μm; manufactured by Dai Nippon Printing Co., Ltd.).
After that, an ester adhesive (trade name, RU-004/H-1; manufactured by Rock Paint Co., Ltd.) was applied onto the base material layer of the laminated film, and the base material layer was joined to a low-density polyethylene film (trade name, SR-WN2AS; thickness, 130 μm; manufactured by Dai Nippon Printing Co., Ltd.).

In such a manner, a laminate in Example 7-7, in which a sealant layer, an adhesive agent layer, a base material layer, a vapor deposition film, a printed layer, an adhesive agent layer, and a sealant layer were laminated in this order, was obtained. In the laminate in Example 7-7, the thicknesses of the adhesive agent layers were both 3 μm.
The main use of the laminate in Example 7-7 is a laminated tube.

[EXAMPLE 7-8]

A laminate in Example 7-8 was obtained in the same manner as in Example 7-3, except that a biaxially oriented polyethylene terephthalate film (trade name, E5202; thickness, 12 μm; manufactured by Toyobo Co., Ltd.) was used instead of a biaxially oriented nylon film, and that the cast polypropylene film was one having a thickness of 70 μm.
The main use of the laminate in Example 7-8 is microwaveable packaging.

[EXAMPLE 7-9]

Two laminated films as in Example 6-1 were prepared.
On the surface of a first laminated film where the vapor deposition film was formed, a printed layer was formed by applying a urethane printing ink (trade name, FINART BM; manufactured by DIC Graphics Corporation) by gravure roll coating. The thickness of the printed layer after drying was 1 μm.
Subsequently, an ester adhesive (trade name, RU-77T/H-7; manufactured by Rock Paint Co., Ltd.) was applied onto the printed layer, and the printed layer was joined to the surface of the second laminated film where the vapor deposition film was formed.
After that, an ester adhesive (trade name, RU-77T/H-7; manufactured by Rock Paint Co., Ltd.) was applied onto the base material layer of the second laminated film, and the base material layer was joined to a linear low-density polyethylene film (trade name, XMTN; thickness, 80 μm; manufactured by Futamura Chemical Co., Ltd.).
In such a manner, a laminate in Example 7-9, in which a base material layer, a vapor deposition film, a printed layer, an adhesive agent layer, a vapor deposition film, a base material layer, an adhesive agent layer, and a sealant layer were laminated in this order, was obtained. In the laminate in Example 7-9, the thicknesses of the adhesive agent layers were both 3 μm.
The main use of the laminate in Example 7-9 is artificial dialysis fluid packaging.

[EXAMPLE 7-10]

A laminate in Example 7-10 was obtained in the same manner as in Example 7-9, except that a biaxially oriented nylon film (trade name, N1202; thickness, 15 μm; manufactured by Toyobo Co., Ltd.) was used instead of the second laminated film.
The main use of the laminate in Example 7-10 is a refill container, ink packaging, or an aerosol spray container.

[Measurement of Lamination Strength]

For each of the laminates in Examples 7-2 to 7-4, 7-6, 7-8, and 7-10, lamination strength between the laminated film and the support was measured according to JIS Z 0238: 1998.
Specifically, the laminate was cut into a strip shape such that the length in the transverse direction of the base material layer constituting the laminated film was 15 mm, and that the length in the machine direction of the base material layer constituting the laminated film was greater than the length in the transverse direction, yielding a test specimen. For the resulting test specimen, the laminated film and the support were partially separated. Using a tensile tester (trade name, TENSILON STA-1150; manufactured by A&D Co., Ltd.), the laminated film and the support in the test specimen were held by pinching each of them with a chuck of the tensile tester. Then the film and the support were pulled apart by the T-peel method in the vertical direction for 10 mm at 50 mm/min, and the average tensile strength was measured.
The same operation was attempted ten times, and the average of the measurements from the ten attempts was reported as the lamination strength between the laminated film and the support (unit: N/15 mm width).
For the laminate in Example 7-1, lamination strength between the laminated film and the low-density polyethylene film (trade name, DNW-20 Blue; milk-white; thickness, 100 μm; manufactured by Dai Nippon Printing Co., Ltd.) was measured in the same manner.
For the laminate in Example 7-5, lamination strength between the laminated film and the linear low-density polyethylene film (trade name, MTNST; thickness, 40 μm; manufactured by Futamura Chemical Co., Ltd.) was measured in the same manner.
For the laminate in Example 7-7, lamination strength between the laminated film and the low-density polyethylene film (trade name, SR-WN2 White; milk-white; thickness, 130 μm; manufactured by Dai Nippon Printing Co., Ltd.)

was measured in the same manner.

For the laminate in Example 7-9, lamination strength between the two laminated films was measured in the same manner.

Along with the layer configurations and main uses of the laminates in Examples 7-1 to 7-10, the results are presented in Table 9.

[Table 9]

| Table 9 | | | Example 7-1 | Example 7-2 | Example 7-3 | Example 7-4 | Example 7-5 | Example 7-6 | Example 7-7 | Example 7-8 | Example 7-9 | Example 7-10 |
|---|---|---|---|---|---|---|---|---|---|---|---|---|
| Layer configuration | Sealant layer | Type | LLDPE | - | - | - | - | - | LLDPE | - | - | - |
| | | Thickness [μm] | 80 | | | | | | 130 | | | |
| | Adhesive resin layer | Type | LDPE | - | - | - | - | - | - | - | - | - |
| | | Thickness [μm] | 25 | | | | | | | | | |
| | Support | Type | PET | - | - | OPP | - | OPP | - | - | - | - |
| | | Thickness [μm] | 12 | | | 20 | | 20 | | | | |
| | Adhesive agent layer | Type | - | - | - | Ester adhesive | - | - | Ester adhesive | - | - | - |
| | | Thickness [μm] | | | | 3 | | | 3 | | | |
| | Base material layer | Type | - | Polyester film in Example 2-1 | Polyester film in Example 2-1 | - | Polyester film in Example 2-1 | - | Polyester film in Example 2-1 | Polyester film in Example 2-1 | Polyester film in Example 2-1 | Polyester film in Example 2-1 |
| | | Thickness [μm] | | 12 | 12 | | 12 | | 12 | 12 | 12 | 12 |
| | Vapor deposition film | Type | - | Aluminum oxide | Aluminum oxide | - | Aluminum oxide | - | Aluminum oxide | Aluminum oxide | Aluminum oxide | Aluminum oxide |
| | | Thickness [nm] | | 12 | 12 | | 12 | | 12 | 12 | 12 | 12 |
| | Printed layer | Type | Urethane printing ink | Urethane printing ink | Urethane printing ink | Urethane printing ink | Urethane printing ink | Urethane printing ink | Urethane printing ink | Urethane printing ink | Urethane printing ink | Urethane printing ink |
| | | Thickness [μm] | 1 | 1 | 1 | 1 | 1 | 1 | 1 | 1 | 1 | 1 |
| | Adhesive agent layer | Type | - | Ester adhesive | Ester adhesive | - | Ether adhesive | Ester adhesive | Ester adhesive | Ester adhesive | Ester adhesive | Ester adhesive |
| | | Thickness [μm] | | 3 | 3 | | 3 | 3 | 3 | 3 | 3 | 3 |

(continued)

| Table 9 | | | Example 7-1 | Example 7-2 | Example 7-3 | Example 7-4 | Example 7-5 | Example 7-6 | Example 7-7 | Example 7-8 | Example 7-9 | Example 7-10 |
|---|---|---|---|---|---|---|---|---|---|---|---|---|
| | Adhesive resin layer | Type | LDPE | - | - | - | - | - | - | - | - | - |
| | | Thickness [μm] | 25 | | | | | | | | | |
| | Support | Type | LDPE | ONY | ONY | - | - | - | - | PET | - | ONY |
| | | Thickness [μm] | 100 | 15 | 15 | | | | | 12 | | 15 |
| | Adhesive agent layer | Type | Ether adhesive | - | - | - | - | - | - | - | - | - |
| | | Thickness [μm] | 3 | | | | | | | | | |
| | Vapor deposition film | Type | Aluminum oxide | - | - | Aluminum oxide | - | Aluminum oxide | - | - | Aluminum oxide | - |
| | | Thickness [nm] | 12 | | | 12 | | 12 | | | 12 | |
| | Base material layer | Type | Polyester film in Example 2-1 | - | - | Polyester film in Example 2-1 | - | Polyester film in Example 2-1 | - | - | Polyester film in Example 2-1 | - |
| | | Thickness [μm] | 12 | | | 12 | | 12 | | | 12 | |
| | Adhesive agent layer | Type | - | Ester adhesive | Ester adhesive | Ester adhesive | - | - | - | Ester adhesive | Ester adhesive | Ester adhesive |
| | | Thickness [μm] | | 3 | 3 | 3 | | | | 3 | 3 | 3 |
| | Adhesive resin layer | Type | EMAA | - | - | - | - | LDPE | - | - | - | - |
| | | Thickness [μm] | 25 | | | | | 15 | | | | |
| | Sealant layer | Type | LLDPE | LLDPE | LLDPE | LLDPE | LLDPE | LLDPE | LLDPE | LLDPE | LLDPE | LLDPE |
| | | Thickness [μm] | 80 | 60 | 60 | 40 | 40 | 30 | 130 | 70 | 80 | 80 |
| Lamination strength [N/15 mm width] | | | 2.1 | 2.8 | 2.8 | 2.8 | 2.1 | 2.8 | 2.8 | 2.8 | 2.8 | 2.8 |

(continued)

| Table 9 | Example 7-1 | Example 7-2 | Example 7-3 | Example 7-4 | Example 7-5 | Example 7-6 | Example 7-7 | Example 7-8 | Example 7-9 | Example 7-10 |
|---|---|---|---|---|---|---|---|---|---|---|
| Main use | Laminated tube | Refill container | Microwaveable packaging | Deep-draw container lid | Wet-wipe packaging *Tsukemono* packaging | *Katsuobushi* packaging | Laminated tube | Microwaveable packaging | Dialysis fluid packaging | Refill container Ink packaging Aerosol spray container |

ONY: Biaxially oriented nylon film
PET: Polyethylene terephthalate film
OPP: Biaxially oriented polypropylene film
CPP: Cast polypropylene film
EMAA: Ethylene-methacrylic acid copolymer
LDPE: Low-density polyethylene
LLDPE: Linear low-density polyethylene

As is clear from Examples 7-1 to 7-10 above, it can be understood that laminates can be manufactured from laminated films made using polyesters in which the diol unit is ethylene glycol made using carbon monoxide gas as a raw material.

The lamination strengths measured with the laminates in Examples 7-1 to 7-10, furthermore, were comparable to lamination strength measured with a laminate made using a known polyester in which the diol unit is fossil fuel-derived ethylene glycol. It can be, therefore, understood that the laminates in Examples 7-1 to 7-10 exhibit physical characteristics that are not inferior in terms of mechanical properties.

[EXAMPLE 8-1]

The polyester film in Example 2-1 and an aluminum foil (thickness, 7 μm; manufactured by Toyo Aluminium K.K.) were sandwich-laminated with an adhesive resin layer having a thickness of 15 μm formed by melt extrusion of low-density polyethylene (trade name, LC600A; manufactured by Japan Polyethylene Corporation) interposed therebetween.

Subsequently, on the aluminum foil, a sealant layer having a thickness of 30 μm was formed by melt extrusion lamination with low-density polyethylene (trade name, LC600A; manufactured by Japan Polyethylene Corporation).

In such a manner, a laminate in Example 8-1, in which a polyester film, an adhesive resin layer, a barrier layer, and a sealant layer were laminated in this order, was obtained.

The main use of the laminate in Example 8-1 is packaging bags for divided Chinese herbal medicine or stick-shaped packaging.

[EXAMPLE 8-2]

An ester adhesive (trade name, RU-77T/H-7; manufactured by Rock Paint Co., Ltd.) was applied onto the polyester film in Example 2-1, and the film was joined to an aluminum foil (thickness, 7 μm; manufactured by Toyo Aluminium K.K.).

Subsequently, an ester adhesive (trade name, RU-77T/H-7; manufactured by Rock Paint Co., Ltd.) was applied onto the aluminum foil, and the aluminum foil was joined to a biaxially oriented nylon film (trade name, N1202; thickness, 15 μm; manufactured by Toyobo Co., Ltd.).

After that, an ester adhesive (trade name, RU-77T/H-7; manufactured by Rock Paint Co., Ltd.) was applied onto the biaxially oriented nylon film, and the film was joined to a linear low-density polyethylene film (trade name, MTNST; thickness, 80 μm; manufactured by Futamura Chemical Co., Ltd.).

In such a manner, a laminate in Example 8-2, in which a polyester film, an adhesive agent layer, a barrier layer, an adhesive agent layer, a support, an adhesive agent layer, and a sealant layer were laminated in this order, was obtained. In the laminate in Example 8-2, the thicknesses of the adhesive agent layers were all 3 μm.

The main use of the laminate in Example 8-2 is a pouch with a capped spout, or a lid.

[EXAMPLE 8-3]

An ether adhesive (trade name, RU-3600/H-689; manufactured by Rock Paint Co., Ltd.) was applied onto the polyester film in Example 2-1, and the film was joined to the surface of an aluminum deposition film formed on one surface of a cast polypropylene film (trade name, 2703; thickness, 25 μm; manufactured by Toray Advanced Film Co., Ltd.). The thickness of the adhesive agent layer was 3 μm.

In such a manner, a laminate in Example 8-3, in which a polyester film, an adhesive agent layer, a barrier layer, and a sealant layer were laminated in this order, was obtained.

The main use of the laminate in Example 8-3 is individual chocolate packaging.

[EXAMPLE 8-4]

The polyester film in Example 2-1 and an aluminum foil (thickness, 7 μm; manufactured by Toyo Aluminium K.K.) were sandwich-laminated with an adhesive resin layer having a thickness of 15 μm formed by melt extrusion of low-density polyethylene (trade name, LC600A; manufactured by Japan Polyethylene Corporation) interposed therebetween.

The surface of the aluminum foil in the laminate obtained in such a manner and a low-density polyethylene film (trade name, L-140AS-1; thickness, 40 μm; an antistatic film; manufactured by Aicello Corporation) were sandwich-laminated with an adhesive resin layer having a thickness of 15 μm formed by melt extrusion of low-density polyethylene (trade name, LC600A; manufactured by Japan Polyethylene Corporation) interposed therebetween.

In such a manner, a laminate in Example 8-4, in which a polyester film, an adhesive resin layer, a barrier layer, an adhesive resin layer, and a sealant layer were laminated in this order, was obtained.

The main use of the laminate in Example 8-4 is stick-shaped packaging.

[EXAMPLE 8-5]

Easy-open processing was applied to the polyester film in Example 2-1 by creating microscopic through-holes in part of the film (the opening position when the film is made into a pillow-seal packaging bag).
A laminate in Example 8-5 was obtained in the same manner as in Example 8-4, except that the polyester film in Example 2-1 that had undergone the easy-open processing was used.
The main use of the laminate in Example 8-5 is stick-shaped packaging.

[EXAMPLE 8-6]

An ester adhesive (trade name, RU-004/H-1; manufactured by Rock Paint Co., Ltd.) was applied onto the polyester film in Example 2-1, and the film was joined to a biaxially oriented nylon film (trade name, N1202; thickness, 15 μm; manufactured by Toyobo Co., Ltd.).
Subsequently, an ester adhesive (trade name, RU-004/H-1; manufactured by Rock Paint Co., Ltd.) was applied onto the biaxially oriented nylon film, and the film was joined to an aluminum foil (thickness, 7 μm; manufactured by Toyo Aluminium K.K.).
After that, an ester adhesive (trade name, RU-004/H-1; manufactured by Rock Paint Co., Ltd.) was applied onto the aluminum foil, and the aluminum foil was joined to a cast polypropylene film (trade name, ZK99S; thickness, 80 μm; manufactured by Toray Advanced Film Co., Ltd.).
In such a manner, a laminate in Example 8-6, in which a polyester film, an adhesive agent layer, a support, an adhesive agent layer, a barrier layer, an adhesive agent layer, and a sealant layer were laminated in this order, was obtained. In the laminate in Example 8-6, the thicknesses of the adhesive agent layers were all 3 μm.
The main use of the laminate in Example 8-6 is hotpot soup base packaging.

[EXAMPLE 8-7]

An ester adhesive (trade name, RU-77T/H-7; manufactured by Rock Paint Co., Ltd.) was applied onto the polyester film in Example 2-1, and the film was joined to an aluminum foil (thickness, 7 μm; manufactured by Toyo Aluminium K.K.).
Subsequently, an ester adhesive (trade name, RU-77T/H-7; manufactured by Rock Paint Co., Ltd.) was applied onto the aluminum foil, and the aluminum foil was joined to a linear low-density polyethylene film (trade name, MTNST; thickness, 80 μm; manufactured by Futamura Chemical Co., Ltd.).
In such a manner, a laminate in Example 8-7, in which a polyester film, an adhesive agent layer, a barrier layer, an adhesive agent layer, and a sealant layer were laminated in this order, was obtained. In the laminate in Example 8-7, the thicknesses of the adhesive agent layers were both 3 μm.
The main use of the laminate in Example 8-7 is dietary supplement packaging or a refill container.

[EXAMPLE 8-8]

An ester adhesive (trade name, RU-004/H-1; manufactured by Rock Paint Co., Ltd.) was applied onto the polyester film in Example 2-1, and the film was joined to an aluminum foil (thickness, 7 μm; manufactured by Toyo Aluminium K.K.).
Subsequently, an ester adhesive (trade name, RU-004/H-1; manufactured by Rock Paint Co., Ltd.) was applied onto the aluminum foil, and the aluminum foil was joined to a cast polypropylene film (trade name, TAF502C; thickness, 25 μm; manufactured by RM Tohcello Co., Ltd.).
In such a manner, a laminate in Example 8-8, in which a polyester film, an adhesive agent layer, a barrier layer, an adhesive agent layer, and a sealant layer were laminated in this order, was obtained. In the laminate in Example 8-8, the thicknesses of the adhesive agent layers were both 3 μm.
The main use of the laminate in Example 8-8 is individual bath-additive packaging.

[EXAMPLE 8-9]

An ester adhesive (trade name, RU-77T/H-7; manufactured by Rock Paint Co., Ltd.) was applied onto the polyester film in Example 2-1, and the film was joined to an aluminum foil (thickness, 7 μm; manufactured by Toyo Aluminium K.K.).
Subsequently, an ester adhesive (trade name, RU-77T/H-7; manufactured by Rock Paint Co., Ltd.) was applied onto the aluminum foil, and the aluminum foil was joined to a biaxially oriented polyethylene terephthalate film (trade name, E5202; thickness, 12 μm; manufactured by Toyobo Co., Ltd.).

After that, an ester adhesive (trade name, RU-77T/H-7; manufactured by Rock Paint Co., Ltd.) was applied onto the biaxially oriented polyethylene terephthalate film, and the film was joined to a linear low-density polyethylene film (trade name, LR-124; thickness, 60 μm; manufactured by Okamoto Industries, Inc.).
In such a manner, a laminate in Example 8-9, in which a polyester film, an adhesive agent layer, a barrier layer, an adhesive agent layer, a support, an adhesive agent layer, and a sealant layer were laminated in this order, was obtained. In the laminate in Example 8-9, the thicknesses of the adhesive agent layers were all 3 μm.
The main use of the laminate in Example 8-9 is pet-food sachets.

[EXAMPLE 8-10]

An ester adhesive (trade name, RU-004/H-1; manufactured by Rock Paint Co., Ltd.) was applied onto the polyester film in Example 2-1, and the film was joined to a biaxially oriented nylon film (trade name, N1202; thickness, 15 μm; manufactured by Toyobo Co., Ltd.).
Subsequently, an ester adhesive (trade name, RU-004/H-1; manufactured by Rock Paint Co., Ltd.) was applied onto the biaxially oriented nylon film, and the film was joined to an aluminum foil (thickness, 40 μm; manufactured by MA Aluminum Corporation).
On the aluminum foil in the laminate obtained in such a manner, an adhesive resin layer having a thickness of 40 μm and a sealant layer having a thickness of 40 μm were formed by coextruding polypropylene modified with maleic anhydride and random polypropylene.
In such a manner, a laminate in Example 8-10, in which a polyester film, an adhesive agent layer, a support, an adhesive agent layer, a barrier layer, an adhesive resin layer, and a sealant layer were laminated in this order, was obtained. In the laminate in Example 8-10, the thicknesses of the adhesive agent layers were both 3 μm.
The main use of the laminate in Example 8-10 is a battery pouch.

[COMPARATIVE EXAMPLES 8-1 to 8-10]

Laminates in Comparative Examples 8-1 to 8-10 were obtained in the same manner as in Examples 8-1 to 8-10, except that the polyester film in Comparative Example 2-1 was used instead of the polyester film in Example 2-1.

[Measurement of Lamination Strength]

For each of the laminates in Examples 8-1 to 8-10 and Comparative Examples 8-1 to 8-10, lamination strength between the polyester film and the layer adjacent to the polyester film with an adhesive resin layer or an adhesive agent layer interposed therebetween was measured according to JIS Z 0238: 1998.
Specifically, the laminate was cut into a strip shape such that the length in the transverse direction of the polyester film was 15 mm, and that the length in the machine direction of the polyester film was greater than the length in the transverse direction, yielding a test specimen. For the resulting test specimen, the polyester film and the layer adjacent to the polyester film with an adhesive resin layer or an adhesive agent layer interposed therebetween were partially separated. Using a tensile tester (trade name, TENSILON STA-1150; manufactured by A&D Co., Ltd.), the polyester film and the layer adjacent to the polyester film with an adhesive resin layer or an adhesive agent layer interposed therebetween in the test specimen were held by pinching each of them with a chuck of the tensile tester. Then the film and the adjacent layer were pulled apart by the T-peel method in the vertical direction for 10 mm at 50 mm/min, and the average tensile strength was measured.
The same operation was attempted ten times, and the average of the measurements from the ten attempts was reported as the lamination strength between the polyester film and the layer adjacent to the polyester film with an adhesive resin layer or an adhesive agent layer interposed therebetween (unit: N/15 mm width).
Along with the layer configurations and main uses of the laminates in Examples 8-1 to 8-10 and Comparative Examples 8-1 to 8-10, the results are presented in Table 10 and Table 11.

[Table 10]

| Table 10 | | | Example 8-1 | Example 8-2 | Example 8-3 | Example 8-4 | Example 8-5 | Example 8-6 | Example 8-7 | Example 8-8 | Example 8-9 | Example 8-10 |
|---|---|---|---|---|---|---|---|---|---|---|---|---|
| Layer configuration | Polyester film | Type | Polyester film in Example 2-1 | Polyester film in Example 2-1 | Polyester film in Example 2-1 | Polyester film in Example 2-1 | Polyester film in Example 2-1, with easy-open processing | Polyester film in Example 2-1 | Polyester film in Example 2-1 | Polyester film in Example 2-1 | Polyester film in Example 2-1 | Polyester film in Example 2-1 |
| | | Thickness [μm] | 12 | 12 | 12 | 12 | 12 | 12 | 12 | 12 | 12 | 12 |
| | Adhesive resin layer | Type | LDPE | - | - | LDPE | LDPE | - | - | - | - | - |
| | | Thickness [μm] | 15 | | | 15 | 15 | | | | | |
| | Adhesive agent layer | Type | | Ester adhesive | Ether adhesive | | | Ester adhesive | Ester adhesive | Ester adhesive | Ester adhesive | Ester adhesive |
| | | Thickness [μm] | | 3 | 3 | | | 3 | 3 | 3 | 3 | 3 |
| | Support | Type | - | - | - | - | - | ONY | - | - | - | ONY |
| | | Thickness [μm] | | | | | | 15 | | | | 15 |
| | Adhesive agent layer | Type | - | - | - | - | - | Ester adhesive | - | - | - | Ester adhesive |
| | | Thickness [μm] | | | | | | 3 | | | | 3 |
| | Barrier layer | Type | Aluminum foil | Aluminum foil | Aluminum deposition film | Aluminum foil | Aluminum foil | Aluminum foil | Aluminum foil | Aluminum foil | Aluminum foil | Aluminum foil |
| | | Thickness [μm] | 7 | 7 | | 7 | 7 | 7 | 7 | 7 | 7 | 40 |
| | Adhesive agent layer | Type | - | Ester adhesive | - | - | - | Ester adhesive | Ester adhesive | Ester adhesive | Ester adhesive | - |

(continued)

| Table 10 | | | Example 8-1 | Example 8-2 | Example 8-3 | Example 8-4 | Example 8-5 | Example 8-6 | Example 8-7 | Example 8-8 | Example 8-9 | Example 8-10 |
|---|---|---|---|---|---|---|---|---|---|---|---|---|
| | | Thickness [μm] | | 3 | | | | 3 | 3 | 3 | 3 | |
| | Support | Type | - | ONY | - | - | - | - | - | - | PET | - |
| | | Thickness [μm] | | 15 | | | | | | | 12 | |
| | Adhesive agent layer | Type | - | Ester adhesive | - | - | - | - | - | - | Ester adhesive | - |
| | | Thickness [μm] | | 3 | | | | | | | 3 | |
| | Adhesive resin layer | Type | - | - | - | LDPE | LDPE | - | - | - | - | Acid-modified PP |
| | | Thickness [μm] | | | | 15 | 15 | | | | | 40 |
| | Sealant layer | Type | LDPE | LLDPE | CPP | Antistatic LDPE | Antistatic LDPE | CPP | LLDPE | CPP | LLDPE | Random PP |
| | | Thickness [μm] | 30 | 80 | 25 | 40 | 40 | 80 | 80 | 25 | 60 | 40 |
| Lamination strength [N/15 mm width] | | | 2.1 | 3.2 | 1.5 | 2.1 | 2,1 | 3.5 | 3.2 | 3.5 | 3.2 | 9.4 |

(continued)

| Table 10 | Example 8-1 | Example 8-2 | Example 8-3 | Example 8-4 | Example 8-5 | Example 8-6 | Example 8-7 | Example 8-8 | Example 8-9 | Example 8-10 |
|---|---|---|---|---|---|---|---|---|---|---|
| Main use | Packaging bags for divided Chinese herbal medicine Stick-shaped packaging | Pouch with a capped spout Lid | Individual chocolate packaging | Stick-shaped packaging | Stick-shaped packaging | Hotpot soup base packaging | Dietary supplement packaging Refill container | Individual bath-additive packaging | Pet-food sachets | Battery pouch |

ONY: Biaxially oriented nylon film
PET: Polyethylene terephthalate film
CPP: Cast polypropylene film
LDPE: Low-density polyethylene
LLDPE: Linear low-density polyethylene
Acid-modified PP: Polypropylene modified with maleic anhydride
Random PP: Random polypropylene

[Table 11]

| Table 11 | | | Comparative Example 8-1 | Comparative Example 8-2 | Comparative Example 8-3 | Comparative Example 8-4 | Comparative Example 8-5 | Comparative Example 8-6 | Comparative Example 8-7 | Comparative Example 8-8 | Comparative Example 8-9 | Comparative Example 8-10 |
|---|---|---|---|---|---|---|---|---|---|---|---|---|
| Layer configuration | Polyester film | Type | Polyester film in Comparative Example 2-1 | Polyester film in Comparative Example 2-1 | Polyester film in Comparative Example 2-1 | Polyester film in Comparative Example 2-1 | Polyester film in Comparative Example 2-1, with easy-open processing | Polyester film in Comparative Example 2-1 | Polyester film in Comparative Example 2-1 | Polyester film in Comparative Example 2-1 | Polyester film in Comparative Example 2-1 | Polyester film in Comparative Example 2-1 |
| | | Thickness [μm] | 12 | 12 | 12 | 12 | 12 | 12 | 12 | 12 | 12 | 12 |
| | Adhesive resin layer | Type | LDPE | - | - | LDPE | LDPE | - | - | - | - | - |
| | | Thickness [μm] | 15 | | | 15 | 15 | | | | | |
| | Adhesive agent layer | Type | - | Ester adhesive | Ether adhesive | - | - | Ester adhesive | Ester adhesive | Ester adhesive | Ester adhesive | Ester adhesive |
| | | Thickness [μm] | | 3 | 3 | | | 3 | 3 | 3 | 3 | 3 |
| | Support | Type | - | - | - | - | - | ONY | - | - | - | ONY |
| | | Thickness [μm] | | | | | | 15 | | | | 15 |
| | Adhesive agent layer | Type | - | - | - | - | - | Ester adhesive | - | - | - | Ester adhesive |
| | | Thickness [μm] | | | | | | 3 | | | | 3 |
| | Barrier layer | Type | Aluminum foil | Aluminum foil | Aluminum deposition film | Aluminum foil | Aluminum foil | Aluminum foil | Aluminum foil | Aluminum foil | Aluminum foil | Aluminum foil |
| | | Thickness [μm] | 7 | 7 | | 7 | 7 | 7 | 7 | 7 | 7 | 40 |
| | Adhesive agent layer | Type | - | Ester adhesive | - | - | - | Ester adhesive | Ester adhesive | Ester adhesive | Ester adhesive | - |
| | | Thickness [μm] | | 3 | | | | 3 | 3 | 3 | 3 | |

(continued)

| Table 11 | | | Comparative Example 8-1 | Comparative Example 8-2 | Comparative Example 8-3 | Comparative Example 8-4 | Comparative Example 8-5 | Comparative Example 8-6 | Comparative Example 8-7 | Comparative Example 8-8 | Comparative Example 8-9 | Comparative Example 8-10 |
|---|---|---|---|---|---|---|---|---|---|---|---|---|
| | Support | Type | - | ONY | - | - | - | - | - | - | PET | - |
| | | Thickness [μm] | | 15 | | | | | | | 12 | |
| | Adhesive agent layer | Type | - | Ester adhesive | - | - | - | - | - | - | Ester adhesive | - |
| | | Thickness [μm] | | 3 | | | | | | | 3 | |
| | Adhesive resin layer | Type | - | - | - | LDPE | LDPE | - | - | - | - | Acid-modified PP |
| | | Thickness [μm] | | | | 15 | 15 | | | | | 40 |
| | Sealant layer | Type | LDPE | LLDPE | CPP | Antistatic LDPE | Antistatic LDPE | CPP | LLDPE | CPP | LLDPE | Random PP |
| | | Thickness [μm] | 30 | 80 | 25 | 40 | 40 | 80 | 80 | 25 | 60 | 40 |
| Lamination strength [N/15 mm width] | | | 2.1 | 3.2 | 1.5 | 2.1 | 2,1 | 3.5 | 3.2 | 3.5 | 3.2 | 9.4 |
| Main use | | | Packaging bags for divided Chinese herbal medicine Stick-shaped packaging | Pouch with a capped spout Lid | Individual chocolate packaging | Stick-shaped packaging | Stick-shaped packaging | Hotpot soup base packaging | Dietary supplement packaging Refill container | Individual bath-additive packaging | Pet-food sachets | Battery pouch |

ONY: Biaxially oriented nylon film
PET: Polyethylene terephthalate film
CPP: Cast polypropylene film
LDPE: Low-density polyethylene
LLDPE: Linear low-density polyethylene
Acid-modified PP: Polypropylene modified with maleic anhydride
Random PP: Random polypropylene

As is clear from Table 10 and Table 11, it can be understood that Examples 8-1 to 8-10, which were laminates including a polyester film produced using a polyester in which the diol unit was ethylene glycol made using carbon monoxide gas as a raw material, exhibit comparable lamination strength, and thus physical characteristics that are not inferior in terms of mechanical properties, compared with Comparative Examples 8-1 to 8-10, which were laminates including a polyester film produced using a known polyester in which the diol unit was fossil fuel-derived ethylene glycol.

Reference Signs List

1 reducing catalyst
11 substrate
12 reflective layer
13 reducing electrode layer
14 first solar cell
14a n-type a-Si layer
14b intrinsic a-SiGe layer
14c p-type μc-Si layer
15 second solar cell
15a n-type a-Si layer
15b intrinsic a-SiGe layer
15c p-type μc-Si layer
16 third solar cell
16a n-type a-Si layer
16b intrinsic a-Si layer
16c p-type μc-Si layer
17 multi-junction solar cell
18 oxidizing electrode layer
19 oxidizing catalyst layer
20 reducing catalyst layer
30 photochemical reaction cell
31 electrolytic cells
31a oxidation reaction electrolytic cell
31b reduction reaction electrolytic cell
41 electrolytic cell channel
42 circulating mechanism
43 ion-exchange membrane
44 temperature adjustment mechanism
51 opening
52 opening
100 spacer organic molecule layer
101 current collector
102 metal layer
109 reactive functional group
110 backbone
111 amino group
112 organic molecule containing quaternary nitrogen cation(s) (modifying organic molecule)
200 chemical reaction apparatus
210 chemical reaction apparatus
220 chemical reaction apparatus
L light
1X film
30X preform
31X mouth
32X body
33X bottom
34X thread
35X bead

36X support ring
40X bottle
41X mouth
42X body
43X bottom
44X neck
45X horizontal groove
46X thread
47X support ring
48X shoulder
49X heel
50X spoon
51X measuring section
52X handle
53X measuring space
10Y polyester film
20A to 20G laminate
21Y sealant layer
21A first sealant layer
21B second sealant layer
30Y stand-up pouch
31Y side sheet
32Y bottom sheet
33Y support
34Y printed layer
41Y pillow bag
42Y three-side-seal bag
43Y four-side-seal bag
50Y tube container
51Y head
52Y cylindrical body
53Y shoulder
54Y outlet
55Y adhesive resin layer
56Y bottom seal
60Y paper container for liquids
61Y body
62Y bottom
63Y top
63a sloping panel
64Y tucked portion
65Y bonding margin
66Y paper layer
70Y paper cup
71Y flange
72Y body
72' body blank
72a' ends
73Y bottom
73' bottom blank
73a' bent portion
75Y body seam
10Z base material layer
11Z vapor deposition film
12Z gas barrier coating film
13Z surface coating layer
15Z laminated film
20Z laminate

21Z sealant layer
33Z support

## Claims

1. Ethylene glycol made using at least one gas selected from the group consisting of carbon monoxide and carbon dioxide as a raw material.

2. The ethylene glycol according to claim 1, wherein a quantity of carbon dioxide molecules is $2.2 \times 10^{18}$ molecules/g or more and $1.5 \times 10^{19}$ molecules/g or less.

3. The ethylene glycol according to claim 1 or 2, wherein an amount of Kjeldahl nitrogen is 700 μg/g or less.

4. A polyester comprising a diol unit and a dicarboxylic acid unit, wherein:
the diol unit is the ethylene glycol according to any one of claims 1 to 3.

5. The polyester according to claim 4, wherein the dicarboxylic acid unit is at least one selected from the group consisting of fossil fuel-derived terephthalic acid, biomass-derived terephthalic acid, and terephthalic acid made using carbon dioxide gas as a raw material.

6. Polyester pellets comprising the polyester according to claim 4 or 5, wherein:
an intrinsic viscosity of the polyester pellets is 0.50 dL/g or more and 0.90 dL/g or less.

7. The polyester pellets according to claim 6, wherein an amount of Kjeldahl nitrogen is 260 μg/g or less.

8. The polyester pellets according to claim 6 or 7, wherein a quantity of carbon dioxide molecules is $4.8 \times 10^{17}$ molecules/g or more and $3.0 \times 10^{18}$ molecules/g or less.

9. The polyester pellets according to any one of claims 6 to 8, wherein a quantity of water molecules is $1.8 \times 10^{19}$ molecules/g or more and $1.0 \times 10^{20}$ molecules/g or less.

10. A molded article made using the polyester pellets according to any one of claims 6 to 9.

11. A film made using the polyester pellets according to any one of claims 6 to 9.

12. Fibers made using the polyester pellets according to claim 6.

13. The fibers according to claim 12, wherein an amount of heat of fusion of the polyester pellets during a second heating run obtained by differential scanning calorimetry (DSC) according to JIS K7122: 2012 is 8 J/g or more.

14. The fibers according to claim 12 or 13, wherein an amount of heat of solidification of the polyester pellets during a second cooling run obtained by differential scanning calorimetry (DSC) according to JIS K7122: 2012 is 7 J/g or more.

15. The fibers according to any one of claims 12 to 14, wherein an amount of Kjeldahl nitrogen in the polyester pellets is 90 μg/g or less.

16. The fibers according to any one of claims 12 to 15, wherein a quantity of hydrogen molecules in the polyester pellets is $7.5 \times 10^{17}$ molecules/g or less.

17. A fiber article made using the fibers according to any one of claims 12 to 16.

18. An article molded from the polyester according to claim 4 or 5.

19. The molded article according to claim 18, wherein a quantity of carbon dioxide molecules is $9.2 \times 10^{17}$ molecules/g or more and $3.0 \times 10^{18}$ molecules/g or less.

20. The molded article according to claim 18 or 19, wherein an amount of Kjeldahl nitrogen is 18 μg/g or less.

**21.** The molded article according to any one of claims 18 to 20, wherein the molded article is a container.

**22.** A preform for use in manufacture of the molded article according to claim 21.

**23.** A polyester film comprising the polyester according to claim 4 or 5.

**24.** The polyester film according to claim 23, wherein a quantity of carbon dioxide molecules is $1.1 \times 10^{18}$ molecules/g or more and $3.0 \times 10^{18}$ molecules/g or less.

**25.** The polyester film according to claim 23 or 24, wherein a quantity of water molecules is $8.6 \times 10^{19}$ molecules/g or more and $2.5 \times 10^{20}$ molecules/g or less.

**26.** A laminate comprising the polyester film according to any one of claims 23 to 25.

**27.** A laminated film comprising at least a base material layer and a vapor deposition film, wherein:

the base material layer contains the polyester according to claim 4 or 5; and
the vapor deposition film is a vapor deposition film of a metal or a metal oxide.

**28.** The laminated film according to claim 27, wherein a quantity of carbon dioxide molecules in the base material layer is $1.1 \times 10^{18}$ molecules/g or more and $3.0 \times 10^{18}$ molecules/g or less.

**29.** The laminated film according to claim 27 or 28, wherein a quantity of water molecules in the base material layer is $8.6 \times 10^{19}$ molecules/g or more and $2.5 \times 10^{20}$ molecules/g or less.

**30.** The laminated film according to any one of claims 27 to 29, further comprising a surface coating layer between the base material layer and the vapor deposition film, wherein:
the surface coating layer contains a resin material having a polar group.

**31.** The laminated film according to any one of claims 27 to 30, wherein:

the laminated film includes the base material layer, the vapor deposition film, and a gas barrier coating film in this order; and
the gas barrier coating film contains an alkoxide and at least one water-soluble polymer selected from the group consisting of a polyvinyl alcohol resin and an ethylene-vinyl alcohol copolymer.

**32.** A laminate comprising the laminated film according to any one of claims 27 to 31 and a sealant layer.

**33.** The laminate according to claim 32, further comprising a support, wherein:
the support is a stretched polyester resin layer, a stretched polyamide resin layer, or a stretched polypropylene resin layer.

**34.** The laminate according to claim 33, wherein the laminate includes the support, the base material layer, the vapor deposition film, and the sealant layer in this order.

**35.** A laminate comprising at least the polyester film according to any one of claims 23 to 25 and a sealant layer.

**36.** The laminate according to claim 35, wherein a resin constituting the sealant layer is low-density polyethylene, linear low-density polyethylene, or polypropylene.

**37.** The laminate according to claim 35 or 36, further comprising a barrier layer between the polyester film and the sealant layer.

**38.** The laminate according to claim 37, wherein the barrier layer is a metal foil.

**39.** A package comprising the laminate according to any one of claims 32 to 38.

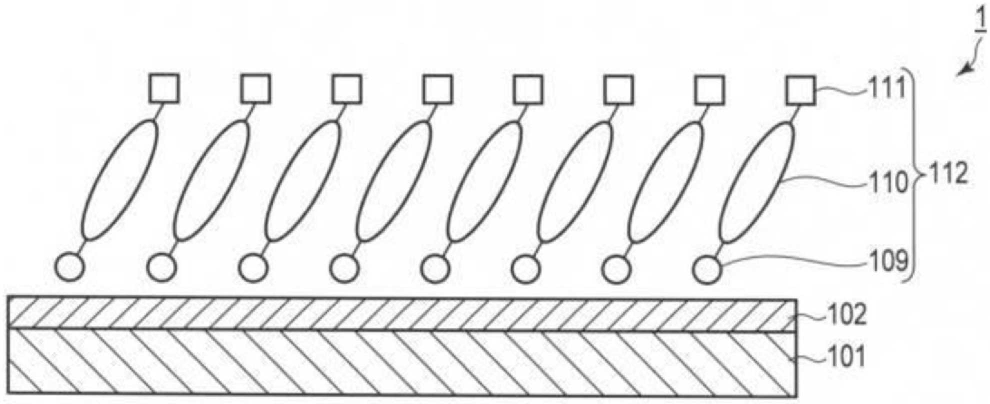


*Fig. 1*

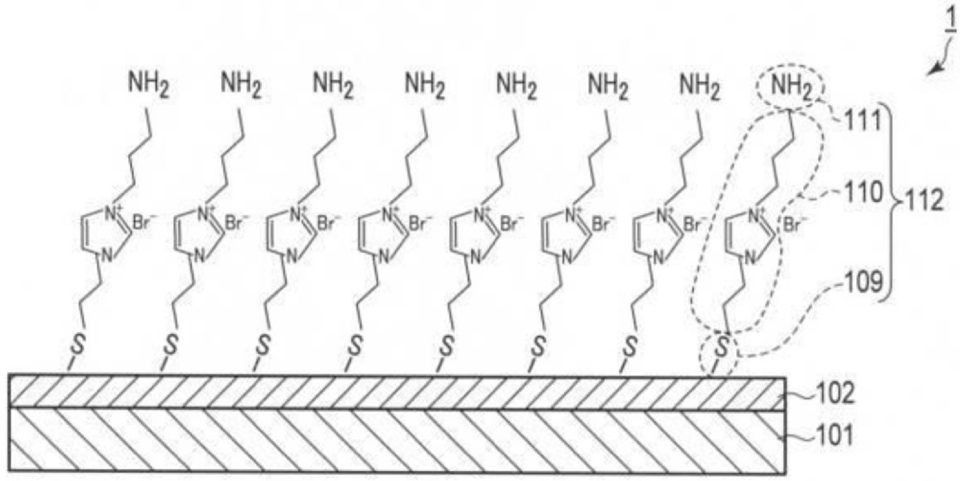


*Fig. 2*

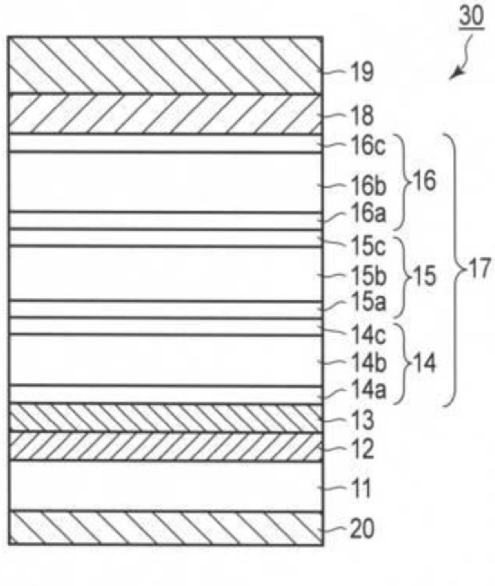


*Fig. 3*

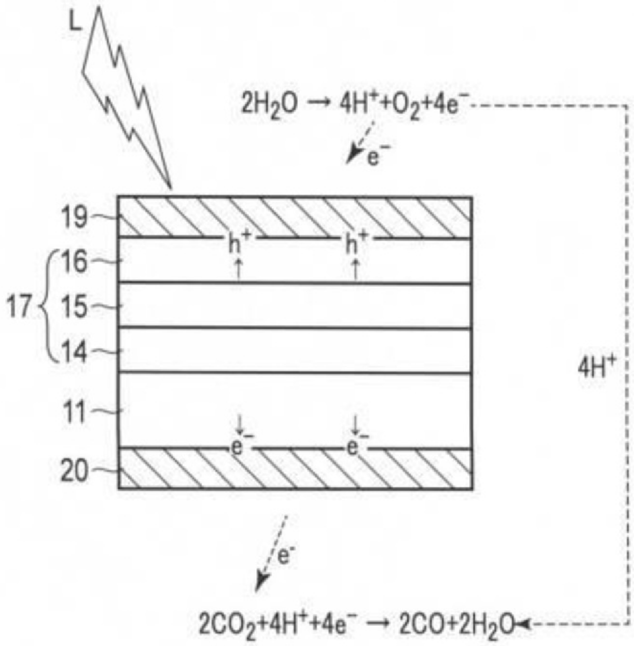
L
2H2O → 4H+ +O2+4e−
e−
19
16
17
15
14
11
20
h+
e−
4H+
e-
2CO2+4H+ +4e− → 2CO+2H2O

*Fig. 4*

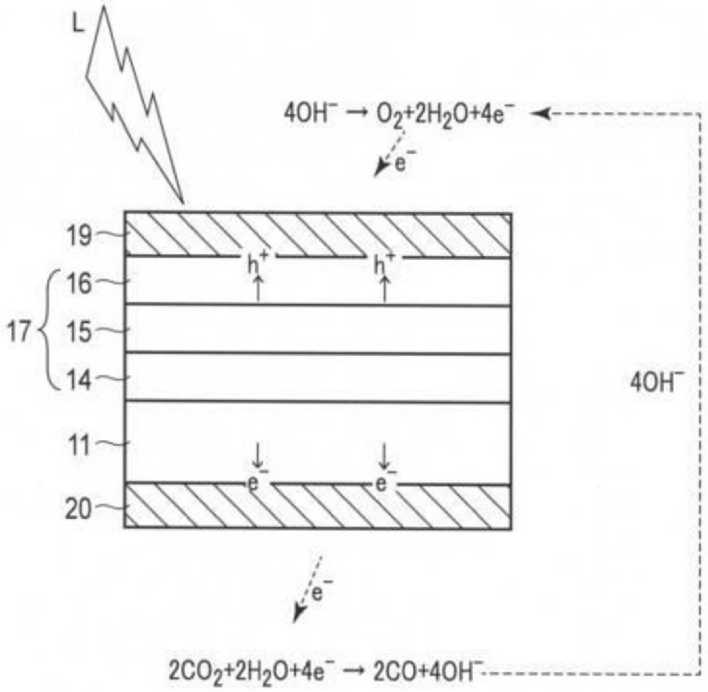
L
4OH− → O2+2H2O+4e−
e−
19
16
17
15
14
11
20
h+
e−
4OH−
e−
2CO2+2H2O+4e− → 2CO+4OH−

*Fig. 5*

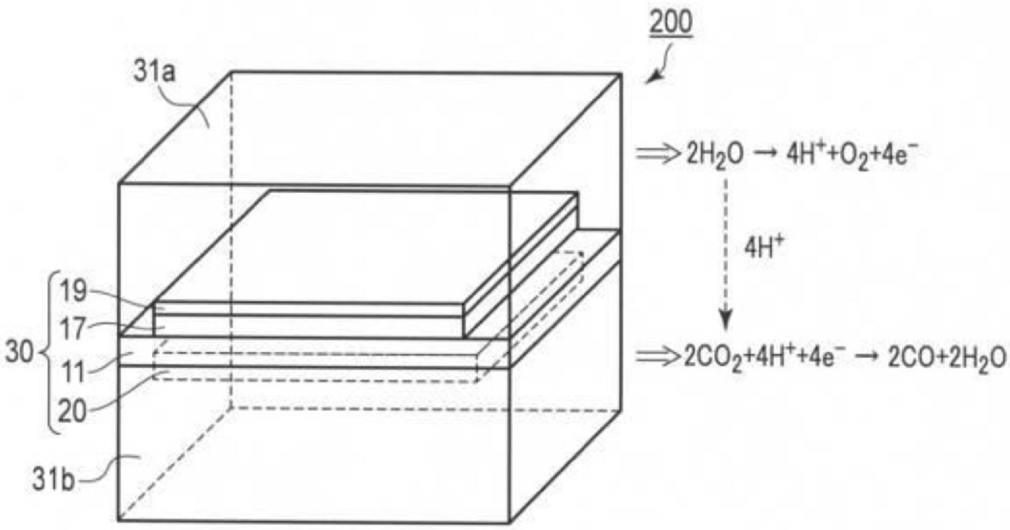
200
31a
$\Rightarrow 2H_2O \rightarrow 4H^+ + O_2 + 4e^-$
$4H^+$
19
17
30
11
20
$\Rightarrow 2CO_2 + 4H^+ + 4e^- \rightarrow 2CO + 2H_2O$
31b

*Fig. 6*

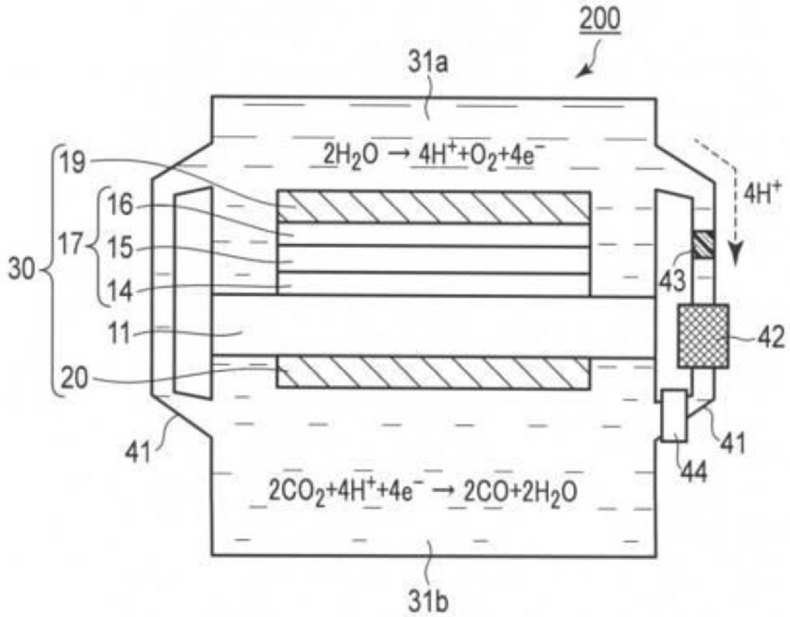
200
31a
$2H_2O \rightarrow 4H^+ + O_2 + 4e^-$
$4H^+$
19
16
17
15
30
14
43
11
42
20
41
41
44
$2CO_2 + 4H^+ + 4e^- \rightarrow 2CO + 2H_2O$
31b

*Fig. 7*

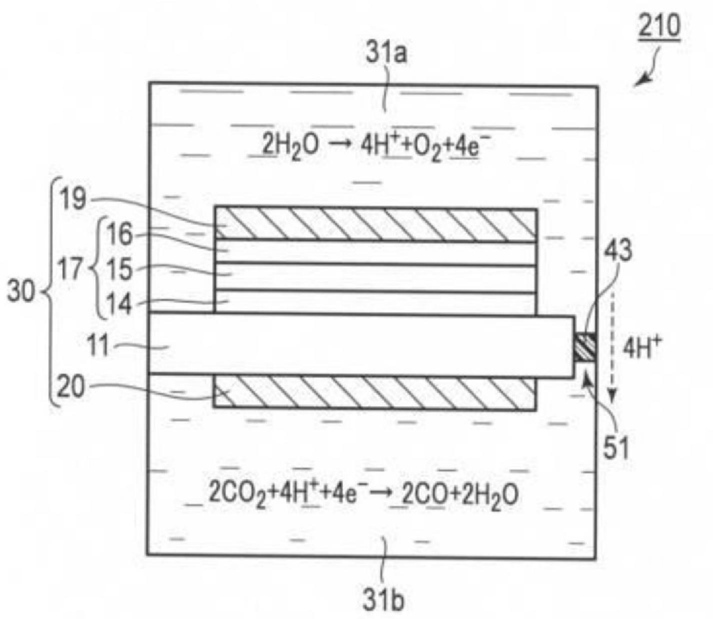
210
31a
$2H_2O \rightarrow 4H^+ + O_2 + 4e^-$
19
16
17
15
30
14
11
20
43
4H+
51
$2CO_2 + 4H^+ + 4e^- \rightarrow 2CO + 2H_2O$
31b

*Fig. 8*

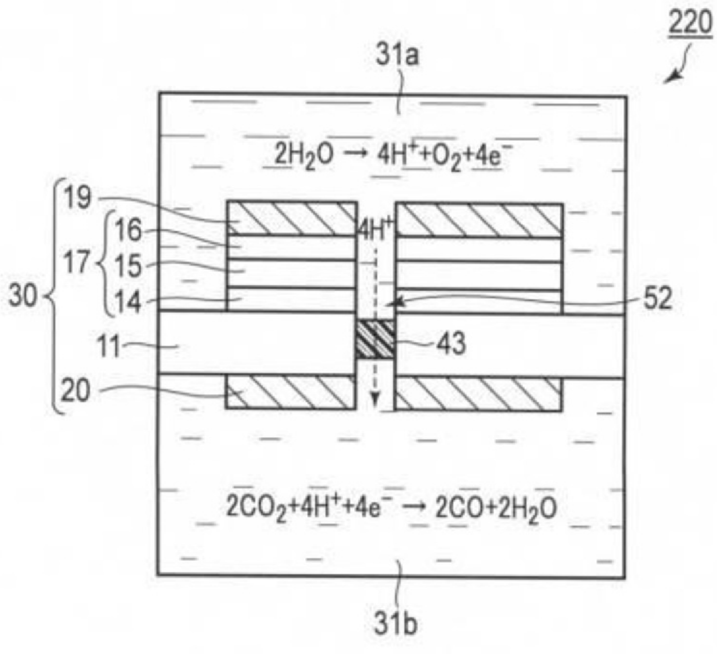
220
31a
$2H_2O \rightarrow 4H^+ + O_2 + 4e^-$
19
16
17
15
30
14
11
20
4H+
52
43
$2CO_2 + 4H^+ + 4e^- \rightarrow 2CO + 2H_2O$
31b

*Fig. 9*

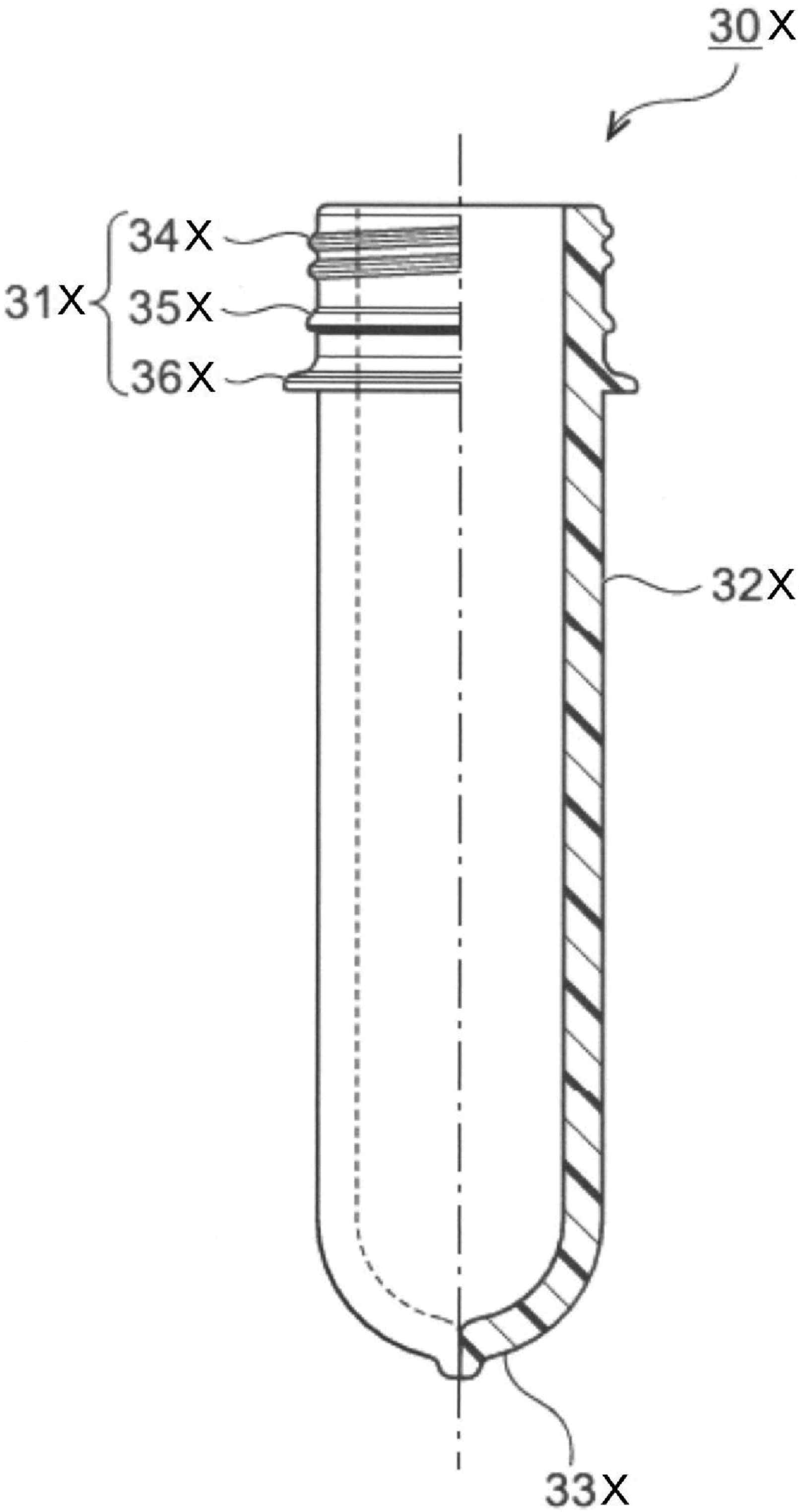


Fig. 10

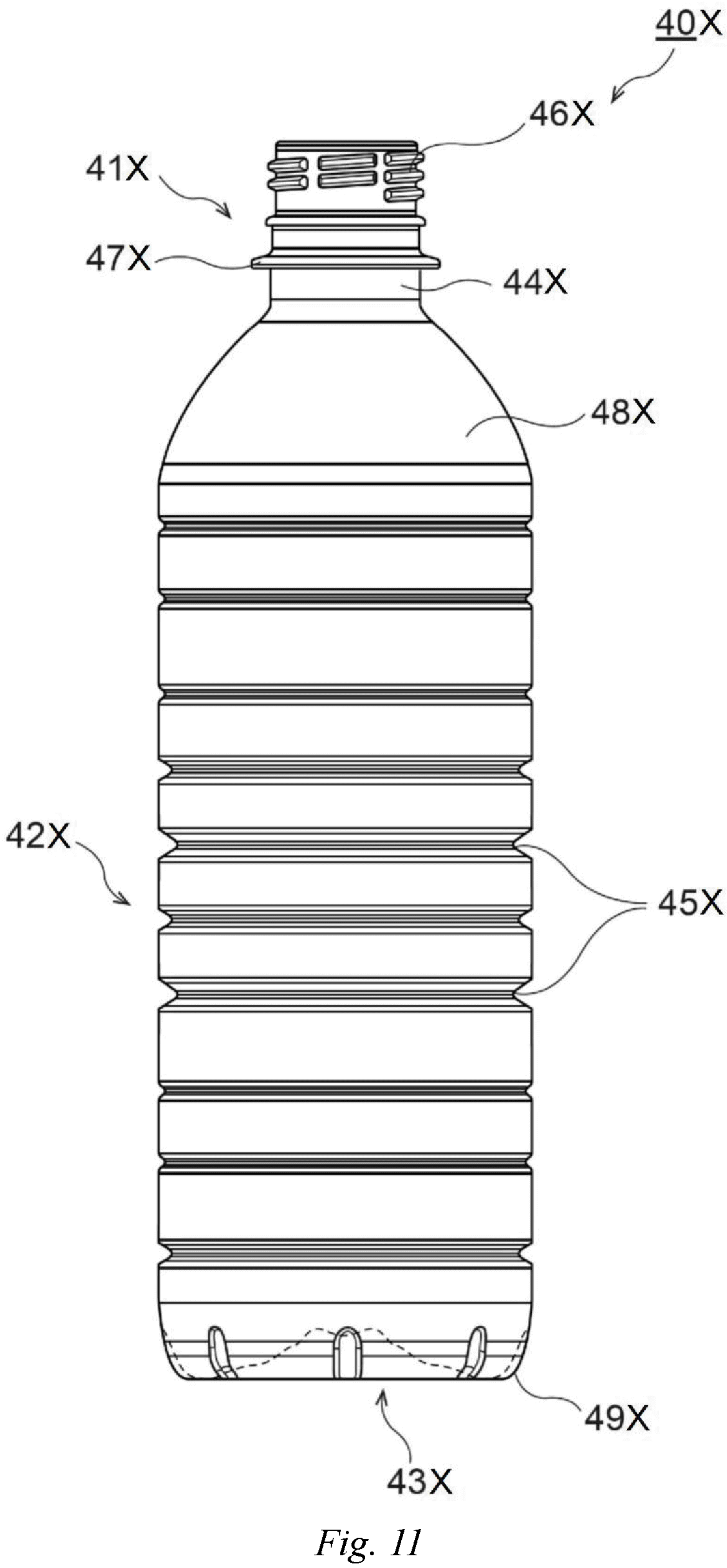


*Fig. 11*

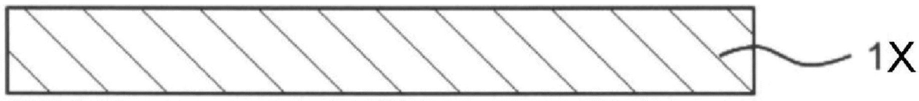


*Fig. 12*

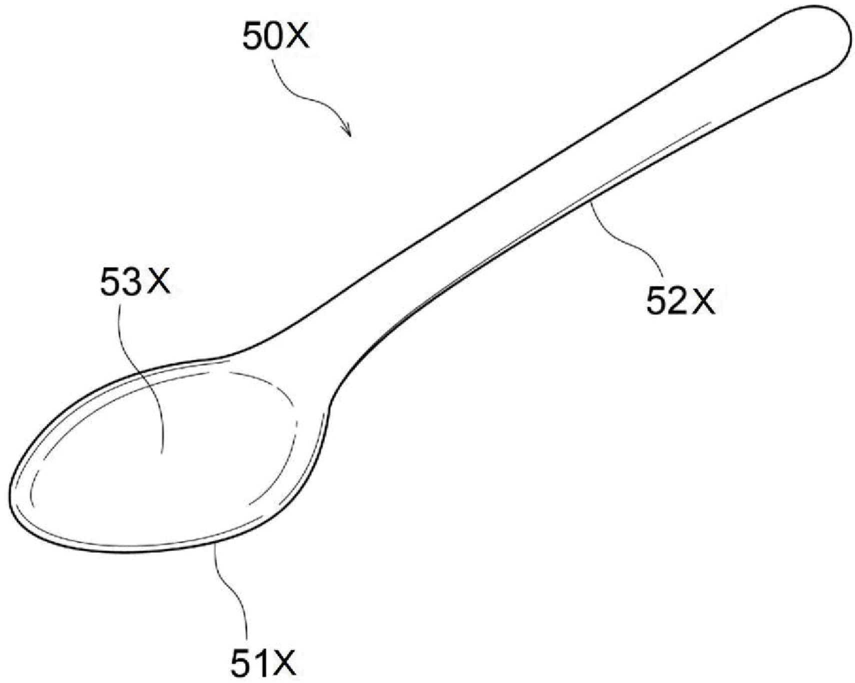


*Fig. 13*

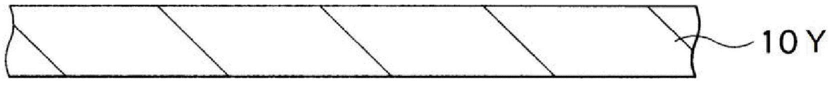


*Fig. 14*

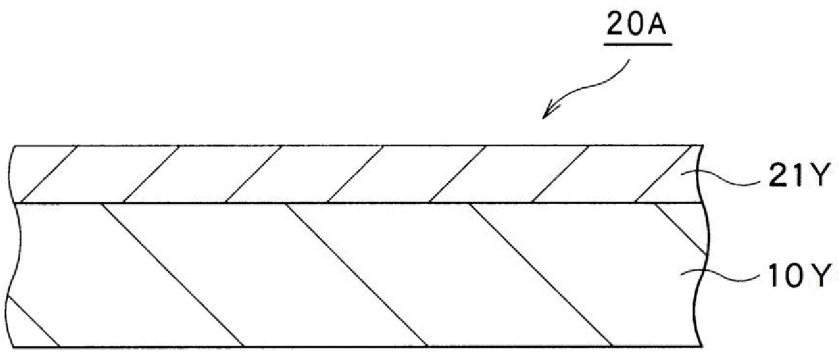


*Fig. 15*

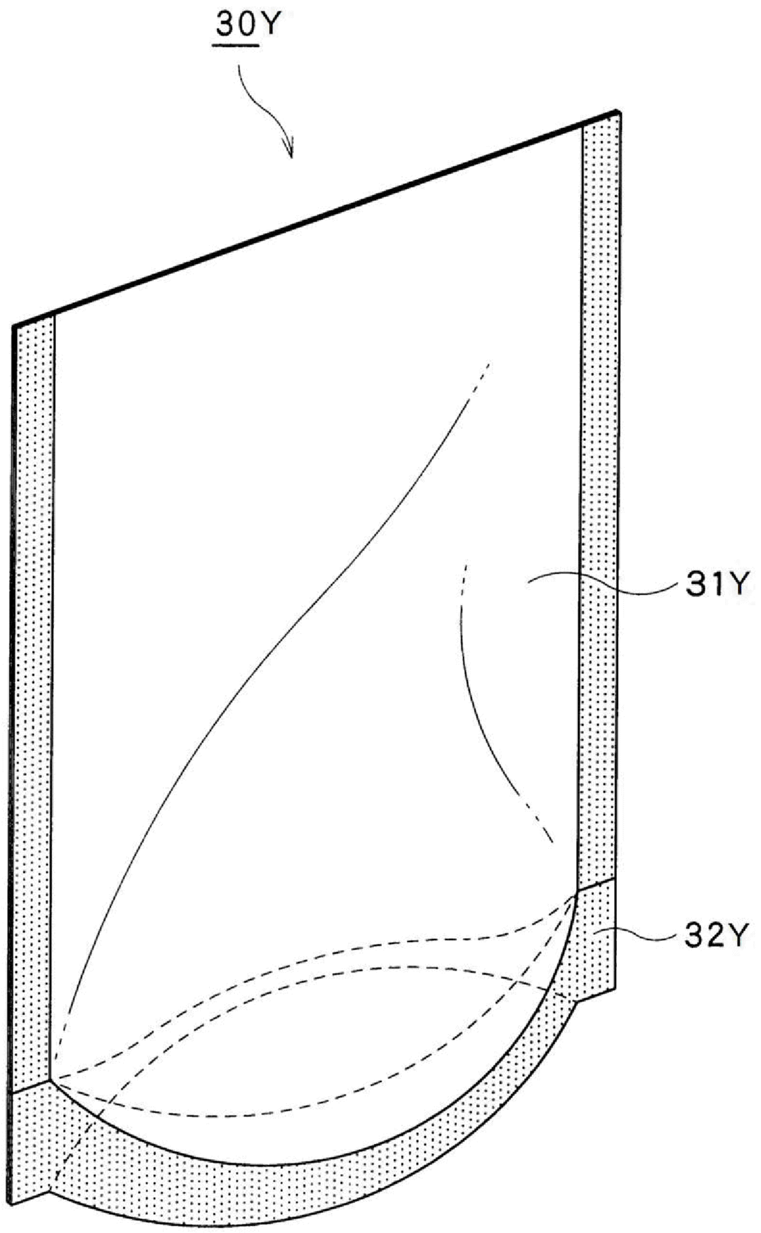


*Fig. 16*

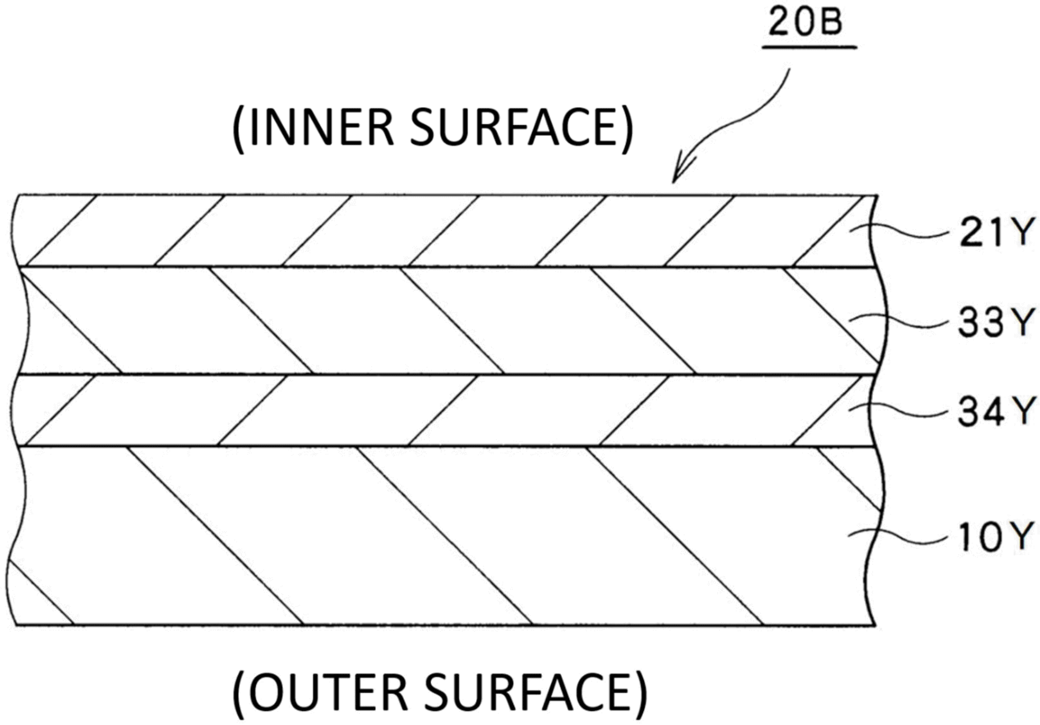


*Fig. 17*

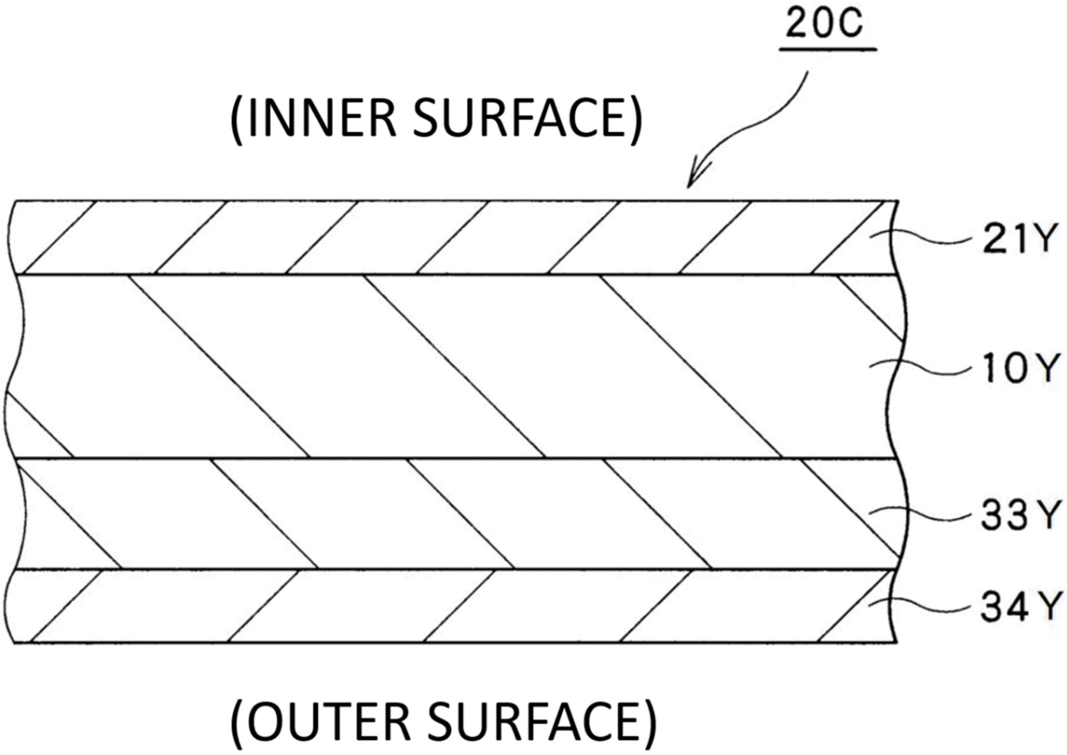


*Fig. 18*

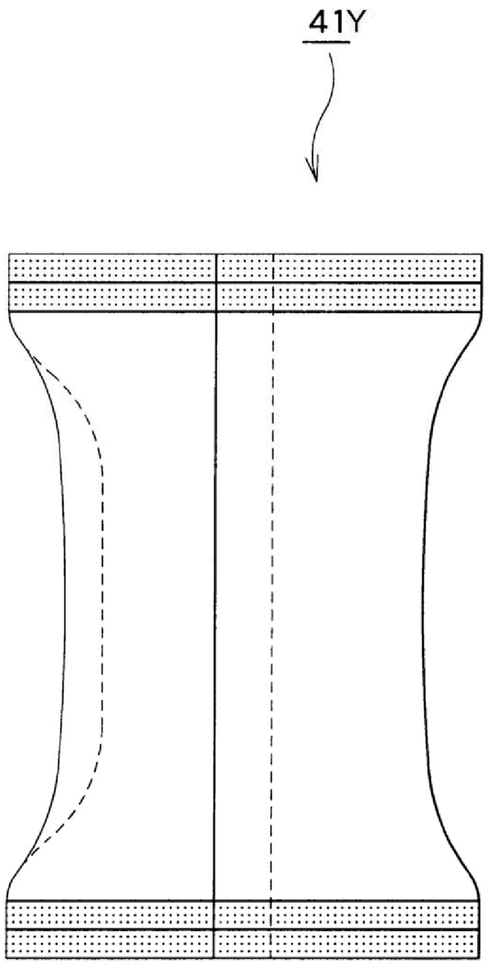


Fig. 19

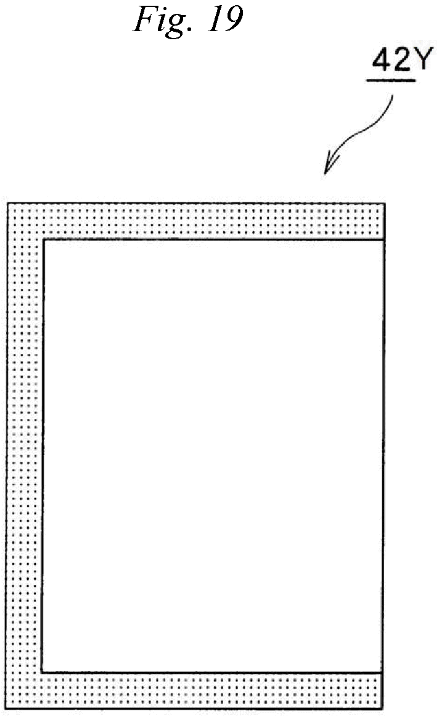


Fig. 20

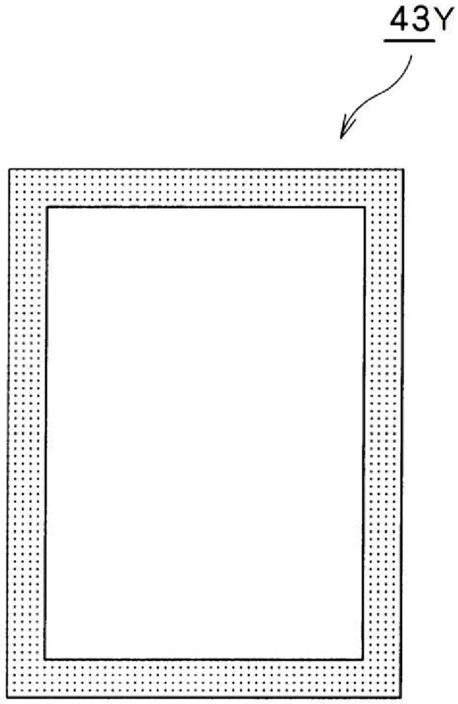


Fig. 21

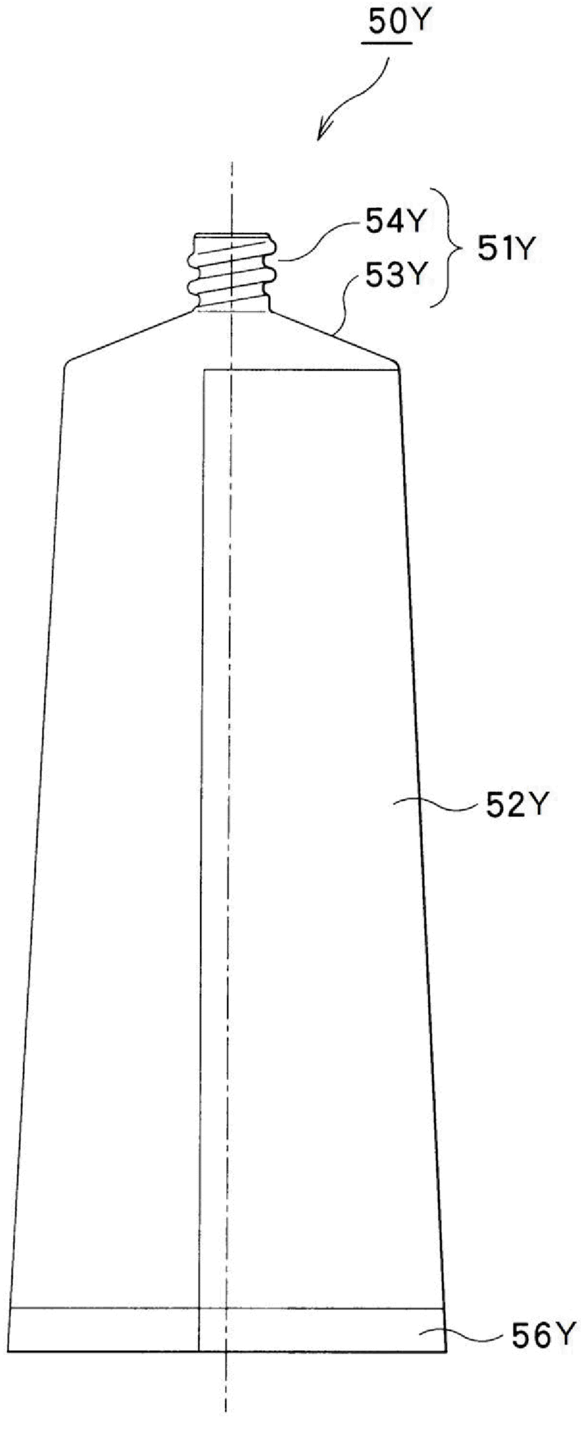


*Fig. 22*

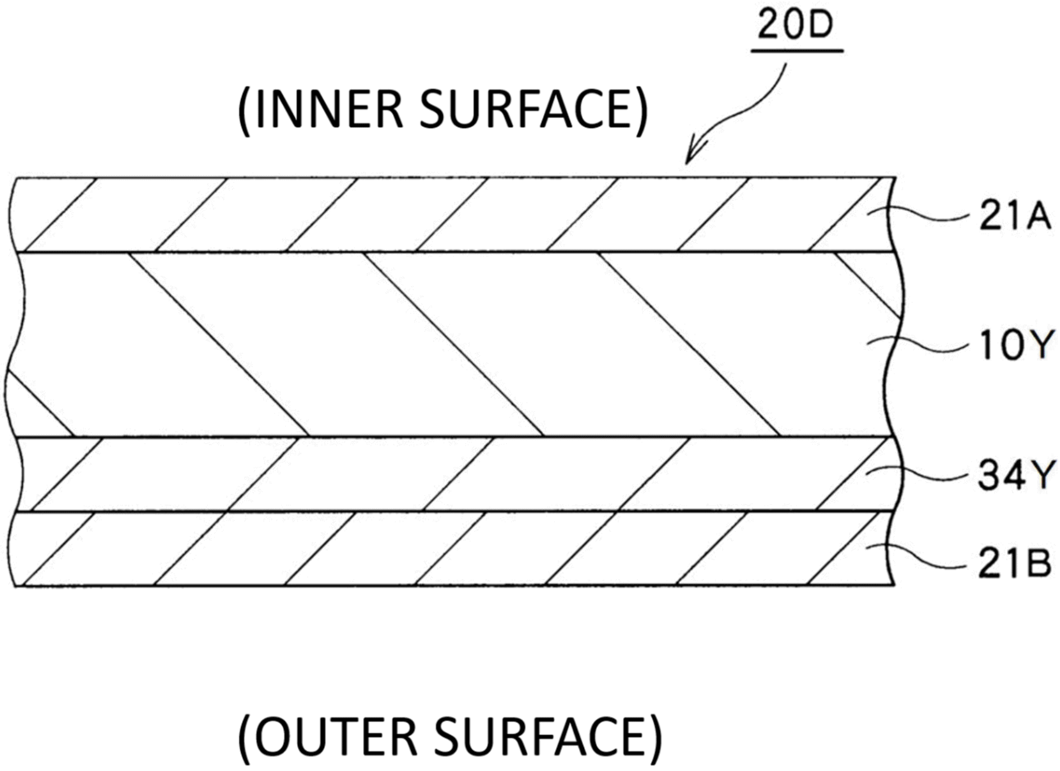


*Fig. 23*

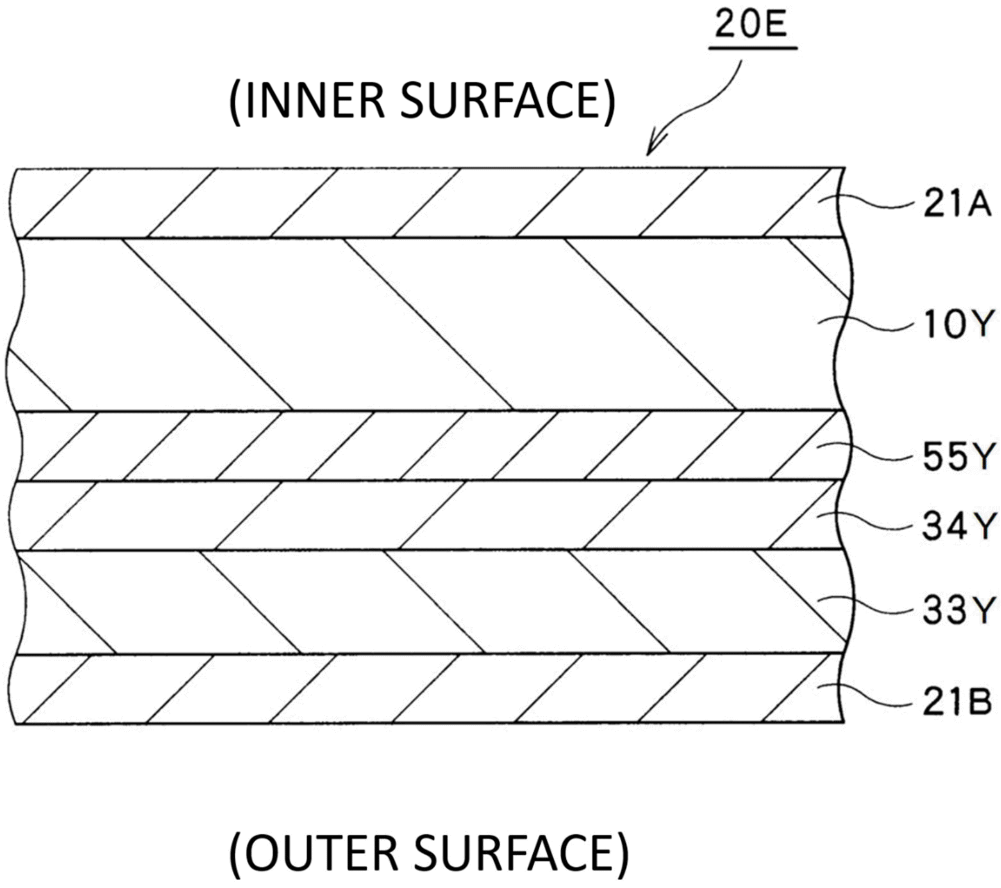


*Fig. 24*

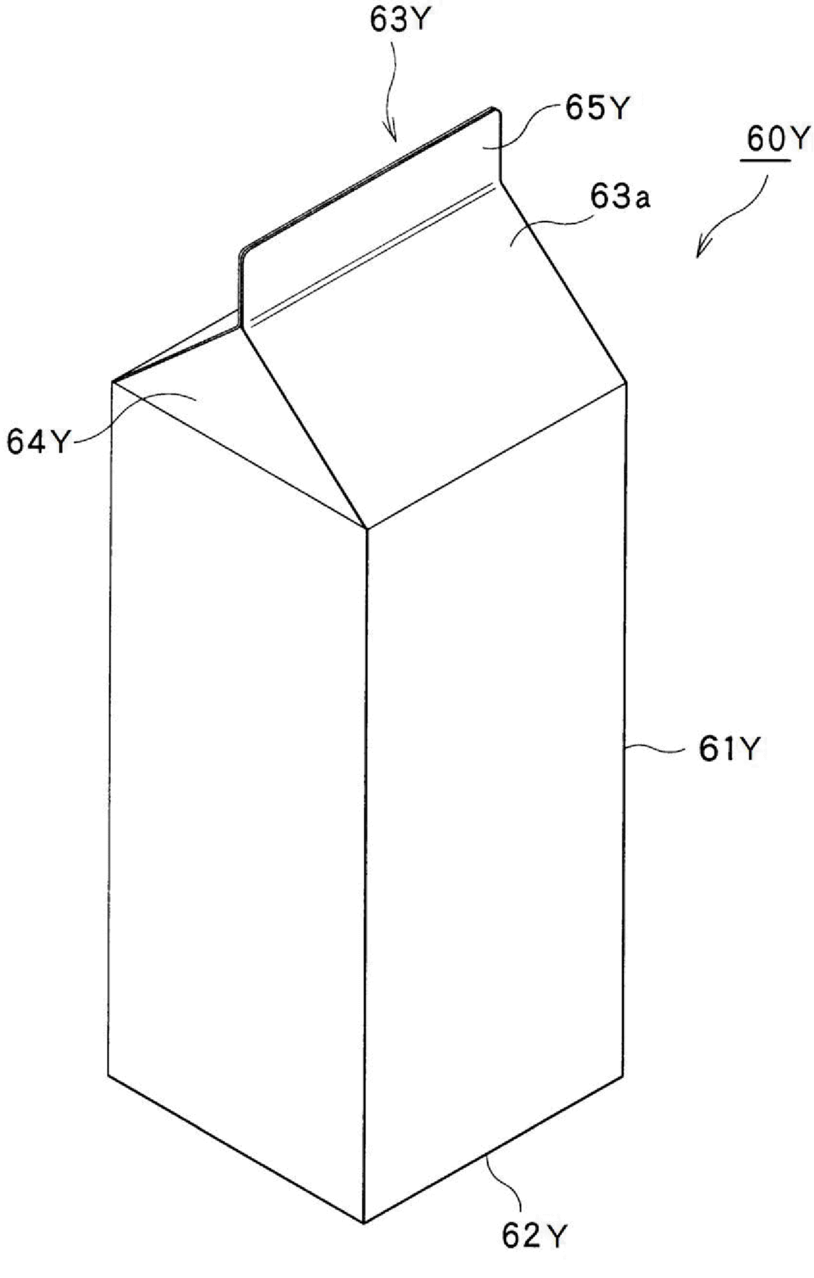


*Fig. 25*

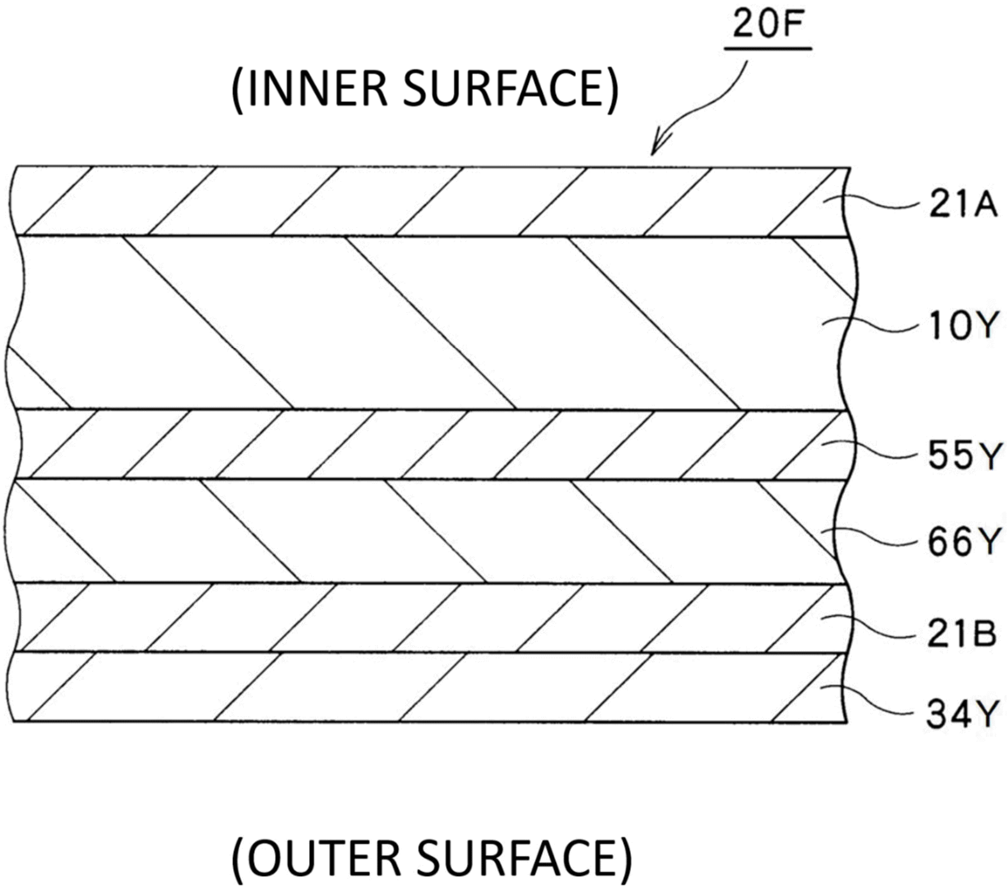


*Fig. 26*

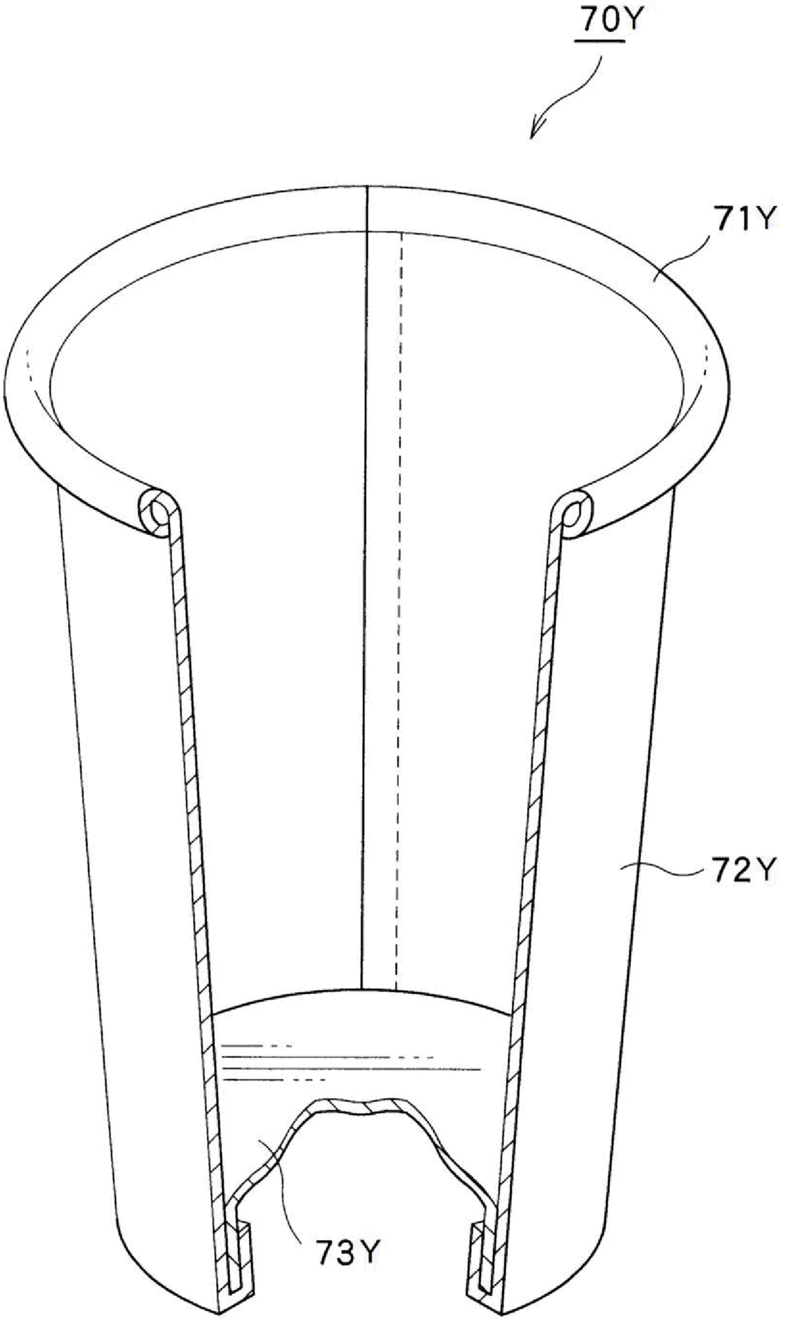


*Fig. 27*

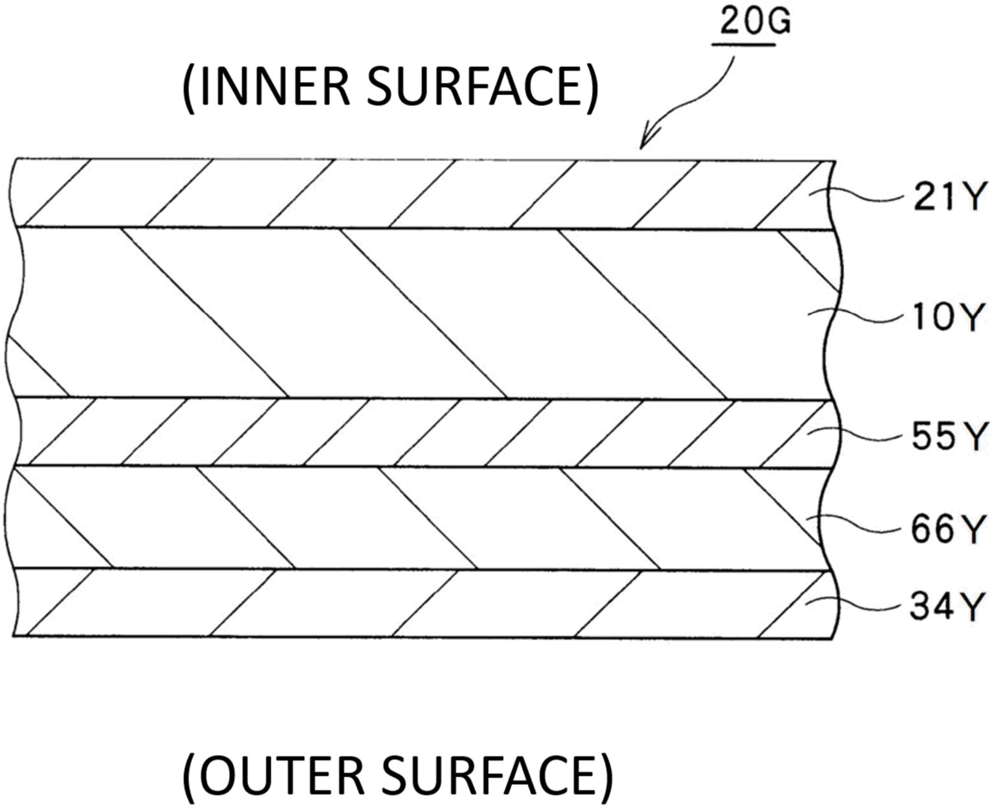


*Fig. 28*

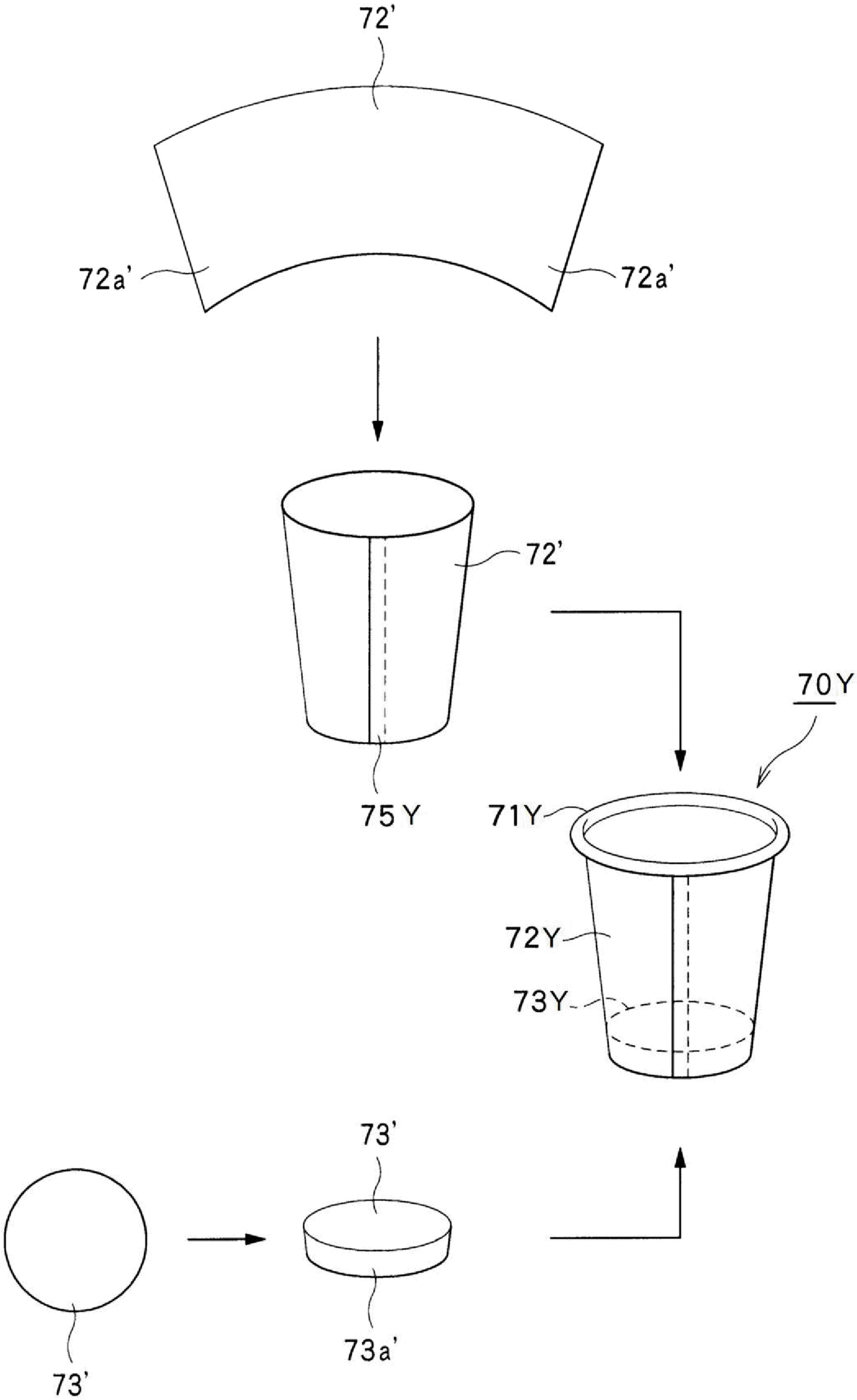


*Fig. 29*

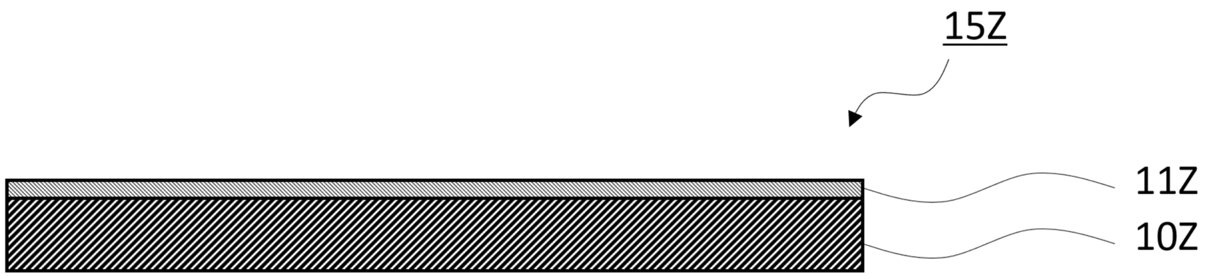


Fig. 30

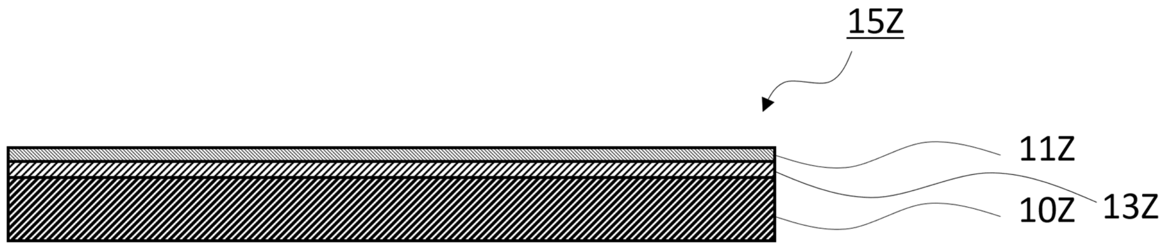


Fig. 31

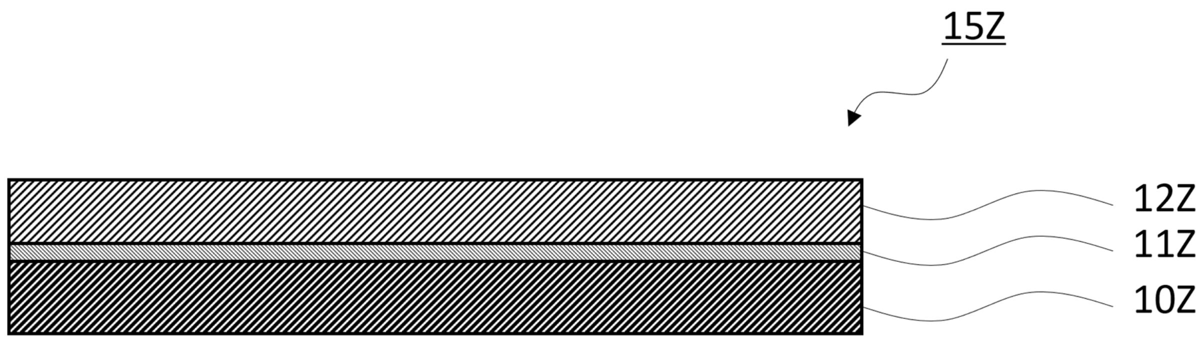


Fig. 32

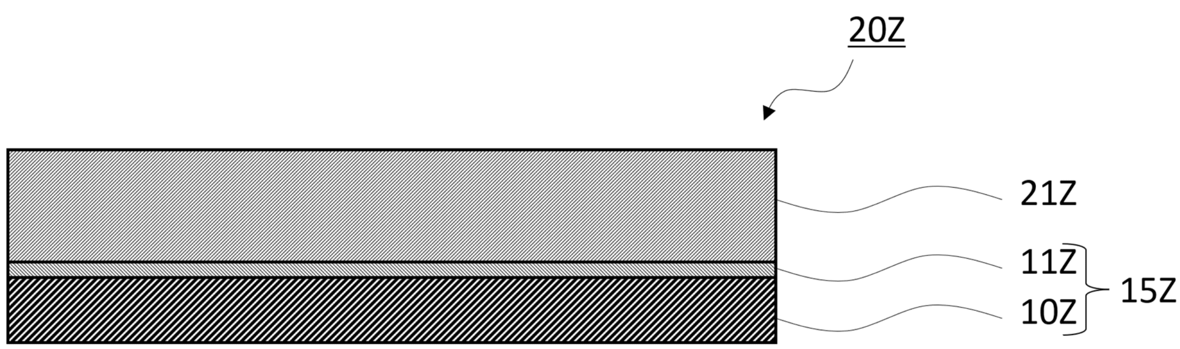


Fig. 33

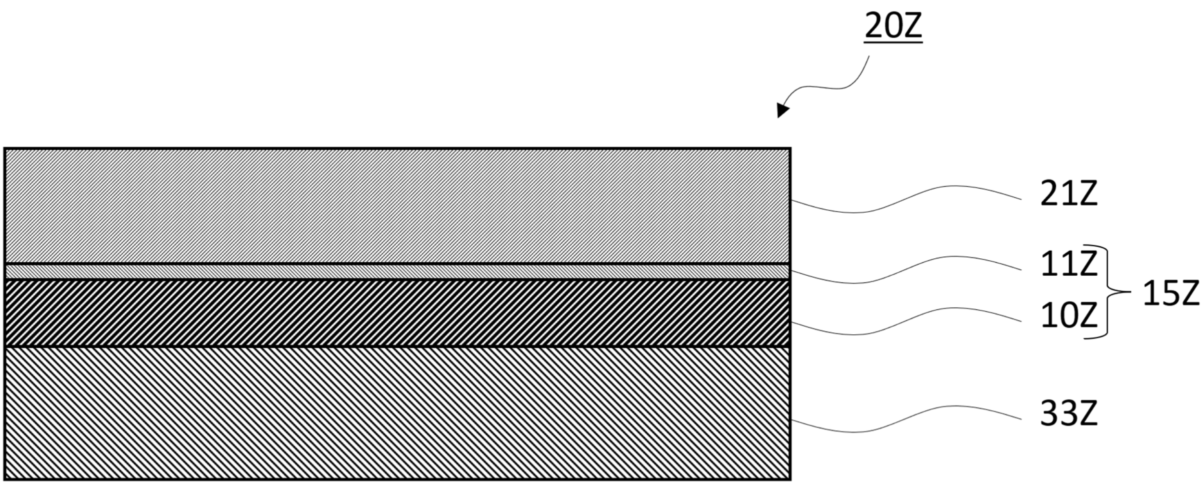


*Fig. 34*

## INTERNATIONAL SEARCH REPORT

International application No.
**PCT/JP2024/033946**

**A. CLASSIFICATION OF SUBJECT MATTER**

***C07C 31/20***(2006.01)i; ***B32B 15/09***(2006.01)i; ***B32B 27/00***(2006.01)i; ***B32B 27/32***(2006.01)i; ***B32B 27/36***(2006.01)i; ***B65D 65/40***(2006.01)i; ***C07C 29/10***(2006.01)i; ***C07C 43/10***(2006.01)i; ***C08G 63/12***(2006.01)i; ***C08G 63/16***(2006.01)i; ***C08G 63/183***(2006.01)i; ***C08L 67/02***(2006.01)i; ***C12P 7/06***(2006.01)i; ***C12P 7/625***(2022.01)i; ***C23C 14/20***(2006.01)i; ***C25B 3/03***(2021.01)i; ***C25B 3/07***(2021.01)i; ***C25B 3/26***(2021.01)i; ***C25B 9/00***(2021.01)i; ***D01F 6/62***(2006.01)i

FI: C07C31/20 A; C08G63/16; C08G63/183; C08L67/02; C25B3/26; C25B3/03; C25B3/07; C25B9/00 G; C12P7/06; C08G63/12; C12P7/625; C07C29/10; B32B27/32; B65D65/40 D; B32B27/00 H; C23C14/20 A; C07C43/10; B32B27/36; B32B15/09 A; D01F6/62 301Z

According to International Patent Classification (IPC) or to both national classification and IPC

**B. FIELDS SEARCHED**

Minimum documentation searched (classification system followed by classification symbols)

B32B1/00-43/00, C07B31/00-63/04, C07C1/00-409/44, C08G63/00-64/42, C12P1/00-41/00, C25B1/00-9/77, C25B13/00-15/08, D01F1/00-6/96, D01F9/00-9/04

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | WO 2016/186005 A1 (KABUSHIKI KAISHA TOSHIBA) 24 November 2016 (2016-11-24) claims, examples | 1-3 |
| Y | | 4-39 |
| X | JP 2017-057491 A (KABUSHIKI KAISHA TOSHIBA) 23 March 2017 (2017-03-23) claims | 1-3 |
| Y | | 4-39 |
| X | JP 2014-1257 A (TORAY INDUSTRIES, INC.) 09 January 2014 (2014-01-09) claims, paragraphs [0020], [0070], [0082], [0083] | 1-26 |
| Y | | 4-39 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 December 2024** | **17 December 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (July 2022)

**INTERNATIONAL SEARCH REPORT**

International application No.
**PCT/JP2024/033946**

**C. DOCUMENTS CONSIDERED TO BE RELEVANT**

| Category* | Citation of document, with indication, where appropriate, of the relevant passages | Relevant to claim No. |
|---|---|---|
| X<br><br>Y | JP 2020-78941 A (DAI NIPPON PRINTING CO., LTD.) 28 May 2020 (2020-05-28)<br>claims, paragraphs [0111]-[0118], [0122], [0127], examples 1, 2 | 1-11, 18-21, 23-29, 31-39<br><br>1-11, 18-21, 23-39 |
| Y | JP 2011-527348 A (THE COCA-COLA COMPANY) 27 October 2011 (2011-10-27)<br>claims, paragraph [0025] | 1-39 |
| Y | WO 2019/188838 A1 (SEKISUI CHEMICAL CO., LTD.) 03 October 2019 (2019-10-03)<br>claims, paragraphs [0066]-[0071], [0095], [0096] | 1-39 |
| Y | WO 2022/044502 A1 (TOPPAN PRINTING CO., LTD.) 03 March 2022 (2022-03-03)<br>claims, paragraphs [0027]-[0029], [0059] | 30 |

Form PCT/ISA/210 (second sheet) (July 2022)

**INTERNATIONAL SEARCH REPORT**
**Information on patent family members**

International application No.
**PCT/JP2024/033946**

| Patent document cited in search report | Publication date (day/month/year) | Patent family member(s) | Publication date (day/month/year) |
|---|---|---|---|
| WO 2016/186005 A1 | 24 November 2016 | US 2017/0369405 A1<br>claims, examples | |
| JP 2017-057491 A | 23 March 2017 | US 2017/0073827 A1<br>claims | |
| JP 2014-1257 A | 09 January 2014 | (Family: none) | |
| JP 2020-78941 A | 28 May 2020 | JP 2012-096469 A | |
| JP 2011-527348 A | 27 October 2011 | US 2009/0246430 A1<br>claims, paragraph [0022]<br>WO 2009/120457 A2<br>EP 2265659 A1<br>CN 101970530 A | |
| WO 2019/188838 A1 | 03 October 2019 | US 2021/0024963 A1<br>claims, paragraphs [0080]-[0085], [0109], [0110]<br>EP 3778907 A1<br>CN 111819287 A | |
| WO 2022/044502 A1 | 03 March 2022 | US 2023/0220536 A1<br>claims, paragraphs [0038]-[0040], [0072]<br>EP 4205974 A1<br>CN 115867435 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2011527348 W
- JP 2012519748 W
- JP 2011512869 W
- JP 2012112001 A
- JP 2009079213 A
- JP 2019205969 A
- JP 2012251006 A
- JP 2012096469 A
- JP 2008307847 A


### Non-patent literature cited in the description


- *Chemische Technik*, 1986, vol. 38 (3), 116-119